# EUROPEAN PATENT APPLICATION

(11) **EP 4 464 694 A1**
(43) Date of publication of application: **20.11.2024**
(21) Application number: 23740105.4
(22) Date of filing: 16.01.2023
(51) Int. Cl.: C07D 213/64, C07D 213/72, C07D 401/14, C07D 405/14, C07D 409/14, A61K 31/444, A61K 31/501, A61K 31/513, A61K 31/497, A61P 29/00, A61P 35/00

(54) **PYRIDINE-CONTAINING POLYCYCLIC DERIVATIVE, AND PREPARATION METHOD THEREFOR AND USE THEREOF**

(30) Priority: 14.01.2022 CN 202210041733; 21.10.2022 CN 202211294865; 22.11.2022 CN 202211474196
(71) Applicant: Shanghai Hansoh Biomedical Co., Ltd., Shanghai 201203 (CN); Jiangsu Hansoh Pharmaceutical Group Co., Ltd., Lianyungang, Jiangsu 222047 (CN)
(72) Inventor: DONG, Jiaqiang, Shanghai 201203 (CN); DENG, Xinxian, Shanghai 201203 (CN); YU, Wensheng, Shanghai 201203 (CN); JIN, Fangfang, Shanghai 201203 (CN); GONG, Zhen, Shanghai 201203 (CN)
(74) Representative: EP&C
(86) International application number: PCT/CN2023/072370
(87) International publication number: WO 2023/134764

(57) **Abstract**

The present invention relates to a pyridine-containing polycyclic derivative, and a preparation method therefor and the use thereof. In particular, the present invention relates to a compound as represented by general formula (I), a preparation method therefor, a pharmaceutical composition containing the compound, and the use thereof as a biological regulator in the preparation of a medicament for the treatment of autoimmune diseases, chronic inflammatory diseases, acute inflammatory diseases, autoinflammatory diseases, atherosclerosis, diabetes, fibrotic diseases, metabolic diseases, cancers, tumors, leukemia and lymphoma, wherein the definition of each substituent in general formula (I) is the same as that in the description.

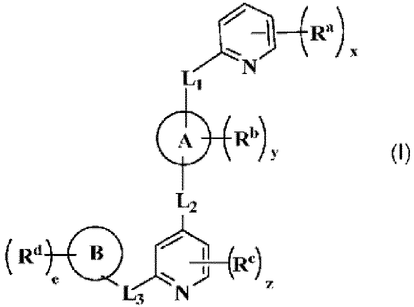

## Description

### Technical Field

The present invention belongs to the field of biomedicine, and specifically relates to a pyridine-containing polycyclic derivative, a preparation method therefor, and the use thereof.

### Background Art

MK2 (MAPK activated protein kinase 2), as an important downstream molecule of the classical inflammatory signal transduction pathway MAPK, is a protein kinase which regulates the expression of inflammatory factors. After cells undergo inflammatory stimulation, the activated p38 MAPK protein phosphorylates and activates MK2, which leads to phosphorylation of the MK2 substrate TTP and contributes to the mRNA stability of pro-inflammatory cytokines, thus improving the protein expression level of inflammatory cytokines.

MK2 is activated in both a mouse arthritis model and chondrocytes in patients with osteoarthritis. Inhibition of p38-MK2 pathway by either genetic or pharmacological means can inhibit the expression level of inflammatory factors, indicating that MK2 participates in mediating inflammatory regulation of arthritis. p38-MK2 inhibitors are promising in treating patients with rheumatoid arthritis who are relapsed, refractory, and poorly responsive to existing therapies.

At present, only the p38-MK2 inhibitor ATI-450 from Aclaris Therapeutics has entered the phase IIb clinical trial of rheumatoid arthritis in the world. The preliminary clinical trial results have shown that ATI-450 is safe and tolerable in patients with rheumatoid arthritis and has outstanding and lasting efficacy. Other MK2 inhibitors in clinical trials further include CC-99677 from BMS and MMI-0100 from Moerae Matrix, both of which can directly inhibit the activity of MK2 in a mechanism different from that of ATI-450.

Existing published patent applications regarding p38-MK2 (or MK2) inhibitors include: patents from Aclaris Therapeutics (WO-2012078684, WO-2013086208, WO-2014197846, WO-2021022186, WO-2012078687, WO 2012078673, and WO 2012078674); patents from BMS (WO-2020236636, WO-2018170199, WO-2016044463, WO-2018170201, WO-2018170200, WO-2018170203, US 20210198276, US 20210139501, US 20200148701, and US 20200102326); patents from Moerae Matrix (WO-2016112292, WO-2016145234, US-10336788, WO-2014040074, WO-2018231722, WO-2012142320, WO-2016033432, WO-2013134636, and WO-2011149964), etc.

P38-MK2 inhibitors as drugs have a good application prospect in the pharmaceutical industry. P38-MK2 inhibitors are provided as a safer and more effective treatment for patients with rheumatoid arthritis who are relapsed, refractory, and poorly responsive to existing therapies. P38-MK2 inhibitors have a broad-spectrum anti-inflammatory effect (TNF/IL-6/IL-1), which can be used to treat a variety of inflammatory diseases and autoimmune diseases in theory. P38-MK2 inhibitors are small molecule inhibitors. Compared with macromolecular drugs such as TNF monoclonal antibodies, such p38-MK2 inhibitors have the advantages of oral convenience, good patient compliance, etc. In addition, no reports regarding toxic and side effects of JAK inhibitors such as infection, tumor, thrombosis and cardiotoxicity have been found in p38-MK2 inhibitors in clinical trials. Therefore, there is a huge clinical need for the development of novel p38-MK2 inhibitors.

### Summary of the Invention

An objective of the present invention is to provide a compound represented by general formula (I), or a stereoisomer or pharmaceutically acceptable salt thereof, wherein the compound represented by general formula (I) has a structure as follows: 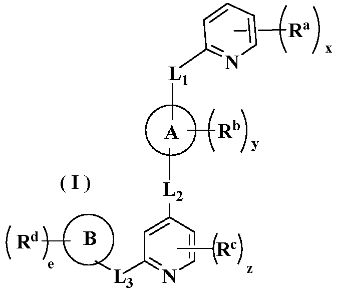 wherein:
ring A is selected from cycloalkyl, heterocyclyl, aryl, or heteroaryl;
ring B is selected from cycloalkyl, heterocyclyl, aryl, or heteroaryl;
L₁ is selected from a bond, substituted or unsubstituted alkenylene, substituted or unsubstituted alkynylene, -(CH₂)ₙ-, -(CH₂)ₙC(O)(CRₐₐR_{bb})ₙ₁-, -(CH₂)ₙC(O)NR_{cc}(CH₂)ₙ₁-, -(CH₂)ₙ(CRₐₐR_{bb})ₙ₁-, - (CRₐₐR_{bb})ₙO(CH₂)ₙ₁-, -(CH₂)ₙO(CRₐₐR_{bb})ₙ₁-, -(CRₐₐR_{bb})ₙS(CH₂)ₙ₁-, -(CH₂)ₙS(CRₐₐR_{bb})ₙ₁-, - (CRₐₐR_{bb})ₙ(CH₂)ₙ₁NR_{cc}-, -(CH₂)ₙNR_{cc}(CRₐₐR_{bb})ₙ₁-, -(CH₂)ₙNR_{cc}C(O)-, -(CH₂)ₙP(O)ₚRₐₐ-, -(CH₂)ₙS(O)ₘ-, - (CH₂)ₙS(O)ₘNR_{cc}-, and -(CH₂)ₙNR_{cc}S(O)ₘ-;
Rₐₐ, R_{bb}, and R_{cc} are each independently selected from hydrogen, deuterium, halogen, nitro, hydroxy, mercapto, cyano, amino, oxo, thio, carboxyl, alkyl, deuteroalkyl, haloalkyl, hydroxyalkyl, alkoxy, deuteroalkoxy, haloalkoxy, alkenyl, alkynyl, cycloalkyl, heterocyclyl, aryl, or heteroaryl, wherein the amino, alkyl, deuteroalkyl, haloalkyl, hydroxyalkyl, alkoxy, deuteroalkoxy, haloalkoxy, alkenyl, alkynyl, cycloalkyl, heterocyclyl, aryl, and heteroaryl may be optionally further substituted;
or any two of Rₐₐ, R_{bb}, and R_{cc} are connected to form cycloalkyl, heterocyclyl, aryl, or heteroaryl, wherein the cycloalkyl, heterocyclyl, aryl, and heteroaryl may be optionally further substituted; L₂ and L₃ are each independently selected from a bond, substituted or unsubstituted alkenyl, substituted or unsubstituted alkynyl, -(CH₂)ₙ₂-, -(CH₂)ₙ₂C(O)(CRₐ₁R_{b1})ₙ₃-, -(CH₂)ₙ₂C(O)NR_{c1}(CH₂)ₙ₃-, - (CH₂)ₙ₂(CRₐ₁R_{b1})ₙ₃-, -(CRₐ₁R_{b1})ₙ₂O(CH₂)ₙ₃-, -(CH₂)ₙ₂O(CRₐ₁R_{b1})ₙ₃-, -(CRₐ₁R_{b1})ₙ₂S(CH₂)ₙ₃-, - (CH₂)ₙ₂S(CRₐ₁R_{b1})ₙ₃-, -(CRₐ₁R_{b1})ₙ₂(CH₂)ₙ₃NR_{c1}-, -(CH₂)ₙ₂NR_{c1}(CRₐ₁R_{b1})ₙ₃-, -(CH₂)ₙ₂NR_{c1}C(O)-, - (CH₂)ₙ₂P(O)_{b1}Rₐ₁-, -(CH₂)ₙ₂S(O)ₘ₁-, -(CH₂)ₙ₂S(O)ₘ₁NR_{c1}-, and -(CH₂)ₙ₂NR_{c1}S(O)ₘ₁-;
Rₐ₁, R_{b1}, and R_{c1} are each independently selected from hydrogen, deuterium, halogen, nitro, hydroxy, mercapto, cyano, amino, oxo, thio, carboxyl, alkyl, deuteroalkyl, haloalkyl, hydroxyalkyl, alkoxy, deuteroalkoxy, haloalkoxy, alkenyl, alkynyl, cycloalkyl, heterocyclyl, aryl, or heteroaryl, wherein the amino, alkyl, deuteroalkyl, haloalkyl, hydroxyalkyl, alkoxy, deuteroalkoxy, haloalkoxy, alkenyl, alkynyl, cycloalkyl, heterocyclyl, aryl, and heteroaryl may be optionally further substituted;
each R^{a} is independently selected from hydrogen, deuterium, halogen, nitro, hydroxy, mercapto, cyano, amino, oxo, thio, carboxyl, alkyl, deuteroalkyl, haloalkyl, hydroxyalkyl, alkoxy, deuteroalkoxy, haloalkoxy, alkenyl, alkynyl, cycloalkyl, heterocyclyl, aryl, or heteroaryl, wherein the amino, alkyl, deuteroalkyl, haloalkyl, alkoxy, haloalkoxy, hydroxyalkyl, alkenyl, alkynyl, cycloalkyl, heterocyclyl, aryl, and heteroaryl may be optionally further substituted;
each R^{b} is independently selected from hydrogen, deuterium, halogen, nitro, hydroxy, mercapto, cyano, amino, oxo, thio, carboxyl, alkyl, deuteroalkyl, haloalkyl, hydroxyalkyl, alkoxy, deuteroalkoxy, haloalkoxy, alkenyl, alkynyl, cycloalkyl, heterocyclyl, aryl, or heteroaryl, wherein the amino, alkyl, deuteroalkyl, haloalkyl, alkoxy, haloalkoxy, hydroxyalkyl, alkenyl, alkynyl, cycloalkyl, heterocyclyl, aryl, and heteroaryl may be optionally further substituted;
each R^{c} is independently selected from hydrogen, deuterium, halogen, nitro, hydroxy, mercapto, cyano, amino, oxo, thio, carboxyl, alkyl, deuteroalkyl, haloalkyl, hydroxyalkyl, alkoxy, deuteroalkoxy, haloalkoxy, alkenyl, alkynyl, cycloalkyl, heterocyclyl, aryl, or heteroaryl, wherein the amino, alkyl, deuteroalkyl, haloalkyl, alkoxy, haloalkoxy, hydroxyalkyl, alkenyl, alkynyl, cycloalkyl, heterocyclyl, aryl, and heteroaryl may be optionally further substituted;
R^{b} and R^{c} are connected to form cycloalkyl, heterocyclyl, aryl, or heteroaryl, wherein the cycloalkyl, heterocyclyl, aryl, and heteroaryl may be optionally further substituted;
each R^{d} is independently selected from hydrogen, deuterium, halogen, nitro, hydroxy, mercapto, cyano, amino, oxo, thio, carboxyl, alkyl, bridged cycloalkyl, deuteroalkyl, haloalkyl, hydroxyalkyl, alkoxy, deuteroalkoxy, haloalkoxy, alkenyl, alkynyl, cycloalkyl, heterocyclyl, aryl, heteroaryl, - (CRₑₑR_{ff})ₙ₄C(O)R_{gg}, -(CRₑₑR_{ff})ₙ₄NRₕₕC(O)R_{gg}, -(CRₑₑR_{ff})ₙ₄S(O)₂R_{gg}, -(CRₑₑR_{ff})ₙ₄C(O)NRₕₕR_{gg}, or - (CRₑₑR_{ff})ₙ₄S(O)₂NRₕₕR_{gg}, wherein the amino, alkyl, bridged cycloalkyl, deuteroalkyl, haloalkyl, alkoxy, haloalkoxy, hydroxyalkyl, alkenyl, alkynyl, cycloalkyl, heterocyclyl, aryl, and heteroaryl may be optionally further substituted;
Rₑₑ, R_{ff}, R_{gg}, and Rₕₕ are each independently selected from hydrogen, deuterium, halogen, nitro, hydroxy, mercapto, cyano, amino, carboxyl, C₁₋₈ alkyl, C₁₋₈ deuteroalkyl, C₁₋₈ haloalkyl, C₁₋₈ hydroxyalkyl, C₁₋₈ alkoxy, C₁₋₈ deuteroalkoxy, C₁₋₈ haloalkoxy, C₂₋₈ alkenyl, C₂₋₈ alkynyl, C₃₋₁₂ cycloalkyl, 3- to 12-membered heterocyclyl, C₆₋₁₄ aryl, or 5- to 14-membered heteroaryl; preferably, Rₑₑ, R_{ff}, R_{gg}, and Rₕₕ are each independently selected from hydrogen, deuterium, halogen, nitro, hydroxy, mercapto, cyano, amino, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₈ cycloalkyl, 3- to 8-membered heterocyclyl, C₆₋₁₀ aryl, or 5- to 10-membered heteroaryl;
or Rₑₑ and R_{ff} are connected to form cycloalkyl, heterocyclyl, aryl, or heteroaryl, wherein the cycloalkyl, heterocyclyl, aryl, and heteroaryl may be optionally further substituted;
n4 is 0, 1, 2, 3, or 4;
preferably,
each R^{d} is independently selected from hydrogen, deuterium, halogen, nitro, hydroxy, mercapto, cyano, amino, oxo, thio, carboxyl, alkyl, deuteroalkyl, haloalkyl, hydroxyalkyl, alkoxy, deuteroalkoxy, haloalkoxy, alkenyl, alkynyl, cycloalkyl, heterocyclyl, aryl, or heteroaryl, wherein the amino, alkyl, deuteroalkyl, haloalkyl, alkoxy, haloalkoxy, hydroxyalkyl, alkenyl, alkynyl, cycloalkyl, heterocyclyl, aryl, and heteroaryl may be optionally further substituted;
or any two adjacent or non-adjacent R^{d} are connected to form cycloalkyl, heterocyclyl, aryl, or heteroaryl, wherein the cycloalkyl, heterocyclyl, aryl, and heteroaryl may be optionally further substituted;
or L₃ and R^{d} are connected to form cycloalkyl, heterocyclyl, aryl, or heteroaryl, wherein the cycloalkyl, heterocyclyl, aryl, and heteroaryl may be optionally further substituted;
or R^{c} and R^{d} are connected to form cycloalkyl, heterocyclyl, aryl, or heteroaryl, wherein the cycloalkyl, heterocyclyl, aryl, and heteroaryl may be optionally further substituted;
x is an integer of 0-6;
y is an integer of 0-6;
z is an integer of 0-6;
e is an integer of 0-6;
n is 0, 1, 2, or 3;
n1 is 0, 1, 2, or 3;
n2 is 0, 1, 2, or 3;
n3 is 0, 1, 2, or 3;
m is 0, 1, 2, or 3;
p is 0, 1, 2, or 3;
m1 is 0, 1, 2, or 3; and
p1 is 0, 1, 2, or 3.

In a preferred embodiment of the present invention, the compound is further as represented by general formula (I'-1): 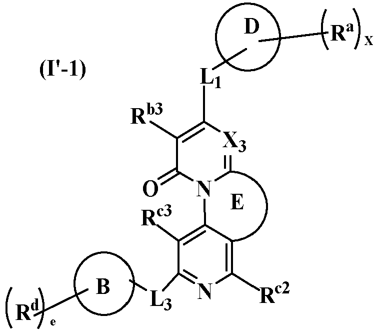 wherein:
ring D is selected from cycloalkyl, heterocyclyl, aryl, or heteroaryl;
ring E is heterocyclyl;
ring B is selected from cycloalkyl, heterocyclyl, aryl, or heteroaryl;
X₃ is selected from N or CR^{b1};
L₁ is selected from a bond, substituted or unsubstituted alkenylene, substituted or unsubstituted alkynylene, -(CH₂)ₙ-, -(CH₂)ₙC(O)(CRₐₐR_{bb})ₙ₁-, -(CH₂)ₙC(O)NR_{cc}(CH₂)ₙ₁-, -(CH₂)ₙ(CRₐₐR_{bb})ₙ₁-, - (CRₐₐR_{bb})ₙO(CH₂)ₙ₁-, -(CH₂)ₙO(CRₐₐR_{bb})ₙ₁-, -(CRₐₐR_{bb})ₙS(CH₂)ₙ₁-, -(CH₂)ₙS(CRₐₐR_{bb})ₙ₁-, - (CRₐₐR_{bb})ₙ(CH₂)ₙ₁NR_{cc}-, -(CH₂)ₙNR_{cc}(CRₐₐR_{bb})ₙ₁-, -(CH₂)ₙNR_{cc}C(O)-, -(CH₂)ₙP(O)ₚRₐₐ-, -(CH₂)ₙS(O)ₘ-, - (CH₂)ₙS(O)ₘNR_{cc}-, and -(CH₂)ₙNR_{cc}S(O)ₘ-;
Rₐₐ, R_{bb}, and R_{cc} are each independently selected from hydrogen, deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl, wherein the amino, C₁₋₆ alkyl, C₁₋₆ deuteroalkyl, C₁₋₆ haloalkyl, C₁₋₆ alkoxy, C₁₋₆ haloalkoxy, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
L₃ is selected from a bond, substituted or unsubstituted alkenyl, substituted or unsubstituted alkynyl, -(CH₂)ₙ₂-, -(CH₂)ₙ₂C(O)(CRₐ₁R_{b1})ₙ₃-, -(CH₂)ₙ₂C(O)NR_{c1}(CH₂)ₙ₃-, -(CH₂)ₙ₂(CRₐ₁R_{b1})ₙ₃, - (CRₐ₁R_{b1})ₙ₂O(CH₂)ₙ₃-, -(CH₂)ₙ₂O(CRₐ₁R_{b1})ₙ₃-, -(CRₐ₁R_{b1})ₙ₂S(CH₂)ₙ₃-, -(CH₂)ₙ₂S(CRₐ₁R_{b1})ₙ₃-, - (CRₐ₁R_{b1})ₙ₂(CH₂)ₙ₃NR_{c1}-, -(CH₂)ₙ₂NR_{c1}(CRₐ₁R_{b1})ₙ₃-, -(CH₂)ₙ₂NR_{c1}C(O)-, -(CH₂)ₙ₂P(O)_{b1}Rₐ₁-, - (CH₂)ₙ₂S(O)ₘ₁-, -(CH₂)ₙ₂S(O)ₘ₁NR_{c1}-, and -(CH₂)ₙ₂NR_{c1}S(O)ₘ₁-;
Rₐ₁, R_{b1}, and R_{c1} are each independently selected from hydrogen, deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl, wherein the amino, C₁₋₆ alkyl, C₁₋₆ deuteroalkyl, C₁₋₆ haloalkyl, C₁₋₆ alkoxy, C₁₋₆ haloalkoxy, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
each R^{a} is independently selected from hydrogen, deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl, wherein the amino, C₁₋₆ alkyl, C₁₋₆ deuteroalkyl, C₁₋₆ haloalkyl, C₁₋₆ alkoxy, C₁₋₆ haloalkoxy, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
or any two adjacent or non-adjacent R^{a} are connected to form C₃₋₁₂ cycloalkyl, 3- to 12-membered heterocyclyl, C₆₋₁₄ aryl, or 5- to 14-membered heteroaryl, wherein the C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
or L₁ and R^{a} are connected to form C₃₋₁₂ cycloalkyl, 3- to 12-membered heterocyclyl, C₆₋₁₄ aryl, or 5- to 14-membered heteroaryl, wherein the C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
each R^{b1} or R^{b3} is independently selected from hydrogen, deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl, wherein the amino, C₁₋₆ alkyl, C₁₋₆ deuteroalkyl, C₁₋₆ haloalkyl, C₁₋₆ alkoxy, C₁₋₆ haloalkoxy, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
each R^{c2} or R^{c3} is independently selected from hydrogen, deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl, wherein the amino, C₁₋₆ alkyl, C₁₋₆ deuteroalkyl, C₁₋₆ haloalkyl, C₁₋₆ alkoxy, C₁₋₆ haloalkoxy, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
each R^{d} is independently selected from hydrogen, deuterium, halogen, nitro, hydroxy, mercapto, cyano, amino, carboxyl, C₁₋₆ alkyl, C₁₋₆ deuteroalkyl, C₁₋₆ bridged cycloalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, - (CRₑₑR_{ff})ₙ₄C(O)R_{gg}, -(CRₑₑR_{ff})ₙ₄NRₕₕC(O)R_{gg}, -(CRₑₑR_{ff})ₙ₄NRₕₕOR_{gg}, -(CRₑₑR_{ff})ₙ₄S(O)₂R_{gg}, - (CRₑₑR_{ff})ₙ₄S(O)R_{gg}, -(CRₑₑR_{ff})ₙ₄SR_{gg}, -(CRₑₑR_{ff})ₙ₄C(O)NRₕₕR_{gg}, -(CRₑₑR_{ff})ₙ₄S(O)NRₕₕR_{gg}, - (CRₑₑR_{ff})ₙ₄SNRₕₕR_{gg}, or -(CRₑₑR_{ff})ₙ₄S(O)₂NRₕₕR_{gg}, wherein the amino, C₁₋₆ alkyl, C₁₋₆ deuteroalkyl, C₁₋₆ bridged cycloalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
Rₑₑ, R_{ff}, R_{gg}, and Rₕₕ are each independently selected from hydrogen, deuterium, halogen, nitro, hydroxy, mercapto, cyano, amino, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl;
or Rₑₑ and R_{ff} are connected to form C₃₋₁₂ cycloalkyl, 3- to 12-membered heterocyclyl, C₆₋₁₄ aryl, or 5- to 14-membered heteroaryl, wherein the C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
x is an integer of 0-6;
e is an integer of 0-6;
n is 0, 1, 2, or 3;
n1 is 0, 1, 2, or 3;
n2 is 0, 1, 2, or 3;
n3 is 0, 1, 2, or 3;
n4 is 0, 1, 2, 3, or 4;
n6 is 0, 1, 2, 3, or 4;
m is 0, 1, 2, or 3;
p is 0, 1, 2, or 3;
m1 is 0, 1, 2, or 3; and
p1 is 0, 1, 2, or 3.

In a further preferred embodiment of the present invention, ring D as described in the present invention is selected from C₃₋₁₂ cycloalkyl, 3- to 12-membered heterocyclyl, C₆₋₁₄ aryl, or 5- to 14-membered heteroaryl; preferably, ring D is selected from C₆₋₁₀ aryl, 5- to 6-membered monocyclic heteroaryl, or 7- to 10-membered bicyclic heteroaryl; and more preferably, ring D is selected from pyrazolyl, thienyl, thiazolyl, oxazolyl, pyrimidinyl, pyridinyl, phenyl, 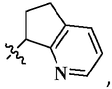 or

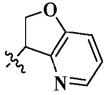

In a further preferred embodiment of the present invention, ring E as described in the present invention is 6- to 12-membered heterocyclyl; and preferably, ring E is selected from

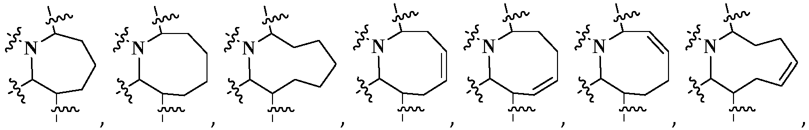

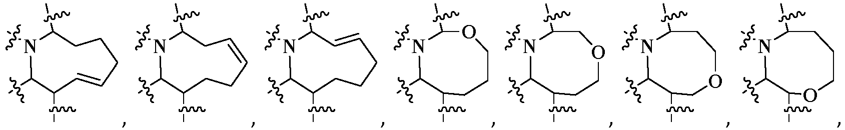

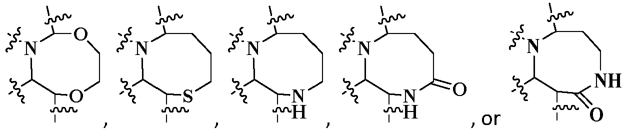

In a preferred embodiment of the present invention, the compound is further as represented by general formula (XIV): 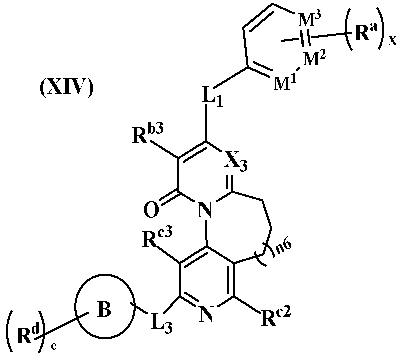 wherein:
M¹ is selected from C, CH, or N, or is absent;
M² is selected from C, CH, or N, or is absent;
M³ is selected from C, CH, or N, or is absent;
X₃ is selected from N or CR^{b1};
ring B is selected from cycloalkyl, heterocyclyl, aryl, or heteroaryl;
L₁ is selected from a bond, substituted or unsubstituted alkenylene, substituted or unsubstituted alkynylene, -(CH₂)ₙ-, -(CH₂)ₙC(O)(CRₐₐR_{bb})ₙ₁-, -(CH₂)ₙC(O)NR_{cc}(CH₂)ₙ₁-, -(CH₂)ₙ(CRₐₐR_{bb})ₙ₁-, - (CRₐₐR_{bb})ₙO(CH₂)ₙ₁-, -(CH₂)ₙO(CRₐₐR_{bb})ₙ₁-, -(CRₐₐR_{bb})ₙS(CH₂)ₙ₁-, -(CH₂)ₙS(CRₐₐR_{bb})ₙ₁-, - (CRₐₐR_{bb})ₙ(CH₂)ₙ₁NR_{cc}-, -(CH₂)ₙNR_{cc}(CRₐₐR_{bb})ₙ₁-, -(CH₂)ₙNR_{cc}C(O)-, -(CH₂)ₙP(O)ₚRₐₐ-, -(CH₂)ₙS(O)ₘ-, - (CH₂)ₙS(O)ₘNR_{cc}-, and -(CH₂)ₙNR_{cc}S(O)ₘ-;
Rₐₐ, R_{bb}, and R_{cc} are each independently selected from hydrogen, deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl, wherein the amino, C₁₋₆ alkyl, C₁₋₆ deuteroalkyl, C₁₋₆ haloalkyl, C₁₋₆ alkoxy, C₁₋₆ haloalkoxy, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
L₃ is selected from a bond, substituted or unsubstituted alkenyl, substituted or unsubstituted alkynyl, -(CH₂)ₙ₂-, -(CH₂)ₙ₂C(O)(CRₐ₁R_{b1})ₙ₃-, -(CH₂)ₙ₂C(O)NRₑ₁(CH₂)ₙ₃-, -(CH₂)ₙ₂(CRₐ₁R_{b1})ₙ₃, - (CRₐ₁R_{b1})ₙ₂O(CH₂)ₙ₃-, -(CH₂)ₙ₂O(CRₐ₁R_{b1})ₙ₃-, -(CRₐ₁R_{b1})ₙ₂S(CH₂)ₙ₃-, -(CH₂)ₙ₂S(CRₐ₁R_{b1})ₙ₃-, - (CRₐ₁R_{b1})ₙ₂(CH₂)ₙ₃NR_{c1}-, -(CH₂)ₙ₂NRₑ₁(CRₐ₁R_{b1})ₙ₃-, -(CH₂)ₙ₂NRₑ₁C(O)-, -(CH₂)ₙ₂P(O)ₚ₁Rₐ₁-, - (CH₂)ₙ₂S(O)ₘ₁-, -(CH₂)ₙ₂S(O)ₘ₁NR_{c1}-, and -(CH₂)ₙ₂NR_{c1}S(O)ₘ₁-;
Rₐ₁, R_{b1}, and R_{c1} are each independently selected from hydrogen, deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl, wherein the amino, C₁₋₆ alkyl, C₁₋₆ deuteroalkyl, C₁₋₆ haloalkyl, C₁₋₆ alkoxy, C₁₋₆ haloalkoxy, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
each R^{a} is independently selected from hydrogen, deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl, wherein the amino, C₁₋₆ alkyl, C₁₋₆ deuteroalkyl, C₁₋₆ haloalkyl, C₁₋₆ alkoxy, C₁₋₆ haloalkoxy, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
or any two adjacent or non-adjacent R^{a} are connected to form C₃₋₁₂ cycloalkyl, 3- to 12-membered heterocyclyl, C₆₋₁₄ aryl, or 5- to 14-membered heteroaryl, wherein the C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
or L₁ and R^{a} are connected to form C₃₋₁₂ cycloalkyl, 3- to 12-membered heterocyclyl, C₆₋₁₄ aryl, or 5- to 14-membered heteroaryl, wherein the C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
each R^{b1} or R^{b3} is independently selected from hydrogen, deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl, wherein the amino, C₁₋₆ alkyl, C₁₋₆ deuteroalkyl, C₁₋₆ haloalkyl, C₁₋₆ alkoxy, C₁₋₆ haloalkoxy, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
each R^{c2} or R^{c3} is independently selected from hydrogen, deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl, wherein the amino, C₁₋₆ alkyl, C₁₋₆ deuteroalkyl, C₁₋₆ haloalkyl, C₁₋₆ alkoxy, C₁₋₆ haloalkoxy, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
each R^{d} is independently selected from hydrogen, deuterium, halogen, nitro, hydroxy, mercapto, cyano, amino, carboxyl, C₁₋₆ alkyl, C₁₋₆ deuteroalkyl, C₁₋₆ bridged cycloalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, - (CRₑₑR_{ff})ₙ₄C(O)R_{gg}, -(CRₑₑR_{ff})ₙ₄NRₕₕC(O)R_{gg}, -(CRₑₑR_{ff})ₙ₄NRₕₕOR_{gg}, -(CRₑₑR_{ff})ₙ₄S(O)₂R_{gg}, - (CRₑₑR_{ff})ₙ₄S(O)R_{gg}, -(CRₑₑR_{ff})ₙ₄SR_{gg}, -(CRₑₑR_{ff})ₙ₄C(O)NRₕₕR_{gg}, -(CRₑₑR_{ff})ₙ₄S(O)NRₕₕR_{gg}, - (CRₑₑR_{ff})ₙ₄SNRₕₕR_{gg}, or -(CRₑₑR_{ff})ₙ₄S(O)₂NRₕₕR_{gg}, wherein the amino, C₁₋₆ alkyl, C₁₋₆ deuteroalkyl, C₁₋₆ bridged cycloalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
Rₑₑ, R_{ff}, R_{gg}, and Rₕₕ are each independently selected from hydrogen, deuterium, halogen, nitro, hydroxy, mercapto, cyano, amino, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl;
or Rₑₑ and R_{ff} are connected to form C₃₋₁₂ cycloalkyl, 3- to 12-membered heterocyclyl, C₆₋₁₄ aryl, or 5- to 14-membered heteroaryl, wherein the C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
x is an integer of 0-6;
e is an integer of 0-6;
n is 0, 1, 2, or 3;
n1 is 0, 1, 2, or 3;
n2 is 0, 1, 2, or 3;
n3 is 0, 1, 2, or 3;
n4 is 0, 1, 2, 3, or 4;
n6 is 0, 1, 2, 3, or 4;
m is 0, 1, 2, or 3;
p is 0, 1, 2, or 3;
m1 is 0, 1, 2, or 3; and
p1 is 0, 1, 2, or 3.

In a more preferred embodiment of the present invention, ring A as described in the present invention is selected from C₃₋₁₂ cycloalkyl, 3- to 12-membered heterocyclyl, C₆₋₁₄ aryl, or 5- to 14-membered heteroaryl;
preferably, ring A is selected from 6- to 12-membered bicyclic heterocyclyl, 5- to 6-membered monocyclic heteroaryl, or 7- to 10-membered bicyclic heteroaryl; and
more preferably, ring A is selected from pyrazolyl, thienyl, thiazolyl, oxazolyl, pyrimidinyl, pyridinyl, phenyl, tetrahydroquinolinyl, benzocyclopentyl, pyrrolopyrimidinyl, pyrrolopyridinyl, pyrazolopyridine, or imidazopyridine.

In a more preferred embodiment of the present invention, ring B as described in the present invention is selected from C₃₋₁₂ cycloalkyl, 3- to 12-membered heterocyclyl, C₆₋₁₄ aryl, or 5- to 14-membered heteroaryl;
preferably, ring B is selected from 3-7-membered monocyclic heterocyclyl, 6- to 12-membered bicyclic heterocyclyl, 5-membered monocyclic heteroaryl, 6-membered monocyclic heteroaryl, or 7- to 14-membered heteroaryl fused ring;
more preferably, ring B is selected from 5-membered monocyclic nitrogen-containing heteroaryl, 6-membered monocyclic nitrogen-containing heteroaryl, 5- to 6-membered heteroaryl fused to 5- to 6-membered cycloalkyl, 5- to 6-membered heteroaryl fused to 5- to 6-membered heteroaryl, or 5- to 6-membered heteroaryl fused to phenyl;
further preferably, ring B is selected from pyrimidine, pyridocyclohexyl, pyridocyclopentyl, pyrrolotriazinyl, pyrrolopyrimidinyl, pyridocyclopentenyl, pyridine, thiazole, pyrazole, pyridopyrrolidone, pyridofuranone, pyridosulfolane, 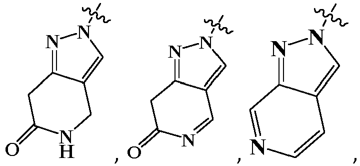 or 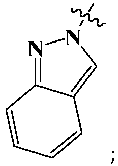 and
further more preferably, ring B is selected from thiazolyl, oxazolyl, pyrazolyl, imidazolyl, 1,2,4-thiadiazolyl, 1,3,4-oxadiazolyl, pyrimidinyl, pyridocyclohexyl, pyridocyclopentyl, pyrrolotriazinyl, pyrrolopyrimidinyl, pyridocyclopentenyl, pyridinyl, pyridopyrrolidone, pyridofuranone, or pyridosulfolane.

In a further preferred embodiment of the present invention, L₁ described in the present invention is selected from a bond, substituted or unsubstituted alkenylene, substituted or unsubstituted alkynylene, -(CH₂)ₙ-, -(CH₂)ₙC(O)(CRₐₐR_{bb})ₙ₁-, -(CH₂)ₙC(O)NR_{cc}(CH₂)ₙ₁-, - (CH₂)ₙ(CRₐₐR_{bb})ₙ₁-, -(CRₐₐR_{bb})ₙO(CH₂)ₙ₁-, -(CH₂)ₙO(CRₐₐR_{bb})ₙ₁-, -(CRₐₐR_{bb})ₙS(CH₂)ₙ₁-, - (CH₂)ₙS(CRₐₐR_{bb})ₙ₁-, -(CRₐₐR_{bb})ₙ(CH₂)ₙ₁NR_{cc}-, -(CH₂)ₙNR_{cc}(CRₐₐR_{bb})ₙ₁-, -(CH₂)ₙNR_{cc}C(O)-, - (CH₂)ₙP(O)ₚRₐₐ-, -(CH₂)ₙS(O)ₘ-, -(CH₂)ₙS(O)ₘNR_{cc}-, and -(CH₂)ₙNR_{cc}S(O)ₘ-;
preferably, L₁ is selected from a bond, substituted or unsubstituted alkenylene, substituted or unsubstituted alkynylene, -(CH₂)ₙ-, -(CH₂)ₙC(O)-, -(CRₐₐR_{bb})ₙO-, -O(CRₐₐR_{bb})ₙ₁-, -(CRₐₐR_{bb})ₙS-, - S(CRₐₐR_{bb})ₙ₁-, -(CH₂)ₙ₁NR_{cc}-, -NR_{cc}(CRₐₐR_{bb})ₙ₁-, or-NR_{cc}C(O)-;
more preferably, L₁ is selected from a bond, -CH₂-, -OCH₂-, -OCD₂-, -NH-, -C(O)NH-, -OCH(CH₃)-, - OC(CH₃)₂-, -NH-CH₂-, or 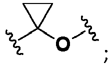
Rₐₐ, R_{bb}, and R_{cc} are each independently selected from hydrogen, deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl, wherein the amino, C₁₋₆ alkyl, C₁₋₆ deuteroalkyl, C₁₋₆ haloalkyl, C₁₋₆ alkoxy, C₁₋₆ haloalkoxy, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
preferably, Rₐₐ, R_{bb}, and R_{cc} are each independently selected from hydrogen, deuterium, halogen, nitro, hydroxy, mercapto, cyano, amino, oxo, thio, carboxyl, C₁₋₃ alkyl, C₁₋₃ deuteroalkyl, C₁₋₃ haloalkyl, C₁₋₃ hydroxyalkyl, C₁₋₃ alkoxy, C₁₋₃ deuteroalkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl, or 5- to 10-membered heteroaryl, wherein the amino, C₁₋₃ alkyl, C₁₋₃ deuteroalkyl, C₁₋₃ haloalkyl, C₁₋₃ alkoxy, C₁₋₃ haloalkoxy, C₁₋₃ hydroxyalkyl, C₂₋₄ alkenyl, C₂₋₄ alkynyl, C₃₋₈ cycloalkyl, 3- to 8-membered heterocyclyl, C₆₋₁₀ aryl, and 5- to 10-membered heteroaryl may be optionally further substituted with one or more substituents of deuterium, halogen, nitro, hydroxy, mercapto, cyano, amino, oxo, thio, carboxyl, C₁₋₃ alkyl, C₁₋₃ deuteroalkyl, C₁₋₃ haloalkyl, C₁₋₃ hydroxyalkyl, C₁₋₃ alkoxy, C₁₋₃ deuteroalkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl, and 5- to 10-membered heteroaryl;
or any two of Rₐₐ, R_{bb}, and R_{cc} are connected to form C₃₋₁₂ cycloalkyl, 3- to 12-membered heterocyclyl, C₆₋₁₄ aryl, or 5- to 14-membered heteroaryl, wherein the C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, and C₁₋₆ haloalkoxy; and
preferably, any two of Rₐₐ, R_{bb}, and R_{cc} are connected to form C₃₋₈ cycloalkyl, 3- to 8-membered heterocyclyl, C₆₋₁₀ aryl, or 5- to 10-membered heteroaryl, wherein the C₃₋₈ cycloalkyl, 3- to 8-membered heterocyclyl, C₆₋₁₀ aryl, and 5- to 10-membered heteroaryl may be optionally further substituted with one or more substituents of deuterium, halogen, nitro, hydroxy, mercapto, cyano, amino, oxo, thio, carboxyl, C₁₋₃ alkyl, C₁₋₃ deuteroalkyl, C₁₋₃ haloalkyl, C₁₋₃ hydroxyalkyl, C₁₋₃ alkoxy, C₁₋₃ deuteroalkoxy, and C₁₋₃ haloalkoxy.

In a further preferred embodiment of the present invention, the compound is further as represented by general formula (XV): 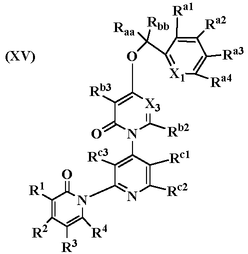 wherein:
X₁ is selected from N or CR^{a5};
X₃ is selected from N or CR^{b1};
each R^{a1}, R^{a2}, R^{a3}, R^{a4}, or R^{a5} is independently selected from hydrogen, deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl, wherein the amino, C₁₋₆ alkyl, C₁₋₆ deuteroalkyl, C₁₋₆ haloalkyl, C₁₋₆ alkoxy, C₁₋₆ haloalkoxy, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
or any two adjacent or non-adjacent R^{a1}, R^{a2}, R^{a3}, R^{a4}, and R^{a5} are connected to form C₃₋₁₂ cycloalkyl, 3- to 12-membered heterocyclyl, C₆₋₁₄ aryl, or 5- to 14-membered heteroaryl, wherein the C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered each heteroaryl;
each Rₐₐ or R_{bb} is independently selected from hydrogen, deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl, wherein the amino, C₁₋₆ alkyl, C₁₋₆ deuteroalkyl, C₁₋₆ haloalkyl, C₁₋₆ alkoxy, C₁₋₆ haloalkoxy, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
or Rₐₐ and R_{bb} are connected to form C₃₋₁₂ cycloalkyl, 3- to 12-membered heterocyclyl, C₆₋₁₄ aryl, or 5- to 14-membered heteroaryl, wherein the C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
each R^{b1}, R^{b2}, or R^{b3} is independently selected from hydrogen, deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl, wherein the amino, C₁₋₆ alkyl, C₁₋₆ deuteroalkyl, C₁₋₆ haloalkyl, C₁₋₆ alkoxy, C₁₋₆ haloalkoxy, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
or the substituents R^{b1} and R^{b2} are connected to form C₃₋₁₂ cycloalkyl, 3- to 12-membered heterocyclyl, C₆₋₁₄ aryl, or 5- to 14-membered heteroaryl, wherein the C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
each R^{c1}, R^{c2}, or R^{c3} is independently selected from hydrogen, deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl, wherein the amino, C₁₋₆ alkyl, C₁₋₆ deuteroalkyl, C₁₋₆ haloalkyl, C₁₋₆ alkoxy, C₁₋₆ haloalkoxy, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
or the substituents R^{c1} and R^{c2} are connected to form C₃₋₁₂ cycloalkyl, 3- to 12-membered heterocyclyl, C₆₋₁₄ aryl, or 5- to 14-membered heteroaryl, wherein the C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
or the substituents R^{c1} and R^{b2} are connected to form 3- to 12-membered heterocyclyl or 5- to 14-membered heteroaryl, wherein the 3- to 12-membered heterocyclyl and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
R¹ is selected from hydrogen, deuterium, halogen, nitro, hydroxy, mercapto, cyano, amino, oxo, thio, carboxyl, C₁₋₆ alkyl, C₁₋₆ deuteroalkyl, C₁₋₆ bridged cycloalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, -(CRₑₑR_{ff})ₙ₄C(O)R_{gg},-(CRₑₑR_{ff})ₙ₄NRₕₕC(O)R_{gg}, -(CRₑₑR_{ff})ₙ₄NRₕₕOR_{gg}, -(CRₑₑR_{ff})ₙ₄S(O)₂R_{gg}, -(CRₑₑR_{ff})ₙ₄S(O)R_{gg}, -(CRₑₑR_{ff})ₙ₄SR_{gg}, -(CRₑₑR_{ff})ₙ₄C(O)NRₕₕR_{gg}, -(CRₑₑR_{ff})ₙ₄S(O)NRₕₕR_{gg}, -(CRₑₑR_{ff})ₙ₄SNRₕₕR_{gg}, or -(CRₑₑR_{ff})ₙ₄S(O)₂NRₕₕR_{gg}, wherein the amino, C₁₋₆ alkyl, C₁₋₆ deuteroalkyl, C₁₋₆ bridged cycloalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
Rₑₑ, R_{ff}, R_{gg}, and Rₕₕ are each independently selected from hydrogen, deuterium, halogen, nitro, hydroxy, mercapto, cyano, amino, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl;
or Rₑₑ and R_{ff} are connected to form C₃₋₁₂ cycloalkyl, 3- to 12-membered heterocyclyl, C₆₋₁₄ aryl, or 5- to 14-membered heteroaryl, wherein the C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
each R², R³, or R⁴ is independently selected from hydrogen, deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl, wherein the amino, C₁₋₆ alkyl, C₁₋₆ deuteroalkyl, C₁₋₆ haloalkyl, C₁₋₆ alkoxy, C₁₋₆ haloalkoxy, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
or any two adjacent or non-adjacent R¹, R², R³, and R⁴ are connected to form C₃₋₁₂ cycloalkyl, 3-to 12-membered heterocyclyl, C₆₋₁₄ aryl, or 5- to 14-membered heteroaryl, wherein the C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
n4 is 0, 1, 2, 3, or 4; and
the 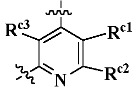 is not

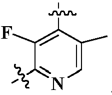

In a further preferred embodiment of the present invention, the compound is further as represented by general formula (XIV-A), (XIV-B), or (XIV-C): 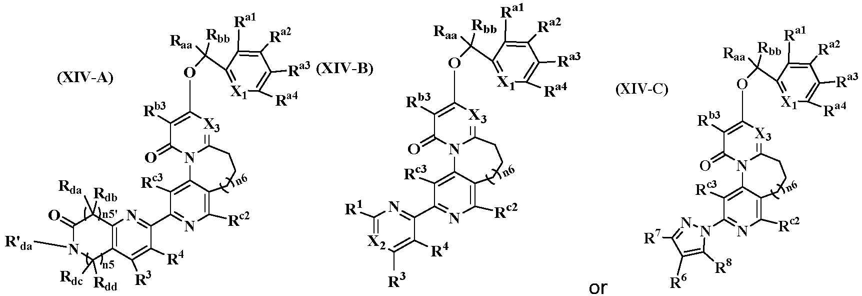 wherein:
X₁ is selected from N or CR^{a5};
X₂ is selected from N or CR²;
X₃ is selected from N or CR^{b1};
each R^{a1}, R^{a2}, R^{a3}, R^{a4}, or R^{a5} is independently selected from hydrogen, deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl, wherein the amino, C₁₋₆ alkyl, C₁₋₆ deuteroalkyl, C₁₋₆ haloalkyl, C₁₋₆ alkoxy, C₁₋₆ haloalkoxy, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
or any two adjacent or non-adjacent R^{a1}, R^{a2}, R^{a3}, R^{a4}, and R^{a5} are connected to form C₃₋₁₂ cycloalkyl, 3- to 12-membered heterocyclyl, C₆₋₁₄ aryl, or 5- to 14-membered heteroaryl, wherein the C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered each heteroaryl;
each Rₐₐ or R_{bb} is independently selected from hydrogen, deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl, wherein the amino, C₁₋₆ alkyl, C₁₋₆ deuteroalkyl, C₁₋₆ haloalkyl, C₁₋₆ alkoxy, C₁₋₆ haloalkoxy, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
or Rₐₐ and R_{bb} are connected to form C₃₋₁₂ cycloalkyl, 3- to 12-membered heterocyclyl, C₆₋₁₄ aryl, or 5- to 14-membered heteroaryl, wherein the C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
each R^{b1} or R^{b3} is independently selected from hydrogen, deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl, wherein the amino, C₁₋₆ alkyl, C₁₋₆ deuteroalkyl, C₁₋₆ haloalkyl, C₁₋₆ alkoxy, C₁₋₆ haloalkoxy, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
each R^{c2} or R^{c3} is independently selected from hydrogen, deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl, wherein the amino, C₁₋₆ alkyl, C₁₋₆ deuteroalkyl, C₁₋₆ haloalkyl, C₁₋₆ alkoxy, C₁₋₆ haloalkoxy, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
each R_{da}, R_{db}, or R'_{da} is independently selected from hydrogen, deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl, wherein the amino, C₁₋₆ alkyl, C₁₋₆ deuteroalkyl, C₁₋₆ haloalkyl, C₁₋₆ alkoxy, C₁₋₆ haloalkoxy, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
or R_{da} and R_{db} are connected to form C₃₋₁₂ cycloalkyl, 3- to 12-membered heterocyclyl, C₆₋₁₄ aryl, or 5- to 14-membered heteroaryl, wherein the C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
each R_{dc} or R_{dd} is independently selected from hydrogen, deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl, wherein the amino, C₁₋₆ alkyl, C₁₋₆ deuteroalkyl, C₁₋₆ haloalkyl, C₁₋₆ alkoxy, C₁₋₆ haloalkoxy, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
or R_{dc} and R_{dd} are connected to form C₃₋₁₂ cycloalkyl, 3- to 12-membered heterocyclyl, C₆₋₁₄ aryl, or 5- to 14-membered heteroaryl, wherein the C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
R¹ is selected from hydrogen, deuterium, halogen, nitro, hydroxy, mercapto, cyano, amino, oxo, thio, carboxyl, C₁₋₆ alkyl, C₁₋₆ deuteroalkyl, C₁₋₆ bridged cycloalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, -(CRₑₑR_{ff})ₙ₄C(O)R_{gg},-(CRₑₑR_{ff})ₙ₄NRₕₕC(O)R_{gg}, -(CRₑₑR_{ff})ₙ₄NRₕₕOR_{gg}, -(CRₑₑR_{ff})ₙ₄S(O)₂R_{gg}, -(CRₑₑR_{ff})ₙ₄S(O)R_{gg}, -(CRₑₑR_{ff})ₙ₄SR_{gg}, -(CRₑₑR_{ff})ₙ₄C(O)NRₕₕR_{gg}, -(CRₑₑR_{ff})ₙ₄S(O)NRₕₕR_{gg}, -(CRₑₑR_{ff})ₙ₄SNRₕₕR_{gg}, or -(CRₑₑR_{ff})ₙ₄S(O)₂NRₕₕR_{gg}, wherein the amino, C₁₋₆ alkyl, C₁₋₆ deuteroalkyl, C₁₋₆ bridged cycloalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
Rₑₑ, R_{ff}, R_{gg}, and Rₕₕ are each independently selected from hydrogen, deuterium, halogen, nitro, hydroxy, mercapto, cyano, amino, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl;
or Rₑₑ and R_{ff} are connected to form C₃₋₁₂ cycloalkyl, 3- to 12-membered heterocyclyl, C₆₋₁₄ aryl, or 5- to 14-membered heteroaryl, wherein the C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
each R², R³, or R⁴ is independently selected from hydrogen, deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl, wherein the amino, C₁₋₆ alkyl, C₁₋₆ deuteroalkyl, C₁₋₆ haloalkyl, C₁₋₆ alkoxy, C₁₋₆ haloalkoxy, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
or any two adjacent or non-adjacent R¹, R², R³, and R⁴ are connected to form C₃₋₁₂ cycloalkyl, 3-to 12-membered heterocyclyl, C₆₋₁₄ aryl, or 5- to 14-membered heteroaryl, wherein the C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
R⁷ is selected from hydrogen, deuterium, halogen, nitro, hydroxy, mercapto, cyano, amino, oxo, thio, carboxyl, C₁₋₆ alkyl, C₁₋₆ deuteroalkyl, C₁₋₆ bridged cycloalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, -(CRₑₑR_{ff})ₙ₄C(O)R_{gg},-(CRₑₑR_{ff})ₙ₄NRₕₕC(O)R_{gg}, -(CR_{gg}R_{ff})ₙ₄NRₕₕOR_{gg}, -(CRₑₑR_{ff})ₙ₄S(O)₂R_{gg}, -(CRₑₑR_{ff})ₙ₄S(O)R_{gg}, -(CRₑₑR_{ff})ₙ₄SR_{gg}, -(CRₑₑR_{ff})ₙ₄C(O)NRₕₕR_{gg}, -(CRₑₑR_{ff})ₙ₄S(O)NRₕₕR_{gg}, -(CRₑₑR_{ff})ₙ₄SNRₕₕR_{gg}, or -(CRₑₑR_{ff})ₙ₄S(O)₂NRₕₕR_{gg}, wherein the amino, C₁₋₆ alkyl, C₁₋₆ deuteroalkyl, C₁₋₆ bridged cycloalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
Rₑₑ, R_{ff}, R_{gg}, and Rₕₕ are each independently selected from hydrogen, deuterium, halogen, nitro, hydroxy, mercapto, cyano, amino, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl;
or Rₑₑ and R_{ff} are connected to form C₃₋₁₂ cycloalkyl, 3- to 12-membered heterocyclyl, C₆₋₁₄ aryl, or 5- to 14-membered heteroaryl, wherein the C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
each R⁶ or R⁸ is independently selected from hydrogen, deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl, wherein the amino, C₁₋₆ alkyl, C₁₋₆ deuteroalkyl, C₁₋₆ haloalkyl, C₁₋₆ alkoxy, C₁₋₆ haloalkoxy, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
or any two adjacent or non-adjacent R⁶, R⁷, and R⁸ are connected to form C₃₋₁₂ cycloalkyl, 3- to 12-membered heterocyclyl, C₆₋₁₄ aryl, or 5- to 14-membered heteroaryl, wherein the C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
n4 is 0, 1, 2, 3, or 4;
n5 is 0, 1, or 2;
n5' is 0, 1, or 2;
n5 and n5' are not both 0; and
n6 is 0, 1, 2, 3, or 4.

In a further preferred embodiment of the present invention, the compound is further as represented by general formula (I'-1-1), (I'-1-2), (XIV-1), (XIV-2), (XIV-A-1), (XIV-A-2), (XIV-B-1), (XIV-B-2), (XIV-C-1), (XIV-C-2), (XV-1), or (XV-2):

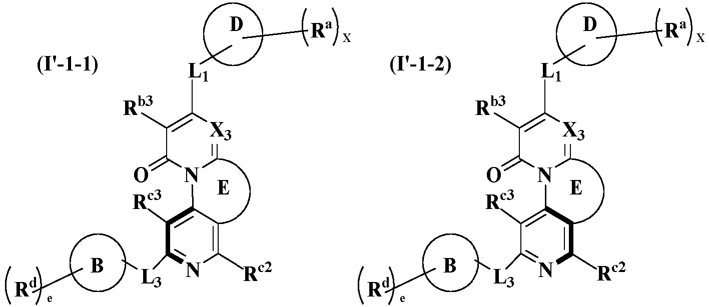

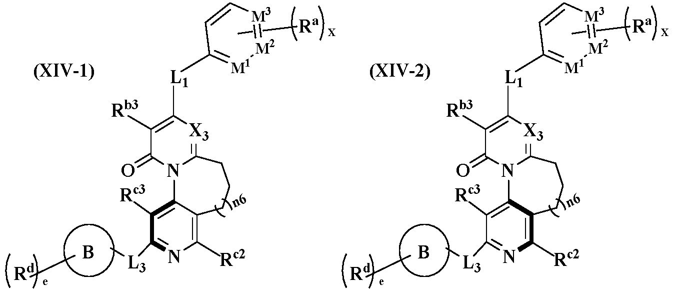

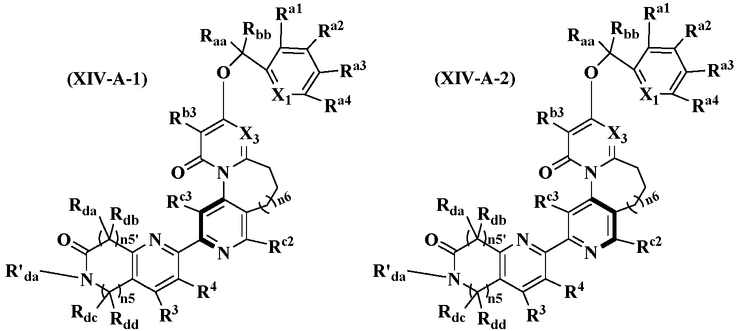

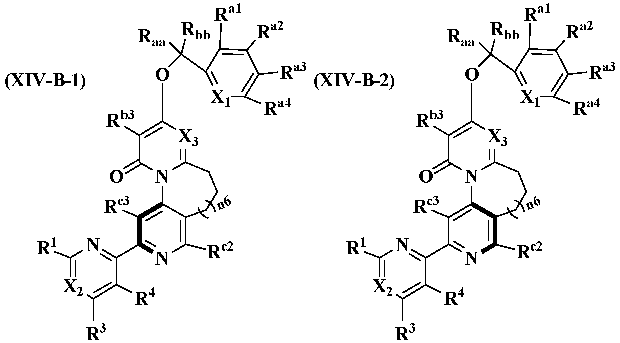

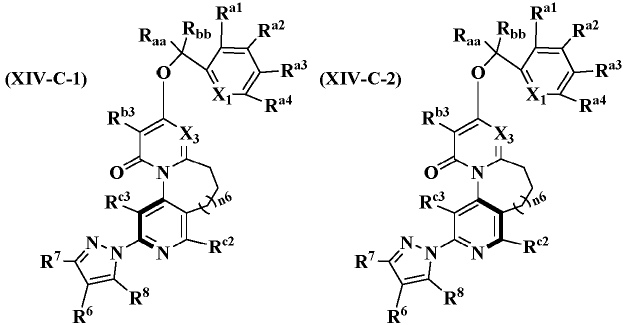

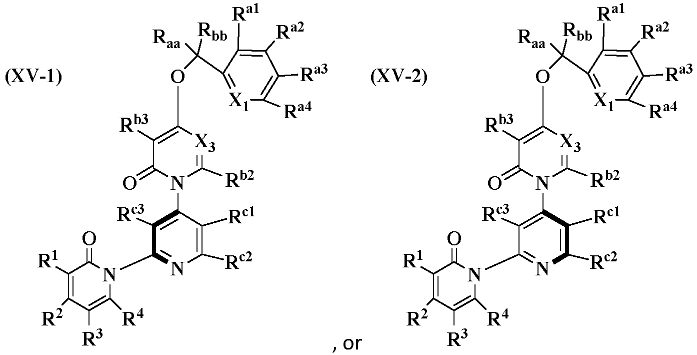

In a further preferred embodiment of the present invention, each R^{a} described in the present invention is independently selected from hydrogen, deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl, wherein the amino, C₁₋₆ alkyl, C₁₋₆ deuteroalkyl, C₁₋₆ haloalkyl, C₁₋₆ alkoxy, C₁₋₆ haloalkoxy, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl; and
preferably, each R^{a} is independently selected from hydrogen, deuterium, halogen, nitro, hydroxy, mercapto, cyano, amino, oxo, thio, carboxyl, C₁₋₃ alkyl, C₁₋₃ deuteroalkyl, C₁₋₃ haloalkyl, C₁₋₃ hydroxyalkyl, C₁₋₃ alkoxy, C₁₋₃ deuteroalkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl, or 5- to 10-membered heteroaryl, wherein the amino, C₁₋₃ alkyl, C₁₋₃ deuteroalkyl, C₁₋₃ haloalkyl, C₁₋₃ alkoxy, C₁₋₃ haloalkoxy, C₁₋₃ hydroxyalkyl, C₂₋₄ alkenyl, C₂₋₄ alkynyl, C₃₋₈ cycloalkyl, 3- to 8-membered heterocyclyl, C₆₋₁₀ aryl, and 5- to 10-membered heteroaryl may be optionally further substituted with one or more substituents of deuterium, halogen, nitro, hydroxy, mercapto, cyano, amino, oxo, thio, carboxyl, C₁₋₃ alkyl, C₁₋₃ deuteroalkyl, C₁₋₃ haloalkyl, C₁₋₃ hydroxyalkyl, C₁₋₃ alkoxy, C₁₋₃ deuteroalkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl, and 5- to 10-membered heteroaryl.

In a further preferred embodiment of the present invention, each R^{b} described in the present invention is independently selected from hydrogen, deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl, wherein the amino, C₁₋₆ alkyl, C₁₋₆ deuteroalkyl, C₁₋₆ haloalkyl, C₁₋₆ alkoxy, C₁₋₆ haloalkoxy, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl; and
preferably, each R^{b} is independently selected from hydrogen, deuterium, halogen, nitro, hydroxy, mercapto, cyano, amino, oxo, thio, carboxyl, C₁₋₃ alkyl, C₁₋₃ deuteroalkyl, C₁₋₃ haloalkyl, C₁₋₃ hydroxyalkyl, C₁₋₃ alkoxy, C₁₋₃ deuteroalkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl, or 5- to 10-membered heteroaryl, wherein the amino, C₁₋₃ alkyl, C₁₋₃ deuteroalkyl, C₁₋₃ haloalkyl, C₁₋₃ alkoxy, C₁₋₃ haloalkoxy, C₁₋₃ hydroxyalkyl, C₂₋₄ alkenyl, C₂₋₄ alkynyl, C₃₋₈ cycloalkyl, 3- to 8-membered heterocyclyl, C₆₋₁₀ aryl, and 5- to 10-membered heteroaryl may be optionally further substituted with one or more substituents of deuterium, halogen, nitro, hydroxy, mercapto, cyano, amino, oxo, thio, carboxyl, C₁₋₃ alkyl, C₁₋₃ deuteroalkyl, C₁₋₃ haloalkyl, C₁₋₃ hydroxyalkyl, C₁₋₃ alkoxy, C₁₋₃ deuteroalkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl, and 5- to 10-membered heteroaryl.

In a further preferred embodiment of the present invention, each R^{c} described in the present invention is independently selected from hydrogen, deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl, wherein the amino, C₁₋₆ alkyl, C₁₋₆ deuteroalkyl, C₁₋₆ haloalkyl, C₁₋₆ alkoxy, C₁₋₆ haloalkoxy, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
preferably, each R^{c} is independently selected from hydrogen, deuterium, halogen, nitro, hydroxy, mercapto, cyano, amino, oxo, thio, carboxyl, C₁₋₃ alkyl, C₁₋₃ deuteroalkyl, C₁₋₃ haloalkyl, C₁₋₃ hydroxyalkyl, C₁₋₃ alkoxy, C₁₋₃ deuteroalkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl, or 5- to 10-membered heteroaryl, wherein the amino, C₁₋₃ alkyl, C₁₋₃ deuteroalkyl, C₁₋₃ haloalkyl, C₁₋₃ alkoxy, C₁₋₃ haloalkoxy, C₁₋₃ hydroxyalkyl, C₂₋₄ alkenyl, C₂₋₄ alkynyl, C₃₋₈ cycloalkyl, 3- to 8-membered heterocyclyl, C₆₋₁₀ aryl, and 5- to 10-membered heteroaryl may be optionally further substituted with one or more substituents of deuterium, halogen, nitro, hydroxy, mercapto, cyano, amino, oxo, thio, carboxyl, C₁₋₃ alkyl, C₁₋₃ deuteroalkyl, C₁₋₃ haloalkyl, C₁₋₃ hydroxyalkyl, C₁₋₃ alkoxy, C₁₋₃ deuteroalkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl, and 5- to 10-membered heteroaryl;
or R^{b} and R^{c} are connected to form C₃₋₁₂ cycloalkyl, 3- to 12-membered heterocyclyl, C₆₋₁₄ aryl, or 5- to 14-membered heteroaryl, wherein C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl.

In a further preferred embodiment of the present invention, each R^{d} described in the present invention is independently selected from hydrogen, deuterium, halogen, nitro, hydroxy, mercapto, cyano, amino, oxo, thio, carboxyl, C₁₋₆ alkyl, 5- to 10-membered bridged cycloalkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, -(CRₑₑR_{ff})ₙ₄C(O)R_{gg}, -(CRₑₑR_{ff})ₙ₄NRₕₕC(O)R_{gg}, -(CRₑₑR_{ff})ₙ₄S(O)₂R_{gg},-(CRₑₑR_{ff})ₙ₄C(O)NRₕₕR_{gg}, or -(CRₑₑR_{ff})ₙ₄S(O)₂NRₕₕR_{gg}, wherein the amino, C₁₋₆ alkyl, 5- to 10-membered bridged cycloalkyl, C₁₋₆ deuteroalkyl, C₁₋₆ haloalkyl, C₁₋₆ alkoxy, C₁₋₆ haloalkoxy, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
Rₑₑ, R_{ff}, R_{gg}, and Rₕₕ are each independently selected from hydrogen, deuterium, halogen, nitro, hydroxy, mercapto, cyano, amino, carboxyl, C₁₋₈ alkyl, C₁₋₈ deuteroalkyl, C₁₋₈ haloalkyl, C₁₋₈ hydroxyalkyl, C₁₋₈ alkoxy, C₁₋₈ deuteroalkoxy, C₁₋₈ haloalkoxy, C₂₋₈ alkenyl, C₂₋₈ alkynyl, C₃₋₁₂ cycloalkyl, 3- to 12-membered heterocyclyl, C₆₋₁₄ aryl, or 5- to 14-membered heteroaryl; preferably, Rₑₑ, R_{ff}, R_{gg}, and Rₕₕ are each independently selected from hydrogen, deuterium, halogen, nitro, hydroxy, mercapto, cyano, amino, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₈ cycloalkyl, 3- to 8-membered heterocyclyl, C₆₋₁₀ aryl, or 5- to 10-membered heteroaryl;
or Rₑₑ and R_{ff} are connected to form cycloalkyl, heterocyclyl, aryl, or heteroaryl, wherein the cycloalkyl, heterocyclyl, aryl, and heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl; and
n4 is 0, 1, 2, 3, or 4.

Preferably,
each R^{d} is independently selected from hydrogen, deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl, wherein the amino, C₁₋₆ alkyl, C₁₋₆ deuteroalkyl, C₁₋₆ haloalkyl, C₁₋₆ alkoxy, C₁₋₆ haloalkoxy, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl; and
preferably, each R^{d} is independently selected from hydrogen, deuterium, halogen, nitro, hydroxy, mercapto, cyano, amino, oxo, thio, carboxyl, C₁₋₃ alkyl, C₁₋₃ deuteroalkyl, C₁₋₃ haloalkyl, C₁₋₃ hydroxyalkyl, C₁₋₃ alkoxy, C₁₋₃ deuteroalkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl, or 5- to 10-membered heteroaryl, wherein the amino, C₁₋₃ alkyl, C₁₋₃ deuteroalkyl, C₁₋₃ haloalkyl, C₁₋₃ alkoxy, C₁₋₃ haloalkoxy, C₁₋₃ hydroxyalkyl, C₂₋₄ alkenyl, C₂₋₄ alkynyl, C₃₋₈ cycloalkyl, 3- to 8-membered heterocyclyl, C₆₋₁₀ aryl, and 5- to 10-membered heteroaryl may be optionally further substituted with one or more substituents of deuterium, halogen, nitro, hydroxy, mercapto, cyano, amino, oxo, thio, carboxyl, C₁₋₃ alkyl, C₁₋₃ deuteroalkyl, C₁₋₃ haloalkyl, C₁₋₃ hydroxyalkyl, C₁₋₃ alkoxy, C₁₋₃ deuteroalkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl, and 5- to 10-membered heteroaryl.

Further preferably, each R^{d} is independently selected from hydrogen, deuterium, halogen, nitro, hydroxy, mercapto, cyano, amino, oxo, thio, carboxyl, C₁₋₃ alkyl, C₁₋₃ deuteroalkyl, C₁₋₃ haloalkyl, C₁₋₃ hydroxyalkyl, C₁₋₃ alkoxy, C₁₋₃ deuteroalkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl, 5- to 10-membered heteroaryl, -(CRₑₑR_{ff})ₙ₄C(O)R_{gg}, -(CRₑₑR_{ff})ₙ₄NRₕₕC(O)R_{gg}, -(CRₑₑR_{ff})ₙ₄S(O)₂R_{gg}, -(CRₑₑR_{ff})ₙ₄C(O)NRₕₕR_{gg}, or-(CRₑₑR_{ff})ₙ₄S(O)₂NRₕₕR_{gg};
Rₑₑ, R_{ff}, R_{gg}, and Rₕₕ are each independently selected from hydrogen, deuterium, halogen, nitro, hydroxy, mercapto, cyano, amino, carboxyl, C₁₋₃ alkyl, C₁₋₃ deuteroalkyl, C₁₋₃ haloalkyl, C₁₋₃ hydroxyalkyl, C₁₋₃ alkoxy, C₁₋₃ deuteroalkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl, or 5- to 10-membered heteroaryl; and n4 is 0, 1, 2, 3, or 4.

In a further preferred embodiment of the present invention, R^{a1}, R^{a2}, R^{a3}, R^{a4}, R^{a5}, R^{b1}, R^{b2}, R^{b3}, R^{c1}, R^{c2}, and R^{c3} described in the present invention are each indenpendently selected from hydrogen, deuterium, halogen, nitro, hydroxy, mercapto, cyano, amino, oxo, thio, carboxyl, C₁₋₃ alkyl, C₁₋₃ deuteroalkyl, C₁₋₃ haloalkyl, C₁₋₃ hydroxyalkyl, C₁₋₃ alkoxy, C₁₋₃ deuteroalkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl, or 5- to 10-membered heteroaryl, wherein the amino, C₁₋₃ alkyl, C₁₋₃ deuteroalkyl, C₁₋₃ haloalkyl, C₁₋₃ alkoxy, C₁₋₃ haloalkoxy, C₁₋₃ hydroxyalkyl, C₂₋₄ alkenyl, C₂₋₄ alkynyl, C₃₋₈ cycloalkyl, 3- to 8-membered heterocyclyl, C₆₋₁₀ aryl, and 5- to 10-membered heteroaryl may be optionally further substituted with one or more substituents of deuterium, halogen, nitro, hydroxy, mercapto, cyano, amino, oxo, thio, carboxyl, C₁₋₃ alkyl, C₁₋₃ deuteroalkyl, C₁₋₃ haloalkyl, C₁₋₃ hydroxyalkyl, C₁₋₃ alkoxy, C₁₋₃ deuteroalkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl, and 5- to 10-membered heteroaryl.

In a further preferred embodiment of the present invention, R_{da}, R_{db}, R'_{da}, R_{dc}, and R_{dd} described in the present invention are each independently selected from hydrogen, deuterium, halogen, nitro, hydroxy, mercapto, cyano, amino, oxo, thio, carboxyl, C₁₋₃ alkyl, C₁₋₃ deuteroalkyl, C₁₋₃ haloalkyl, C₁₋₃ hydroxyalkyl, C₁₋₃ alkoxy, C₁₋₃ deuteroalkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl, or 5- to 10-membered heteroaryl, wherein the amino, C₁₋₃ alkyl, C₁₋₃ deuteroalkyl, C₁₋₃ haloalkyl, C₁₋₃ alkoxy, C₁₋₃ deuteroalkoxy, C₁₋₃ hydroxyalkyl, C₂₋₄ alkenyl, C₂₋₄ alkynyl, C₃₋₈ cycloalkyl, 3- to 8-membered heterocyclyl, C₆₋₁₀ aryl, and 5- to 10-membered heteroaryl may be optionally further substituted with one or more substituents of deuterium, halogen, nitro, hydroxy, mercapto, cyano, amino, oxo, thio, carboxyl, C₁₋₃ alkyl, C₁₋₃ deuteroalkyl, C₁₋₃ haloalkyl, C₁₋₃ hydroxyalkyl, C₁₋₃ alkoxy, C₁₋₃ deuteroalkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl, and 5- to 10-membered heteroaryl.

In a further preferred embodiment of the present invention, R¹ and R⁷ described in the present invention are each independently selected from hydrogen, deuterium, halogen, nitro, hydroxy, mercapto, cyano, amino, oxo, thio, carboxyl, C₁₋₃ alkyl, C₁₋₃ deuteroalkyl, C₁₋₃ haloalkyl, C₁₋₃ hydroxyalkyl, C₁₋₃ alkoxy, C₁₋₃ deuteroalkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl, 5- to 10-membered heteroaryl,-(CRₑₑR_{ff})ₙ₄C(O)R_{gg}, -(CRₑₑR_{ff})ₙ₄NRₕₕC(O)R_{gg}, -(CRₑₑR_{ff})ₙ₄S(O)₂R_{gg}, -(CRₑₑR_{ff})ₙ₄C(O)NRₕₕR_{gg}, or-(CRₑₑR_{ff})ₙ₄S(O)₂NRₕₕR_{gg};
Rₑₑ, R_{ff}, R_{gg}, and Rₕₕ are each independently selected from hydrogen, deuterium, halogen, nitro, hydroxy, mercapto, cyano, amino, carboxyl, C₁₋₃ alkyl, C₁₋₃ deuteroalkyl, C₁₋₃ haloalkyl, C₁₋₃ hydroxyalkyl, C₁₋₃ alkoxy, C₁₋₃ deuteroalkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl, or 5- to 10-membered heteroaryl; and n4 is 0, 1, 2, 3, or 4.

In a further preferred embodiment of the present invention, R², R³, R⁴, R⁶, and R⁸ described in the present invention are each independently selected from hydrogen, deuterium, halogen, nitro, hydroxy, mercapto, cyano, amino, oxo, thio, carboxyl, C₁₋₃ alkyl, C₁₋₃ deuteroalkyl, C₁₋₃ haloalkyl, C₁₋₃ hydroxyalkyl, C₁₋₃ alkoxy, C₁₋₃ deuteroalkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl, or 5- to 10-membered heteroaryl, wherein the amino, C₁₋₃ alkyl, C₁₋₃ deuteroalkyl, C₁₋₃ haloalkyl, C₁₋₃ alkoxy, C₁₋₃ haloalkoxy, C₁₋₃ hydroxyalkyl, C₂₋₄ alkenyl, C₂₋₄ alkynyl, C₃₋₈ cycloalkyl, 3- to 8-membered heterocyclyl, C₆₋₁₀ aryl, and 5- to 10-membered heteroaryl may be optionally further substituted with one or more substituents of deuterium, halogen, nitro, hydroxy, mercapto, cyano, amino, oxo, thio, carboxyl, C₁₋₃ alkyl, C₁₋₃ deuteroalkyl, C₁₋₃ haloalkyl, C₁₋₃ hydroxyalkyl, C₁₋₃ alkoxy, C₁₋₃ deuteroalkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl, and 5- to 10-membered heteroaryl.

In a further preferred embodiment of the present invention, each R² described in the present invention is selected from halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl, wherein the amino, C₁₋₆ alkyl, C₁₋₆ deuteroalkyl, C₁₋₆ haloalkyl, C₁₋₆ alkoxy, C₁₋₆ haloalkoxy, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
preferably, R² is selected from halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, or C₂₋₆ alkynyl; and
more preferably, R² is fluorine.

In a further preferred embodiment of the present invention, each R⁶ described in the present invention is selected from halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl, wherein the amino, C₁₋₆ alkyl, C₁₋₆ deuteroalkyl, C₁₋₆ haloalkyl, C₁₋₆ alkoxy, C₁₋₆ haloalkoxy, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
preferably, R⁶ is selected from halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, or C₂₋₆ alkynyl; and
more preferably, R⁶ is fluorine.

The present invention provides a compound represented by general formula (M-1) or (M-2), or a stereoisomer or pharmaceutically acceptable salt thereof: 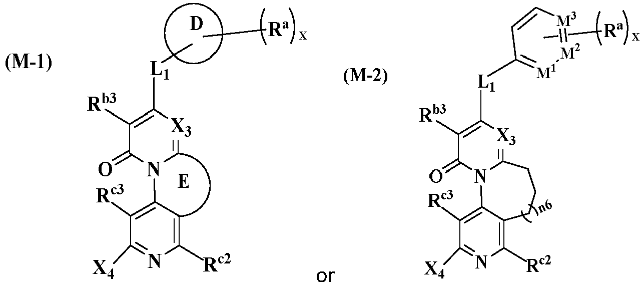 wherein:
X₄ is selected from halogen, preferably chlorine or bromine; and
ring D, ring E, M¹, M², M³, n6, L₁, R^{a}, R^{b3}, R^{c2}, R^{c3}, X₃, and x are as described above.

The present invention further provides a method for preparing the above-mentioned compound of general formula (I'-1) or the stereoisomer and pharmaceutically acceptable salt thereof, comprising the following steps: 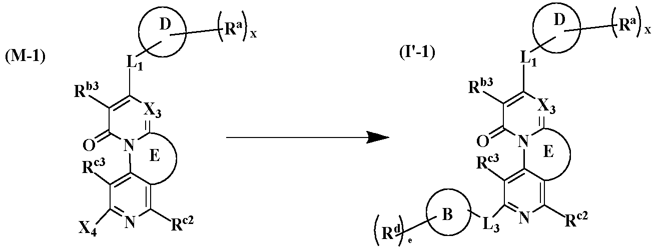
reacting a compound represented by general formula (M-1) to obtain a target compound represented by general formula (I'-1);
wherein
X₄ is selected from halogen, preferably chlorine or bromine; and
ring D, ring E, ring B, L₁, L₃, R^{a}, R^{b3}, R^{c2}, R^{c3}, X₃, R^{d}, x, and e are as described above.

The present invention further provides a method for preparing the above-mentioned compound of general formula (XIV) or the stereoisomer and pharmaceutically acceptable salt thereof, comprising the following steps: 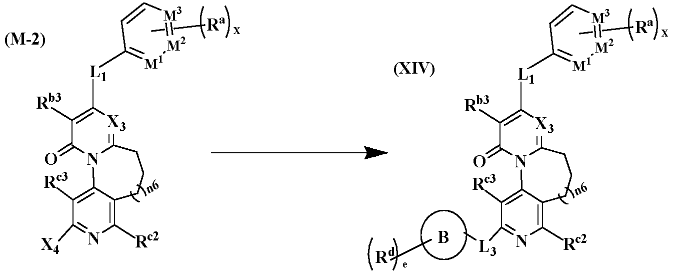
reacting a compound represented by general formula (M-2) to obtain a target compound represented by general formula (XIV);
wherein
X₄ is selected from halogen, preferably chlorine or bromine; and
M¹, M², M³, L₁, L₃, R^{a}, R^{b3}, R^{c2}, R^{c3}, X₃, R^{d}, x, e, and n6 are as described above.

The present invention further provides a method for preparing the above-mentioned compound of general formula (XV) or the stereoisomer and pharmaceutically acceptable salt thereof, comprising the following steps: 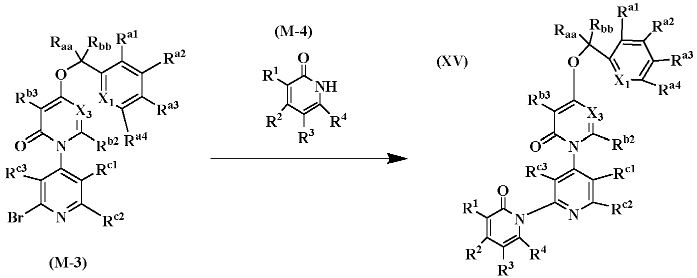
reacting general formula (M-3) with general formula (M-4) to obtain a target compound represented by general formula (XV);
wherein
X₁, X₃, R^{a1}, R^{a2}, R^{a3}, R^{a4}, Rₐₐ, R_{bb}, R^{b2}, R^{b3}, R^{c1}, R^{c2}, R^{c3}, R¹, R², R³, and R⁴ are as described above.

The present invention further relates to a pharmaceutical composition comprising a therapeutically effective dose of the compound or the stereoisomer or pharmaceutically acceptable salt thereof, and one or more pharmaceutically acceptable carriers, diluents, or excipients; and furthermore, the weight percentage of the compound or the stereoisomer or pharmaceutically acceptable salt thereof in the composition is 0.1-95%, preferably 0.5-85%, more preferably 1-60%, further preferably 10-50%, further more preferably 15-40%, further more preferably 20-30%, and further more preferably 20-25% (based on the total weight of the pharmaceutical composition).

The present invention further relates to the use of the compound or the stereoisomer or pharmaceutically acceptable salt thereof, or the pharmaceutical composition in the preparation of a medicament for the treatment of a p38 kinase-mediated disease.

The present invention further relates to the use of the compound, or the stereoisomer or pharmaceutically acceptable salt thereof, or the pharmaceutical composition in the preparation of a medicament for the treatment of autoimmune diseases, metabolic diseases, tumors, etc., wherein the autoimmune diseases are selected from chronic inflammatory diseases, acute inflammatory diseases, autoimmune diseases, atherosclerosis, etc.; the metabolic diseases are selected from diabetes, fibrotic diseases, etc.; and the tumors are selected from leukemia, lymphoma, etc. The present invention further relates to a method for treating or preventing and/or treating autoimmune diseases, metabolic diseases, tumors, etc., comprising administering a therapeutically effective dose of the compound of the present invention, or the stereoisomer or pharmaceutically acceptable salt thereof, or the pharmaceutical composition thereof to a patient; wherein the autoimmune diseases are selected from chronic inflammatory diseases, acute inflammatory diseases, autoimmune diseases, atherosclerosis, etc.; the metabolic diseases are selected from diabetes, fibrotic diseases, etc.; and the tumors are selected from leukemia, lymphoma, etc.

The present invention further provides a method for treating a disease condition with the compound or pharmaceutical composition of the present invention. The disease condition includes, but is not limited to, conditions related to p38 kinase-mediated diseases.

### Detailed Description of the Invention

Unless stated to the contrary, the terms used in the specification and claims have the following meanings.

The term "alkyl" refers to a saturated aliphatic hydrocarbon group, which is a linear or branched chain group containing 1 to 20 carbon atoms, preferably alkyl containing 1 to 12 carbon atoms, more preferably alkyl containing 1 to 8 carbon atoms, further preferably alkyl containing 1 to 6 carbon atoms, and most preferably alkyl containing 1 to 3 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 4-heptyl, 1-propylbutyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, their respective branched chain isomers, etc. Lower alkyl groups containing 1 to 6 carbon atoms are more preferred, and non-limiting examples thereof include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, n-heptyl, 4-heptyl, 1-propylbutyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, etc. Alkyl may be substituted or unsubstituted, and when it is substituted, the substituents may be at any available point of attachment. The substituents are preferably one or more groups independently selected from alkyl, alkenyl, alkynyl, alkoxy, alkylthio, alkylamino, halogen, mercapto, hydroxy, nitro, cyano, cycloalkyl, heterocycloalkyl, aryl, heteroaryl, heterocycloalkoxy, cycloalkylthio, heterocycloalkylthio, oxo, carboxyl or carboxylate group, and methyl, ethyl, isopropyl, tert-butyl, haloalkyl, deuteroalkyl, alkoxy-substituted alkyl and hydroxy-substituted alkyl are preferred in the present invention.

The term "alkylene" refers to bivalent alkyl formed by further replacing one hydrogen atom of alkyl, wherein the alkyl is as defined above. For example, "methylene" refers to -CH₂-, "ethylene" refers to -(CH₂)₂-, "propylene" refers to -(CH₂)₃-, and "butylene" refers to -(CH₂)₄-, etc. The point of attachment of an alkylene chain to the rest of a molecule and to a group can be via one carbon or any two carbons in the chain. Alkylene may be substituted or unsubstituted, and when it is substituted, the substituents may be at any available point of attachment. The substituents are preferably one or more groups independently selected from alkyl, alkenyl, alkynyl, alkoxy, alkylthio, alkylamino, halogen, mercapto, hydroxy, nitro, cyano, cycloalkyl, heterocycloalkyl, aryl, heteroaryl, heterocycloalkoxy, cycloalkylthio, heterocycloalkylthio, oxo, carboxyl or carboxylate group, and methyl, ethyl, isopropyl, tert-butyl, haloalkyl, deuteroalkyl, alkoxy-substituted alkyl and hydroxy-substituted alkyl are preferred in the present invention.

The term "alkenyl" refers to alkyl as defined above consisting of at least two carbon atoms and at least one carbon-carbon double bond, which is a linear or branched group containing 2 to 20 carbon atoms, preferably alkenyl containing 2 to 12 carbon atoms, more preferably alkenyl containing 2 to 8 carbon atoms, further preferably alkenyl containing 2 to 6 carbon atoms, and most preferably alkenyl containing 2 to 4 carbon atoms. Examples are vinyl, 1-propenyl, 2-propenyl, 1-butenyl, 2-butenyl, or 3-butenyl, etc. Alkenyl can be substituted or unsubstituted. When it is substituted, the substituent is preferably one or more of groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, or heterocycloalkylthio.

The term "alkenylene" refers to bivalent alkenyl formed by further replacing one hydrogen atom of alkenyl, wherein the alkenyl group is defined as above, for example, vinylidene, propenylidene, and butenylidene. Alkenylene can be substituted or unsubstituted. When it is substituted, the substituent is preferably one or more of groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, or heterocycloalkylthio.

The term "alkynyl" refers to alkyl as defined above consisting of at least two carbon atoms and at least one carbon-carbon triple bond, which is a linear or branched group containing 2 to 20 carbon atoms, preferably alkynyl containing 2 to 12 carbon atoms, more preferably alkynyl containing 2 to 8 carbon atoms, further preferably alkynyl containing 2 to 6 carbon atoms, and most preferably alkynyl containing 2 to 4 carbon atoms. Examples are ethynyl, propynyl, 1-butynyl, 2-butynyl, or 3-butynyl, etc. Alkynyl can be substituted or unsubstituted. When it is substituted, the substituent is preferably one or more of groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, or heterocycloalkylthio.

The term "alkynylene" refers to bivalent alkynyl formed by further replacing one hydrogen atom of alkynyl, wherein the alkynyl is as defined above. Examples are ethynylene, propynylene, butynylene, etc. Alkynylene can be substituted or unsubstituted. When it is substituted, the substituent is preferably one or more of groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, or heterocycloalkylthio.

The term "cycloalkyl" refers to a saturated or partially unsaturated monocyclic or polycyclic cyclic hydrocarbon substituent, and the cycloalkyl comprises 3 to 20 carbon atoms, preferably 3 to 12 carbon atoms, more preferably 3 to 8 carbon atoms, and further preferably 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, etc. Polycyclic cycloalkyl includes spiro, fused and bridged cycloalkyl, preferably cyclopropyl, cyclobutyl, cyclohexyl, cyclopentyl and cycloheptyl.

The term "spirocycloalkyl" refers to a 5- to 20-membered polycyclic group with monocyclic rings sharing one carbon atom (called a spiro atom). It may contain one or more double bonds, but no ring has a completely conjugated π electron system. Preferably, it is 6- to 14-membered and more preferably 7- to 10-membered. According to the number of shared spiro atoms between rings, the spirocycloalkyl is divided into monospirocycloalkyl, bispirocycloalkyl or polyspirocycloalkyl, preferably monospirocycloalkyl and bispirocycloalkyl. More preferably, it is 3-membered/6-membered, 3-membered/5-membered, 4-membered/4-membered, 4-membered/5-membered, 4-membered/6-membered, 5-membered/5-membered or 5-membered/6-membered monospirocycloalkyl. Non-limiting examples of spirocycloalkyl include: 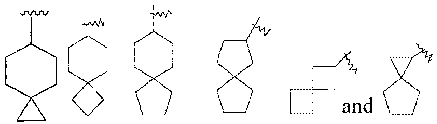 etc.;
and also included are spirocycloalkyl in which monospirocycloalkyl and heterocycloalkyl share a spiro atom. Non-limiting examples include: 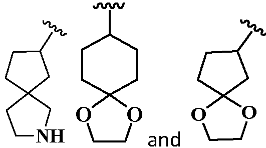 etc.

The term "fused cycloalkyl" refers to a 5- to 20-membered all-carbon polycyclic group in which each ring in the system shares a pair of carbon atoms neighboring another ring in the system, one or more ring of which may contain one or more double bonds, but no ring has a fully conjugated π electron system. Preferably, it is 6- to 14-membered and more preferably 7-to 10-membered. According to the number of constituent rings, it can be divided into bicyclic, tricyclic, tetracyclic or polycyclic fused cycloalkyl, preferably bicyclic or tricyclic, and more preferably 5-membered/5-membered or 5-membered/6-membered bicyclic cycloalkyl. Non-limiting examples of fused cycloalkyl include: 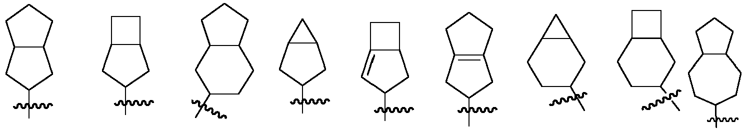 etc.

The term "bridged cycloalkyl" refers to a 5- to 20-membered all-carbon polycyclic group with any two rings sharing two carbon atoms that are not directly connected. It may contain one or more double bonds, but no ring has a fully conjugated π electron system. It is preferably 6- to 14- or 5- to 14-membered and more preferably 7- to 10-membered or 5- to 10-membered. According to the number of constituent rings, it can be divided into bicyclic, tricyclic, tetracyclic or polycyclic bridged cycloalkyl, preferably bicyclic, tricyclic or tetracyclic, and more preferably bicyclic or tricyclic. Non-limiting examples of bridged cycloalkyl include:

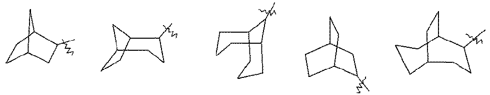

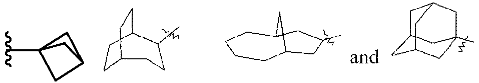

The cycloalkyl ring can be fused to an aryl, heteroaryl or heterocycloalkyl ring, wherein the ring connected to the parent structure is cycloalkyl, and non-limiting examples include indanyl, tetrahydronaphthyl, benzocycloheptyl, 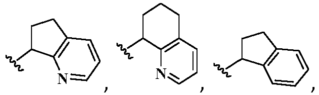 etc. Cycloalkyl may be optionally substituted or unsubstituted, and when it is substituted, the substituent is preferably one or more groups independently selected from alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio, heterocycloalkylthio, oxo, carboxyl, or carboxylate group.

The term "cycloalkylene" refers to bivalent cycloalkyl formed by further replacing one hydrogen atom of cycloalkyl, wherein the cycloalkylene is optionally substituted or unsubstituted, and the cycloalkyl is defined as above.

The term "heterocyclyl" refers to a saturated or partially unsaturated monocyclic or polycyclic cyclic hydrocarbon substituent, which comprises 3 to 20 ring atoms, wherein one or more ring atoms are heteroatoms selected from nitrogen, oxygen, C(O), or S(O)ₘ (wherein m is an integer of 0 to 2), but excluding ring moieties of -O-O-, -O-S-, or -S-S-, and the remaining ring atoms are carbon. Preferably, it comprises 3 to 12 ring atoms, of which 1-4 are heteroatoms; more preferably, it comprises 3 to 8 ring atoms or 6 to 12 ring atoms; most preferably, it comprises 3 to 6 ring atoms; and further preferably, it is a 3- to 8-membered heterocyclyl containing 1-3 nitrogen atoms, optionally substituted with 1-2 oxygen atoms, sulfur atoms, or oxo, including monocyclic heterocyclyl, spirocyclic heterocyclyl, or fused heterocyclyl.

Non-limiting examples of monocyclic heterocyclyl include oxetanyl, azetidinyl, thietanyl, pyrrolidinyl, imidazolidinyl, tetrahydrofuryl, tetrahydrothienyl, tetrahydropyranyl, dihydroimidazolyl, dihydrofuryl, dihydropyrazolyl, dihydropyrrolyl, piperidyl, piperazinyl, morpholinyl, thiomorpholinyl, homopiperazinyl, azepanyl, 1,4-diazacycloheptyl, pyridonyl, pyranyl, tetrahydrothiopyranyl dioxide group, 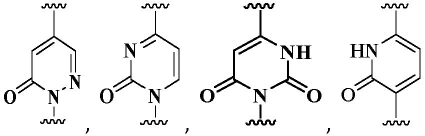 etc.

Polycyclic heterocyclyl includes spiro, fused and bridged heterocyclyl; the involved spiro, fused and bridged heterocyclyl are optionally connected to other groups through a single bond, or further fused to other cycloalkyl, heterocyclyl, aryl and heteroaryl through any two or more atoms on the ring.

The term "spiroheterocyclyl" refers to a 5- to 20-membered polycyclic heterocyclic group with monocyclic rings sharing one atom (called a spiro atom), wherein one or more ring atoms are heteroatoms selected from nitrogen, oxygen, or S(O)ₘ (wherein m is an integer of 0 to 2), and the remaining ring atoms are carbon. Spiroheterocyclyl may contain one or more double bonds, but no ring has a fully conjugated π electron system. Preferably, it is 6- to 14-membered and more preferably 8- to 12-membered. According to the number of shared spiro atoms between rings, the spiro heterocyclyl is divided into monospiroheterocyclyl, bispiroheterocyclyl or polyspiroheterocyclyl, preferably monospiroheterocyclyl and bispiroheterocyclyl. More preferably, it is 3-membered/5-membered, 3-membered/6-membered, 4-membered/4-membered, 4-membered/5-membered, 4-membered/6-membered, 5-membered/5-membered, or 5-membered/6-membered monospiro heterocyclyl. Non-limiting examples of spiro heterocyclyl include: 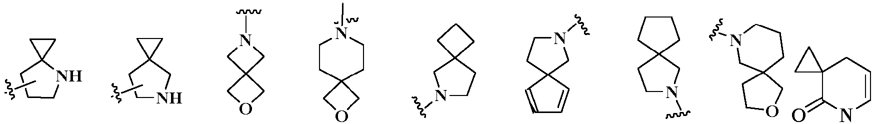 etc.

The term "fused heterocyclyl" refers to a 5- to 20-membered polycyclic heterocyclic group with each ring in the system sharing a pair of atoms neighboring other rings in the system, and one or more rings may contain one or more double bonds, but no ring has a fully conjugated π electron system, wherein one or more ring atoms are heteroatoms selected from nitrogen, oxygen, or S(O)ₘ (wherein m is an integer of 0 to 2), and the remaining ring atoms are carbon. Preferably, it is 6- to 14-membered and more preferably 8- to 12-membered. According to the number of constituent rings, it can be divided into bicyclic, tricyclic, tetracyclic or polycyclic fused heterocyclyl, preferably bicyclic or tricyclic, more preferably 4-membered/5-membered, 5-membered/5-membered, 5-membered/6-membered, 6-membered/6-membered, and 6-membered/7-membered bicyclic fused heterocyclyl. Non-limiting examples of fused heterocyclyl include: 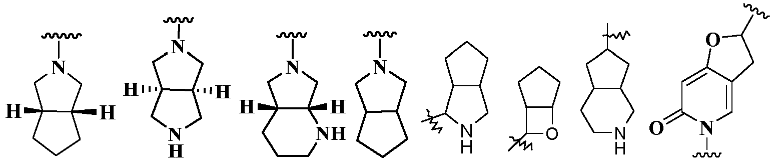 etc.

The term "bridged heterocyclyl" refers to a 5- to 14-membered polycyclic heterocyclic group with any two rings sharing two atoms that are not directly connected. It may contain one or more double bonds, but no ring has a fully conjugated π electron system, wherein one or more ring atoms are heteroatoms selected from nitrogen, oxygen, or S(O)ₘ (wherein m is an integer of 0 to 2), and the remaining ring atoms are carbon. Preferably, it is 6- to 14-membered and more preferably 7- to 10-membered. According to the number of constituent rings, it can be divided into bicyclic, tricyclic, tetracyclic or polycyclic bridged heterocyclyl, preferably bicyclic, tricyclic or tetracyclic, and more preferably bicyclic or tricyclic. Non-limiting examples of bridged heterocyclyl include: 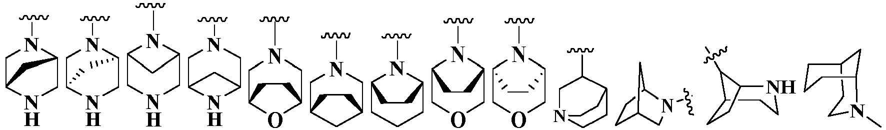 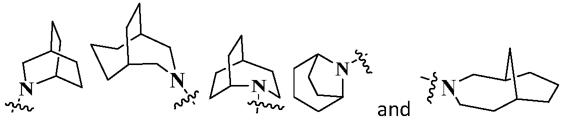 etc.

The heterocyclyl ring can be fused to an aryl, heteroaryl or cycloalkyl ring, wherein the ring connected to the parent structure is heterocyclyl, and the non-limiting examples thereof include: 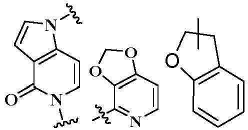 etc.

Heterocyclyl may be optionally substituted or unsubstituted, and when it is substituted, the substituent is preferably one or more groups independently selected from alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio, heterocycloalkylthio, oxo, carboxyl, or carboxylate group.

The term "heterocyclylalkylene" refers to heterocyclyl connected to alkylene to form "heterocyclyl-alkylene-", wherein the heterocyclyl or alkyl may be optionally substituted or unsubstituted, and the heterocyclyl and alkylene are as defined above.

The term "heterocyclylene" refers to bivalent heterocyclyl formed by further replacing one hydrogen atom of cycloalkyl, wherein the heterocyclylene may be optionally substituted or unsubstituted, and the heterocyclyl is defined as above.

The term "aryl" refers to a 6- to 14-membered all-carbon monocyclic or fused polycyclic (i.e., rings sharing an adjacent pair of carbon atoms) group having a conjugated π electron system, preferably 6- to 10-membered aryl, such as phenyl and naphthyl. Phenyl is more preferred. An aryl ring can be fused to heteroaryl, heterocyclyl or cycloalkyl ring, including benzo 5- to 10-membered heteroaryl, benzo 3- to 8-membered cycloalkyl and benzo 3- to 8-membered heteroalkyl, preferably benzo 5- to 6-membered heteroaryl, benzo 3- to 6-membered cycloalkyl and benzo 3- to 6-membered heteroalkyl, wherein the heterocyclyl is heterocyclyl containing 1-3 nitrogen atoms, oxygen atoms, or sulfur atoms; or further including a three-membered nitrogen-containing fused ring containing a benzene ring,
wherein the ring connected to the parent structure is an aryl ring, and the non-limiting examples thereof include: 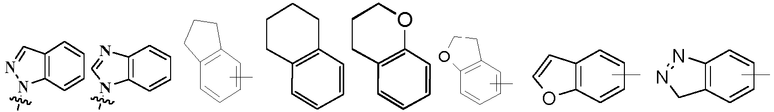 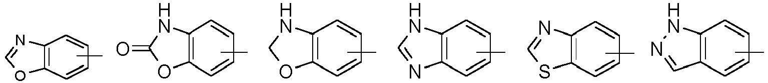 etc.

Aryl may be substituted or unsubstituted, and when it is substituted, the substituent is preferably one or more groups independently selected from alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio, heterocycloalkylthio, carboxyl, or carboxylate group.

The term "arylene" refers to bivalent aryl formed by further replacing one hydrogen atom of cycloalkyl, wherein the arylene is optionally substituted or unsubstituted, and the aryl is defined as above.

The term "heteroaryl" refers to a heteroaromatic system containing 1 to 4 heteroatoms and 5 to 14 ring atoms, where the heteroatoms are selected from oxygen, sulfur and nitrogen. The heteroaryl is preferably 5- to 6-membered monocyclic heteroaryl, or 8- to 12-membered bicyclic heteroaryl, e.g., imidazolyl, furyl, thienyl, thiazolyl, pyrazolyl, oxazolyl, pyrrolyl, triazolyl, tetrazolyl, pyridinyl, pyrimidinyl, thiadiazolyl, pyrazinyl, etc., preferably pyridinyl, oxadiazolyl, triazolyl, thienyl, imidazolyl, pyrazolyl, oxazolyl, thiazolyl, pyrimidinyl, or thiazolyl; and tetrazolyl, pyridinyl, oxadiazolyl, pyrazolyl, pyrrolyl, thiazolyl, and oxazolyl are more preferred.

Non-limiting examples of bicyclic heteroaryl include:

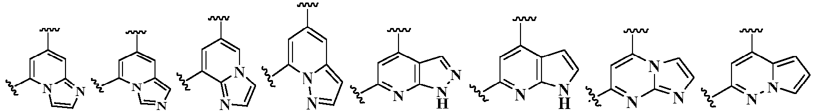

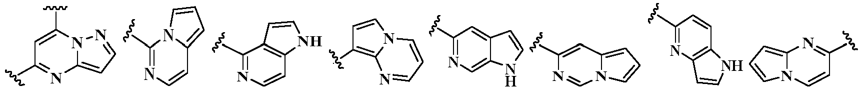

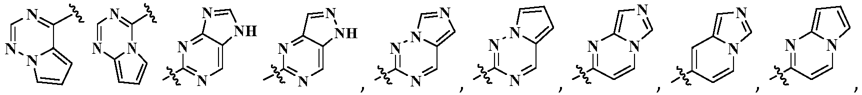

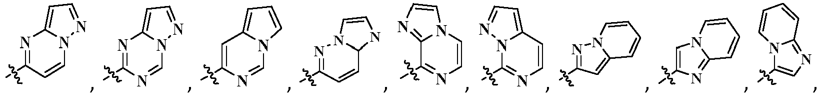

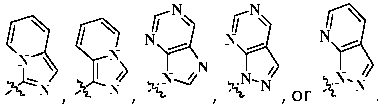

The heteroaryl ring can be fused to an aryl, heterocyclyl or cycloalkyl ring, wherein the ring connected to the parent structure is heteroaryl ring, and the non-limiting examples thereof include: 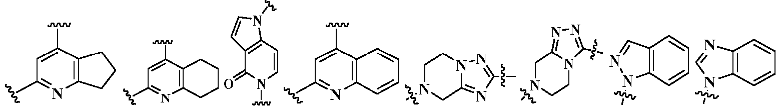 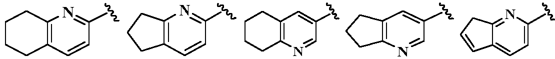 etc.

Heteroaryl may be optionally substituted or unsubstituted, and when it is substituted, the substituent is preferably one or more groups independently selected from alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio, heterocycloalkylthio, carboxyl or carboxylate group.

The term "heteroarylene" refers to bivalent heteroaryl formed by further replacing one hydrogen atom of cycloalkyl, wherein the heteroarylene is optionally substituted or unsubstituted, and the heteroaryl is defined as above.

The term "alkoxy" refers to -O-(alkyl) and -O-(unsubstituted cycloalkyl), where the alkyl is as defined above. Non-limiting examples of alkoxy include: methoxy, ethoxy, propoxy, butoxy, cyclopropyloxy, cyclobutoxy, cyclopentyloxy, and cyclohexyloxy. Alkoxy may be optionally substituted or unsubstituted, and when it is substituted, the substituent is preferably one or more groups independently selected from alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio, heterocycloalkylthio, carboxyl, or carboxylate group.

"Haloalkyl" refers to alkyl substituted with one or more halogens, wherein the alkyl is as defined above. Non-limiting examples of haloalkyl include: trifluoromethyl, -CH₂CF₃, 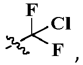 or

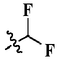

"Haloalkoxy" refers to alkoxy substituted with one or more halogens, wherein the alkoxy is as defined above.

"Hydroxyalkyl" refers to alkyl substituted with hydroxy, wherein the alkyl is as defined above.

The term "alkenylcarbonyl" refers to -C(O)-(alkenyl), wherein the alkenyl is as defined above. Non-limiting examples of alkenylcarbonyl include: ethenylcarbonyl, propenylcarbonyl, and butenylcarbonyl. Alkenylcarbonyl can be optionally substituted or unsubstituted. When it is substituted, the substituent is preferably one or more groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl, or a carboxylate group.

"Aminocarbonyl" refers to NH₂-C(O)-.

"Alkylaminocarbonyl" means that one or both of two hydrogens on aminocarbonyl (NH₂-C(O)-) are substituted by alkyl, wherein the alkyl is as described above.

"Alkylamino" means that one or both of two hydrogens on the amino are substituted by alkyl, wherein the alkyl is as described above.

"Alkylcarbonyl" or "acyl" refers to (alkyl)-C(O)-, wherein the alkyl is as described above.

"Hydroxyl" refers to a -OH group.

"Halogen" refers to fluorine, chlorine, bromine, or iodine.

"Amino" refers to -NH₂.

"Cyano" refers to -CN.

"Nitro" refers to -NO₂.

"Carbonyl" refers to -C(O)-.

"Carboxyl" refers to -C(O)OH.

"THF" refers to tetrahydrofuran.

"Ethyl acetate" refers to ethyl acetate.

"MeOH" refers to methanol.

"DMF" refers to N,N-dimethylformamide.

"DIPEA" refers to diisopropylethylamine.

"TFA" refers to trifluoroacetic acid.

"TEA" refers to triethylamine.

"MeCN" refers to acetonitrile.

"DMA" refers to N,N-dimethylacetamide.

"EtzO" refers to diethyl ether.

"DCM" refers to dichloromethane.

"DMAP" refers to 4-dimethylaminopyridine.

"DCC" refers to dicyclohexylcarbodiimide.

"DCE" refers to 1,2-dichloroethane.

"DIPEA" refers to N,N-diisopropylethylamine.

"NBS" refers to N-bromosuccinimide.

"NIS" refers to N-iodosuccinimide.

"Cbz-CI" refers to benzyl chloroformate.

"Pd₂(dba)₃" refers to tris(dibenzylideneacetone)dipalladium.

"Dppf" refers to 1,1'-bisdiphenylphosphine ferrocene.

"HATU" refers to 2-(7-oxidobenzotriazole)-N,N,N',N'-tetramethyluronium hexafluorophosphate.

"KHMDS" refers to potassium hexamethyldisilazide.

"LiHMDS" refers to lithium bis(trimethylsilyl)amide.

"MeLi" refers to methyl lithium.

"n-BuLi" refers to n-butyl lithium.

"NaBH(OAc)₃" refers to sodium triacetoxyborohydride.

Different expressions such as "X is selected from A, B, or C", "X is selected from A, B, and C", "X is A, B, or C", and "X is A, B, and C" all express the same meaning, i.e., X can be any one or more of A, B, and C.

The hydrogen atoms described in the present invention can be replaced by its isotope deuterium, and any hydrogen atom in the example compounds involved in the present invention can also be replaced by deuterium atom.

"Optional" or "optionally" means that the event or circumstance subsequently described may but need not to occur, and the description includes the occasions where the events or circumstances occur or do not occur. For example, "heterocyclic group optionally substituted with alkyl" means the alkyl may but need not be present, the description includes the case where the heterocyclic group is substituted with alkyl and the case where the heterocyclic group is not substituted with alkyl.

"Substituted" refers to one or more hydrogen atoms in the group, preferably at most 5, more preferably 1-3 hydrogen atoms each independently substituted with a corresponding number of substituents. It goes without saying, the substituents may be only in their possible chemical positions, a person skilled in the art can determine the possible or impossible substitutions (by experiment or theory) without paying too much effort. For example, amino having a free hydrogen or a hydroxy group may be unstable when combined the carbon atoms having an unsaturated (e.g., olefinic) bond.

"Pharmaceutical composition" denotes a mixture containing one or more of the compounds as described herein or physiologically/pharmaceutically acceptable salts or prodrug thereof and other chemical components, as well as other components, such as a physiologically/pharmaceutically acceptable carrier and an excipient. The purpose of pharmaceutical compositions is to facilitate administration to living organisms and facilitate the absorption of active ingredients to exert biological activity.

"Pharmaceutically acceptable salt" refers to a salt of the compound of the present invention, which are safe and effective when used in mammals, and have appropriate biological activity.

### Detailed Description of Embodiments

The present invention will be further described below with reference to examples, but these examples do not limit the scope of the present invention.

### Example

The structure of the compound of the present invention was determined by nuclear magnetic resonance (NMR) or/and liquid chromatography-mass spectrometry (LC-MS). NMR chemical shift (δ) was given in parts per million (ppm) unit. NMR was determined using Bruker AVANCE-400 nuclear magnetic instrument. The solvents for determination were deuterated dimethyl sulfoxide (DMSO-d6), deuterated methanol (CD₃OD), deuterated chloroform (CDCl₃), or deuterium water (D₂O), and the internal standard was tetramethylsilane (TMS).

Agilent 1200 Infinity Series mass spectrometer was used for Liquid chromatography-mass spectrometry LC-MS determination. HPLC determination used Agilent 1200DAD high-pressure liquid chromatograph instrument (Sunfire C18 150 × 4.6 mm chromatographic column) and Waters 2695-2996 high-pressure liquid chromatograph instrument (Gimini C18 150 × 4.6 mm chromatographic column).

Yantai Huanghai HSGF254 or Qingdao GF254 silica gel plate was used as a thin layer chromatography silica plate, and the specification used for the TLC was 0.15-0.20 mm, and the specification when separating and purifying a product by thin layer chromatography is 0.4-0.5 mm. For the column chromatography, Yantai Huanghai silica gel of 200-300 mesh silica gel was generally used as a carrier.

The starting materials in the examples of the present invention were known and can be purchased on the market, or can be synthesized using or according to methods known in the art.

Unless otherwise specified, all reactions of the present invention were carried out under continuous magnetic stirring in a dry nitrogen or argon atmosphere, the solvent was a dry solvent, and the reaction temperature unit was degrees Celsius.

Eluent systems for silica gel column chromatography and developer systems for thin layer chromatography, which were used for the purification of compounds in the intermediates and examples, included: A: dichloromethane and methanol system, B: n-hexane and ethyl acetate system, and C: dichloromethane and acetone system, wherein the volume ratio between the solvents could be adjusted depending on the polarity of the compound, or could also be adjusted by adding a small amount of basic or acidic reagents such as triethylamine and acetic acid.

Unless otherwise specified, in the examples of the present invention, ratios in mobile phases in HPLC chiral resolution conditions and HPLC chiral analysis conditions were volume ratios.

### Intermediate 1

### 2',3-Dichloro-4-((3,5-difluoropyridin-2-yl)methoxy)-5',6-dimethyl-2H-[1,4'-bipyridin]-2-one

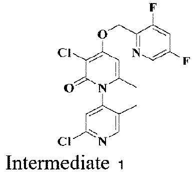

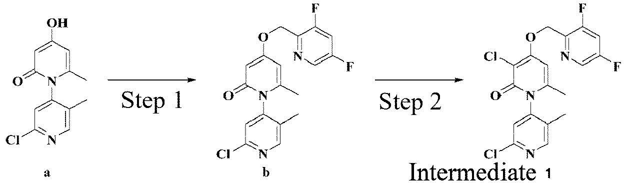

### Step 1

2'-Chloro-4-hydroxy-5',6-dimethyl-2*H*-[1,4'-bipyridin]-2-one a (200 mg, 0.8 mmol, referring to WO 2014197846 A1 for the synthesis method therefor), 2-(chloromethyl)-3,5-difluoro-pyridine (157 mg, 0.96 mmol), potassium carbonate (276 mg, 1.99 mmol), and 18-crown-6 (21 mg, 0.08 mmol) were dissolved in *N,N*-dimethylformamide (4 mL). The reaction was heated to 60°C and stirred for 16 hours. Water (20 mL) was added to the reaction liquid, the aqueous phase was extracted with ethyl acetate (20 mL × 2), and the organic phases were combined, dried, and concentrated. The residue was subjected to separation by silica gel column chromatography (eluent system A) to obtain the product 2'-chloro-4-((3,5-difluoropyridin-2-yl)methoxy)-5',6-dimethyl-2*H*-[1,4'-bipyridin]-2-one **b** (301 mg), yield: 99%.

MS m/z (ESI): 378.1 [M+H]⁺.

### Step 2

2'-Chloro-4-((3,5-difluoropyridin-2-yl)methoxy)-5',6-dimethyl-2*H*-[1,4'-bipyridin]-2-one b (301 mg, 0.8 mmol) and *N*-chlorosuccinimide (117 mg, 0.88 mmol) were dissolved in isopropanol (6 mL), and the reaction was heated to 60°C and stirred for 16 hours. After cooling to room temperature, the reaction liquid was concentrated, and the residue was subjected to separation by silica gel column chromatography (eluent system A) to obtain the product 2',3-dichloro-4-((3,5-difluoropyridin-2-yl)methoxy)-5',6-dimethyl-2*H*-[1,4'-bipyridin]-2-one intermediate 1 (190 mg), yield: 57.9%.

MS m/z (ESI): 412.1 [M+H]⁺.

¹H NMR (400 MHz, DMSO-*d*₆) *δ* 8.60 (s, 1H), 8.54 (s, 1H), 8.07-8.13 (m, 1H), 7.69 (s, 1H), 6.80 (s, 1H), 5.48 (s, 2H), 1.98 (s, 3H), 1.96 (s, 3H).

### Intermediate 2

### 2-(1H-pyrazol-3-yl)propan-2-ol

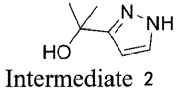

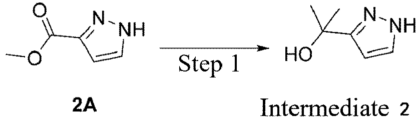

### Step 1

Methyl 1H-pyrazole-3-carboxylate (2 g, 15.9 mmol) was dissolved in tetrahydrofuran (50 mL) and cooled to 0°C. Methyl magnesium chloride (1 M, 47.58 mL) was dropwise added to the reaction liquid with stirring. After the dropwise addition was completed, the reaction was heated to room temperature and stirred for 2 hours. Ethyl acetate (100 mL) was added to the reaction liquid, and the organic phase was washed with water and saturated ammonium chloride, dried, and concentrated to obtain the product 2-(1H-pyrazol-3-yl)propan-2-ol **intermediate 2** (840 mg), yield: 42%.

MS m/z (ESI): 127.1 [M+H]⁺.

¹H NMR (400 MHz, CDCl₃) δ 7.51 (d, 1H), 6.64 (s, 2H), 6.18 (d, 1H), 1.62 (s, 6H).

### Intermediate 3

### 2',3-Dichloro-4-hydroxy-5',6-dimethyl-2H-[1,4'-bipyridin]-2-one

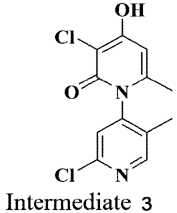

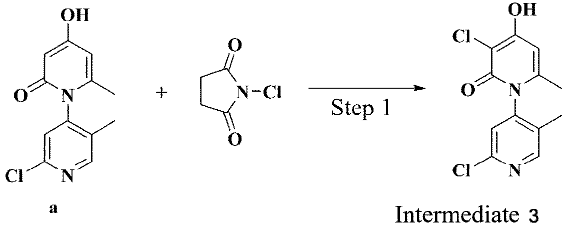

### Step 1

a (10 g, 40 mmol) was dissolved in a mixed solution of dichloroethane (600 mL) and isopropanol (400 mL). The reaction liquid was heated to 60°C, N-chlorosuccinamide (6.4 g, 50 mmol) was added to the reaction liquid in portions, and the reaction continued to be stirred at 60°C for 2 hours. The reaction liquid was concentrated, isopropanol (50 mL) was added to the residue, and the mixed solution was stirred at 60°C for 1 hour. The mixed solution was cooled and filtered, and the filter cake was washed with isopropanol. The filter cake was dried to obtain the product 2',3-dichloro-4-hydroxy-5',6-dimethyl-2H-[1,4'-bipyridin]-2-one **intermediate 3** (8.0 g), yield: 71%.

MS m/z (ESI): 285.0 [M+H] ⁺;
¹H NMR (400 MHz, DMSO-*d*₆) δ 11.61 (s, 1H), 8.52 (s, 1H), 7.64 (s, 1H), 6.17 (d, 1H), 1.97 (s, 3H), 1.86 (s, 3H).

### Intermediate 4

### 1-(Chloromethyl-d2)-2,4-difluorobenzene

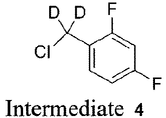

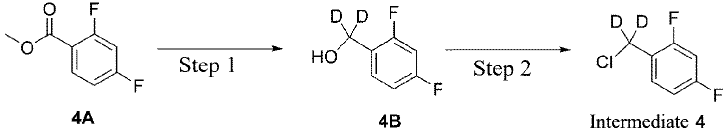

### Step 1

Methyl 2,4-difluorobenzoate **4A** (0.5 g, 2.90 mmol) was dissolved in tetrahydrofuran (8 mL) and stirred. A solution of lithium aluminum deuteride (122 mg, 2.90 mmol) in tetrahydrofuran (5 mL) was added to the reaction liquid in an ice bath. The reaction was stirred in the ice bath for 1 hour, the reaction liquid was quenched with saturated ammonium chloride, the aqueous phase was extracted with ethyl acetate (20 mL × 3), the organic phases were combined, washed with saturated sodium chloride, dried, and concentrated, and the residue was subjected to silica gel column chromatography (elution system B) to obtain (2,4-difluorophenyl)methan-*d*₂-ol **4B** (0.4 g), yield: 94%.

MS m/z (ESI): 147.1 [M+H]⁺.

### Step 2

(2,4-Difluorophenyl)methan-*d*₂-ol **4B** (400 mg, 2.74 mmol) and *N,N-*dimethylformamide (0.05 mL) were dissolved in dichloromethane (10 mL) and stirred, and sulfoxide chloride (749 mg, 6.30 mmol) was slowly dropwise added to the reaction liquid in an ice bath. The reaction was transferred to room temperature and stirred for 1 hour. The reaction liquid was concentrated, the residue was diluted with a saturated sodium bicarbonate solution, the aqueous phase was extracted with dichloromethane (20 mL × 3), and the organic phases were combined, washed with saturated sodium chloride, dried, and concentrated to obtain 1-(chloromethyl-d2)-2,4-difluorobenzene **intermediate 4** (0.42 g).

### Intermediate 5

### (2-(2-Hydroxypropan-2-yl)thiazol-4-yl)boronic acid

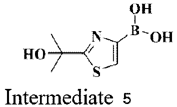

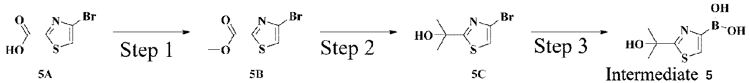

### Step 1

4-Bromothiazol-2-carboxylic acid **5A** (1 g, 4.81 mmol) was dissolved in methanol (10 mL), and thionyl chloride (1.72 g, 14.42 mmol) was slowly dropwise added to the reaction liquid at room temperature. After the dropwise addition was completed, the reaction was heated to 50°C and stirred for 3.5 hours. The reaction was returned to room temperature and directly concentrated, and the residue was subjected to separation by silica gel column chromatography (eluent system A) to obtain the product methyl 4-bromothiazol-2-carboxylate **5B** (1.02 g), yield: 95. 6%.

MS m/z (ESI): 222.1, 224.1 [M+H] ⁺.

### Step 2

Methyl 4-bromothiazol-2-carboxylate **5B** (970 mg, 4.37 mmol) was dissolved in tetrahydrofuran (30 mL) and cooled to 0°C. Magnesium methyl bromide (3 M, 4.37 mL) was dropwise added to the reaction liquid, and the mixture was returned to room temperature and continued to be stirred for 5 hours. A saturated ammonium chloride (15 mL) solution was added to the reaction liquid, which was extracted with ethyl acetate (15 mL × 2), the organic phases were combined, dried, and concentrated, and the residue was subjected to separation by silica gel column chromatography (eluent system A) to obtain the product 2-(4-bromothiazol-2-yl)propan-2-ol **5C** (805 mg,), yield: 83.0%.

MS m/z (ESI): 222.1, 224.1 [M+H]⁺.

### Step 3

2-(4-Bromothiazol-2-yl)propan-2-ol **5C** (100 mg, 0.45 mmol), bis(pinacolato)diboron (137.2 mg, 0.54 mmol), tris(dibenzylideneacetone)dipalladium (20.6 mg, 0.02 mmol), 2-dicyclohexylphosphine-2',4',6'-triisopropylbiphenyl (25.8 mg, 0.05 mmol), and potassium acetate (88.4 mg, 0.9 mmol) were dissolved in anhydrous 1,4-dioxane (9 mL), and after displacement with nitrogen for 1 minute, the reaction was heated to 110°C and stirred for 2 hours. After cooling to room temperature, the reaction liquid was filtered, the filtrate was concentrated, and the residue was subjected to reversed-phase HPLC (formic acid system) to prepare the title product (2-(2-hydroxypropan-2-yl)thiazol-4-yl)boronic acid **intermediate 5** (65 mg), yield: 77.2%.

MS m/z (ESI): 188.1 [M+H]⁺.

### Intermediate 6

### 2-(Chloromethyl-d2)-3,5-difluoropyridine

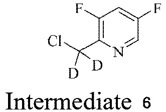

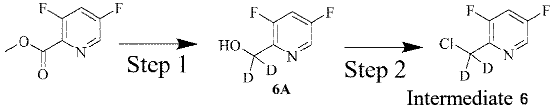

### Step 1

Lithium aluminum deuteride (388 mg, 9.24 mmol) was added to a solution of methyl 3,5-difluoropyridine-2-carboxylate (1.00 g, 5.78 mmol) in tetrahydrofuran (20 mL) in portions in an ice bath. The reaction was stirred at room temperature for 2 hours. The reaction was quenched with ice cubes, and the reaction liquid was filtered. The filtrate was concentrated, and the residue was subjected to separation by silica gel column chromatography (eluent system B) to obtain the product (3,5-difluoropyridin-2-yl)methan-d2-ol **6A** (345 mg), yield: 40.6%.

MS m/z (ESI): 148.0 [M+H] ⁺.

### Step 2

With stirring, thionyl dichloride (839 mg, 7.06 mmol) was dropwise added to a solution of (3,5-difluoropyridin-2-yl)methan-d2-ol **6A** (346 mg, 2.35 mmol) in dichloromethane (5 mL) and N,N-dimethylformamide (0.1 mL). The reaction was stirred at room temperature for 3 hours. The reaction liquid was diluted with ethyl acetate (160 mL), and the organic phase was washed with saturated sodium bicarbonate and water. The organic phase was concentrated, and the residue was subjected to separation by silica gel column chromatography (eluent system B) to obtain the product 2-(chloromethyl-d2)-3,5-difluoropyridine **intermediate 6** (150 mg), yield: 38.5%.

MS m/z (ESI): 166.0 [M+H] ⁺.

### Intermediate 7

### 1-(2-Bromo-5-methyl-4-pyridinyl)-3-chloro-4-hydroxy-6-methyl-pyridin-2-one

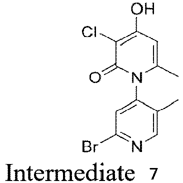

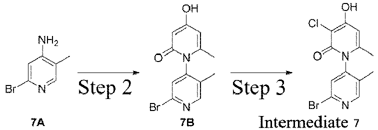

### Step 1

2-Bromo-5-methyl-pyridin-4-amine **7A** (2.03 g, 10.86 mmol) and 2,2-dimethyl-6-(2-carbonylpropyl)-4H-1,3-dioxin-4-one (2 g, 10.86 mmol) was dissolved in 1,4-dioxane (80 mL) and stirred. The reaction was placed in a reactor preheated to 90°C and stirred for 3 hours. Concentrated sulfuric acid (1.8 g, 18.35 mmol) was added to the reaction liquid, and stirring was continued for 1 hour. The reaction liquid was cooled, the supernatant was removed, and water was added to the residual oil. The mixture was stirred at room temperature for 30 minutes and filtered to obtain 2'-bromo-4-hydroxy-5',6-dimethyl-2H-[1,4'-bipyridin]-2-one **7B** (1.7 g), yield: 53%.

MS m/z (ESI): 296.1 [M+H] ⁺.

### Step 3

2'-Bromo-4-hydroxy-5',6-dimethyl-2H-[1,4'-bipyridin]-2-one **7B** (1.7 g, 5.76 mmol) was dissolved in isopropanol (40 mL) and 1,2-dichloroethane (60 mL), heated to 60°C, and stirred. N-Chlorosuccinamide (923 mg, 6.91 mmol) was added to the reaction liquid in portions, and the mixture was stirred for 2 hours. The reaction liquid was concentrated, isopropanol (20 mL) was added to the residue, and the mixture was heated to 60°C and stirred for 0.5 hours. The reaction liquid was cooled and filtered to obtain 1-(2-bromo-5-methyl-4-pyridinyl)-3-chloro-4-hydroxy-6-methyl-pyridin-2-one **intermediate 7** (1.71 g), yield: 90.1%.

MS m/z (ESI): 330.9 [M+H] ⁺.

### Intermediate 8

### 2'-Bromo-3-chloro-4-((3,5-difluoropyridin-2-yl)methoxy-d2)-5',6-dimethyl-2H-[1,4'-bipyridin]-2-one

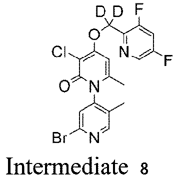

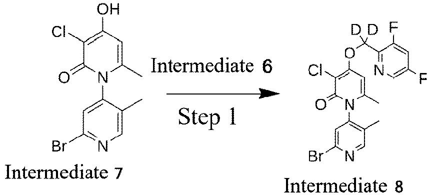

### Step 1

1-(2-Bromo-5-methyl-4-pyridinyl)-3-chloro-4-hydroxy-6-methyl-pyridin-2-one **intermediate 7**(550 g, 1.67 mmol), **intermediate 6** (387 mg, 2.34 mmol), potassium carbonate (577 mg, 4.17 mmol), and 18-crown-6 ether (662 mg, 2.50 mmol) were dissolved in *N,N-*dimethylformamide (25 mL), heated to 70°C, and stirred for 3 hours. Ethyl acetate (50 mL) was added to the reaction liquid, and the organic phase was washed with water and saturated sodium chloride, dried, and concentrated. The residue was purified by silica gel column (elution system A) to obtain 2'-bromo-3-chloro-4-((3,5-difluoropyridin-2-yl)methoxy-d2)-5',6-dimethyl-2H-[1,4'-bipyridin]-2-one **intermediate 8** (540 mg), yield: 70.6%.

MS m/z (ESI): 458.0 [M+H] ⁺.

### Intermediate 9

### N-(2-(4-fluoro-1H-pyrazol-3-yl)propan-2-yl)acetamide

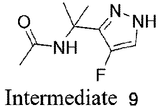

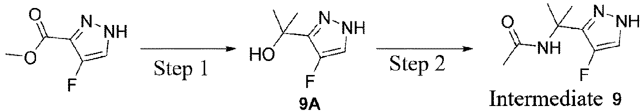

### Step 1

In an ice bath, methyl 4-fluoro-1*H*-pyrazol-3-carboxylate (600 mg, 4.16 mmol) was dissolved in anhydrous tetrahydrofuran (20 mL), a solution of methyl magnesium bromide (3 M, 6.9 mL, 20.7 mmol) in tetrahydrofuran was dropwise added to the reaction liquid while stirring, and the reaction was stirred at room temperature for 3 hours. The reaction liquid was poured into a saturated ammonium chloride solution (50 mL), and the aqueous phase was extracted with ethyl acetate (50 mL × 3). The organic phases were combined, washed with water (50 mL) and a saturated sodium chloride solution (50 mL) in sequence, dried, and concentrated to obtain 2-(4-fluoro-1*H*-pyrazol-3-yl)propan-2-ol **9A** (600 mg), and the product was directly used in the next reaction without purification.

MS m/z (ESI): 145.1 [M+H]⁺.

### Step 2

**9A** (600 mg, 4.17 mmol) was dissolved in acetonitrile (15 mL) and stirred, concentrated sulfuric acid (5 mL) was dropwise added to the reaction liquid at 0°C, and the reaction was heated to room temperature and stirred for 3 hours. The reaction liquid was poured into a saturated sodium bicarbonate solution (100 mL), and the aqueous phase was extracted with ethyl acetate (50 mL × 3). The organic phases were combined, washed with water (50 mL) and a saturated sodium chloride solution (50 mL) in sequence, dried, and concentrated, and the residue was purified by silica gel column chromatography (elution system B) to obtain *N*-(2-(4-fluoro-1*H*-pyrazol-3-yl)propan-2-yl)acetamide **intermediate 9** (510 mg), yield: 86.8 %.

MS m/z (ESI): 186.1 [M+H]⁺.

### Intermediate 10

### 2'-Bromo-3-chloro-4-((2,4-difluorophenyl)methoxy-d2)-5',6-dimethyl-2H-[1,4'-bipyridin]-2-one

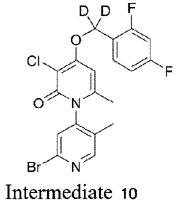

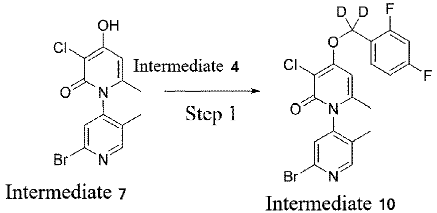

### Step 1

Under nitrogen protection, **intermediate 7** (400 mg, 1.21 mmol), potassium carbonate (503 mg, 3.64 mmol), and 18-crown-6 (481 mg, 1.82 mmol) were dissolved in anhydrous *N,N-*dimethylformamide (6 mL), heated to 75°C, and stirred, and **intermediate 4** (300 mg, 1.82 mmol) was dropwise added to the reaction liquid. The reaction was stirred at 75°C for 1 hour. Water (50 mL) was added to the reaction liquid, and the aqueous phase was extracted with ethyl acetate (50 mL × 2). The organic phases were combined, washed with water (50 mL) and a saturated sodium chloride solution (50 mL) in sequence, and dried, the filtrate was concentrated, and the residue was subjected to separation by silica gel column chromatography (elution system B) to obtain 2'-bromo-3-chloro-4-((2,4-difluorophenyl)methoxy-*d*2)-5',6-dimethyl-2*H*-[1,4'-bipyridin]-2-one **intermediate 10** (480 mg), yield: 86.4%.

MS m/z (ESI): 457.0 [M+H]⁺.

### Reference Example 1

### 3-Chloro-6-cyclopropyl-4-((3,5-difluoropyridin-2-yl)methoxy)-2'-(2-(2-hydroxypropan-2-yl)pyrimidin-4-yl)-5'-methyl-2H-[1,4'-bipyridin]-2-one

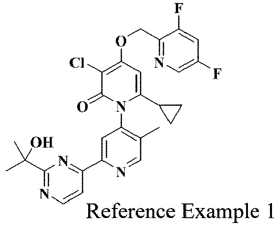

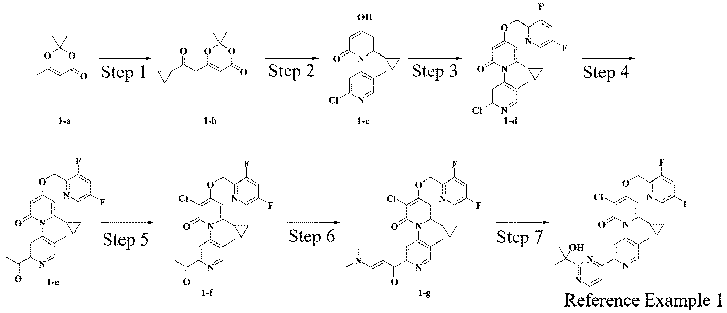

### Step 1

2,2,6-Trimethyl-4*H*-1,3-dioxin-4-one **1-a** (8.16 g, 57.40 mmol) was dissolved in tetrahydrofuran (60 mL) and cooled to -78°C. Lithium bis(trimethylsilyl)amide (1 M, 63 mL) was dropwise added to the reaction liquid, and after the dropwise addition was completed, the reaction was stirred at -78°C for 1 hour. A solution of cyclopropylformyl chloride (3 g, 28.70 mmol, 2.60 mL) in tetrahydrofuran (10 mL) was dropwise added to the reaction liquid, and the reaction was stirred at -78°C for 16 hours. In an ice bath, a saturated ammonium chloride solution (30 mL) was added to the reaction liquid to quench the reaction, and the pH was adjusted to weak acidity with diluted hydrochloric acid. The organic phase was separated, and the aqueous phase was extracted with ethyl acetate (30 mL × 2). The organic phases were combined, dried, and concentrated. The residue was subjected to separation by silica gel column chromatography (eluent system B) to obtain the product 6-(2-cyclopropyl-2-carbonylethyl)-2,2-dimethyl-4*H*-1,3-dioxin-4-one **1-b** (2.93 g), yield: 48%.

MS m/z (ESI): 211.1 [M+H] ⁺.

### Step 2

6-(2-Cyclopropyl-2-carbonylethyl)-2,2-dimethyl-4*H*-1,3-dioxin-4-one**1-b** (700 mg, 3.33 mmol) and 2-chloro-4-amino-5-methylpyridine (617 mg, 4.33 mmol) were dissolved in 1,4-dioxane (10 mL), and the reaction was heated to 90°C and stirred for 3.5 hours. Concentrated sulfuric acid (0.25 mL) was added to thre reaction liquid, and the reaction was stirred at 90°C for 1 hour. Water (10 mL) was added to the reaction liquid, and subjected to separation by preparative reversed-phase HPLC (formic acid system) to obtain the product 2'-chloro-6-cyclopropyl-4-hydroxy-5'-methyl-2*H*-[1,4'-bipyridin]-2-one **1-c** (318 mg), yield: 34.5%.

MS m/z (ESI): 277.1 [M+H]⁺.

### Step 3

2'-Chloro-6-cyclopropyl-4-hydroxy-5'-methyl-2*H*-[1,4'-bipyridin]-2-one **1-c** (318 mg, 1.15 mmol), 2-(chloromethyl)-3,5-difluoro-pyridine (226 mg, 1.38 mmol), potassium carbonate (397 mg, 2.87 mmol), and 18-crown-6 (30 mg, 0.11 mmol) were dissolved in *N,N-*dimethylformamide (5 mL). The reaction was heated to 60°C and stirred for 3 hours. Water (20 mL) was added to the reaction liquid, and the aqueous phase was extracted with ethyl acetate (20 mL × 2). The organic phases were combined, dried, and concentrated. The residue was subjected to separation by silica gel column chromatography (eluent system A) to obtain the product 2'-chloro-6-cyclopropyl-4-((3,5-difluoropyridin-2-yl)methoxy)-5'-methyl-2*H*-[1,4'-bipyridin]-2-one **1-d** (380 mg), yield: 81%.

MS m/z (ESI): 404.1 [M+H] ⁺.

### Step 4

2'-Chloro-6-cyclopropyl-4-((3,5-difluoropyridin-2-yl)methoxy)-5'-methyl-2*H*-[1,4'-bipyridin]-2-one **1-d** (150 mg, 0.37 mmol), tributyl (1-ethoxyvinyl)stannane (148 mg, 0.41 mmol), and bis(triphenylphosphine)palladium dichloride (26 mg, 0.04 mmol) were dissolved in 1,4-dioxane (3 mL) and displaced with nitrogen for 2 minutes. The reaction was heated to 130°C by microwave and stirred for 2 hours. The reaction liquid was filtered, and the filtrate was concentrated. The residue was dissolved with tetrahydrofuran (3 mL), and diluted hydrochloric acid (2 M, 1 mL) was dropwise added for a reaction until the raw materials were completely hydrolyzed. A saturated sodium bicarbonate solution was added to the reaction liquid to adjust the pH to weak alkalinity, and the aqueous phase was extracted with ethyl acetate (20 mL × 2). The organic phases were combined, dried, and concentrated, and the residue was subjected to separation by silica gel column chromatography (eluent system A) to obtain the product 2'-acetyl-6-cyclopropyl-4-((3,5-difluoropyridin-2-yl)methoxy)-5'-methyl-2*H*-[1,4'-bipyridin]-2-one **1-e** (90 mg), yield: 58.9%.

MS m/z (ESI): 412.1 [M+H] ⁺.

### Step 5

2'-Acetyl-6-cyclopropyl-4-((3,5-difluoropyridin-2-yl)methoxy)-5'-methyl-2H-[1,4'-bipyridin]-2-one **1-e** (90 mg, 0.22 mmol) and N-chlorosuccinimide (32 mg, 0.24 mmol) were dissolved in isopropanol (2 mL), and the reaction was heated to 60°C and stirred for 3 hours. The reaction liquid was concentrated, and the residue was subjected to separation by silica gel column chromatography (eluent system B) to obtain the product 2'-acetyl-3-chloro-6-cyclopropyl-4-((3,5-difluoropyridin-2-yl)methoxy)-5'-methyl-2*H*-[1,4'-bipyridin]-2-one **1-f** (90 mg), yield: 92%.

MS m/z (ESI): 446.1 [M+H] ⁺.

### Step 6

2'-Acetyl-3-chloro-6-cyclopropyl-4-((3,5-difluoropyridin-2-yl)methoxy)-5'-methyl-2*H-*[1,4'-bipyridin]-2-one **1-f** (90 mg, 0.2 mmol) and *N,N*-dimethylformamide dimethylacetal (72 mg, 0.61 mmol) were dissolved in *N,N*-dimethylformamide (2 mL), and the reaction was heated to 50°C and stirred for 16 hours. Water (10 mL) was added to the reaction liquid, the aqueous phase was extracted with ethyl acetate (15 mL × 2), the organic phases were combined, dried, and concentrated to obtain the crude product (*E*)-3-chloro-6-cyclopropyl-4-((3,5-difluoropyridin-2-yl)methoxy)-2'-(3-(dimethylamino)acryloyl)-5'-methyl-2*H*-[1,4'-bipyridin]-2-one **1-g** (80 mg), and the product was directly used in the next reaction without purification.

MS m/z (ESI): 501.1 [M+H] ⁺.

### Step 7

(*E*)-3-chloro-6-cyclopropyl-4-((3,5-difluoropyridin-2-yl)methoxy)-2'-(3-(dimethylamino)acryloyl)-5'-methyl-2*H*-[1,4'-bipyridin]-2-one **1-g** (80 mg, 0.16 mmol), 2-hydroxy-2-methylpropionamidine hydrochloride (33 mg, 0.24 mmol), and potassium carbonate (44 mg, 0.32 mmol) were dissolved in *N,N*-dimethylformamide (2 mL), and the reaction was heated to 75°C and stirred for 16 hours. After cooling to room temperature and filtration, the filtrate was subjected to separation by preparative reversed-phase HPLC (formic acid system) to obtain the title product 3-chloro-6-cyclopropyl-4-((3,5-difluoropyridin-2-yl)methoxy)-2'-(2-(2-hydroxypropan-2-yl)pyrimidin-4-yl)-5'-methyl-2*H*-[1,4'-bipyridin]-2-one **Reference Example 1** (55 mg), yield: 63.8%.

MS m/z (ESI): 540.1 [M+H] ⁺.

¹H NMR (400 MHz, DMSO-*d*₆) δ 8.96 (d, 1H), 8.85 (s, 1H), 8.75 (s, 1H), 8.62 (d, 1H), 8.24 (d, 1H), 8.15-8.05 (m, 1H), 6.47 (s, 1H), 5.53 (d, 2H), 5.24 (s, 1H), 2.13 (s, 3H), 1.53 (d, 6H), 1.36-1.23 (m, 1H), 1.02-0.93 (m, 1H), 0.92-0.81 (m, 1H), 0.75-0.63 (m, 2H).

### Reference Example 2

### 3-Chloro-4-((3,5-difluoropyridin-2-yl)methoxy)-2'-(2-(2-hydroxypropan-2-yl)thiazol-4-yl)-5',6-dimethyl-2H-[1,4'-bipyridin]-2-one

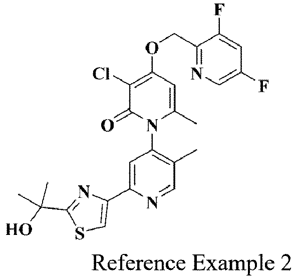

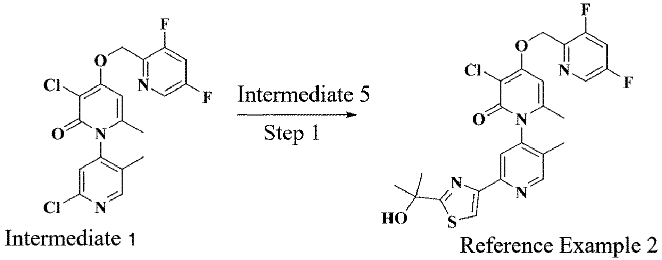

### Step 1

Under nitrogen protection, **intermediate 1** (50 mg, 0.12 mmol), **intermediate 5** (50.8 mg, 0.17 mmol, purity: 62%), 1,1-bis(diphenylphosphine) 1,1'-bisdiphenylphosphineferrocene palladium dichloride (8.9 mg, 0.01 mmol), and cesium carbonate (79 mg, 0.24 mmol) were dissolved in a mixed solvent of 1,4-dioxane (1 mL) and water (0.3 mL). The reaction was heated to 100°C and stirred for 3 hours. The reaction liquid was concentrated, and the residue was subjected to separation by silica gel column chromatography (eluent system B) to obtain the title product 3-chloro-4-((3,5-difluoropyridin-2-yl)methoxy)-2'-(2-(2-hydroxypropan-2-yl)thiazol-4-yl)-5',6-dimethyl-2H-[1,4'-bipyridin]-2-one **Reference Example 2** (32 mg), yield: 50.8%.

MS m/z (ESI): 519.1 [M+H] ⁺.

¹H NMR (400 MHz, DMSO-*d*₆) δ 8.70 (s, 1H), 8.61 (d, 1H), 8.18 (s, 1H), 8.15-8.05 (m, 1H), 7.86 (s, 1H), 6.80 (s, 1H), 6.06 (s, 1H), 5.48 (d, 2H), 2.03 (s, 3H), 1.97 (s, 3H), 1.55 (d, 6H).

### Example 1

### 11-Chloro-10-((3,5-difluoropyridin-2-yl)methoxy-d2)-2-(2-(2-hydroxypropan-2-yl)pyrimidin-4-yl)-5,6,7,8-tetrahydro-12H-dipyrido[1,2-a:3',4'-g]azol-12-one

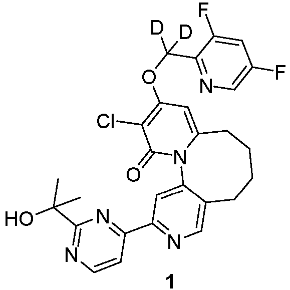

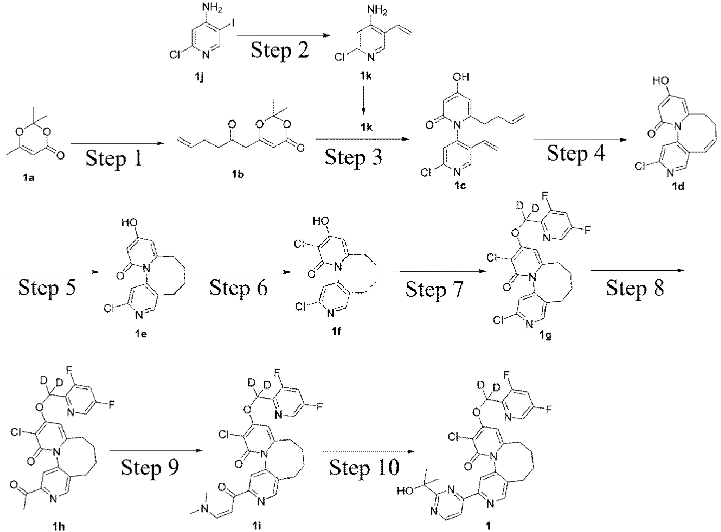

### Step 1

At -50°C, lithium bis(trimethylsilyl)amide (66.1 mL, 1 M in tetrahydrofuran) was dropwise added to a solution of 2,2,6-trimethyl-4H-1,3-dioxy-4-one **1a** (9.50 g, 66.8 mmol) in tetrahydrofuran (20 mL). After the reaction was stirred at -50°C for 1 hour, pent-4-enoyl chloride (4.40 g, 37.11 mmol) was dropwise added to the reaction liquid, and the stirring was continued for 1 hour. The reaction liquid was quenched with formic acid and filtered, the filtrate was concentrated, and the residue was subjected to separation by silica gel column chromatography (eluent system A) to obtain the title product 2,2-dimethyl-6-(2-oxohex-5-en-1-yl)-4H-1,3-dioxy-4-one **1b** (3.60 g), yield: 43.3%.

MS m/z (ESI): 225.1 [M+H]⁺.

### Step 2

Under nitrogen protection, **1j** (1.50 g, 5.89 mmol), tributyl vinyl tin (2.43 g, 7.66 mmol), and 1,1-bis(diphenylphosphine)ferrocene palladium dichloride (431 mg, 0.59 mmol) were dissolved in 1,4-dioxane (15 mL) and sealed in a microwave tube, and the reaction was heated to 100°C by microwave and stirred for 2 hours. The reaction liquid was concentrated, and the residue was subjected to separation by silica gel column chromatography (eluent system A) to obtain the title product 2-chloro-4-amino-5-vinylpyridine **1k** (460 mg), yield: 50.5%.

MS m/z (ESI): 155.0 [M+H] ⁺.

### Step 3

**1b** (6.70 g, 29.88 mmol) and **1k** (3.08 g, 19.92 mmol) were dissolved in 1,4-dioxane (80 mL), heated to 100°C, and stirred for 1.5 hours. Concentrated sulfuric acid (4.88 g, 49.80 mmol) was slowly dropwise added to the reaction liquid, and the reaction was stirred at 100°C for 2 hours. The reaction liquid was concentrated, methanol and water were added to the residue, which was filtered, and the filtrate was concentrated and subjected to separation by preparative reversed-phase HPLC (formic acid system) to obtain the title product 6-(but-3-en-1-yl)-2'-chloro-4-hydroxy-5'-vinyl-2H-[1,4'-bipyridin]-2-one **1c** (3.80 g), yield: 63.0%.

MS m/z (ESI): 303.1 [M+H] ⁺.

### Step 4

Under nitrogen protection, **1c** (334 mg, 1.11 mmol) and Grubbs second-generation catalyst (93 mg, 0.11 mmol) were dissolved in 1,2-dichloroethane (300 mL), and the reaction was heated to 60°C and stirred for 2 hours. Methanol (10 mL) was added to the reaction liquid and concentrated, and the residue was subjected to separation by silica gel column chromatography (eluent system A) to obtain the title product (Z)-2-chloro-10-hydroxy-7,8-dihydro-12H-dipyrido[1,2-a:3',4'-g]azol-12-one **1d** (110 mg), yield: 36.0%.

MS m/z (ESI): 275.0 [M+H] ⁺.

### Step 5

In a hydrogen atmosphere, **1d** (300 mg, 0.19 mmol) and platinum dioxide (25 mg, 0.19 mmol) were dissolved in tetrahydrofuran (20 mL), and the reaction was stirred at 25°C for 3 hours. The reaction liquid was filtered, the filtrate was concentrated, and the residue was subjected to separation by silica gel column chromatography (eluent system A) to obtain the title product 2-chloro-10-hydroxy-5,6,7,8-tetrahydro-12H-dipyrido[1,2-a:3',4'-g]azol-12-one **1e** (200 mg), yield: 66.2%.

MS m/z (ESI): 277.1 [M+H] ⁺.

### Step 6

**1e** (480 mg, 1.76 mmol) and N-chlorosuccinimide (347 mg, 2.61 mmol) were dissolved in isopropanol (20 mL), and the reaction was heated to 35°C and stirred for 1 hour. The reaction liquid was concentrated, and the residue was subjected to separation by silica gel column chromatography (eluent system A) to obtain the title product 2,11-dichloro-10-hydroxy-5,6,7,8-tetrahydro-12H-dipyrido[1,2-a:3',4'-g]azol-12-one **1f** (200 mg), yield: 64.3%.

MS m/z (ESI): 311.0 [M+H]⁺.

### Step 7

**1f** (168 mg, 0.54 mmol), potassium carbonate (149 mg, 1.08 mmol), and **intermediate 6** (119 mg, 0.81 mmol) were dissolved in N,N-dimethylformamide (5 mL), and the reaction was heated to 70°C and stirred for 16 hours. The reaction liquid was concentrated, and the residue was subjected to separation by preparative reversed-phase HPLC (formic acid system) to obtain the title product 2,11-dichloro-10-((3,5-difluoropyridin-2-yl)methoxy-d2)-5,6,7,8-tetrahydro-12H-dipyrido[1,2-a:3',4'-g]azol-12-one **1g** (172 mg), yield: 72.4%.

MS m/z (ESI): 40.1 [M+H] ⁺.

### Step 8

Under nitrogen protection, **1g** (150 mg, 0.34 mmol), tributyl(1-ethoxyvinyl)stannane (185 mg, 0.51 mmol) and bis(triphenylphosphine)palladium dichloride (24 mg, 0.03 mmol) were dissolved in a solution of 1,4-dioxane (5 mL), and the reaction was heated to 123°C by microwave and stirred for 2 hours. The reaction liquid was quenched with diluted hydrochloric acid (0.6 M, 0.5 mL), stirred at room temperature for 30 minutes, and concentrated, and the residue was subjected to separation by preparative reversed-phase HPLC (formic acid system) to obtain the title product 2-acetyl-11-chloro-10-((3,5-difluoropyridin-2-yl)methoxy-d2)-5,6,7,8-tetrahydro-12H-dipyrido[1,2-a:3',4'-g]azol-12-one **1h** (132 mg), yield: 86.5%.

MS m/z (ESI): 448.1 [M+H]⁺.

### Step 9

A solution of **1h** (160 mg, 0.36 mmol) in N,N-dimethylformamide (2 mL) and 1,1-dimethoxy-N,N-dimethylmethylamine (2 mL) was warmed to 70°C and stirred for 16 hours. The reaction liquid was concentrated, and the residue was subjected to separation by preparative reversed-phase HPLC (formic acid system) to obtain the title product (Z)-11-chloro-10-((3,5-difluoropyridin-2-yl)methoxy-d2)-2-(3-(dimethylamino)acryloyl)-5,6,7,8-tetrahydro-12H-dipyrido[1,2-a:3',4'-g]azol-12-one **1i** (85 mg), yield: 47.3%.

MS m/z (ESI): 503.2 [M+H] ⁺.

### Step 10

**1i** (85 mg, 0.17 mmol), 2-hydroxy-2-methylpropionamidine hydrochloride (70 mg, 0.51 mmol), and potassium carbonate (70 mg, 0.51 mmol) were dissolved in N,N-dimethylformamide (3 mL), and the reaction was heated to 75°C and stirred for 16 hours. The reaction liquid was cooled to room temperature and filtered, and the filtrate was subjected to separation by preparative reversed-phase HPLC (formic acid system) to obtain the title product 11-chloro-10-((3,5-difluoropyridin-2-yl)methoxy-d2)-2-(2-(2-hydroxypropan-2-yl)pyrimidin-4-yl)-5,6,7,8-tetrahydro-12H-dipyrido[1,2-a:3',4'-g]azol-12-one **1** (50 mg), yield: 54.6%.

MS m/z (ESI): 542.2 [M+H] +.

¹H NMR (400 MHz, CD₃OD) δ 8.90 (d, 1H), 8.86 (s, 1H), 8.54 (s, 1H), 8.46 (d, 1H), 8.29 (d, 1H), 7.73 (m, 1H), 6.84 (s, 1H), 2.90 (m, 2H), 2.26 (m, 3H), 2.04 (m, 1H), 1.62 (m, 8H).

### Resolution of Example 1

### (S)-11-Chloro-10-((3,5-difluoropyridin-2-yl)methoxy-d2)-2-(2-(2-hydroxypropan-2-yl)pyrimidin-4-yl)-5,6,7,8-tetrahydro-12H-dipyrido[1,2-a:3',4'-g]azol-12-one and (R)-11-chloro-10-((3,5-difluoropyridin-2-yl)methoxy-d2)-2-(2-(2-hydroxypropan-2-yl)pyrimidin-4-yl)-5,6,7,8-tetrahydro-12H-dipyrido[1,2-a:3',4'-g]azol-12-one

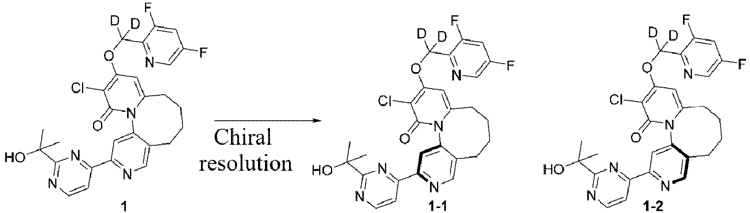

**Example 1** (50 mg, 0.091 mmol) was subjected to chiral resolution (OD column) to obtain **1-1** (20 mg, R.T = 3.807 min), yield: 40.0%; **1-2** (20 mg, R.T = 7.060 min), yield: 40.0%.

**Example 1-1:** MS m/z (ESI): 542.2 [M+H] ⁺;
¹H NMR (400 MHz, CD₃OD) δ 8.90 (d, 1H), 8.86 (s, 1H), 8.54 (s, 1H), 8.46 (d, 1H), 8.29 (d, 1H), 7.73 (m, 1H), 6.84 (s, 1H), 2.90 (m, 2H), 2.26 (m, 3H), 2.04 (m, 1H), 1.62 (m, 8H).

**Example 1-2:** MS m/z (ESI): 542.2 [M+H] ⁺;
¹H NMR (400 MHz, CD₃OD) δ 8.90 (d, 1H), 8.86 (s, 1H), 8.54 (s, 1H), 8.46 (d, 1H), 8.29 (d, 1H), 7.73 (m, 1H), 6.84 (s, 1H), 2.90 (m, 2H), 2.26 (m, 3H), 2.04 (m, 1H), 1.62 (m, 8H).

### HPLC chiral resolution conditions:

| | |
|---|---|
| Instrument | HPLC (Waters) |
| Column type | DAICEL CHIPAKPAK^{®}OD 250 * 20 mm 5.0 µm |
| Column pressure | 800 psi |
| Mobile phase | Hexane: MeOH (+0.1% DEA) = 70 : 30 |
| Flow rate | 15 mL/min |
| Detection wavelength | UV 214 nm |
| Column temperature | R.T. |

### HPLC chiral analysis conditions:

| | |
|---|---|
| Instrument | HPLC (Waters) |
| Column type | DAICEL CHIRALCEL OD-H 4.6 mm I.D × 150 mmL 5.0 µm |
| Column pressure | 45 psi |
| Mobile phase | Hexane : MeOH (+0.1% DEA) = 70 : 30 |
| Flow rate | 1 mL/min |
| Detection wavelength | UV 214 nm |
| Column temperature | 35°C |

### Example 2

### 11-Chloro-10-((3,5-difluoropyridin-2-yl)methoxy-d2)-2-(3-(2-hydroxypropan-2-yl)-1H-pyrazol-1-yl)-5,6,7,8-tetrahydro-12H-dipyrido[1,2-a:3',4'-g]azol-12-one

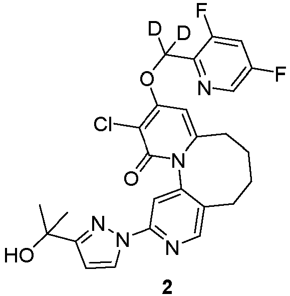

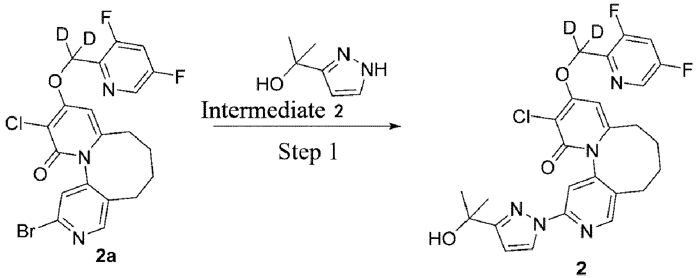

### Step 1

Under nitrogen protection, 2-bromo-11-chloro-10-((3,5-difluoropyridin-2-yl)methoxy-d2)-5,6,7,8-tetrahydro-12H-dipyrido[1,2-a:3',4'-g]azol-12-one **2a** (50 mg, 0.10 mmol; for the synthesis method therefor, reference could be made to the synthesis of **1g** in Example **1**), **intermediate 2** (20 mg, 0.15 mmol), cesium carbonate (50 mg, 0.15 mmol), and cuprous iodide (10 mg, 0.05 mmol) were dissolved in 1,4-dioxane (3 mL), and the reaction was heated to 100°C and stirred for 2 hours. The reaction liquid was concentrated, and the residue was subjected to separation by silica gel column chromatography (eluent system A) to obtain 11-chloro-10-((3,5-difluoropyridin-2-yl)methoxy-d2)-2-(3-(2-hydroxypropan-2-yl)-1H-pyrazol-1-yl)-5,6,7,8-tetrahydro-12H-dipyrido[1,2-a:3',4'-g]azol-12-one **2** (41 mg), yield: 75.0%.

MS m/z (ESI): 530.2 [M+H] ⁺.

¹H NMR (400 MHz, DMSO-*d*₆) δ 8.60 (m, 2H), 8.52 (d, 1H), 8.10 (m, 1H), 7.70 (s, 1H), 6.80 (s, 1H), 6.56 (d, 1H), 5.13 (s, 1H), 2.85 (m, 2H), 2.09 (m, 3H), 1.90 (m, 1H), 1.47 (m, 8H).

### Resolution of Example 2

### (S)-11-chloro-10-((3,5-difluoropyridin-2-yl)methoxy-d2)-2-(3-(2-hydroxypropan-2-yl)-1H-pyrazol-1-yl)-5,6,7,8-tetrahydro-12H-dipyrido[1,2-a:3',4'-g]azol-12-one and (R)-11-chloro-10-((3,5-difluoropyridin-2-yl)methoxy-d2)-2-(3-(2-hydroxypropan-2-yl)-1H-pyrazol-1-yl)-5,6,7,8-tetrahydro-12H-dipyrido[1,2-a:3',4'-g]azol-12-one

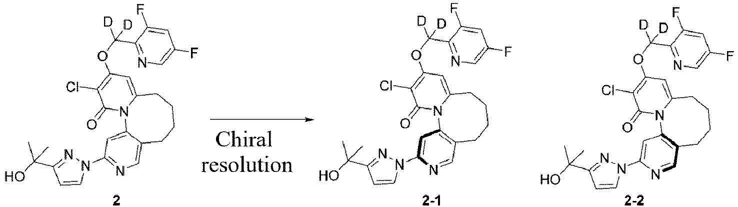

**Example 2** (80 mg, 0.151 mmol) was subjected to chiral resolution (IB column) to obtain **2-1** (35 mg, R.T = 3.677 min), yield: 43.7%; **2-2** (30 mg, R.T = 6.300 min), yield: 37.5%.

**Example 2-1:** MS m/z (ESI): 530.2 [M+H] ⁺;
¹H NMR (400 MHz, DMSO-*d*₆) δ 8.60 (m, 2H), 8.52 (d, 1H), 8.10 (m, 1H), 7.70 (s, 1H), 6.80 (s, 1H), 6.56 (d, 1H), 5.13 (s, 1H), 2.85 (m, 2H), 2.09 (m, 3H), 1.90 (m, 1H), 1.47 (m, 8H).

**Example 2-2:** MS m/z (ESI): 530.2 [M+H] ⁺;
¹H NMR (400 MHz, DMSO-*d*₆) δ 8.60 (m, 2H), 8.52 (d, 1H), 8.10 (m, 1H), 7.70 (s, 1H), 6.80 (s, 1H), 6.56 (d, 1H), 5.13 (s, 1H), 2.85 (m, 2H), 2.09 (m, 3H), 1.90 (m, 1H), 1.47 (m, 8H).

### HPLC chiral resolution conditions:

| | |
|---|---|
| Instrument | HPLC (Waters) |
| Column type | DAICEL CHIPAKPAK^{®}IB 250 * 20 mm 5.0 µm |
| Column pressure | 800 psi |
| Mobile phase | Hexane : EtOH : MeOH (+0.1% DEA) = 70 : 15 : 15 |
| Flow rate | 15 mL/min |
| Detection wavelength | UV 214 nm |
| Column temperature | R.T. |

### HPLC chiral analysis conditions:

| | |
|---|---|
| Instrument | HPLC (Waters) |
| Column type | DAICEL CHIRALCEL IB-H 4.6 mm I.D × 150 mmL 5.0 µm |
| Column pressure | 45 psi |
| Mobile phase | Hexane: EtOH : MeOH (+0.1% DEA) = 70:15:15 |
| Flow rate | 1 mL/min |
| Detection wavelength | UV 214 nm |
| Column temperature | 35°C |

### Example 3

### N-(2-(1-(11-chloro-10-((3,5-difluoropyridin-2-yl)methoxy-d2)-12-carbonyl-6,7,8,12-tetrahydro-5H-dipyrido[1,2-a:3',4'-g]azocin-2-yl)-4-fluoro-1H-pyrazol-3-yl)propan-2-yl)acetamide

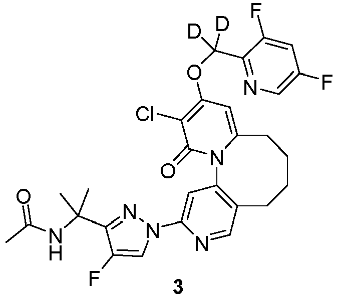

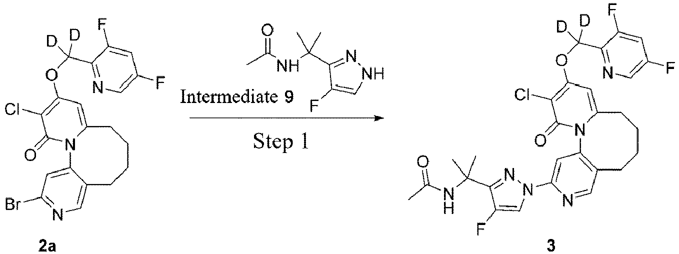

### Step 1

Under nitrogen protection, **2a** (100 mg, 0.206 mmol), **intermediate 9** (52 mg, 0.280 mmol), cuprous iodide (19.64 mg, 0.103 mmol), (1R,2R)-N1,N2-dimethylcyclohexane-1,2-diamine (23.48 mg, 0.165 mmol), and cesium carbonate (100.58 mg, 0.309 mmol) were dissolved in 1,4-dioxane (2 mL), and the reaction liquid was heated to 100°C and stirred for 0.5 hours. The reaction liquid was filtered and concentrated. The residue was purified by silica gel column chromatography (elution system C) to obtain N-(2-(1-(11-chloro-10-((3,5-difluoropyridin-2-yl)methoxy-d2)-12-carbonyl-6,7,8,12-tetrahydro-5H-dipyrido[1,2-a:3',4'-g]azocin-2-yl)-4-fluoro-1H-pyrazol-3-yl)propan-2-yl)acetamide **3** (90 mg), yield: 74.1%.

MS m/z (ESI): 589.2 [M+H]⁺.

### Resolution of Example 3

### (S)-N-(2-(1-(11-chloro-10-((3,5-difluoropyridin-2-yl)methoxy-d2)-12-carbonyl-6,7,8,12-tetrahydro-5H-dipyrido[1,2-a:3',4'-g]azocin-2-yl)-4-fluoro-1H-pyrazol-3-yl)propan-2-yl)acetamide and (R)- N-(2-(1-(11-chloro-10-((3,5-difluoropyridin-2-yl)methoxy-d2)-12-carbonyl-6,7,8,12-tetrahydro-5H-dipyrido[1,2-a:3',4'-g]azocin-2-yl)-4-fluoro-1H-pyrazol-3-yl)propan-2-yl)acetamide

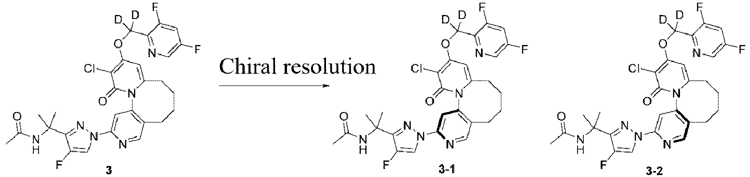

**Example 3** (90 mg, 0.152 mmol) was subjected to chiral resolution (OD column) to obtain **3-1** (20.4 mg, R.T = 3.403 min), yield: 22.35%; **3-2** (21.9 mg, R.T = 4.447 min), yield: 23.56%.

**Example 3-1:** MS m/z (ESI): 589.2 [M+H] ⁺;
¹H NMR (400 MHz, DMSO-*d₆*) δ 8.64 - 8.51 (m, 3H), 8.15 - 8.04 (m, 2H), 7.68 (s, 1H), 6.79 (s, 1H), 2.93 - 2. 76 (m, 2H), 2.15 - 1.89 (m, 4H), 1.79 (s, 3H), 1.57 (d, 6H), 1.53 - 1.29 (m, 2H).

**Example 3-2:** MS m/z (ESI): 589.2 [M+H] ⁺;
¹H NMR (400 MHz, DMSO-*d₆*) δ 8.64 - 8.54 (m, 3H), 8.15 - 8.06 (m, 2H), 7.68 (s, 1H), 6.79 (s, 1H), 2.93 - 2. 76 (m, 2H), 2.16 - 1.89 (m, 4H), 1.79 (s, 3H), 1.57 (d, 6H), 1.50 - 1.29 (m, 2H).

### HPLC chiral resolution conditions:

| | |
|---|---|
| Instrument | Waters HPLC |
| Column type | DAICEL CHIRALCEL^{®}OD 20 mm I.D × 250 mm 5.0 µm |
| Column pressure | 900 psi |
| Mobile phase | Hexane:EtOH (+0.1% DEA) = 70:30 |
| Flow rate | 15 mL/min |
| Detection wavelength | 254 nm |
| Column temperature | R.T. |

### HPLC chiral analysis method:

| | |
|---|---|
| Instrument | Thermo Ultimate 3000 |
| Column type | DAICEL CHIRALCEL^{®}OD 4.6 mm I.D × 150 mm 5.0 µm |
| Column pressure | 55 bar |
| Mobile phase | Hexane:EtOH (+0.1% DEA) = 70:30 |
| Flow rate | 1 mL/min |
| Detection wavelength | 254 nm |
| Column temperature | 35°C |

### Example 4

### N-(2-(4-(11-chloro-10-((3,5-difluoropyridin-2-yl)methoxy-d2)-12-carbonyl-6,7,8,12-tetrahydro-5H-dipyrido[1,2-a:3',4'-g]azocin-2-yl)pyrimidin-2-yl)propan-2-yl)acetamide

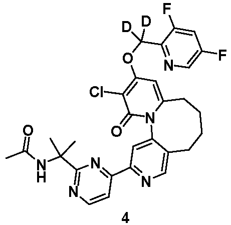

Referring to the synthesis method of **Example 1,** the target product N-(2-(4-(11-chloro-10-((3,5-difluoropyridin-2-yl)methoxy-d2)-12-carbonyl-6,7,8,12-tetrahydro-5H-dipyrido[1,2-a:3',4'-g]azocin-2-yl)pyrimidin-2-yl)propan-2-yl)acetamide **4** was synthesized.

MS m/z (ESI): 583.2 [M+H]⁺;

### Resolution of Example 4

### (S)-N-(2-(4-(11-chloro-10-((3,5-difluoropyridin-2-yl)methoxy-d2)-12-carbonyl-6,7,8,12-tetrahydro-5H-dipyrido[1,2-a:3',4'-g]azocin-2-yl)pyrimidin-2-yl)propan-2-yl)acetamide and (R)-N-(2-(4-(11-chloro-10-((3,5-difluoropyridin-2-yl)methoxy-d2)-12-carbonyl-6,7,8,12-tetrahydro-5H-dipyrido[1,2-a:3',4'-g]azocin-2-yl)pyrimidin-2-yl)propan-2-yl)acetamide

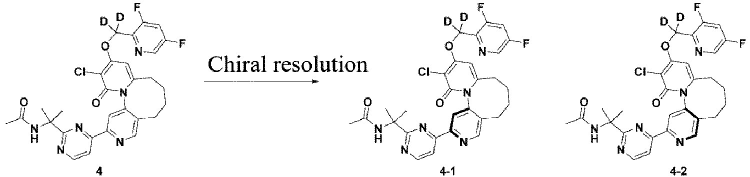

**Example 4** (26 mg, 0.047 mmol) was subjected to chiral resolution (IB column) to obtain **4-1** (5 mg, R.T = 4.387 min), yield: 19.23%; **4-2** (5 mg, R.T = 5.183 min), yield: 19.23%.

**Example 4-1:** MS m/z (ESI): 583.2 [M+H] ⁺;
**Example 4-2:** MS m/z (ESI): 583.2 [M+H] ⁺;
¹H NMR (400 MHz, DMSO-*d₆*) δ 8.94 - 8.83 (m, 2H), 8.61 (d, 1H), 8.24 (d, 2H), 8.16 (d, 1H), 8.10 (ddd, 1H), 6.81 (s, 1H), 2.98 (dd, 1H), 2.80 (dd, 1H), 2.21 - 2.05 (m, 3H), 1.95 - 1.82 (m, 1H), 1.77 (s, 3H), 1.60 (d, 6H), 1.48 - 1.29 (m, 2H).

### HPLC chiral resolution conditions:

| | |
|---|---|
| Instrument | HPLC (Waters) |
| Column type | DAICEL CHIPAKPAK^{®}IB 250 * 20 mm 5.0 µm |
| Column pressure | 800 psi |
| Mobile phase | Hexane : EtOH : MeOH (+0.1% DEA) = 70 : 15 : 15 |
| Flow rate | 15 mL/min |
| Detection wavelength | UV 214 nm |
| Column temperature | R.T. |

### HPLC chiral analysis conditions:

| | |
|---|---|
| Instrument | HPLC (Waters) |
| Column type | DAICEL CHIRALCEL IB-H 4.6 mm I.D × 150 mmL 5.0 µm |
| Column pressure | 45 psi |
| Mobile phase | Hexane:EtOH:MeOH (+0.1% DEA) = 70:15:15 |
| Flow rate | 1 mL/min |
| Detection wavelength | UV 214 nm |
| Column temperature | 35°C |

### Example 5

### N-(2-(3"-chloro-4"-((3,5-difluoropyridin-2-yl)methoxy-d2)-5',6"-dimethyl-2,2"-dicarbonyl-2H,2"H-[1,2':4',1"-terpyridin]-3-yl)propan-2-yl)acetamide

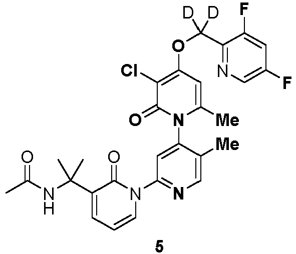

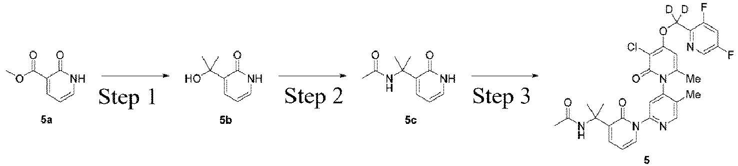

### Step 1

At 0°C, methyl magnesium bromide (3 M, 2.61 mL) was dropwise added to a solution of methyl 2-oxo-1,2-dihydro-3-pyridinecarboxylate **5a** (300 mg, 1.96 mmol) in tetrahydrofuran (8 mL), and the reaction was warmed to room temperature and stirred for 1.5 hours. The reaction liquid was cooled to 0°C, and a saturated ammonium chloride solution was dropwise added to the reaction liquid to quench the reaction. The aqueous phase was extracted with ethyl acetate (25 mL × 4), the organic phases were combined, dried, and concentrated, and the residue was subjected to separation by silica gel column chromatography (elution system B) to obtain 3-(2-hydroxypropan-2-yl)pyridin-2(1*H*)-one **5b** (173 mg), yield: 57.65%.

MS m/z (ESI): 154.1 [M+H]⁺;

### Step 2

Concentrated sulfuric acid (960 mg, 9.8 mmol) was dropwise added to a solution of **5b** (150 mg, 0.98 mmol) in acetonitrile (2 mL), and the reaction was warmed to room temperature and stirred for 3 hours. At 0°C, a saturated sodium bicarbonate solution was added to the above reaction liquid to adjust pH to weak alkalinity. The reaction liquid was concentrated, and the residue was subjected to separation by silica gel column chromatography (elution system B) to obtain *N*-(2-(2-carbonyl-1,2-dihydropyridin-3-yl)propan-2-yl)acetamide **5c** (168 mg), yield: 88.33%.

MS m/z (ESI): 195.2 [M+H]⁺;

### Step 3

Under nitrogen protection, **intermediate 8** (60 mg, 0.13 mmol), **5c** (33 mg, 0.17 mmol), cuprous iodide (10 mg, 0.05 mmol), and potassium carbonate (18 mg, 0.13 mmol) were dissolved in *N,N*dimethylacetamide (1 mL), and the reaction was heated to 120°C and stirred for 6 hours. A saturated ammonium chloride solution was added to the reaction liquid, the aqueous phase was extracted with ethyl acetate (20 mL × 2), the organic phases were combined, dried, and concentrated, and the residue was subjected to preparation by reversed-phase HPLC to obtain *N*-(2-(3"-chloro-4"-((3,5-difluoropyridin-2-yl)methoxy-*d*2)-5',6"-dimethyl-2,2"-dicarbonyl-2*H*,2"*H*-[1,2':4',1"-terpyridin]-3-yl)propan-2-yl)acetamide **5** (35 mg), yield: 46.78%.
MS m/z (ESI): 572.2 [M+H] ⁺;

### Resolution of Example 5

### (S)-N-(2-(3"-chloro-4"-((3,5-difluoropyridin-2-yl)methoxy-d2)-5',6"-dimethyl-2,2"-dicarbonyl-2H,2"H-[1,2':4',1"-terpyridin]-3-yl)propan-2-yl)acetamide and (R)-N-(2-(3"-chloro-4"-((3,5-difluoropyridin-2-yl)methoxy-d2)-5',6"-dimethyl-2,2"-dicarbonyl-2H,2"H-[1,2':4',1"-terpyridin]-3-yl)propan-2-yl)acetamide

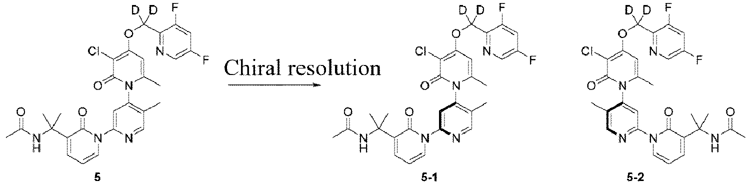

**Example 5** (33 mg, 0.06 mmol) was subjected to chiral resolution (IB column) to obtain the title product **5-1** (11.1 mg, R.T = 5.100 min), yield: 33.64%; **5-2** (11 mg, R.T = 6.173 min), yield: 33.33%.

**Example 5-1,** MS m/z (ESI): 572.2 [M+H] ⁺;
¹H NMR (400 MHz, DMSO-*d*₆) δ 8.68 (s, 1H), 8.60 (d, 1H), 8.14-8.04 (m, 1H), 7.88-7.80 (m, 2H), 7.75 (s, 1H), 7.39-7.30 (m, 1H), 6.79 (s, 1H), 6.36 (t, 1H), 2.07 (s, 3H), 2.00 (s, 3H), 1.81 (s, 3H), 1.61-1.58 (m, 6H).

**Example 5-2**, MS m/z (ESI): 572.2 [M+H] ⁺;
¹H NMR (400 MHz, DMSO-*d*₆) δ 8.68 (s, 1H), 8.60 (d, 1H), 8.14-8.04 (m, 1H), 7.88-7.80 (m, 2H), 7.75 (s, 1H), 7.39-7.30 (m, 1H), 6.79 (s, 1H), 6.36 (t, 1H), 2.07 (s, 3H), 2.00 (s, 3H), 1.81 (s, 3H), 1.61-1.58 (m, 6H).

### HPLC chiral resolution conditions:

| | |
|---|---|
| Instrument | HPLC (Waters) |
| Column type | DAICEL CHIRALPAK^{®}IB 270 * 30 mm 10.0 µm |
| Column pressure | 800 psi |
| Mobile phase | Hexane/EtOH (+0.1% DEA) = 70/30 |
| Flow rate | 15 mL/min |
| Detection wavelength | UV 214 nm |
| Column temperature | 25°C |

### HPLC chiral analysis method:

| | |
|---|---|
| Instrument | HPLC (Waters) |
| Column type | DAICEL CHIRALPAK^{®}IB N-5 150 * 4.6 mm 5.0 µm |
| Column pressure | 35 bar |
| Mobile phase | Hexane/EtOH (+0.1% DEA) = 70/30 |
| Flow rate | 1 mL/min |
| Detection wavelength | 214 nm |
| Column temperature | 35°C |

### Example 6

### N-(2-(1-(11-chloro-10-((3,5-difluoropyridin-2-yl)methoxy-d₂)-1-fluoro-12-carbonyl-6,7,8,12-tetrahydro-5H-dipyrido[1,2-a:3',4'-g]azocin-2-yl)-4-fluoro-1H-pyrazol-3-yl)propan-2-yl)acetamide

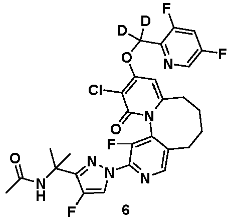

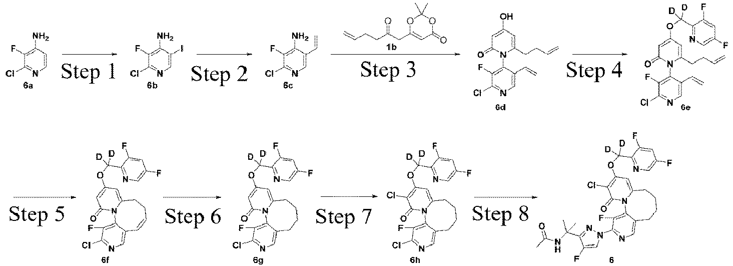

### Step 1

Under nitrogen protection, **6a** (4 g, 27.3 mmol) was dissolved in anhydrous acetonitrile (60 mL), N-iodosuccinimide (7.37 g, 32.8 mmol) and p-toluenesulfonic acid (235 mg, 1.36 mmol) were added, and the reaction was heated to 80°C and stirred for 16 hours. The reaction liquid was cooled to room temperature, diluted with ethyl acetate (100 mL), then washed with a saturated sodium sulfite solution (80 mL), water (80 mL), and a saturated sodium chloride solution (80 mL) in sequence, dried, and concentrated, and the residue was purified by silica gel column chromatography (elution system A) to obtain 2-chloro-3-fluoro-5-iodo-pyridin-4-amine **6b** (6.5 g), yield: 87.4%.

MS m/z (ESI): 272.9 [M+H] ⁺;
¹H NMR (400 MHz, DMSO-*d*₆) δ 8.10 (s, 1H), 6.69 (s, 2H).

### Step 2

Under nitrogen protection, **6b** (4 g, 14.7 mmol) was dissolved in 1,4-dioxane (60 mL), and water (12 mL), potassium vinyltrifluoroborate (2.36 g, 17.6 mmol), 1,1-bis(diphenylphosphine)ferrocene palladium dichloride (533 mg, 0.734 mmol), and sodium carbonate (3.89 g, 36.7 mmol) were added, and the reaction was heated to 80°C and stirred for 4 hours. The reaction liquid was cooled to room temperature, diluted with ethyl acetate (100 mL), then washed with water (60 mL) and a saturated sodium chloride solution (60 mL) in sequence, dried, filtered, and concentrated, and the residue was purified by silica gel column chromatography (elution system B) to obtain 2-chloro-3-fluoro-5-vinyl-pyridin-4-amine **6c** (2.1 g), yield: 82.9%.

MS m/z (ESI): 173.0 [M+H] ⁺;
¹H NMR (400 MHz, DMSO-*d*₆) δ 7.98 (s, 1H), 6.82 (dd, 1H), 6.62 (s, 2H), 5.78 (dd, 1H), 5.33 (dd, 1H).

### Step 3

**6c** (1 g, 5.79 mmol) was dissolved in *1,4-*dioxane (15 mL), **1b** (1.30 g, 5.79 mmol) was added, and the reaction was heated to 100°C and stirred for 2 hours. Concentrated sulfuric acid (1.42 g, 0.772 mL) was added to the reaction liquid, and the reaction continued to be stirred at 100°C for 0.5 hours. The reaction liquid was cooled to room temperature, diluted with ethyl acetate (50 mL), then washed with water (50 mL) and a saturated sodium chloride solution (50 mL) in sequence, dried, and concentrated, and the residue was purified by silica gel column chromatography (elution system B) to obtain 6-(but-3-en-1-yl)-2'-chloro-3'-fluoro-4-hydroxy-5'-vinyl-2H-[1,4'-bipyridin]-2-one **6d** (1.1 g), yield: 59.2%.

MS m/z (ESI): 321.0 [M+H]⁺;
¹H NMR (400 MHz, DMSO-*d*₆) δ 11.13 (s, 1H), 8.80 (s, 1H), 6.29 - 6.22 (m, 1H), 6.08 - 6.00 (m, 2H), 5.67 - 5.58 (m, 3H), 4.95 - 4.89 (m, 2H), 2.19 - 2.16 (m, 2H), 2.13 - 2.09 (m, 2H).

### Step 4

**6d** (100 mg, 0.312 mmol) was dissolved in *N*,*N*-dimethylformamide (3 mL), **intermediate 6** (77 mg, 0.468 mmol), 18-crown-6 (124 mg, 0.468 mmol), and potassium carbonate (129 mg, 0.935 mmol) were added, and the reaction was heated to 75°C and stirred for 2 hours. The reaction liquid was cooled to room temperature and extracted with ethyl acetate (50 mL), the organic phase was washed with water (30 mL × 2) and a saturated sodium chloride solution (30 mL) in sequence, dried, and concentrated, and the residue was purified by silica gel column chromatography (elution system B) to obtain 6-(but-3-en-1-yl)-2'-chloro-4-((3,5-difluoropyridin-2-yl)methoxy-d2)-3'-fluoro-5'-vinyl-2H-[1,4'-bipyridin]-2-one **6e** (110 mg), yield: 78.4%.
MS m/z (ESI): 450.1 [M+H]⁺;

### Step 5

**6e** (110 mg, 0.245 mmol) and Grubbs second-generation catalyst (21 mg, 0.0245 mmol) were dissolved in 1,2-dichloroethane (80 mL), and the reaction was heated to 40°C under nitrogen protection and stirred for 1 hour. The reaction liquid was cooled to room temperature and concentrated, and the residue was purified by silica gel column chromatography (elution system B) to obtain (Z)-2-chloro-10-((3,5-difluoropyridin-2-yl)methoxy-d2)-1-fluoro-7,8-dihydro-12H-dipyrido[1,2-a:3',4'-g]azocin-12-one **6f** (95 mg), yield: 92.1%.

MS m/z (ESI): 422.1 [M+H]⁺;
¹H NMR (400 MHz, DMSO-*d*₆) δ 8.59 (d, 1H), 8.32 (s, 1H), 8.10 - 8.05 (m, 1H), 6.43 (d, 1H), 6.19 (d, 1H), 6.04 (d, 1H), 6.03 - 5.98 (m, 1H), 2.72 - 2.64 (m, 2H), 2.59 - 2.52 (m, 1H), 2.47 - 2.41 (m, 1H).

### Step 6

**6f** (95 mg, 0.225 mmol) and platinum dioxide (5 mg, 0.0225 mmol) were dissolved in tetrahydrofuran (3 mL), and the reaction was subjected to displacement with hydrogen 3 times and then stirred at room temperature in a hydrogen atmosphere for 2 hours. The reaction liquid was filtered and concentrated, and the residue was purified by silica gel column chromatography (elution system B) to obtain 2-chloro-10-((3,5-difluoropyridin-2-yl)methoxy-d2)-1-fluoro-5,6,7,8-tetrahydro-12H-dipyrido[1,2-a:3',4'-g]azocin-12-one **6g** (90 mg), yield: 94.3%.
MS m/z (ESI): 424.1 [M+H]⁺;

### Step 7

**6g** (90 mg, 0.212 mmol) and *N*-chlorosuccinimide (34 mg, 0.255 mmol) were dissolved in 1,2-dichloroethane (9 mL) and isopropanol (3 mL), and the reaction was heated to 60°C for 2 hours of reaction. The reaction liquid was cooled to room temperature, washed with water (20 mL) and a saturated sodium chloride solution (20 mL) in sequence, dried, and concentrated, and the residue was purified by silica gel column chromatography (elution system B) to obtain 2,11-dichloro-10-((3,5-difluoropyridin-2-yl)methoxy-d2)-1-fluoro-5,6,7,8-tetrahydro-12H-dipyrido[1,2-a:3',4'-g]azocin-12-one **6h** (90 mg), yield: 92.5%.

MS m/z (ESI): 458.0 [M+H] ⁺;
¹H NMR (400 MHz, CDCl₃) δ 8.39 (d, 1H), 8.26 (s, 1H), 7.35 - 7.30 (m, 1H), 6.43 (s, 1H), 2.93 - 2.88 (m, 1H), 2.80 - 2.75 (m, 1H), 2.28 - 2.17 (m, 3H), 2.05 - 1.95 (m, 1H), 1.62 - 1.45 (m, 2H).

### Step 8

Under nitrogen protection, **6h** (100 mg, 0.218 mmol), **intermediate 9** (81 mg, 0.436 mmol), cesium carbonate (213 mg, 0.655 mmol), cuprous iodide (62 mg, 0.327 mmol), and trans-*N*,*N*-dimethyl-1,2-cyclohexanediamine (31 mg, 0.218 mmol) were dissolved in 1,4-dioxane (2 mL), and the reaction was heated to 100°C and stirred for 16 hours. The reaction liquid was cooled to room temperature and filtered, the filtrate was diluted with ethyl acetate (50 mL), then washed with water (30 mL) and a saturated sodium chloride solution (30 mL) in sequence, dried, and concentrated, and the residue was purified by reversed-phase column chromatography (eluent system C) to obtain *N*-(2-(1-(11-chloro-10-((3,5-difluoropyridin-2-yl)methoxy-*d*₂)-1-fluoro-12-carbonyl-6,7,8,12-tetrahydro-5*H*-dipyrido[*1,2-a:3;4'-g*]azocin-2-yl)-4-fluoro-1*H*-pyrazol-3-yl)propan-2-yl)acetamide **6** (40 mg), yield: 30.2%.
MS m/z (ESI): 607.2 [M+H] ⁺;

### Resolution of Example 6

### (R)-N-(2-(1-(11-chloro-10-((3,5-difluoropyridin-2-yl)methoxy-d₂)-1-fluoro-12-carbonyl-6,7,8,12-tetrahydro-5H-dipyrido[1,2-a:3',4'-g]azocin-2-yl)-4-fluoro-1H-pyrazol-3-yl)propan-2-yl)acetamide and (S)-N-(2-(1-(11-chloro-10-((3,5-difluoropyridin-2-yl)methoxy-d₂)-1-fluoro-12-carbonyl-6,7,8,12-tetrahydro-5H-dipyrido[1,2-a:3',4'-g]azocin-2-yl)-4-fluoro-1H-pyrazol-3-yl)propan-2-yl)acetamide

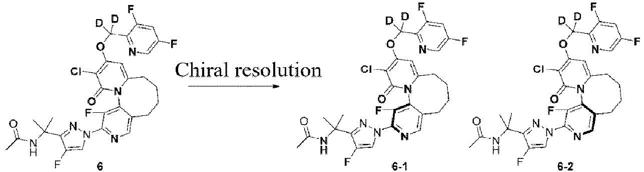

**Example 6** (40 mg, 0.066 mmol) was subjected to chiral resolution (IB column) to obtain **6-1** (13.2 mg, R.T = 4.940 min), yield: 33.0%; **6-2** (15.8 mg, R.T = 7.710 min), yield: 21.3%.

**Example 6-1:** MS m/z (ESI): 607.2 [M+H] ⁺;
¹H NMR (400 MHz, DMSO-*d*₆) δ 8.60 (d, 1H), 8.50 (s, 1H), 8.45 (d, 1H), 8.13 - 8.07 (m, 2H), 6.86 (s, 1H), 3.03 - 2.98 (m, 1H), 2.86 - 2.80 (m, 1H), 2.18 - 1.99 (m, 4H), 1.79 (s, 3H), 1.61 - 1.50 (m, 7H), 1.40 - 1.34 (m, 1H).

**Example 6-2:** MS m/z (ESI): 607.2 [M+H] ⁺;
¹H NMR (400 MHz, DMSO-*d*₆) δ 8.60 (d, 1H), 8.50 (s, 1H), 8.45 (d, 1H), 8.13 - 8.07 (m, 2H), 6.86 (s, 1H), 3.03 - 2.98 (m, 1H), 2.86 - 2.80 (m, 1H), 2.18 - 1.99 (m, 4H), 1.79 (s, 3H), 1.61 - 1.50 (m, 7H), 1.40 - 1.34 (m, 1H).

### HPLC chiral resolution conditions:

| | |
|---|---|
| Instrument | Waters HPLC |
| Column type | DAICEL CHIRALCEL^{®}IB 20 mm I.D × 250 mm 5.0 µm |
| Column pressure | 900 psi |
| Mobile phase | Hexane : EtOH (+0.1% DEA) = 70:30 |
| Flow rate | 15 mL/min |
| Detection wavelength | 214 nm |
| Column temperature | R.T. |

### HPLC chiral analysis method:

| | |
|---|---|
| Instrument | Thermo Ultimate 3000 |
| Column type | DAICEL CHIRALCEL^{®}IB 4.6 mm I.D × 150 mm 5.0 µm |
| Column pressure | 55 bar |
| Mobile phase | Hexane:EtOH (+0.1% DEA) = 70:30 |
| Flow rate | 1 mL/min |
| Detection wavelength | 214 nm |
| Column temperature | 35°C |

### Example 7

### N-(2-(4-(11-chloro-10-((3,5-difluoropyridin-2-yl)methoxy-d₂)-1-fluoro-12-carbonyl-6,7,8,12-tetrahydro-5H-dipyrido[1,2-a:3',4'-g]azocin-2-yl)pyrimidin-2-yl)propan-2-yl)acetamide

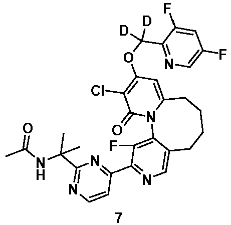

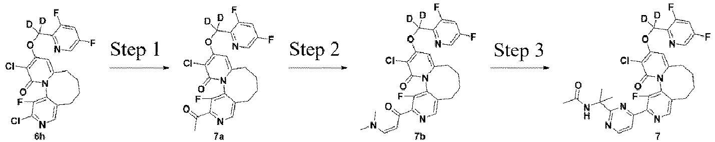

### Step 1

Under nitrogen protection, **6h** (90 mg, 0.196 mmol) was dissolved in 1,4-dioxane (2 mL), tributyl (1-ethoxyvinyl)tin (75 mg, 0.206 mmol) and bis(triphenylphosphine)palladium dichloride (14 mg, 0.019 mmol) were added, and the mixed solution was reacted in a microwave reactor at 125°C for 2 hours. The reaction liquid was cooled to room temperature, and diluted hydrochloric acid (0.2 M, 1 mL) was added and stirred for 1 hour. The reaction liquid was diluted with ethyl acetate (50 mL), then washed with a saturated sodium bicarbonate solution (30 mL) and a saturated sodium chloride solution (30 mL) in sequence, the organic phase was dried and concentrated, and the residue was purified by silica gel column chromatography (elution system B) to obtain 2-acetyl-11-chloro-10-((3,5-difluoropyridin-2-yl)methoxy-*d*₂)-1-fluoro-5,6,7,8-tetrahydro-12*H*-dipyrido[*1,2-a:3',4'-g*]azocin-12-one **7a** (90 mg), yield: 98.4%.
MS m/z (ESI): 466.1 [M+H]⁺.

### Step 2

Under nitrogen protection, **7a** (90 mg, 0.193 mmol) was dissolved in *N,N-*dimethylformamide (2 mL), *N*,*N*-dimethylformamide dimethylacetal (115 mg, 0.966 mmol) was added, and the reaction was heated to 55°C and stirred for 6 hours. The reaction liquid was cooled to room temperature, then washed with ethyl acetate (50 mL), then washed with water (30 mL × 2) and a saturated sodium chloride solution (30 mL) in sequence, dried, and concentrated to obtain 11-chloro-10-((3,5-difluoropyridin-2-yl)methoxy-*d*₂)-2-(3-(dimethylamino)acryloyl)-1-fluoro-5,6,7,8-tetrahydro-12*H*-dipyrido[*1,2-a:3',4'-g*]azocin-12-one **7b** (110 mg), and the product was directly used in the next reaction without purification.
MS m/z (ESI): 521.2 [M+H]⁺.

### Step 3

Under nitrogen protection, **7b** (110 mg, 0.211 mmol) was dissolved in *N,N-*dimethylformamide (3 mL), N-(1-amino-1-imino-2-methylpropan-2-yl)acetamide acetate (90 mg, 0.443 mmol; for the preparation method therefor, reference could be made to the document: Chemistry of Heterocyclic Compounds 2020, 56(10), 1329-1334.) and potassium carbonate (146 mg, 1.06 mmol) were added, and the reaction was heated to 75°C and stirred for 16 hours. The reaction was cooled to room temperature, diluted with ethyl acetate (50 mL), then washed with water (30 mL × 2) and a saturated sodium chloride solution (30 mL) in sequence, dried, and concentrated, and the residue was subjected to separation by preparative HPLC (formic acid system) to obtain *N*-(2-(4-(11-chloro-10-((3,5-difluoropyridin-2-yl)methoxy-*d*₂)-1-fluoro-12-carbonyl-6,7,8,12-tetrahydro-5*H*-dipyrido[*1,2-a:3',4'-g*]azocin-2-yl)pyrimidin-2-yl)propan-2-yl)acetamide **7** (35 mg), yield: 27.6%.
MS m/z (ESI): 601.2 [M+H]⁺;

### Resolution of Example 7

### (R)-N-(2-(4-(11-chloro-10-((3,5-difluoropyridin-2-yl)methoxy-d₂)-1-fluoro-12-carbonyl-6,7,8,12-tetrahydro-5H-dipyrido[1,2-a:3',4'-g]azocin-2-yl)pyrimidin-2-yl)propan-2-yl)acetamide and (S)-N-(2-(4-(11-chloro-10-((3,5-difluoropyridin-2-yl)methoxy-d₂)-1-fluoro-12-carbonyl-6,7,8,12-tetrahydro-5H-dipyrido[1,2-a:3',4'-g]azocin-2-yl)pyrimidin-2-yl)propan-2-yl)acetamide

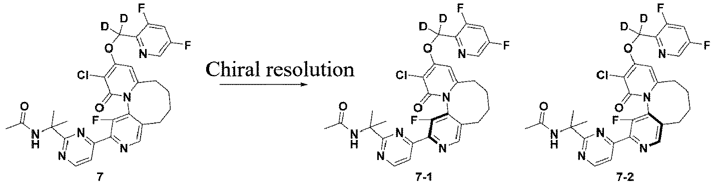

**Example 7** (35 mg, 0.058 mmol) was subjected to chiral resolution (IB column) to obtain **7-1** (10.7 mg, R.T = 4.640 min), yield: 30.45%; **7-2** (10.4 mg, R.T = 5.473 min), yield: 29.54%.

**Example 7-1:** MS m/z (ESI): 601.2 [M+H] ⁺;
¹H NMR (400 MHz, DMSO-*d₆*) δ 8.92 (d, 1H), 8.77 (s, 1H), 8.60 (d, 1H), 8.22 (s, 1H), 8.10 (ddd, 1H), 7.93 (d, 1H), 6.86 (s, 1H), 3.05 (dd, 1H), 2.84 (dd, 1H), 2.16 (q, 3H), 2.07 - 1.94 (m, 1H), 1.76 (s, 3H), 1.59 (d, 7H), 1.49 - 1.35 (m, 1H).

**Example 7-2:** MS m/z (ESI): 601.2 [M+H] ⁺;
¹H NMR (400 MHz, DMSO-*d₆*) δ 8.92 (d, 1H), 8.77 (s, 1H), 8.60 (d, 1H), 8.22 (s, 1H), 8.10 (ddd, 1H), 7.93 (d, 1H), 6.86 (s, 1H), 3.05 (dd, 1H), 2.84 (dd, 1H), 2.16 (q, 3H), 2.07 - 1.94 (m, 1H), 1.76 (s, 3H), 1.59 (d, 7H), 1.49 - 1.35 (m, 1H).

### HPLC chiral resolution conditions:

| | |
|---|---|
| Instrument | Waters HPLC |
| Column type | DAICEL CHIRALCEL^{®}IB 20 mm I.D × 250 mm 5.0 µm |
| Column pressure | 900 psi |
| Mobile phase | Hexane : EtOH : MeOH (+0.1% DEA) = 70 : 15 : 15 |
| Flow rate | 15 mL/min |
| Detection wavelength | 214 nm |
| Column temperature | R.T. |

### HPLC chiral analysis method:

| | |
|---|---|
| Instrument | Thermo Ultimate 3000 |
| Column type | DAICEL CHIRALCEL^{®}IB 4.6 mm I.D × 150 mm 5.0 µm |
| Column pressure | 55 bar |
| Mobile phase | Hexane : EtOH : MeOH (+0.1% DEA) = 70 : 15 : 15 |
| Flow rate | 1 mL/min |
| Detection wavelength | 214 nm |
| Column temperature | 35°C |

### Example 8

### 11-Chloro-10-((3,5-difluoropyridin-2-yl)methoxy-d₂)-1-fluoro-2-(3-(2-hydroxypropan-2-yl)-1H-pyrazol-1-yl)-5,6,7,8-tetrahydro-12H-dipyrido[1,2-a:3',4'-g]azocin-12-one

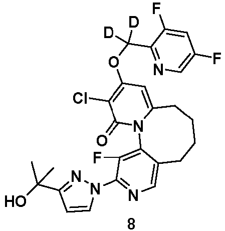

Referring to the synthesis method for **Example 6,** the target product 11-chloro-10-((3,5-difluoropyridin-2-yl)methoxy-*d*₂)-1-fluoro-2-(3-(2-hydroxypropan-2-yl)-1*H*-pyrazol-1-yl)-5,6,7,8-tetrahydro-12*H*-dipyrido[*1,2-a:3',4'-g*]azocin-12-one **8** (20 mg) was synthesized, yield: 32.1%.
MS m/z (ESI): 548.2 [M+H] ⁺;

### Resolution of Example 8

### (R)-11-chloro-10-((3,5-difluoropyridin-2-yl)methoxy-d₂)-1-fluoro-2-(3-(2-hydroxypropan-2-yl)-1H-pyrazol-1-yl)-5,6,7,8-tetrahydro-12H-dipyrido[1,2-a:3',4'-g]azocin-12-one and (S)-11-chloro-10-((3,5-difluoropyridin-2-yl)methoxy-d₂)-1-fluoro-2-(3-(2-hydroxypropan-2-yl)-1H-pyrazol-1-yl)-5,6,7,8-tetrahydro-12H-dipyrido[1,2-a:3',4'-g]azocin-12-one

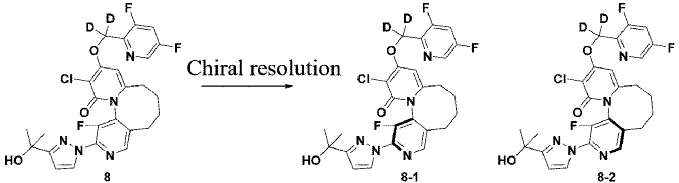

**Example 8** (20 mg, 0.036 mmol) was subjected to chiral resolution (IB column) to obtain **8-1** (10 mg, R.T = 4.923 min), yield: 50.0%; **8-2** (3 mg, R.T = 9.473 min), yield: 15%.

**Example 8-1:** MS m/z (ESI): 548.2 [M+H] ⁺;
¹H NMR (400 MHz, DMSO-*d₆*) δ 8.60 (d, 1H), 8.50 (s, 1H), 8.33 (d, 1H), 8.10 (ddd, 1H), 6.86 (s, 1H), 6.57 (d, 1H), 5.11 (s, 1H), 3.01 (dd, 1H), 2.83 (dd, 1H), 2.20 - 1.97 (m, 4H), 1.55 (d, 1H), 1.46 (d, 6H), 1.41 - 1.31 (m, 1H).

**Example 8-2:** MS m/z (ESI): 548.2 [M+H] ⁺;
¹H NMR (400 MHz, DMSO-*d₆*) δ 8.60 (d, 1H), 8.50 (s, 1H), 8.33 (d, 1H), 8.10 (ddd, 1H), 6.86 (s, 1H), 6.57 (d, 1H), 5.11 (s, 1H), 3.01 (dd, 1H), 2.83 (dd, 1H), 2.20 - 1.97 (m, 4H), 1.55 (d, 1H), 1.46 (d, 6H), 1.41 - 1.31 (m, 1H).

### HPLC chiral resolution conditions:

| | |
|---|---|
| Instrument | Waters HPLC |
| Column type | DAICEL CHIRALCEL^{®}IB 20 mm I.D × 250 mm 5.0 µm |
| Column pressure | 900 psi |
| Mobile phase | Hexane : EtOH (+0.1% DEA) = 70:30 |
| Flow rate | 15 mL/min |
| Detection wavelength | 214 nm |
| Column temperature | R.T. |

### HPLC chiral analysis method:

| | |
|---|---|
| Instrument | Thermo Ultimate 3000 |
| Column type | DAICEL CHIRALCEL^{®}IB 4.6 mm I.D × 150 mm 5.0 µm |
| Column pressure | 55 bar |
| Mobile phase | Hexane : EtOH (+0.1% DEA) = 70:30 |
| Flow rate | 1 mL/min |
| Detection wavelength | 214 nm |
| Column temperature | 35°C |

### Example 9

### 11-Chloro-10-((3,5-difluoropyridin-2-yl)methoxy-d₂)-1-fluoro-2-(2-(2-hydroxypropan-2-yl)pyrimidin-4-yl)-5,6,7,8-tetrahydro-12H-dipyrido[1,2-a:3',4'-g]azocin-12-one

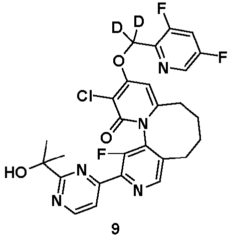

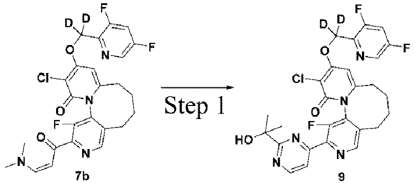

### Step 1

Under nitrogen protection, **7b** (100 mg, 0.192 mmol) was dissolved in *N,N-*dimethylformamide (2 mL), 2-hydroxy-2-methylpropionamidine hydrochloride (80 mg, 0.576 mmol), and potassium carbonate (133 mg,0.960 mmol) were added, and the reaction was heated to 75°C and stirred for 16 hours. The reaction was cooled to room temperature, diluted with ethyl acetate (50 mL), and then washed with water (30 mL × 2) and a saturated sodium chloride solution (30 mL) in sequence, the organic phase was dried and concentrated, and the residue was purified by silica gel column chromatography (elution system B) to obtain 11-chloro-10-((3,5-difluoropyridin-2-yl)methoxy-*d*₂)-1-fluoro-2-(2-(2-hydroxypropan-2-yl)pyrimidin-4-yl)-5,6,7,8-tetrahydro-12*H*-dipyrido[*1,2-a:3,4'-g*]azocin-12-one **9** (50 mg), yield: 46.5 %.

MS m/z (ESI): 560.2 [M+H] ⁺.

### Resolution of Example 9

### (R)-11-chloro-10-((3,5-difluoropyridin-2-yl)methoxy-d₂)-1-fluoro-2-(2-(2-hydroxypropan-2-yl)pyrimidin-4-yl)-5,6,7,8-tetrahydro-12H-dipyrido[1,2-a:3',4'-g]azocin-12-one and (S)-11-chloro-10-((3,5-difluoropyridin-2-yl)methoxy-d₂)-1-fluoro-2-(2-(2-hydroxypropan-2-yl)pyrimidin-4-yl)-5,6,7,8-tetrahydro-12H-dipyrido[1,2-a:3',4'-g]azocin-12-one

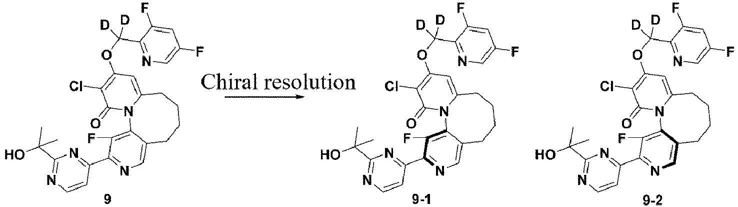

**Example 9** (50 mg, 0.089 mmol) was subjected to chiral resolution (IB column) to obtain **9-1** (15.4 mg, R.T = 4.973 min), yield: 30.8%; **9-2** (15.8 mg, R.T = 8.337 min), yield: 31.6%.

**Example 9-1:** MS m/z (ESI): 560.2 [M+H] ⁺;
¹H NMR (400 MHz, DMSO-*d*₆) δ 9.02 (d, 1H), 8.79 (s, 1H), 8.61 (d, 1H), 8.13 - 8.08 (m, 1H), 8.03 (d, 1H), 6.87 (s, 1H), 5.09 (s, 1H), 3.09 - 3.04 (m, 1H), 2.88 - 2.82 (m, 1H), 2.22 - 2.11 (m, 3H), 2.06 - 1.99 (m, 1H), 1.62 - 1.53 (m, 1H), 1.52 (s, 6H), 1.46 - 1.39 (m, 1H).

**Example 9-2:** MS m/z (ESI): 560.2 [M+H] ⁺;
¹H NMR (400 MHz, DMSO-*d*₆) δ 9.02 (d, 1H), 8.79 (s, 1H), 8.61 (d, 1H), 8.13 - 8.08 (m, 1H), 8.03 (d, 1H), 6.87 (s, 1H), 5.09 (s, 1H), 3.09 - 3.04 (m, 1H), 2.88 - 2.82 (m, 1H), 2.22 - 2.13 (m, 3H), 2.06 - 1.99 (m, 1H), 1.62 - 1.53 (m, 1H), 1.52 (s, 6H), 1.46 - 1.39 (m, 1H).

### HPLC chiral resolution conditions:

| | |
|---|---|
| Instrument | Waters HPLC |
| Column type | DAICEL CHIRALCEL^{®}IB 20 mm I.D × 250 mm 5.0 µm |
| Column pressure | 900 psi |
| Mobile phase | Hexane:MeOH:EtOH (+0.1% DEA) = 70:15:15 |
| Flow rate | 15 mL/min |
| Detection wavelength | 214 nm |
| Column temperature | R.T. |

### HPLC chiral analysis method:

| | |
|---|---|
| Instrument | Thermo Ultimate 3000 |
| Column type | DAICEL CHIRALCEL^{®}IB 4.6 mm I.D × 150 mm 5.0 µm |
| Column pressure | 55 bar |
| Mobile phase | Hexane:MeOH:EtOH (+0.1% DEA) = 70:15:15 |
| Flow rate | 1 mL/min |
| Detection wavelength | 214 nm |
| Column temperature | 35°C |

### Example 10

### 3",5'-Dichloro-4"-((3,5-difluoropyridin-2-yl)methoxy-d2)-3-(2-hydroxypropan-2-yl)-6"-methyl-2H,2"H-[1,2':4',1"-terpyridine]-2,2"-dione

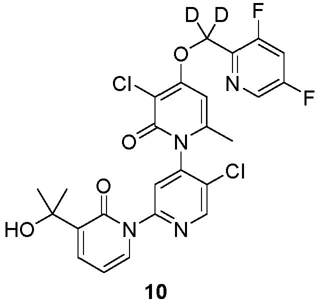

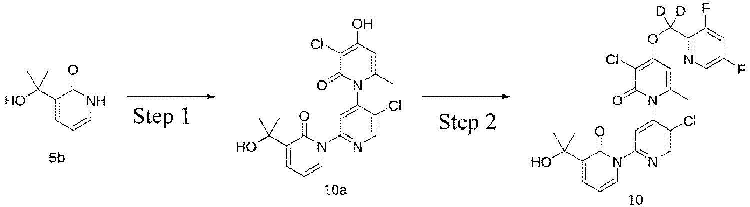

### Step 1

Under nitrogen protection, 2',3,5'-trichloro-4-hydroxy-6-methyl-2*H*-[1,4'-bipyridin]-2-one (150 mg, 0.49 mmol; for the synthesis method therefor, reference could be made to the synthesis of **intermediate 3**), **5b** (90 mg, 0.59 mmol), and cesium carbonate (400 mg, 1.23 mmol) were dissolved in dimethyl sulfoxide (2 mL), and the reaction was heated to 120°C and stirred for 16 hours. The reaction liquid was cooled to room temperature, formic acid was dropwise added to the reaction liquid, the pH was adjusted to weak acidity, the reaction liquid was extracted with a saturated sodium chloride solution and ethyl acetate (25 mL × 2), the organic phases were combined, dried, and concentrated, and the residue was subjected to separation by silica gel column chromatography (elution system B) to obtain 3",5'-dichloro-4"-hydroxy-3-(2-hydroxypropan-2-yl)-6''-methyl-2*H*,2'*'*H-[1,2':4',1''-terpyridine]-2,2''-dione **10a** (147 mg), yield: 70.91%.
MS m/z (ESI): 422.1 [M+H]⁺;

### Step 2

**10a** (147 mg, 0.35 mmol), potassium carbonate (96 mg, 0.7 mmol), and 18-crown-6 (138 mg, 0.52 mmol) were dissolved in *N*,*N*-dimethylformamide (2 mL), and the reaction was heated to 65°C and stirred for 10 minutes. **Intermediate 6** (75 mg, 0.45 mmol) was dropwise added to the reaction liquid, and the reaction was stirred at 65°C for 2 hours. A saturated sodium chloride solution was added to the reaction liquid, the aqueous phase was extracted with ethyl acetate (25 mL × 2), the organic phases were combined, dried, and concentrated, and the residue was subjected to separation by silica gel column chromatography (elution system B) to obtain 3",5'-dichloro-4"-((3,5-difluoropyridin-2-yl)methoxy-*d*2)-3-(2-hydroxypropan-2-yl)-6"-methyl-2*H*,2"*H-*[1,2':4',1"-terpyridine]-2,2"-dione **10** (127 mg), yield: 66.17%.
MS m/z (ESI): 551.1 [M+H]⁺;

### Resolution of Example 10

### (S)-3",5'-dichloro-4"-((3,5-difluoropyridin-2-yl)methoxy-d2)-3-(2-hydroxypropan-2-yl)-6"-methyl-2H,2"H-[1,2':4',1"-terpyridine]-2,2"-dione and (R)-3",5'-dichloro-4"-((3,5-difluoropyridin-2-yl)methoxy-d2)-3-(2-hyd roxypropa n-2-yl)-6" -methyl-2H,2"H-[1,2' :4',1"-terpyridine]-2,2"-dione

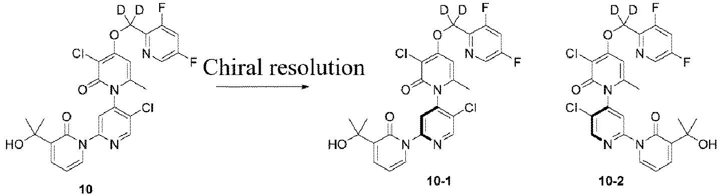

**Example 10** (127 mg, 0.23 mmol) was subjected to chiral resolution (IB column) to obtain the title product **10-1** (26.2 mg, R.T = 5.667 min), yield: 20.63%; **10-2** (22.1 mg, R.T = 7.113 min), yield: 17.40%.

**Example 10-1,** MS m/z (ESI): 551.1 [M+H] ⁺;
¹H NMR (400 MHz, DMSO-*d*₆) δ 8.97 (s, 1H), 8.60 (d, 1H), 8.16-8.06 (m, 2H), 7.92-7.86 (m, 1H), 7.75-7.68 (m, 1H), 6.83 (s, 1H), 6.45 (t, 1H), 5.21 (s, 1H), 2.03 (s, 3H), 1.47 (d, 6H).

**Example 10-2,** MS m/z (ESI): 551.1 [M+H] ⁺;
¹H NMR (400 MHz, DMSO-*d*₆) δ 8.97 (s, 1H), 8.60 (d, 1H), 8.16-8.06 (m, 2H), 7.92-7.86 (m, 1H), 7.75-7.68 (m, 1H), 6.83 (s, 1H), 6.45 (t, 1H), 5.21 (s, 1H), 2.03 (s, 3H), 1.47 (d, 6H).

### HPLC chiral resolution conditions:

| | |
|---|---|
| Instrument | HPLC (Waters) |
| Column type | DAICEL CHIRALPAK^{®}IB 270 * 30 mm 10.0 µm |
| Column pressure | 800 psi |
| Mobile phase | Hexane/EtOH/MeOH (+0.1% DEA) = 70/15/15 |
| Flow rate | 15 mL/min |
| Detection wavelength | UV 214 nm |
| Column temperature | 25°C |

### HPLC chiral analysis method:

| | |
|---|---|
| Instrument | HPLC (Waters) |
| Column type | DAICEL CHIRALPAK^{®}IB N-5 150 * 4.6 mm 5.0 µm |
| Column pressure | 35 bar |
| Mobile phase | Hexane/EtOH/MeOH (+0.1% DEA) = 70/15/15 |
| Flow rate | 1 mL/min |
| Detection wavelength | 214 nm |
| Column temperature | 35°C |

### Example 11

### 3"-Chloro-4"-((3,5-difluoropyridin-2-yl)methoxy-d2)-3-(2-hydroxypropan-2-yl)-5',6"-dimethyl-2H,2"H-[1,2':4',1"-terpyridine]-2,2"-dione

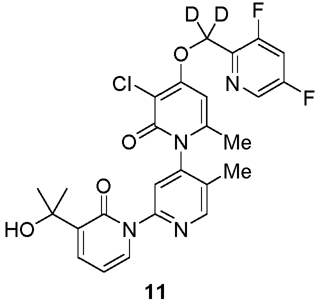

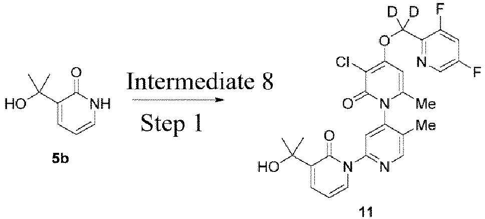

### Step 1

Under nitrogen protection, **intermediate 8** (85 mg, 0.19 mmol), 3-(2-hydroxypropan-2-yl)pyridin-2(1*H*)-one **5b** (37 mg, 0.24 mmol), cuprous iodide (18 mg, 0.09 mmol), and potassium carbonate (26 mg, 0.19 mmol) were dissolved in *N*,*N*-dimethylacetamide (1.5 mL), and the reaction was heated to 120°C and stirred for 5 hours. A saturated ammonia chloride solution was added to the reaction liquid, the aqueous phase was extracted with ethyl acetate (20 mL × 2), the organic phases were combined, dried, and concentrated, and the residue was subjected to preparation by reversed-phase HPLC to obtain 3"-chloro-4"-((3,5-difluoropyridin-2-yl)methoxy-*d*2)-3-(2-hydroxypropan-2-yl)-5',6''-dimethyl-2*H*,2"*H*-[1,2':4',1''-terpyridine]-2,2"-dione **11** (23 mg), yield: 23.38%.
MS m/z (ESI): 531.2 [M+H]⁺;

### Resolution of Example 11

### (S)-3"-chloro-4"-((3,5-difluoropyridin-2-yl)methoxy-d2)-3-(2-hydroxypropan-2-yl)-5',6"-dimethyl-2H,2"H-[1,2:4',1"-terpyridine]-2,2"-dione and (R)-3"-chloro-4"-((3,5-difluoropyridin-2-yl)methoxy-d2)-3-(2-hydroxypropan-2-yl)-5',6"-dimethyl-2H,2"H-[1,2':4',1"-terpyridine]-2,2"-dione

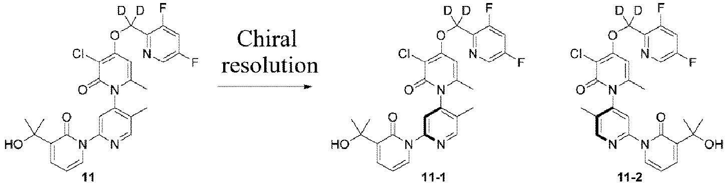

**Example 11** (23 mg, 0.04 mmol) was subjected to chiral resolution (IB column) to obtain the title product **11-1** (7.4 mg, R.T = 4.833 min), yield: 32.17%; **11-2** (8.5 mg, R.T = 6.753 min), yield: 36.96%.

**Example 11-1,** MS m/z (ESI): 531.2 [M+H] ⁺;
¹H NMR (400 MHz, DMSO-*d*₆) δ 8.68 (s, 1H), 8.60 (d, 1H), 8.14-8.05 (m, 1H), 7.88-7.82 (m, 1H), 7.78 (s, 1H), 7.72-7.66 (m, 1H), 6.80 (s, 1H), 6.42 (t, 1H), 5.23 (s, 1H), 2.07 (s, 3H), 2.00 (s, 3H), 1.49-1.44 (m, 6H).

**Example 11-2,** MS m/z (ESI): 531.2 [M+H] ⁺;
¹H NMR (400 MHz, DMSO-*d*₆) δ 8.68 (s, 1H), 8.60 (d, 1H), 8.14-8.05 (m, 1H), 7.88-7.82 (m, 1H), 7.78 (s, 1H), 7.72-7.66 (m, 1H), 6.80 (s, 1H), 6.42 (t, 1H), 5.23 (s, 1H), 2.07 (s, 3H), 2.00 (s, 3H), 1.49-1.44 (m, 6H).

### HPLC chiral resolution conditions:

| | |
|---|---|
| Instrument | HPLC (Waters) |
| Column type | DAICEL CHIRALPAK^{®}IB 270 * 30 mm 10.0 µm |
| Column pressure | 800 psi |
| Mobile phase | Hexane/EtOH (+0.1% DEA) = 70/30 |
| Flow rate | 15 mL/min |
| Detection wavelength | UV 214 nm |
| Column temperature | 25°C |

### HPLC chiral analysis method:

| | |
|---|---|
| Instrument | HPLC (Waters) |
| Column type | DAICEL CHIRALPAK^{®}IB N-5 150 * 4.6 mm 5.0 µm |
| Column pressure | 35 bar |
| Mobile phase | Hexane/EtOH (+0.1% DEA) = 70/30 |
| Flow rate | 1 mL/min |
| Detection wavelength | 214 nm |
| Column temperature | 35°C |

### Example 12

### N-(2-(3",5'-dichloro-4"-((3,5-difluoropyridin-2-yl)methoxy-d2)-6"-methyl-2,2"-dicarbonyl-2H,2"H-[1,2':4',1"-terpyridin]-3-yl)propan-2-yl)acetamide

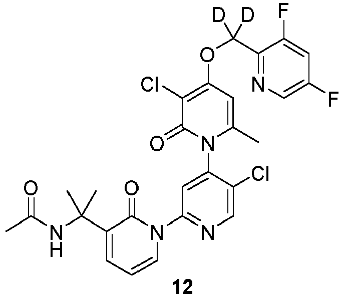

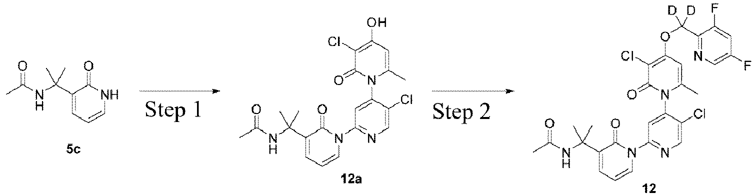

### Step 1

Under nitrogen protection, 2',3,5'-trichloro-4-hydroxy-6-methyl-2*H*-[1,4'-bipyridin]-2-one (100 mg, 0.33 mmol), **5c** (83 mg, 0.43 mmol), and cesium carbonate (320 mg, 0.98 mmol) were dissolved in dimethyl sulfoxide (2 mL), and the reaction was heated to 120°C and stirred for 16 hours. A saturated sodium chloride solution was added to the reaction liquid, the pH was adjusted to weak acidity with formic acid, the reaction liquid was extracted with ethyl acetate (25 mL × 3), and the organic phases are combined, dried, and concentrated to obtain the crude product*N*-(2-(3",5'-dichloro-4"-hydroxy-6"-methyl-2,2"-dicarbonyl-2*H*,2"*H*-[1,2':4',1"-terpyridin]-3-yl)propan-2-yl)acetamide **12a** (100 mg), which was directly used in the next reaction without purification.
MS m/z (ESI): 463.1 [M+H]⁺;

### Step 2

**12a** (27 mg, 0.06 mmol), potassium carbonate (16 mg, 0.12 mmol), and 18-crown-6 (23 mg, 0.09 mmol) were dissolved in *N*,*N*-dimethylformamide (1 mL) and stirred at 65°C for 15 minutes, and **intermediate 6** (14 mg, 0.09 mmol) was added to the above reaction liquid and stirred at 65°C for 1 hour. A saturated sodium chloride solution was added to the reaction liquid, which was extracted with ethyl acetate (25 mL × 2), the organic phases were combined, dried, and concentrated, and the residue was subjected to preparation by reversed-phase HPLC to obtain *N*-(2-(3'',5'-dichloro-4''-((3,5-difluoropyridin-2-yl)methoxy-*d*2)-6''-methyl-2,2''-dicarbonyl-2*H*,2"*H*-[1,2':4',1"-terpyridin]-3-yl)propan-2-yl)acetamide **12** (15.8 mg), yield: 45.77%.

MS m/z (ESI): 592.1 [M+H] ⁺;
¹H NMR (400 MHz, DMSO-*d*₆) δ 8.97 (s, 1H), 8.60 (d, 1H), 8.15-8.06 (m, 2H), 7.92-7.82 (m, 2H), 7.41-7.32 (m, 1H), 6.83 (s, 1H), 6.39 (t, 1H), 2.02 (s, 3H), 1.81 (s, 3H), 1.60 (d, 6H).

### Example 13

### N-(2-(3"-chloro-4"-((2,4-difluorophenyl)methoxy-d2)-5',6"-dimethyl-2,2"-dicarbonyl-2H,2"H-[1,2':4',1"-terpyridin]-3-yl)propan-2-yl)acetamide

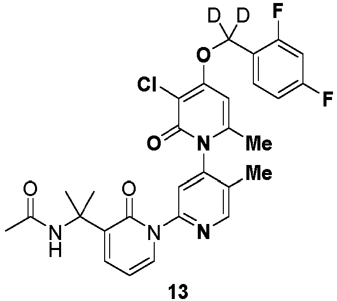

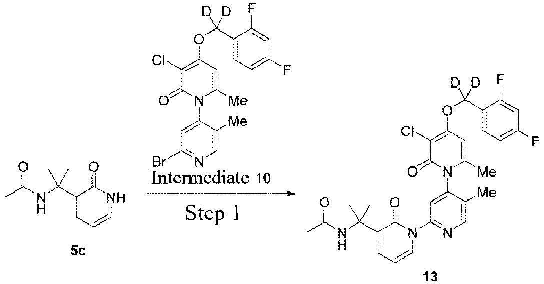

### Step 1

Under nitrogen protection, **intermediate 10** (85 mg, 0.19 mmol), *N*-(2-(2-carbonyl-1,2-dihydropyridin-3-yl)propan-2-yl)acetamide **5c** (43 mg, 0.22 mmol), cuprous iodide (18 mg, 0.09 mmol), and potassium carbonate (26 mg, 0.19 mmol) were dissolved in *N*,*N*-dimethylacetamide (1.5 mL), and the reaction was heated to 120°C and stirred for 3 hours. A saturated sodium chloride solution was added to the reaction liquid, which was extracted with ethyl acetate (25 mL × 2), the organic phases were combined, dried, and concentrated, and the residue was subjected to preparation by reversed-phase HPLC to obtain *N*-(2-(3"-chloro-4"-((2,4-difluorophenyl)methoxy-*d*2)-5',6''-dimethyl-2,2''-dicarbonyl-2*H*,2"*H*-[1,2':4',1"-terpyridin]-3-yl)propan-2-yl)acetamide **13** (40 mg), yield: 37.72%.
MS m/z (ESI): 571.2 [M+H]⁺;

### Resolution of Example 13

### (S)-N-(2-(3"-chloro-4"-((2,4-difluorophenyl)methoxy-d2)-5',6"-dimethyl-2,2"-dicarbonyl-2H,2"H-[1,2':4',1"-terpyridin]-3-yl)propan-2-yl)acetamide and (R)-N-(2-(3"-chloro-4"-((2,4-difluorophenyl)methoxy-d2)-5',6"-dimethyl-2,2"-dicarbonyl-2H,2"H-[1,2':4',1"-terpyridin]-3-yl)propan-2-yl)acetamide

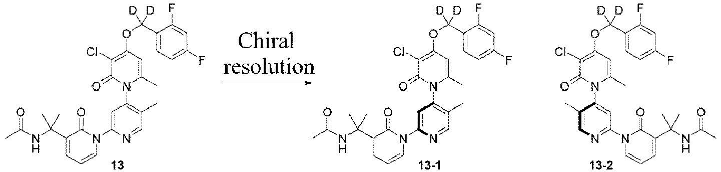

**Example 13** (40 mg, 0.07 mmol) was subjected to chiral resolution (AD column) to obtain the title product **13-1** (14.6 mg, R.T = 5.173 min), yield: 36.50%; **13-2** (15.6 mg, R.T = 8.937 min), yield: 39.00%.

**Example 13-1,** MS m/z (ESI): 571.2 [M+H] ⁺;
¹H NMR (400 MHz,DMSO-*d*₆) δ 8.68 (s, 1H), 7.88-7.80 (m, 2H), 7.77-7.66 (m, 2H), 7.42-7.31 (m, 2H), 7.25-7.15 (m, 1H), 6.82 (s, 1H), 6.36 (t, 1H), 2.07 (s, 3H), 2.02 (s, 3H), 1.81 (s, 3H), 1.60 (d, 6H).

**Example 13-2,** MS m/z (ESI): 571.2 [M+H] ⁺;
¹H NMR (400 MHz,DMSO-*d*₆) δ 8.68 (s, 1H), 7.88-7.80 (m, 2H), 7.77-7.66 (m, 2H), 7.42-7.31 (m, 2H), 7.25-7.15 (m, 1H), 6.82 (s, 1H), 6.36 (t, 1H), 2.07 (s, 3H), 2.02 (s, 3H), 1.81 (s, 3H), 1.60 (d, 6H).

### HPLC chiral resolution conditions:

| | |
|---|---|
| Instrument | HPLC (Waters) |
| Column type | DAICEL CHIRALPAK^{®}AD 250 * 20 mm 5.0 µm |
| Column pressure | 800 psi |
| Mobile phase | Hexane/EtOH (+0.1% DEA) = 70/30 |
| Flow rate | 15 mL/min |
| Detection wavelength | UV 214 nm |
| Column temperature | 25°C |

### HPLC chiral analysis method:

| | |
|---|---|
| Instrument | HPLC (Waters) |
| Column type | DAICEL CHIRALPAK^{®}AD 250*4.6 mm 5.0 µm |
| Column pressure | 35 bar |
| Mobile phase | Hexane/EtOH (+0.1% DEA) = 70/30 |
| Flow rate | 1 mL/min |
| Detection wavelength | 214 nm |
| Column temperature | 35°C |

### Example 14

### 3"-Chloro-4"-((2,4-difluorophenyl)methoxy-d2)-3-(2-hydroxypropan-2-yl)-5',6"-dimethyl-2H,2"H-[1,2':4',1"-terpyridine]-2,2"-dione

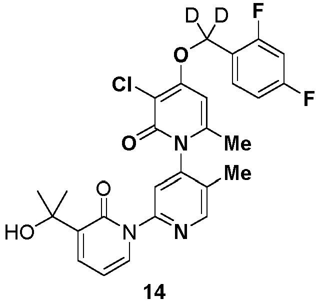

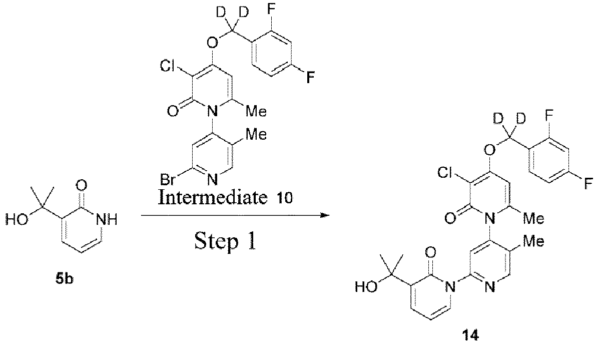

### Step 1

Under nitrogen protection, **intermediate 10** (70 mg, 0.15 mmol), **5b** (28 mg, 0.18 mmol), cuprous iodide (15 mg, 0.08 mmol), and potassium carbonate (21 mg, 0.15 mmol) were dissolved in *N*,*N*-dimethylacetamide (1.5 mL), and the reaction was heated to 120°C and stirred for 3 hours. A saturated sodium chloride solution was added to the reaction liquid, which was extracted with ethyl acetate (25 mL × 2), the organic phases were combined, dried, and concentrated, and the residue was subjected to preparation by reversed-phase HPLC to obtain 3"-chloro-4"-((2,4-difluorophenyl)methoxy-*d*2)-3-(2-hydroxypropan-2-yl)-5',6"-dimethyl-2*H*,2"*H-*[1,2':4',1"-terpyridine]-2,2"-dione **14** (35 mg), yield: 43.18%.
MS m/z (ESI): 530.2 [M+H]⁺;

### Resolution of Example 14

### (S)-3"-chloro-4"-((2,4-difluorophenyl)methoxy-d2)-3-(2-hydroxypropan-2-yl)-5',6"-dimethyl-2H,2"H-[1,2':4',1"-terpyridine]-2,2"-dione and (R)-3"-chloro-4"-((2,4-difluorophenyl)methoxy-d2)-3-(2-hydroxypropan-2-yl)-5',6"-dimethyl-2H,2"H-[1,2':4',1"-terpyridine]-2,2"-dione

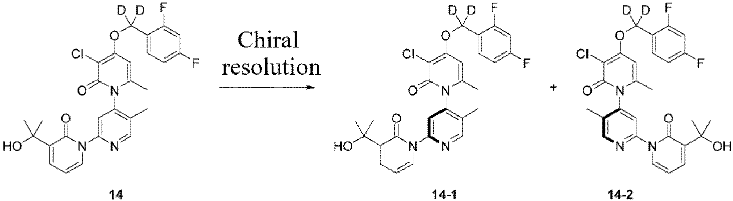

**Example 14** (35 mg, 0.07 mmol) was subjected to chiral resolution (IB column) to obtain the title product **14-1** (12.2 mg, R.T = 5.990 min), yield: 34.86%; **14-2** (11.9 mg, R.T = 9.167 min), yield: 34.00%.

**Example 14-1,** MS m/z (ESI): 530.2 [M+H] ⁺;
¹H NMR (400 MHz, DMSO-*d*₆) δ 8.69 (s, 1H), 7.88-7.82 (m, 1H), 7.78 (s, 1H), 7.74-7.65 (m, 2H), 7.43-7.32 (m, 1H), 7.24-7.16 (m, 1H), 6.82 (s, 1H), 6.42 (t, 1H), 5.23 (s, 1H), 2.07 (s, 3H), 2.02 (s, 3H), 1.46 (d, 6H).

**Example 14-2,** MS m/z (ESI): 530.2 [M+H] ⁺;
¹H NMR (400 MHz, DMSO-*d*₆) δ 8.69 (s, 1H), 7.88-7.82 (m, 1H), 7.78 (s, 1H), 7.74-7.65 (m, 2H), 7.43-7.32 (m, 1H), 7.24-7.16 (m, 1H), 6.82 (s, 1H), 6.42 (t, 1H), 5.23 (s, 1H), 2.07 (s, 3H), 2.02 (s, 3H), 1.46 (d, 6H).

### HPLC chiral resolution conditions:

| | |
|---|---|
| Instrument | HPLC (Waters) |
| Column type | DAICEL CHIRALPAK^{®}IB 270 * 30 mm 10.0 µm |
| Column pressure | 800 psi |
| Mobile phase | Hexane/EtOH (+0.1% DEA) = 70/30 |
| Flow rate | 15 mL/min |
| Detection wavelength | UV 214 nm |
| Column temperature | 25°C |

### HPLC chiral analysis method:

| | |
|---|---|
| Instrument | HPLC (Waters) |
| Column type | DAICEL CHIRALPAK^{®}IB N-5 150 * 4.6 mm 5.0 µm |
| Column pressure | 35 bar |
| Mobile phase | Hexane : EtOH (+0.1% DEA) = 70/30 |
| Flow rate | 1 mL/min |
| Detection wavelength | 214 nm |
| Column temperature | 35°C |

### Example 15

### 11-Chloro-10-((3,5-difluoropyridin-2-yl)methoxy-d2)-2-(3-(2-hydroxypropan-2-yl)-2-carbonylpyridin-1(2H)-yl)-5,6,7,8-tetrahydro-12H-dipyrido[1,2-a:3',4'-g]azocin-12-one

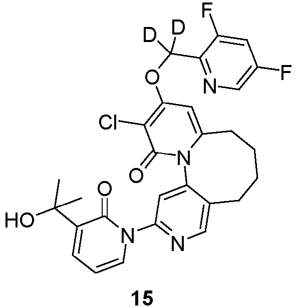

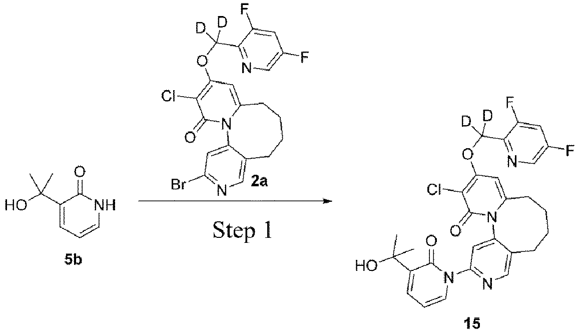

### Step 1

Under nitrogen protection, **2a** (100 mg, 0.21 mmol), **5b** (41 mg, 0.27 mmol), (15,25)-*N*1,*N*2-dimethylcyclohexane-1,2-diamine (31 mg, 0.22 mmol), cuprous iodide (20 mg, 0.1 mmol), and cesium carbonate (134 mg, 0.41 mmol) were dissolved in 1,4-dioxane (1.5 mL), and the reaction was heated to 100°C and stirred for 1.5 hours. A saturated ammonium chloride solution was added to the reaction liquid, which was extracted with ethyl acetate (25 mL × 2), the organic phases were combined, dried, and concentrated, and the residue was subjected to separation by silica gel column chromatography (elution system B) to obtain 11-chloro-10-((3,5-difluoropyridin-2-yl)methoxy-*d*2)-2-(3-(2-hydroxypropan-2-yl)-2-carbonylpyridin-1(2*H*)-yl)-5,6,7,8-tetrahydro-12*H*-dipyrido[1,2-*a*:3',4'-*g*]azocin-12-one **15** as a white solid (105 mg), yield: 91.38%.
MS m/z (ESI): 557.2 [M+H] ⁺;

### Resolution of Example 15

### (S)-11-chloro-10-((3,5-difluoropyridin-2-yl)methoxy-d2)-2-(3-(2-hydroxypropan-2-yl)-2-carbonylpyridin-1(2H)-yl)-5,6,7,8-tetrahydro-12H-dipyrido[1,2-a:3',4'-g]azocin-12-one and (R)-11-chloro-10-((3,5-difluoropyridin-2-yl)methoxy-d2)-2-(3-(2-hydroxypropan-2-yl)-2-carbonylpyridin-1(2H)-yl)-5,6,7,8-tetrahydro-12H-dipyrido[1,2-a:3',4'-g]azocin-12-one

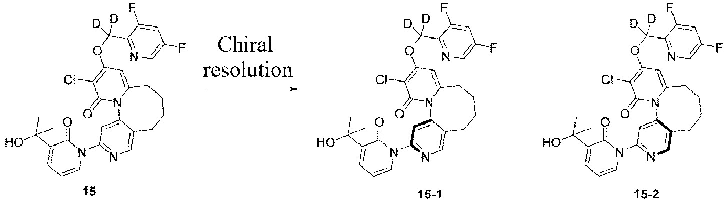

**Example 15** (105 mg, 0.19 mmol) was subjected to chiral resolution (IB column) to obtain the title product **15-1** (36.5 mg, R.T = 3.977 min), yield: 34.76%; **15-2** (34.3 mg, R.T = 5.027 min), yield: 32.67%.

**Example 15-1,** MS m/z (ESI): 557.2 [M+H] ⁺;
¹H NMR (400 MHz, DMSO-*d*₆) δ 8.75 (s, 1H), 8.60 (d, 1H), 8.13-8.06 (m, 1H), 7.88-7.82 (m, 1H), 7.74-7.67 (m, 2H), 6.80 (s, 1H), 6.42 (t, 1H), 5.21 (s, 1H), 3.02-2.90 (m, 1H), 2.88-2.77 (m, 1H), 2.23-2.04 (m, 3H), 1.95-1.82 (m, 1H), 1.58 (d, 1H), 1.47 (s, 3H), 1.45 (s, 3H), 1.42-1.32 (m, 1H).

**Example 15-2,** MS m/z (ESI): 557.2 [M+H] ⁺;
¹H NMR (400 MHz, DMSO-*d*₆) δ 8.75 (s, 1H), 8.60 (d, 1H), 8.13-8.06 (m, 1H), 7.88-7.82 (m, 1H), 7.74-7.67 (m, 2H), 6.80 (s, 1H), 6.42 (t, 1H), 5.21 (s, 1H), 3.02-2.90 (m, 1H), 2.88-2.77 (m, 1H), 2.23-2.04 (m, 3H), 1.95-1.82 (m, 1H), 1.58 (d, 1H), 1.47 (s, 3H), 1.45 (s, 3H), 1.42-1.32 (m, 1H).

### HPLC chiral resolution conditions:

| | |
|---|---|
| Instrument | HPLC (Waters) |
| Column type | DAICEL CHIRALPAK^{®}IB 270 * 30 mm 10.0 µm |
| Column pressure | 800 psi |
| Mobile phase | Hexane : EtOH : MeOH (+0.1% DEA) = 70:15:15 |
| Flow rate | 15 mL/min |
| Detection wavelength | UV 214 nm |
| Column temperature | 25°C |

### HPLC chiral analysis method:

| | |
|---|---|
| Instrument | HPLC (Waters) |
| Column type | DAICEL CHIRALPAK^{®}IB N-5 150 * 4.6 mm 5.0 µm |
| Column pressure | 35 bar |
| Mobile phase | Hexane : EtOH : MeOH (+0.1% DEA) = 70:15:15 |
| Flow rate | 1 mL/min |
| Detection wavelength | 214 nm |
| Column temperature | 35°C |

### Example 16

### (Z)-11-chloro-10-((3,5-difluoropyridin-2-yl)methoxy-d2)-2-(2-(2-hydroxypropan-2-yl)pyrimidin-4-yl)-7,8-dihydro-12H-dipyrido[1,2-a:3',4'-g]azocin-12-one

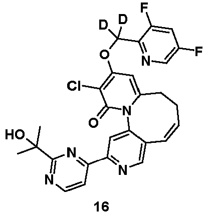

Referring to the synthesis method for **Example 1,** the target product (Z)-11-chloro-10-((3,5-difluoropyridin-2-yl)methoxy-d2)-2-(2-(2-hydroxypropan-2-yl)pyrimidin-4-yl)-7,8-dihydro-12H-dipyrido[1,2-a:3',4'-g]azocin-12-one **16** was synthesized.

MS m/z (ESI): 540.2 [M+H] ⁺.

¹H NMR (400 MHz, DMSO-*d₆*) δ 8.99 (d, 1H), 8.80 (s, 1H), 8.66 - 8.57 (m, 2H), 8.26 (d, 1H), 8.11 (ddd, 1H), 6.81 (s, 1H), 6.53 (dd, 1H), 6.03 (d, 1H), 5.29 (s, 1H), 2.76 (d, 2H), 2.57 (dd, 1H), 2.00 (q, 1H), 1.53 (d, 6H).

### Resolution of Example 16

### (S,Z)-11-chloro-10-((3,5-difluoropyridin-2-yl)methoxy-d2)-2-(2-(2-hydroxypropan-2-yl)pyrimidin-4-yl)-7,8-dihydro-12H-dipyrido[1,2-a:3',4'-g]azocin-12-one and (R,Z)-11-chloro-10-((3,5-difluoropyridin-2-yl)methoxy-d2)-2-(2-(2-hydroxypropan-2-yl)pyrimidin-4-yl)-7,8-dihydro-12H-dipyrido[1,2-a:3,4'-g]azocin-12-one

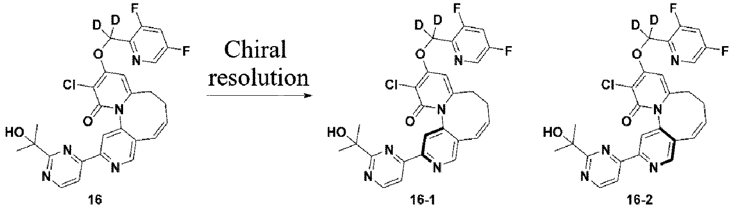

**Example 16** (20 mg, 0.037 mmol) was subjected to chiral resolution (OD column) to obtain the title product **16-1** (8 mg, R.T = 3.623 min), yield: 40%; **16-2** (6 mg, R.T = 7.39 min), yield: 30%.
**Example 16-1,** MS m/z (ESI): 540.2 [M+H] ⁺;
**Example 16-2,** MS m/z (ESI): 540.2 [M+H] ⁺;
¹H NMR (400 MHz, DMSO-*d₆*) δ 8.99 (d, 1H), 8.80 (s, 1H), 8.66 - 8.57 (m, 2H), 8.26 (d, 1H), 8.11 (ddd, 1H), 6.81 (s, 1H), 6.53 (dd, 1H), 6.03 (d, 1H), 5.29 (s, 1H), 2.76 (d, 2H), 2.57 (dd, 1H), 2.00 (q, 1H), 1.53 (d, 6H).

### HPLC chiral resolution conditions:

| | |
|---|---|
| Instrument | Waters HPLC |
| Column type | DAICEL CHIRALCEL^{®}OD 20 mm I.D × 250 mm 5.0 µm |
| Column pressure | 900 psi |
| Mobile phase | Hexane : EtOH : MeOH (+0.1% DEA) = 70 : 15 : 15 |
| Flow rate | 15 mL/min |
| Detection wavelength | 254 nm |
| Column temperature | R.T. |

### HPLC chiral analysis method:

| | |
|---|---|
| Instrument | Thermo Ultimate 3000 |
| Column type | DAICEL CHIRALCEL^{®}OD 4.6 mm I.D × 150 mm 5.0 µm |
| Column pressure | 55 bar |
| Mobile phase | Hexane : EtOH : MeOH (+0.1% DEA) = 70 : 15 : 15 |
| Flow rate | 1 mL/min |
| Detection wavelength | 254 nm |
| Column temperature | 35°C |

### Example 17

### (Z)-11-chloro-10-((3,5-difluoropyridin-2-yl)methoxy-d2)-2-(3-(2-hydroxypropan-2-yl)-1H-pyrazol-1-yl)-7,8-dihydro-12H-dipyrido[1,2-a:3',4'-g]azocin-12-one

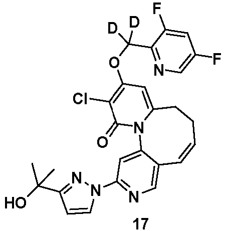

Referring to the synthesis method for **Example 2,** the target product (Z)-11-chloro-10-((3,5-difluoropyridin-2-yl)methoxy-d2)-2-(3-(2-hydroxypropan-2-yl)-1H-pyrazol-1-yl)-7,8-dihydro-12H-dipyrido[1,2-a:3',4'-g]azocin-12-one **17** was synthesized.

MS m/z (ESI): 528.2 [M+H] ⁺.

### Resolution of Example 17

### (S,Z)-11-chloro-10-((3,5-difluoropyridin-2-yl)methoxy-d2)-2-(3-(2-hydroxypropan-2-yl)-1H-pyrazol-1-yl)-7,8-dihydro-12H-dipyrido[1,2-a:3',4'-g]azocin-12-one and (R,Z)-11-chloro-10-((3,5-difluoropyridin-2-yl)methoxy-d2)-2-(3-(2-hydroxypropan-2-yl)-1H-pyrazol-1-yl)-7,8-dihydro-12H-dipyrido[1,2-a:3',4'-g]azocin-12-one

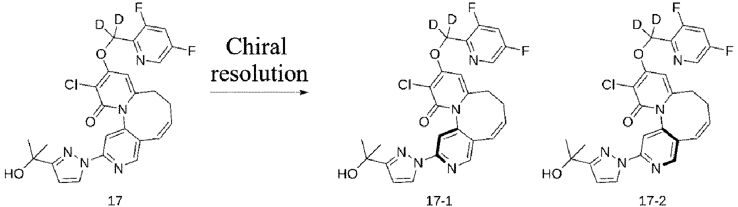

**Example 17** (80 mg, 0.152 mmol) was subjected to chiral resolution (OD column) to obtain the title product **17-1** (36 mg, R.T = 3.553 min), yield: 45%; **17-2** (38 mg, R.T = 5.693 min), yield: 47.5%.
**Example 17-1,** MS m/z (ESI): 528.2 [M+H] ⁺;
**Example 17-2,** MS m/z (ESI): 528.2 [M+H] ⁺;
¹H NMR (400 MHz, DMSO-*d₆*) δ 8.60 (d, 1H), 8.53 (d, 1H), 8.49 (s, 1H), 8.10 (td, 1H), 7.72 (s, 1H), 6.79 (s, 1H), 6.57 (d, 1H), 6.47 - 6.39 (m, 1H), 5.99 - 5.88 (m, 1H), 5.14 (s, 1H), 2.80 - 2.67 (m, 2H), 2.67 - 2.52 (m, 2H), 1.47 (d, 6H).

### HPLC chiral resolution conditions:

| | |
|---|---|
| Instrument | Waters HPLC |
| Column type | DAICEL CHIRALCEL^{®}OD 20 mm I.D × 250 mm 5.0 µm |
| Column pressure | 900 psi |
| Mobile phase | Hexane:EtOH:MeOH (+0.1% DEA) = 70:15:15 |
| Flow rate | 15 mL/min |
| Detection wavelength | 254 nm |
| Column temperature | R.T. |

### HPLC chiral analysis method:

| | |
|---|---|
| Instrument | Thermo Ultimate 3000 |
| Column type | DAICEL CHIRALCEL^{®}OD 4.6 mm I.D × 150 mm 5.0 µm |
| Column pressure | 55 bar |
| Mobile phase | Hexane : EtOH : MeOH (+0.1% DEA) = 70 : 15 : 15 |
| Flow rate | 1 mL/min |
| Detection wavelength | 254 nm |
| Column temperature | 35°C |

### Example 18

### (Z)-N-(2-(1-(11-chloro-10-((3,5-difluoropyridin-2-yl)methoxy-d2)-12-carbonyl-8,12-dihydro-7H-dipyrido[1,2-a:3',4'-g]azocin-2-yl)-4-fluoro-1H-pyrazol-3-yl)propan-2-yl)acetamide

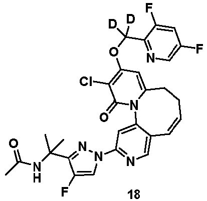

Referring to the synthesis method of **Example 3,** the target product (Z)-N-(2-(1-(11-chloro-10-((3,5-difluoropyridin-2-yl)methoxy-d2)-12-carbonyl-8,12-dihydro-7H-dipyrido[1,2-a:3',4'-g]azocin-2-yl)-4-fluoro-1H-pyrazol-3-yl)propan-2-yl)acetamide **18** was synthesized.

MS m/z (ESI): 587.2 [M+H] ⁺.

### Resolution of Example 18

### (S,Z)-N-(2-(1-(11-chloro-10-((3,5-difluoropyridin-2-yl)methoxy-d2)-12-carbonyl-8,12-dihydro-7H-dipyrido[1,2-a:3',4'-g]azocin-2-yl)-4-fluoro-1H-pyrazol-3-yl)propan-2-yl)acetamide and (R,Z)-N-(2-(1-(11-chloro-10-((3,5-difluoropyridin-2-yl)methoxy-d2)-12-carbonyl-8,12-dihydro-7H-dipyrido[1,2-a:3',4'-g]azocin-2-yl)-4-fluoro-1H-pyrazol-3-yl)propan-2-yl)acetamide

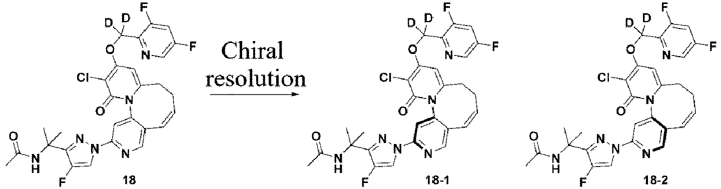

**Example 18** (86 mg, 0.147 mmol) was subjected to chiral resolution (OD column) to obtain the title product **18-1** (32 mg, R.T = 3.280 min), yield: 37.2%; **18-2** (32 mg, R.T = 4.387 min), yield: 37.2%.
**Example 18-1,** MS m/z (ESI): 587.2 [M+H] ⁺;
**Example 18-2,** MS m/z (ESI): 587.2 [M+H] ⁺;
¹H NMR (400 MHz, DMSO-*d₆*) δ 8.59 (dd, 2H), 8.48 (s, 1H), 8.18 - 8.02 (m, 2H), 7.71 (s, 1H), 6.78 (s, 1H), 6.44 (d, 1H), 5.99 - 5.91 (m, 1H), 2.78 - 2.65 (m, 2H), 2.57 (dd, 2H), 1.79 (s, 3H), 1.58 (d, 6H).

### HPLC chiral resolution conditions:

| | |
|---|---|
| Instrument | Waters HPLC |
| Column type | DAICEL CHIRALCEL^{®}OD 20 mm I.D × 250 mm 5.0 µm |
| Column pressure | 900 psi |
| Mobile phase | Hexane:EtOH:MeOH (+0.1% DEA) = 70:15:15 |
| Flow rate | 15 mL/min |
| Detection wavelength | 254 nm |
| Column temperature | R.T. |

### HPLC chiral analysis method:

| | |
|---|---|
| Instrument | Thermo Ultimate 3000 |
| Column type | DAICEL CHIRALCEL^{®}OD 4.6 mm I.D × 150 mm 5.0 µm |
| Column pressure | 55 bar |
| Mobile phase | Hexane:EtOH:MeOH (+0.1% DEA) = 70:15:15 |
| Flow rate | 1 mL/min |
| Detection wavelength | 254 nm |
| Column temperature | 35°C |

### Example 19

### (Z)-11-chloro-10-((3,5-difluoropyridin-2-yl)methoxy-d2)-2-(3-(2-hydroxypropan-2-yl)-2-carbonylpyridin-1(2H)-yl)-7,8-dihydro-12H-dipyrido[1,2-a:3',4'-g]azocin-12-one

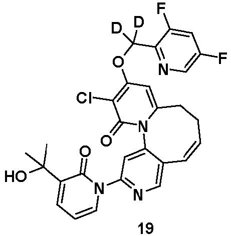

Referring to the synthesis method of **Example 15,** the target product (Z)-11-chloro-10-((3,5-difluoropyridin-2-yl)methoxy-d2)-2-(3-(2-hydroxypropan-2-yl)-2-carbonylpyridin-1(2H)-yl)-7,8-dihydro-12H-dipyrido[1,2-a:3',4'-g]azocin-12-one **19** was synthesized.

MS m/z (ESI): 555.2 [M+H]⁺.

### Resolution of Example 19

### (S,Z)-11-chloro-10-((3,5-difluoropyridin-2-yl)methoxy-d2)-2-(3-(2-hydroxypropan-2-yl)-2-carbonylpyridin-1(2H)-yl)-7,8-dihydro-12H-dipyrido[1,2-a:3',4'-g]azocin-12-one and (R,Z)-11-chloro-10-((3,5-difluoropyridin-2-yl)methoxy-d2)-2-(3-(2-hydroxypropan-2-yl)-2-carbonylpyridin-1(2H)-yl)-7,8-dihydro-12H-dipyrido[1,2-a:3',4'-g]azocin-12-one

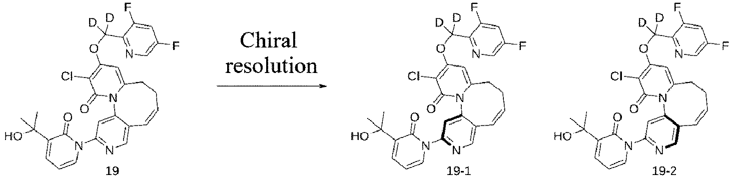

**Example 19** (76 mg, 0.137 mmol) was subjected to chiral resolution (IB column) to obtain the title product **19-1** (33 mg, R.T = 5.147 min), yield: 43.4%; **19-2** (27 mg, R.T = 7.360 min), yield: 35.5%.

**Example 19-1,** MS m/z (ESI): 555.2 [M+H] ⁺;
¹H NMR (400 MHz, DMSO-*d₆*) δ 8.64 (s, 1H), 8.60 (d, 1H), 8.09 (td, 1H), 7.87 (dd, 1H), 7.78 (s, 1H), 7.70 (dd, 1H), 6.80 (s, 1H), 6.53 - 6.38 (m, 2H), 6.06 - 5.94 (m, 1H), 5.20 (s, 1H), 2.78 (d, 2H), 2.59 - 2.51 (m, 2H), 1.46 (d, 6H).

**Example 19-2,** MS m/z (ESI): 555.2 [M+H] ⁺;
¹H NMR (400 MHz, DMSO-*d₆*) δ 8.64 (s, 1H), 8.60 (d, 1H), 8.09 (td, 1H), 7.87 (dd, 1H), 7.78 (s, 1H), 7.70 (dd, 1H), 6.80 (s, 1H), 6.53 - 6.38 (m, 2H), 6.06 - 5.94 (m, 1H), 5.20 (s, 1H), 2.78 (d, 2H), 2.59 - 2.51 (m, 2H), 1.46 (d, 6H).

### HPLC chiral resolution conditions:

| | |
|---|---|
| Instrument | HPLC (Waters) |
| Column type | DAICEL CHIRALPAK^{®}IB 270 * 30 mm 10.0 µm |
| Column pressure | 800 psi |
| Mobile phase | Hexane : EtOH : MeOH (+0.1% DEA) = 70:15:15 |
| Flow rate | 15 mL/min |
| Detection wavelength | UV 214 nm |
| Column temperature | 25°C |

### HPLC chiral analysis method:

| | |
|---|---|
| Instrument | HPLC (Waters) |
| Column type | DAICEL CHIRALPAK^{®}IB N-5 150 * 4.6 mm 5.0 µm |
| Column pressure | 35 bar |
| Mobile phase | Hexane : EtOH : MeOH (+0.1% DEA) = 70:15:15 |
| Flow rate | 1 mL/min |
| Detection wavelength | 214 nm |
| Column temperature | 35°C |

### Example 56

### 11-Chloro-10-((3,5-difluoropyridin-2-yl)methoxy-d2)-1-fluoro-2-(3-(2-hydroxypropan-2-yl)-2-carbonylpyridin-1(2H)-yl)-5,6,7,8-tetrahydro-12H-dipyrido[1,2-a:3',4'-g]azocin-12-one

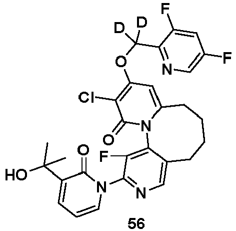

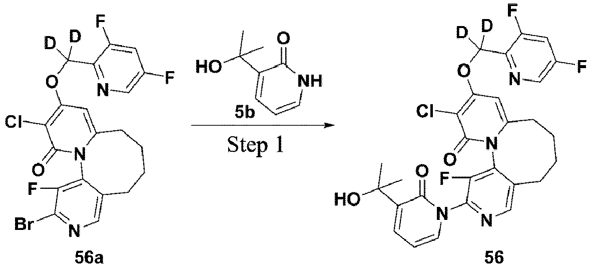

### Step 1

**56a** (150 mg, 0.298 mmol; for the preparation method therefor, reference could be made to the synthesis of 6h in Example 6) was dissolved in dimethyl sulfoxide (1.5 mL), **5b** (137 mg, 0.895 mmol), cesium carbonate (292 mg, 0.895 mmol), ethyl 2-cyclohexanonecarboxylate (51 mg, 0.298 mmol), and cuprous bromide (64 mg, 0.447 mmol) were added, and the reaction was heated to 80°C under nitrogen protection and stirred for 16 hours. The reaction liquid was cooled to room temperature and filtered, the filtrate was diluted with ethyl acetate (30 mL), then washed with water (30 mL) and a saturated sodium chloride solution (30 mL) in sequence, dried, and concentrated, and the residue was purified by reversed-phase chromatographic column (eluent system C) to obtain 11-chloro-10-((3,5-difluoropyridin-2-yl)methoxy-d2)-1-fluoro-2-(3-(2-hydroxypropan-2-yl)-2-carbonylpyridin-1(2H)-yl)-5,6,7,8-tetrahydro-12H-dipyrido[1,2-a:3',4'-g]azocin-12-one **56** (30 mg), yield: 17.5 %.
MS m/z (ESI): 575.1 [M+H] ⁺;

### Resolution of Example 56

### (R)-11-chloro-10-((3,5-difluoropyridin-2-yl)methoxy-d2)-1-fluoro-2-(3-(2-hydroxypropan-2-yl)-2-carbonylpyridin-1(2H)-yl)-5,6,7,8-tetrahydro-12H-dipyrido[1,2-a:3',4'-g]azocin-12-one and (S)-11-chloro-10-((3,5-difluoropyridin-2-yl)methoxy-d2)-1-fluoro-2-(3-(2-hydroxypropan-2-yl)-2-carbonylpyridin-1(2H)-yl)-5,6,7,8-tetrahydro-12H-dipyrido[1,2-a:3',4'-g]azocin-12-one

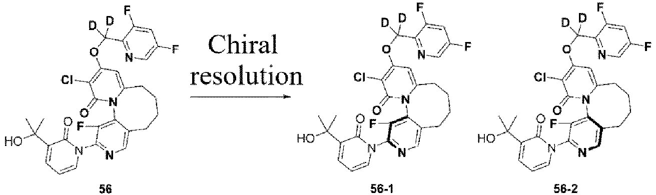

**Example 56** (30 mg, 0.066 mmol) was subjected to chiral resolution (OD column) to obtain **56-1** (13.5 mg, R.T = 3.543 min), yield: 45.0%; **56-2** (13.2 mg, R.T = 5.707 min), yield: 44.0%.

**Example 56-1:** MS m/z (ESI): 575.1 [M+H] ⁺;
¹H NMR (400 MHz, DMSO-*d*₆) δ 8.63 (d, 1H), 8.59 (d, 1H), 8.12 - 8.06 (m, 1H), 7.73 (d, 1H), 7.69 - 7.65 (m, 1H), 6.87 (s, 1H), 6.48 - 6.40 (m, 1H), 5.18 (s, 1H), 3.09 - 3.03 (m, 1H), 2.94 - 2.83 (m, 1H), 2.22 - 2.03 (m, 4H), 1.61 - 1.54 (m, 1H), 1.46 - 1.34 (m, 7H).

**Example 56-2:** MS m/z (ESI): 575.1 [M+H] ⁺;
¹H NMR (400 MHz, DMSO-*d*₆) δ 8.63 (d, 1H), 8.59 (d, 1H), 8.12 - 8.06 (m, 1H), 7.73 (d, 1H), 7.69 - 7.65 (m, 1H), 6.87 (s, 1H), 6.48 - 6.40 (m, 1H), 5.18 (s, 1H), 3.09 - 3.03 (m, 1H), 2.94 - 2.83 (m, 1H), 2.22 - 2.03 (m, 4H), 1.61 - 1.54 (m, 1H), 1.46 - 1.34 (m, 7H).

### HPLC chiral resolution conditions:

| | |
|---|---|
| Instrument | Waters HPLC |
| Column type | DAICEL CHIRALCEL^{®}OD 20 mm I.D × 250 mm 5.0 µm |
| Column pressure | 900 psi |
| Mobile phase | Hexane : EtOH (+0.1% DEA) = 70:30 |
| Flow rate | 15 mL/min |
| Detection wavelength | 214 nm |
| Column temperature | R.T. |

### HPLC chiral analysis method:

| | |
|---|---|
| Instrument | Thermo Ultimate 3000 |
| Column type | DAICEL CHIRALCEL^{®}OD 4.6 mm I.D × 150 mm 5.0 µm |
| Column pressure | 55 bar |
| Mobile phase | Hexane : EtOH (+0.1% DEA) = 70:30 |
| Flow rate | 1 mL/min |
| Detection wavelength | 214 nm |
| Column temperature | 35°C |

### Example 59

### (Z)-11-chloro-10-((3,5-difluoropyridin-2-yl)methoxy-d2)-1-fluoro-2-(2-(2-hydroxypropan-2-yl)pyrimidin-4-yl)-7,8-dihydro-12H-dipyrido[1,2-a:3',4'-g]azocin-12-one

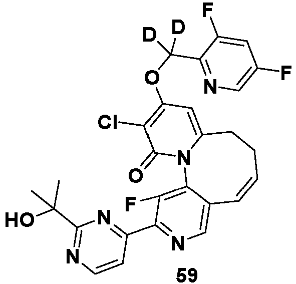

Referring to the synthesis method for **Example 9,** the target product (Z)-11-chloro-10-((3,5-difluoropyridin-2-yl)methoxy-d2)-1-fluoro-2-(2-(2-hydroxypropan-2-yl)pyrimidin-4-yl)-7,8-dihydro-12H-dipyrido[1,2-a:3',4'-g]azocin-12-one **59** was synthesized.

MS m/z (ESI): 558.1 [M+H] ⁺.

¹H NMR (400 MHz, DMSO-*d₆*) δ 9.02 (d, 1H), 8.68 (s, 1H), 8.60 (d, 1H), 8.09 (ddd, 1H), 8.03 (d, 1H), 6.87 (s, 1H), 6.56 (d, 1H), 6.09 (ddd, 1H), 5.07 (s, 1H), 2.80 (d, 2H), 2.72 - 2.52 (m, 2H), 1.59 - 1.44 (m, 6H).

### Example 60

### (Z)-N-(2-(4-(11-chloro-10-((3,5-difluoropyridin-2-yl)methoxy-d2)-1-fluoro-12-carbonyl-8,12-dihydro-7H-dipyrido[1,2-a:3',4'-g]azocin-2-yl)pyrimidin-2-yl)propan-2-yl)acetamide

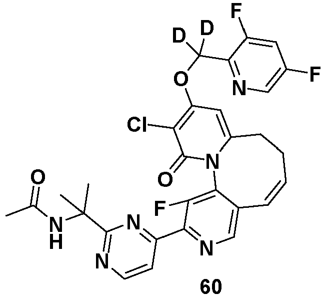

Referring to the synthesis method of **Example 7,** the target product (Z)-N-(2-(4-(11-chloro-10-((3,5-difluoropyridin-2-yl)methoxy-d2)-1-fluoro-12-carbonyl-8,12-dihydro-7H-dipyrido[1,2-a:3',4'-g]azocin-2-yl)pyrimidin-2-yl)propan-2-yl)acetamide **60** was synthesized.

MS m/z (ESI): 599.2 [M+H] ⁺.

¹H NMR (400 MHz, DMSO-*d₆*) δ 8.93 (d, 1H), 8.66 (s, 1H), 8.60 (d, 1H), 8.21 (s, 1H), 8.09 (ddd, 1H), 7.93 (d, 1H), 6.86 (s, 1H), 6.58 - 6.51 (m, 1H), 6.14 - 6.02 (m, 1H), 2.87 - 2.58 (m, 4H), 1.76 (s, 3H), 1.59 (s, 6H).

### Example 61

### (Z)-11-chloro-10-((3,5-difluoropyridin-2-yl)methoxy-d2)-1-fluoro-2-(3-(2-hydroxypropan-2-yl)-1H-pyrazol-1-yl)-7,8-dihydro-12H-dipyrido[1,2-a:3',4'-g]azocin-12-one

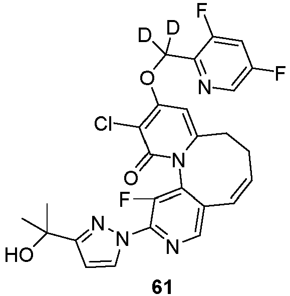

Referring to the synthesis method for **Example 6,** the target product (*Z*)-11-chloro-10-((3,5-difluoropyridin-2-yl)methoxy-*d*2)-1-fluoro-2-(3-(2-hydroxypropan-2-yl)-1*H*-pyrazol-1-yl)-7,8-dihydro-12*H*-dipyrido[1,2-*a*:3',4'-*g*]azocin-12-one **61** was synthesized.

MS m/z (ESI): 546.2 [M+H] ⁺;
¹H NMR (400 MHz, DMSO-*d*₆) δ 8.60 (d, 1H), 8.40 (s, 1H), 8.35 (d, 1H), 8.14-8.06 (m, 1H), 6.86 (s, 1H), 6.59 (d, 1H), 6.50 (d, 1H), 6.08-5.95 (m, 1H), 5.14 (s, 1H), 2.84-2.68 (m, 3H), 2.57-2.52 (m, 1H), 1.46 (d, 6H).

### Example 62

### (Z)-N-(2-(1-(11-chloro-10-((3,5-difluoropyridin-2-yl)methoxy-d2)-1-fluoro-12-carbonyl-8,12-dihydro-7H-dipyrido[1,2-a:3',4'-g]azocin-2-yl)-4-fluoro-1H-pyrazol-3-yl)propan-2-yl)acetamide

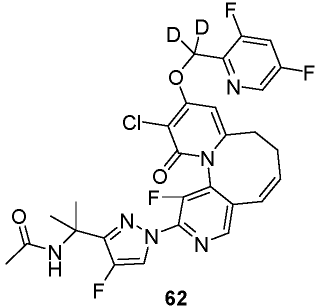

Referring to the synthesis method of **Example 6,** the target product (*Z*)-*N*-(2-(1-(11-chloro-10-((3,5-difluoropyridin-2-yl)methoxy-d2)-1-fluoro-12-carbonyl-8,12-dihydro-7*H-*dipyrido[1,2-*a*:3',4'-g]azocin-2-yl)-4-fluoro-1*H*-pyrazol-3-yl)propan-2-yl)acetamide **62** was synthesized.

MS m/z (ESI): 605.2 [M+H] ⁺;
¹H NMR (400 MHz, CDCl₃) δ 8.39 (d, 1H), 8.24 (s, 1H), 8.18 (d, 1H), 7.37-7.29 (m, 1H), 6.57 (s, 1H), 6.46-6.36 (m, 2H), 6.00-5.89 (m, 1H), 2.89-2.78 (m, 1H), 2.76-2.67 (m, 2H), 2.57-2.42 (m, 1H), 2.00 (s, 3H), 1.79 (s, 6H).

| Examples could also be obtained by referring to the above synthesis method: | | |
|---|---|---|
| Example | Structure | MS m/z (ESI) |
| 20 | 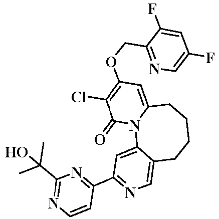 | 540.2 [M+H] ⁺ |
| 21 | 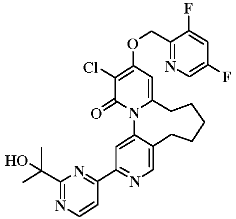 | 554.2 [M+H] ⁺ |
| 22 | 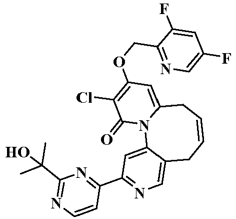 | 538.1 [M+H] ⁺ |
| 23 | 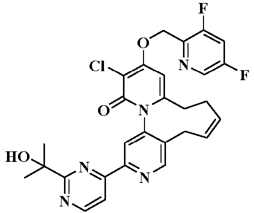 | 552.2 [M+H] ⁺ |
| 24 | 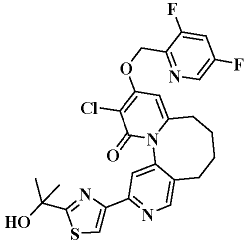 | 545.1 [M+H] ⁺ |
| 25 | 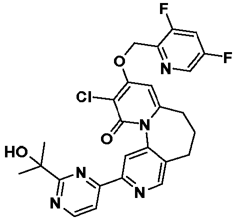 | 526.1 [M+H] ⁺ |
| 26 | 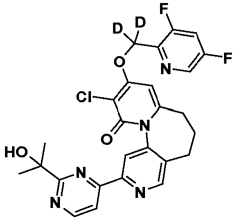 | 528.2 [M+H] ⁺ |
| 27 | 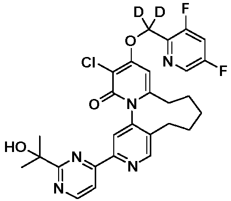 | 556.2 [M+H] ⁺ |
| 28 | 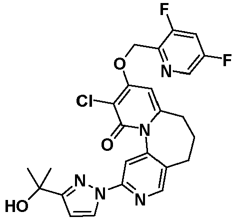 | 514.1 [M+H] ⁺ |
| 29 | 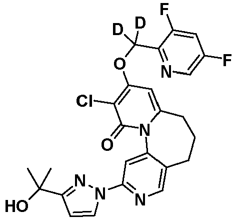 | 516.2 [M+H] ⁺ |
| 30 | 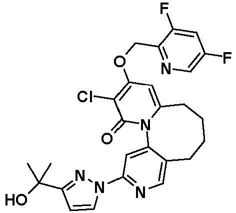 | 528.2 [M+H] ⁺ |
| 31 | 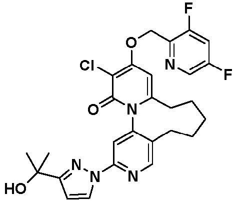 | 542.2 [M+H] ⁺ |
| 32 | 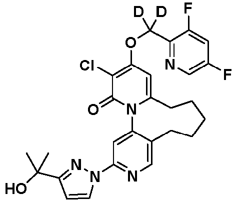 | 544.2 [M+H] ⁺ |
| 33 | 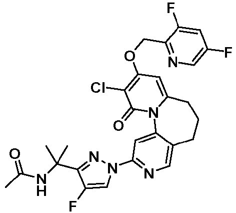 | 573.2 [M+H] ⁺ |
| 34 | 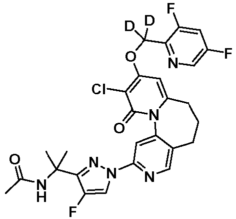 | 575.1 [M+H] ⁺ |
| 35 | 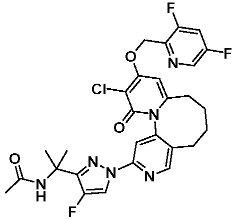 | 587.2 [M+H] ⁺ |
| 36 | 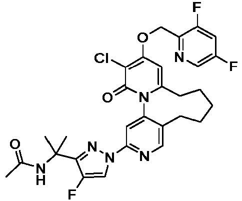 | 601.2 [M+H] ⁺ |
| 37 | 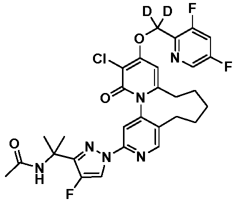 | 603.2 [M+H] ⁺ |
| 38 | 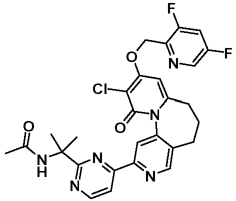 | 567.2 [M+H] ⁺ |
| 39 | 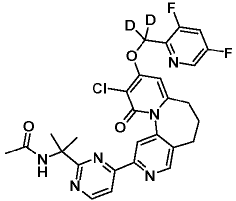 | 569.2 [M+H] ⁺ |
| 40 | 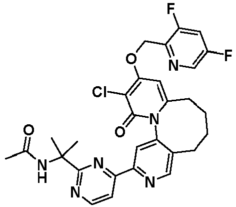 | 581.2 [M+H] ⁺ |
| 41 | 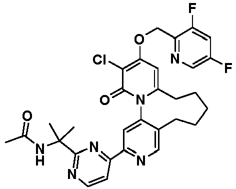 | 595.2 [M+H] ⁺ |
| 42 | 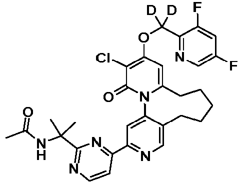 | 597.2 [M+H] ⁺ |
| 43 | 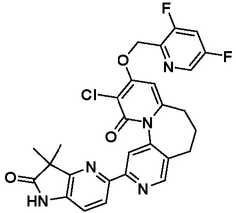 | 550.1 [M+H] ⁺ |
| 44 | 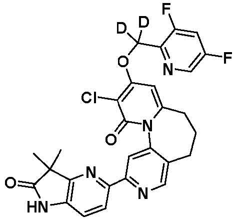 | 552.2 [M+H] ⁺ |
| 45 | 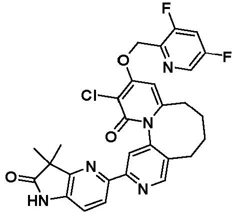 | 564.2 [M+H] ⁺ |
| 46 | 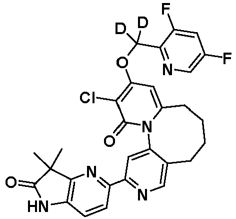 | 566.2 [M+H] ⁺ |
| 47 | 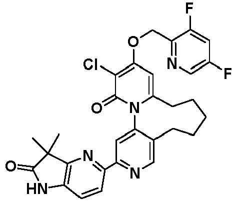 | 558.2 [M+H] ⁺ |
| 48 | 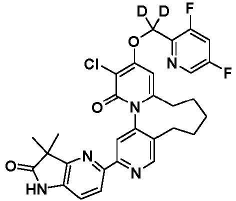 | 580.2 [M+H] ⁺ |
| 49 | 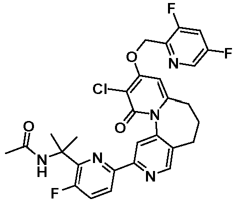 | 584.2 [M+H] ⁺ |
| 50 | 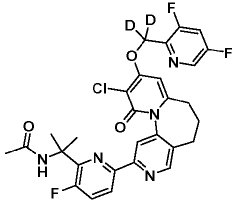 | 586.2 [M+H] ⁺ |
| 51 | 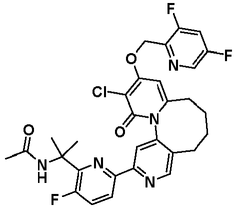 | 598.2 [M+H] ⁺ |
| 52 | 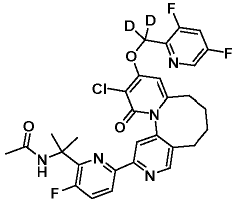 | 600.2 [M+H] ⁺ |
| 53 | 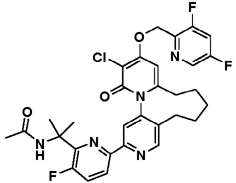 | 612.2 [M+H] ⁺ |
| 54 | 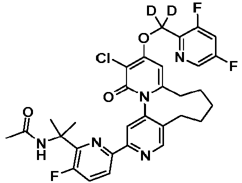 | 614.2 [M+H] ⁺ |
| 55 | 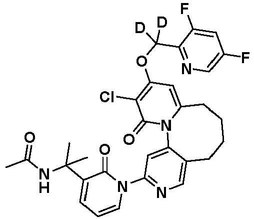 | 598.2 [M+H] ⁺ |
| | | |
| 57 | 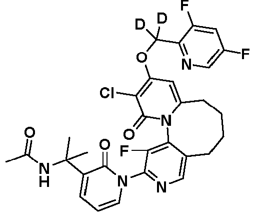 | 616.2 [M+H] ⁺ |
| 58 | 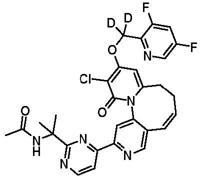 | 581.2 [M+H] ⁺ |
| 63 | 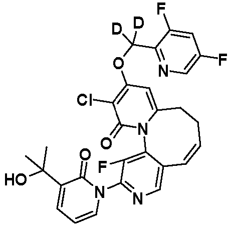 | 573.1 [M+H] ⁺ |
| 64 | 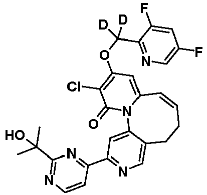 | 540.2 [M+H] ⁺ |
| 65 | 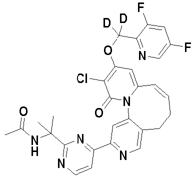 | 581.2 [M+H] ⁺ |
| 66 | 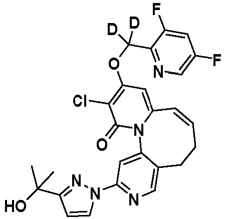 | 528.2 [M+H] ⁺ |
| 67 | 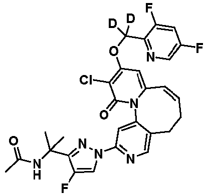 | 587.2 [M+H] ⁺ |
| 68 | 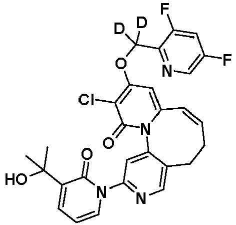 | 555.2 [M+H] ⁺ |
| 69 | 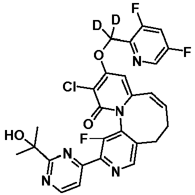 | 558.1 [M+H] ⁺ |
| 70 | 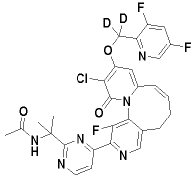 | 599.2 [M+H] ⁺ |
| 71 | 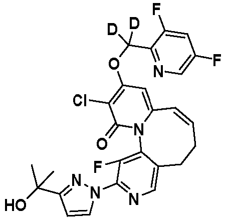 | 546.1 [M+H] ⁺ |
| 72 | 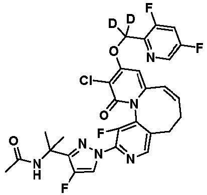 | 605.2 [M+H] ⁺ |
| 73 | 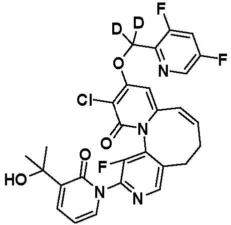 | 573.1 [M+H] ⁺ |
| 74 | 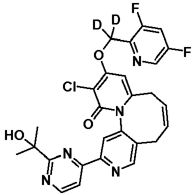 | 540.2 [M+H] ⁺ |
| 75 | 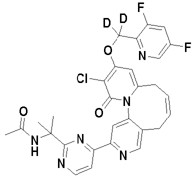 | 581.2 [M+H] ⁺ |
| 76 | 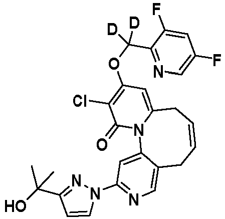 | 528.2 [M+H] ⁺ |
| 77 | 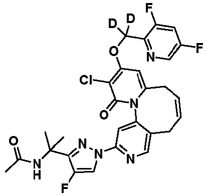 | 587.2 [M+H] ⁺ |
| 78 | 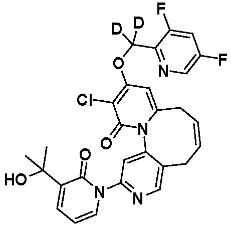 | 555.2 [M+H] ⁺ |
| 79 | 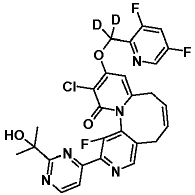 | 558.1 [M+H] ⁺ |
| 80 | 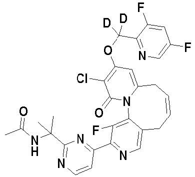 | 599.2 [M+H] ⁺ |
| 81 | 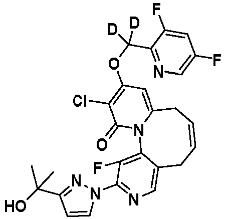 | 546.1 [M+H] ⁺ |
| 82 | 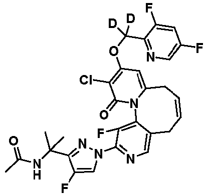 | 605.2 [M+H] ⁺ |
| 83 | 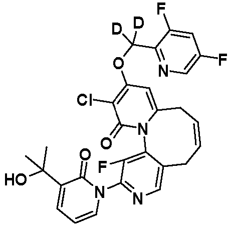 | 573.1 [M+H] ⁺ |
| 84 | 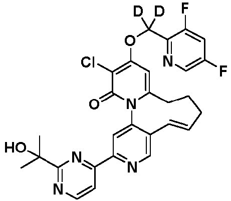 | 554.2 [M+H] ⁺ |
| 85 | 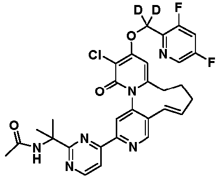 | 595.2 [M+H] ⁺ |
| 86 | 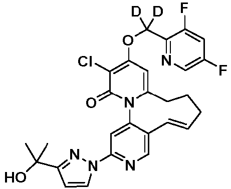 | 542.2 [M+H] ⁺ |
| 87 | 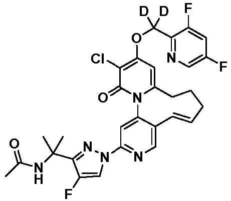 | 601.2 [M+H] ⁺ |
| 88 | 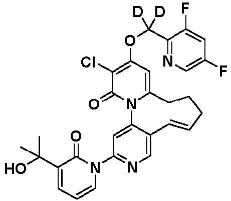 | 567.2 [M+H] ⁺ |
| 89 | 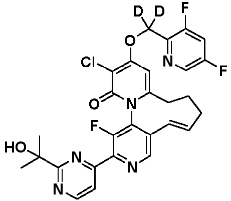 | 572.2 [M+H] ⁺ |
| 90 | 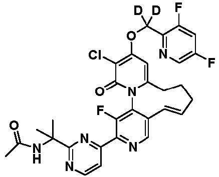 | 613.2 [M+H] ⁺ |
| 91 | 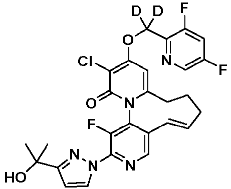 | 560.2 [M+H] ⁺ |
| 92 | 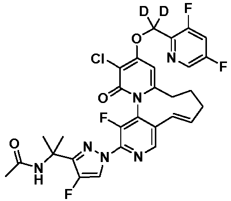 | 619.2 [M+H] ⁺ |
| 93 | 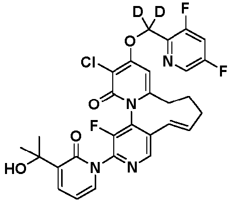 | 587.2 [M+H] ⁺ |
| 94 | 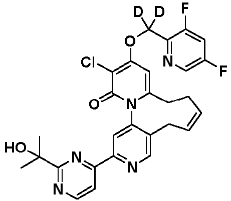 | 554.2 [M+H] ⁺ |
| 95 | 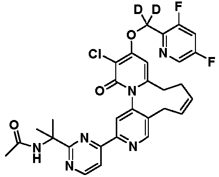 | 595.2 [M+H] ⁺ |
| 96 | 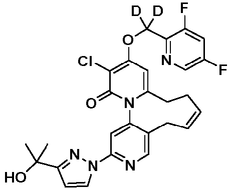 | 542.2 [M+H] ⁺ |
| 97 | 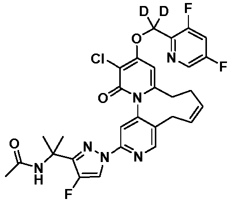 | 601.2 [M+H] ⁺ |
| 98 | 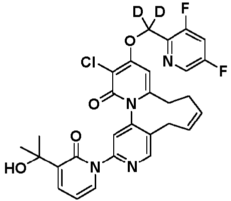 | 569.2 [M+H] ⁺ |
| 99 | 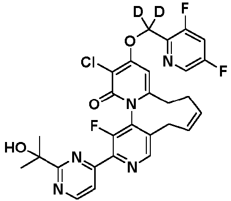 | 572.2 [M+H] ⁺ |
| 100 | 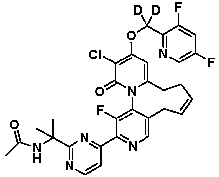 | 613.2 [M+H] ⁺ |
| 101 | 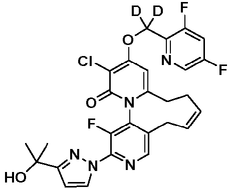 | 560.2 [M+H] ⁺ |
| 102 | 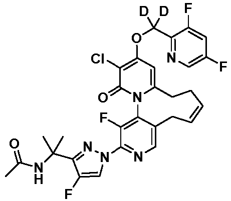 | 619.2 [M+H] ⁺ |
| 103 | 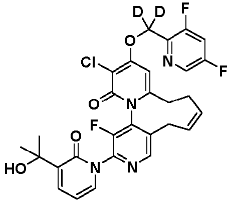 | 587.2 [M+H] ⁺ |
| 104 | 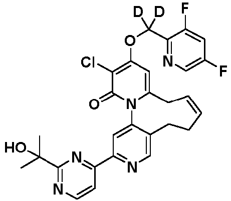 | 554.2 [M+H] ⁺ |
| 105 | 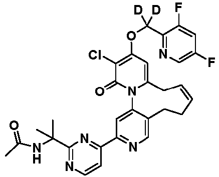 | 595.2 [M+H] ⁺ |
| 106 | 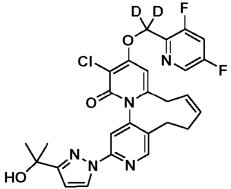 | 542.2 [M+H] ⁺ |
| 107 | 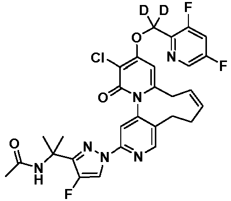 | 601.2 [M+H] ⁺ |
| 108 | 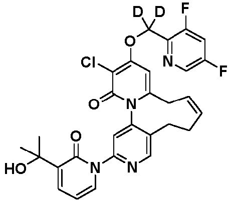 | 569.2 [M+H] ⁺ |
| 109 | 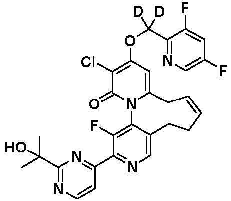 | 572.2 [M+H] ⁺ |
| 110 | 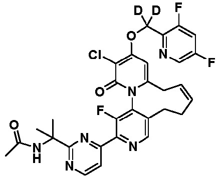 | 613.2 [M+H] ⁺ |
| 111 | 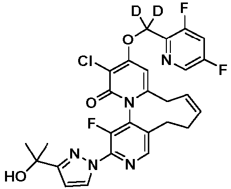 | 560.2 [M+H] ⁺ |
| 112 | 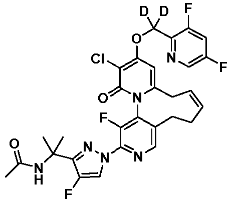 | 619.2 [M+H] ⁺ |
| 113 | 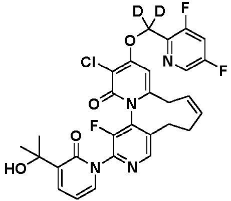 | 587.2 [M+H] ⁺ |
| 114 | 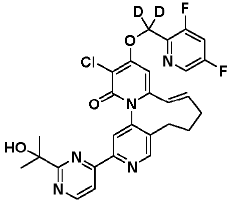 | 554.2 [M+H] ⁺ |
| 115 | 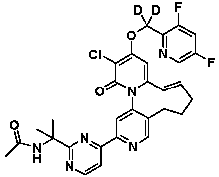 | 595.2 [M+H] ⁺ |
| 116 | 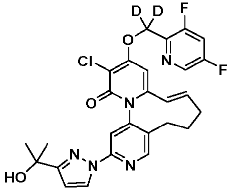 | 542.2 [M+H] ⁺ |
| 117 | 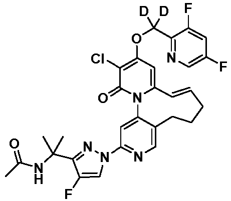 | 601.2 [M+H] ⁺ |
| 118 | 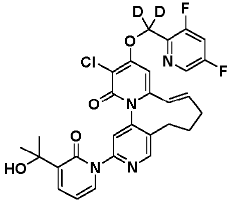 | 569.2 [M+H] ⁺ |
| 119 | 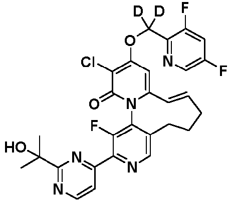 | 572.1 [M+H] ⁺ |
| 120 | 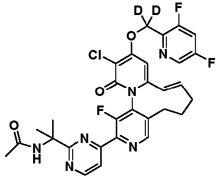 | 613.2 [M+H] ⁺ |
| 121 | 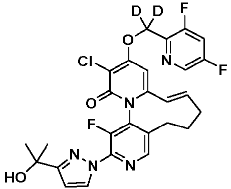 | 560.2 [M+H] ⁺ |
| 122 | 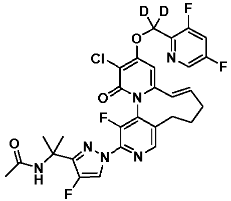 | 619.2 [M+H] ⁺ |
| 123 | 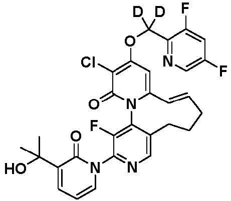 | 587.2 [M+H] ⁺ |
| 124 | 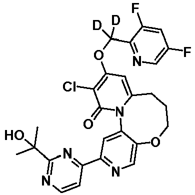 | 544.2 [M+H] ⁺ |
| 125 | 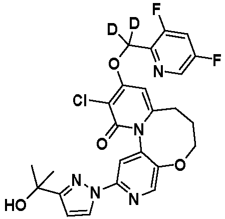 | 532.2 [M+H] ⁺ |
| 126 | 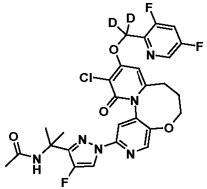 | 591.2 [M+H] ⁺ |
| 127 | 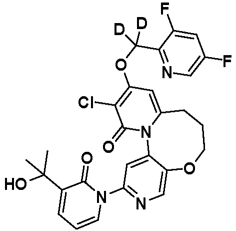 | 559.2 [M+H] ⁺ |
| 128 | 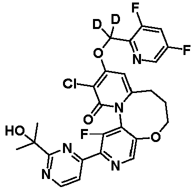 | 562.1 [M+H] ⁺ |
| 129 | 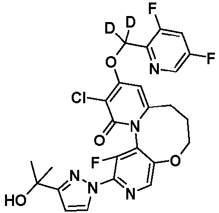 | 550.1 [M+H] ⁺ |
| 130 | 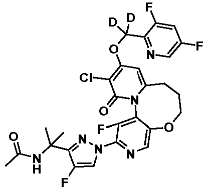 | 609.2 [M+H] ⁺ |
| 131 | 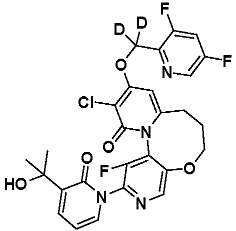 | 577.1 [M+H] ⁺ |
| 132 | 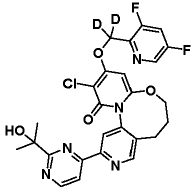 | 544.2 [M+H] ⁺ |
| 133 | 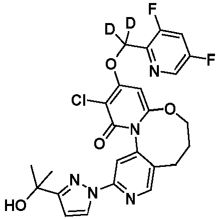 | 532.2 [M+H] ⁺ |
| 134 | 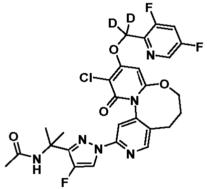 | 591.2 [M+H] ⁺ |
| 135 | 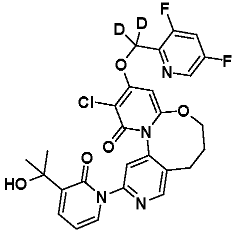 | 559.2 [M+H] ⁺ |
| 136 | 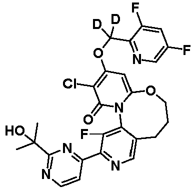 | 562.1 [M+H] ⁺ |
| 137 | 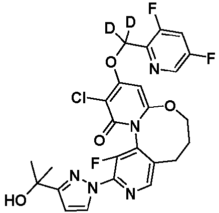 | 550.1 [M+H] ⁺ |
| 138 | 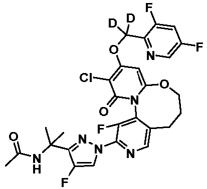 | 609.2 [M+H] ⁺ |
| 139 | 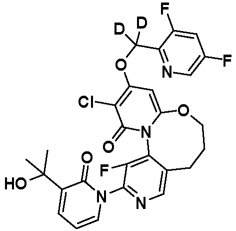 | 577.1 [M+H] ⁺ |
| 140 | 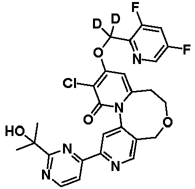 | 544.2 [M+H] ⁺ |
| 141 | 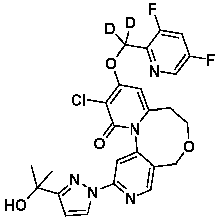 | 532.2 [M+H] ⁺ |
| 142 | 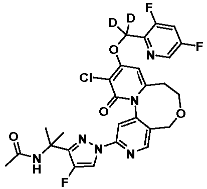 | 591.2 [M+H] ⁺ |
| 143 | 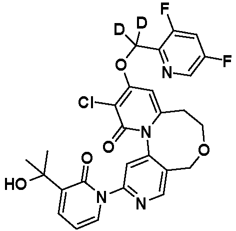 | 559.2 [M+H] ⁺ |
| 144 | 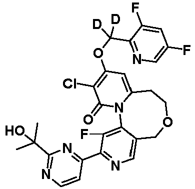 | 562.1 [M+H] ⁺ |
| 145 | 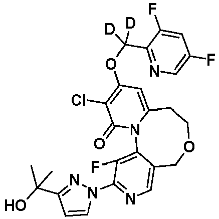 | 550.1 [M+H] ⁺ |
| 146 | 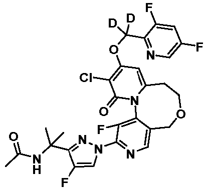 | 609.2 [M+H] ⁺ |
| 147 | 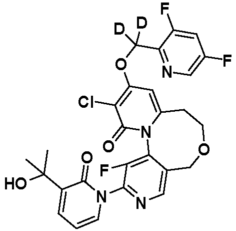 | 577.1 [M+H] ⁺ |
| 148 | 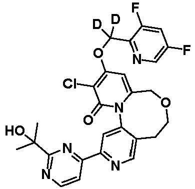 | 544.2 [M+H] ⁺ |
| 149 | 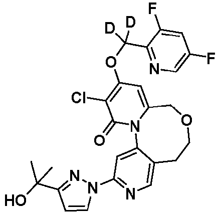 | 532.2 [M+H] ⁺ |
| 150 | 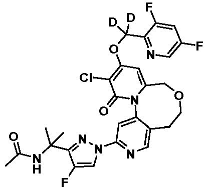 | 591.2 [M+H] ⁺ |
| 151 | 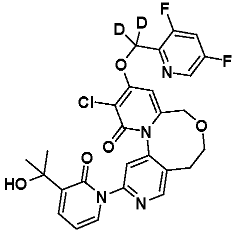 | 559.2 [M+H] ⁺ |
| 152 | 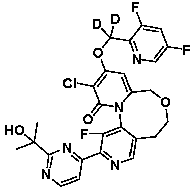 | 562.1 [M+H] ⁺ |
| 153 | 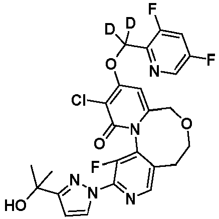 | 550.1 [M+H] ⁺ |
| 154 | 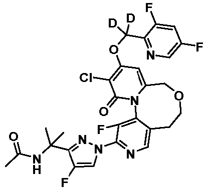 | 609.2 [M+H] ⁺ |
| 155 | 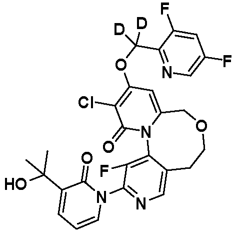 | 577.1 [M+H] ⁺ |
| 156 | 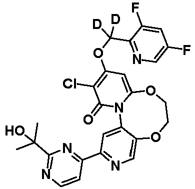 | 546.1 [M+H] ⁺ |
| 157 | 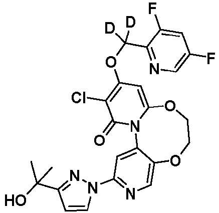 | 534.1 [M+H] ⁺ |
| 158 | 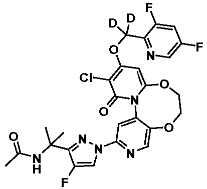 | 593.1 [M+H] ⁺ |
| 159 | 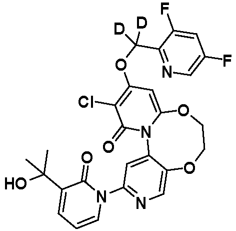 | 561.1 [M+H] ⁺ |
| 160 | 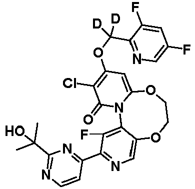 | 564.1 [M+H] ⁺ |
| 161 | 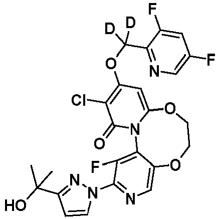 | 552.1 [M+H] ⁺ |
| 162 | 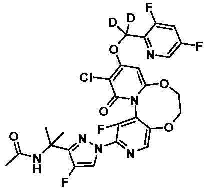 | 611.1 [M+H] ⁺ |
| 163 | 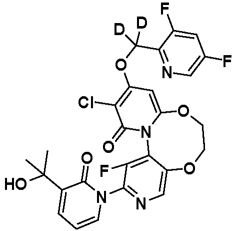 | 579.1 [M+H] ⁺ |
| 164 | 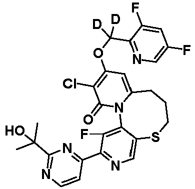 | 578.1 [M+H] ⁺ |
| 165 | 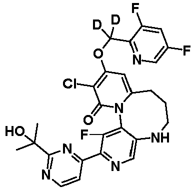 | 561.2 [M+H] ⁺ |
| 166 | 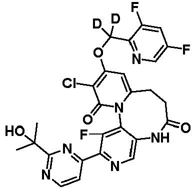 | 575.1 [M+H] ⁺ |
| 167 | 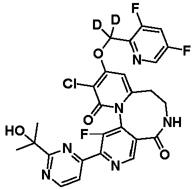 | 575.1 [M+H] ⁺ |
| 168 | 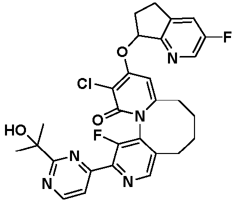 | 566.2 [M+H] ⁺ |
| 169 | 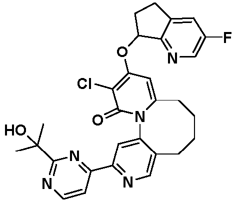 | 548.2 [M+H] ⁺ |
| 170 | 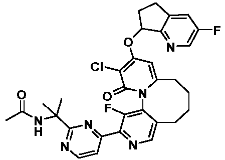 | 607.2 [M+H] ⁺ |
| 171 | 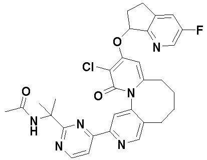 | 589.2 [M+H] ⁺ |
| 172 | 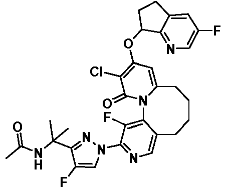 | 613.2 [M+H] ⁺ |
| 173 | 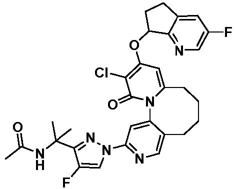 | 595.2 [M+H] ⁺ |
| 174 | 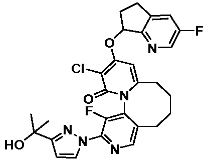 | 554.2 [M+H] ⁺ |
| 175 | 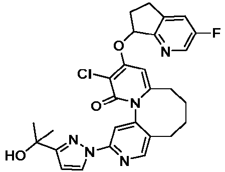 | 536.2 [M+H] ⁺ |
| 176 | 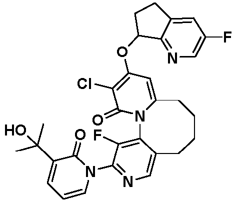 | 581.2 [M+H] ⁺ |
| 177 | 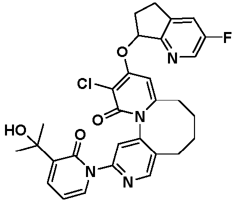 | 563.2 [M+H] ⁺ |
| 178 | 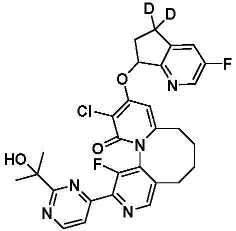 | 568.2 [M+H] ⁺ |
| 179 | 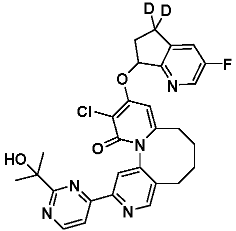 | 550.2 [M+H] ⁺ |
| 180 | 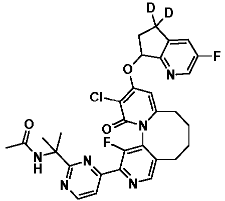 | 609.2 [M+H] ⁺ |
| 181 | 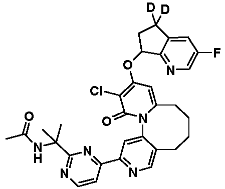 | 591.2 [M+H] ⁺ |
| 182 | 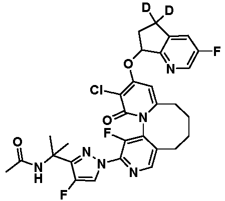 | 615.2 [M+H] ⁺ |
| 183 | 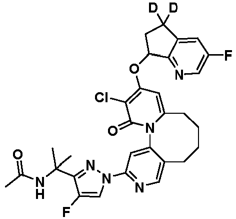 | 597.2 [M+H] ⁺ |
| 184 | 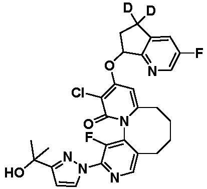 | 556.2 [M+H] ⁺ |
| 185 | 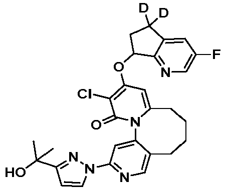 | 538.2 [M+H] ⁺ |
| 186 | 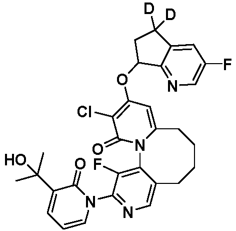 | 583.2 [M+H] ⁺ |
| 187 | 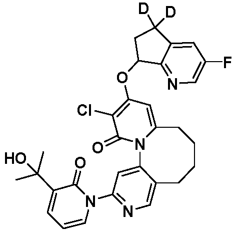 | 565.2 [M+H] ⁺ |
| 188 | 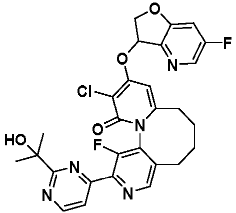 | 568.2 [M+H] ⁺ |
| 189 | 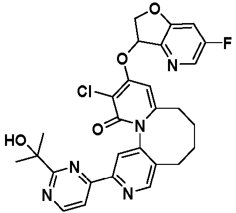 | 550.2 [M+H] ⁺ |
| 190 | 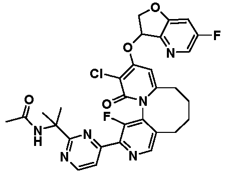 | 609.2 [M+H] ⁺ |
| 191 | 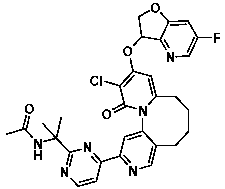 | 591.2 [M+H] ⁺ |
| 192 | 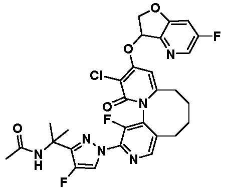 | 615.2 [M+H] ⁺ |
| 193 | 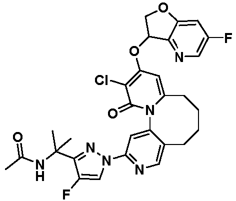 | 597.2 [M+H] ⁺ |
| 194 | 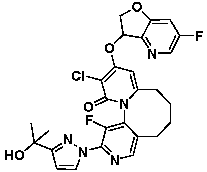 | 556.2 [M+H] ⁺ |
| 195 | 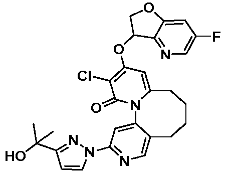 | 538.2 [M+H] ⁺ |
| 196 | 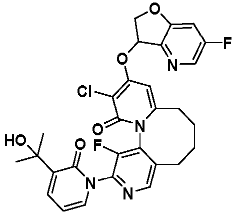 | 583.2 [M+H] ⁺ |
| 197 | 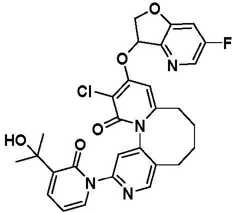 | 565.2 [M+H] ⁺ |
| 198 | 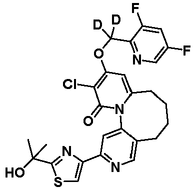 | 547.1 [M+H] ⁺ |
| 199 | 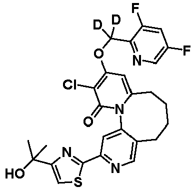 | 547.1 [M+H] ⁺ |
| 200 | 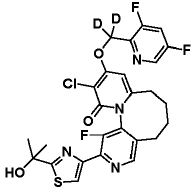 | 565.1 [M+H] ⁺ |
| 201 | 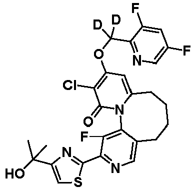 | 565.1 [M+H] ⁺ |
| 202 | 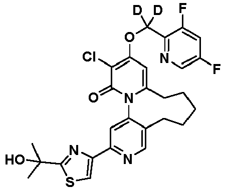 | 561.1 [M+H] ⁺ |
| 203 | 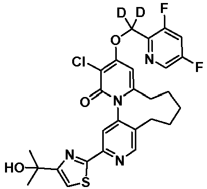 | 561.1 [M+H] ⁺ |
| 204 | 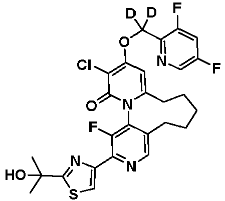 | 579.1 [M+H] ⁺ |
| 205 | 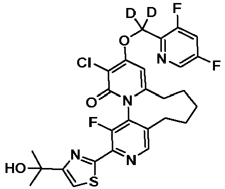 | 579.1 [M+H] ⁺ |
| 206 | 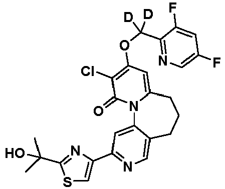 | 533.1 [M+H] ⁺ |
| 207 | 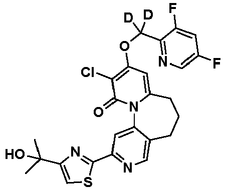 | 533.1 [M+H] ⁺ |
| 208 | 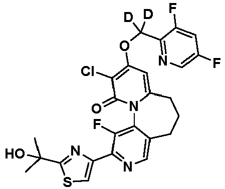 | 551.1 [M+H] ⁺ |
| 209 | 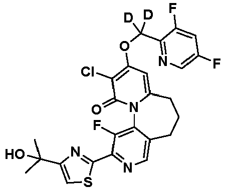 | 551.1 [M+H] ⁺ |
| 210 | 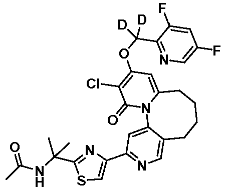 | 588.2 [M+H] ⁺ |
| 211 | 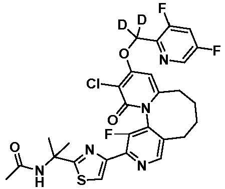 | 606.1 [M+H] ⁺ |
| 212 | 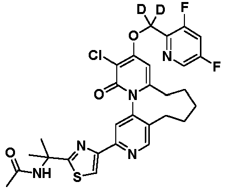 | 602.2 [M+H] ⁺ |
| 213 | 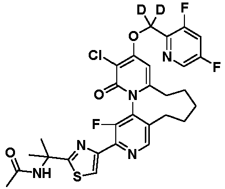 | 620.2 [M+H] ⁺ |
| 214 | 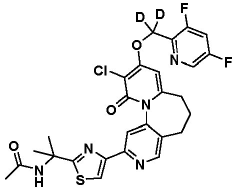 | 573.1 [M+H] ⁺ |
| 215 | 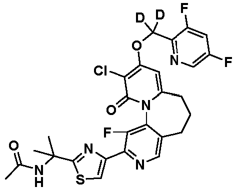 | 592.1 [M+H] ⁺ |

### Test and evaluation of examples

The present invention will be further described and explained below in conjunction with test examples, but these examples are not meant to limit the scope of the present invention.

### Test Example 1. Determination of inhibitory effect of the compound of the present invention on p38α/MK2 enzyme activity

### 1. Experimental purpose:

The purpose of this test example is to determine the ability of the compound to inhibit p38α/MK2 enzyme activity.

### 2. Experimental instruments and reagents:

### 2.1 Instruments:

| **Name of instrument** | **Manufacturer** | **Specification and model** |
|---|---|---|
| EnVision | PerkinElmer | ENVISION 2015 |

### 2.2 Reagents:

| **Reagents and materials** | **Manufacturer** | **Item No.** |
|---|---|---|
| Active p38 alpha Protein | R & D systems | 5477-KS-010 |
| Inactive MK2 | Thermo | PV3316 |
| ULight-CREBtide | Perkinelmer | TRF0107 |
| ATP Solution | Invitrogen | PV3227 |
| DMSO | Sigma | D2650 |
| HEPES(1M) | Gibco | 15630-080 |
| MgCl₂(1M) | Invitrogen | AM9530G |
| Tween 20 | Sigma | P9416 |
| EGTA | Alfa Aesar | J60767 |
| DTT(1M) | Sigma | 43816 |
| Europium-anti-phospho-CREB | PerkinElmer | TRF0200 |
| EDTA(0.5M) | Invitrogen | AM9260G |
| 10X LANCE detection buffer | PerkinElmer | CR97-100 |
| 384-well plate | Perkinelmer | 6007299 |
| 96 V-bottom plate (PS) | Axygen | WIPP02280 |

### 3. Experimental method:

Experimental method for determining the p38/MK2 enzyme activity: 0.06 nM p38α (Active p38 alpha Protein), 1.2 nM MK2 (Inactive MK2), and compounds diluted in a certain concentration gradient were incubated in 1× enzyme reaction buffer (50 mM HEPES pH 7.5, 1 mM EGTA, 10 mM MgCl₂, 2 mM DTT, 0.01% Tween-20) for 1 hour. 50 nM ULight-CREBtide and 25 µM ATP (ATP Solution) were added, and the mixture was reacted at room temperature for 1.5 hours. An equal volume of a detection and termination mixed solution (1×LANCE detection buffer containing 20 mM EDTA and 2 nM Europium-anti-phospho-CREB) was added, the mixture was mixed until uniform and centrifuged, a plate sealing film was attached, and a reaction was performed at room temperature for 1 hour. TR-FRET program of the EnVision instrument was used to read the plate, and the fluorescence value of each well was detected at 665 nm/615 nm.

Experimental method for determining the p38 enzyme activity: 3 nM p38α (Active p38 alpha Protein) and compounds diluted in a certain concentration gradient were incubated in 1× enzyme reaction buffer (50 mM HEPES pH 7.5, 1 mM EGTA, 10 mM MgCl₂, 2 mM DTT, 0.01% Tween-20) for 1 hour. 50 nM ULight-MBP and 50 µM ATP (ATP 10 mM Solution) were added, and the mixture was reacted at room temperature for 1.5 hours. An equal volume of a detection and termination mixed solution (1×LANCE detection buffer containing 20 mM EDTA and 2 nM Europium-anti-P-MBP) was added, the mixture was mixed until uniform and centrifuged, a plate sealing film was attached, and a reaction was performed at room temperature for 1 hour. TR-FRET program of the EnVision instrument was used to read the plate, and the fluorescence value of each well was detected at 665 nm/615 nm.

### 4. Experimental data processing method:

Calculation was performed on the raw data (665 nm/615 nm reading ratio) according to the following formula to obtain the inhibition rate. Inhibition rate % = [(mean value in positive control well - value in sample well) / (mean value in positive control well - mean value in negative control well)] × 100, wherein the positive control well was a reaction well without a compound and an enzyme, and the negative control well was a reaction wells without an enzyme.

Log(inhibitor) vs. response - Variable slope (four parameters) in GraphPad Prism 6 was used for fitting equation analysis of the compound concentration and the corresponding inhibition rate, and the curve was fitted to obtain the IC₅₀ value of the compound.

The fitting calculation equation was Y = Bottom + (Top - Bottom) / (1 + 10^((LogIC₅₀ - X) * HillSlope)).

### 5. Experimental results:

| **Example** | **p38/MK2 IC₅₀ (nM)** | **p38 IC₅₀ (nM)** | **p38/p38/MK2** |
|---|---|---|---|
| 1-1 | 0.71 | 77.4 | 109 |
| 2-1 | 0.42 | / | / |
| 3-1 | 0.18 | / | / |
| 5-1 | 0.79 | 525.8 | 666 |
| 6-1 | 0.10 | 15.3 | 153 |
| 7-1 | 0.34 | 49.1 | 144 |
| 8-1 | 0.25 | / | / |
| 9-1 | 0.14 | 22.9 | 164 |
| 10-1 | 0.13 | / | / |
| 11-1 | 0.28 | 96.9 | 346 |
| 13-1 | 0.12 | 16.5 | 138 |
| 14-1 | 0.05 | 5.1 | 102 |
| 15-1 | 0.2 | 53.6 | 268 |
| 16-1 | 0.49 | / | / |
| 17-1 | 0.27 | / | / |
| 18-1 | 0.12 | / | / |
| 19-1 | 0.12 | / | / |

### 6. Experimental conclusion:

The above data show that the compounds of the examples of the present invention have a stronger ability to inhibit p38α/MK2 enzyme activity and also have good selectivity.

### Test Example 2. Determination of inhibitory effect of the compound of the present invention on p-Hsp27 in U937 cells

### 1. Experimental purpose:

The purpose of this test example is to determine the ability of the compound of the present invention to inhibit p-Hsp27 in U937 cells.

### 2. Experimental instruments and reagents:

### 2.1 Instruments:

| **Name of instrument** | **Manufacturer** | **Specification and model** |
|---|---|---|
| EnVision | PerkinElmer | ENVISION 2015 |

### 2.2 Reagents:

| **Reagents and materials** | **Manufacturer** | **Item No.** |
|---|---|---|
| U937 | ATCC | CRL-1593.2 |
| Phospho-HSP27 (S78/S82) DuoSet IC ELISA | R & D systems | DYC2314-5 |
| DuoSet ELISA Ancillary Reagent Kit 2 | R & D systems | DY008 |
| Phorbol 12-myristate 13-acetate (PMA) | Abcam | Ab120297 |
| Lipopolysaccharides from *Escherichia coli* O111:B4 | Sigma-Aldrich | L4391 |

### 3. Experimental method:

U937 cells were treated with 20 ng/ml PMA for differentiation overnight, and after replacement with a fresh culture medium, the U937 cells were rested overnight. After differentiation, the U937 cell were digested and counted, and 25,000 cells per well were seeded in a 96-well plate until the cells adhered to the wall. A compound concentration gradient was prepared, the cells were pretreated with the compound for 1 hour (the highest final concentration of the compound was 1 µM, diluted in 3-fold concentration gradient), and 10 ng/ml LPS (Lipopolysaccharides from *Escherichia coli* O111:B4) was added for stimulation for 0.5 hours. After the cells were washed with PBS, the cells were lysed with a lysis solution. p-Hsp27 in the cell lysate was detected by using Phospho-HSP27 (S78/S82) DuoSet IC ELISA kit. For specific steps, reference could be made to the standard steps provided by the kit manufacturer.

### 4. Experimental data processing method:

Calculation of p-Hsp27 inhibition rate: Inhibition rate % = [(mean value of p-Hsp27 in positive control well - mean value of p-Hsp27 in sample well) / (mean value of p-Hsp27 in positive control well - mean value of p-Hsp27 in negative control well)] × 100%, wherein the positive control well was a well without a compound (DMSO-treated well), and the negative control well was a well without LPS stimulation.

Dose response curve fitting: Log(inhibitor) vs. response - Variable slope (four parameters) in GraphPad Prism 6 was used for fitting equation analysis of the compound concentration and the corresponding inhibition rate, and the curve was fitted to obtain the IC₅₀ value of the compound.

The fitting calculation equation was Y = Bottom + (Top - Bottom) / (1 + 10^((LogIC₅₀ - X) * HillSlope)).

### 5. Experimental results:

| **Example** | **U937 cell activity test** |
|---|---|
| | **p-HSP27 IC₅₀ (nM)** |
| 1-1 | 0.17 |
| 2-1 | 0.28 |
| 3-1 | 0.15 |
| 4-1 | 0.35 |
| 6-1 | 0.15 |
| 7-1 | 0.35 |
| 8-1 | 0.22 |
| 9-1 | 0.10 |
| 10-1 | 0.13 |
| 11-1 | 0.10 |
| 13-1 | 0.56 |
| 14-1 | 0.13 |
| 15-1 | 0.32 |
| 16-1 | 0.28 |

### 6. Experimental conclusion:

The above data show that the compounds of the examples of the present invention have a stronger ability to inhibit p-Hsp27 in U937 cells.

### Test Example 3. Determination of inhibitory effect of the compound of the present invention on the expression level of TNF-α in U937 cells.

### 1. Experimental purpose:

The purpose of this test example is to determine the ability of the compound of the present invention to inhibit the expression level of TNF-α in U937 cells.

### 2. Experimental instruments and reagents:

### 2.1 Instruments:

| **Name of instrument** | **Manufacturer** | **Specification and model** |
|---|---|---|
| EnVision | PerkinElmer | 2105-0020 |

### 2.2 Reagents:

| **Reagents and materials** | **Manufacturer** | **Item No.** |
|---|---|---|
| U937 | ATCC | CRL-1593.2 |
| Human TNF-alpha DuoSet ELISA | R & D systems | DY210 |
| DuoSet ELISA Ancillary Reagent Kit 2 | R & D systems | DY008 |
| Phorbol 12-myristate 13-acetate (PMA) | Abcam | Ab120297 |
| Lipopolysaccharides from *Escherichia coli* O111:B4 | Sigma-Aldrich | L4391 |

### 3. Experimental method:

U937 cells were treated with 20 ng/ml PMA for differentiation overnight, and after replacement with a fresh culture medium, the U937 cells were rested overnight. After differentiation, the U937 cells were digested and counted, and 50,000 cells per well were seeded in a 96-well plate until the cells adhered to the wall. A compound concentration gradient was prepared, the cells were pretreated with the compound for 1 hour (the highest final concentration of the compound was 1 µM, diluted in 3-fold concentration gradient), and 10 ng/ml LPS (Lipopolysaccharides from *Escherichia coli* O111:B4) was added for stimulation for 4 hours. Thereafter, the culture supernatant was collected by centrifugation and used for subsequent ELISA to detect the concentration of TNF-α in the supernatant. The concentration of TNF-α was detected according to the standard steps provided by the ELISA kit manufacturer.

### 4. Experimental data processing method:

A standard curve was plotted by a linear regression method from a TNF-α standard, and the concentration of TNF-α in a sample was derived by conversion according to the standard curve.

Calculation of inhibition rate: Inhibition rate % = [(mean value of TNF-α concentration in positive control well - mean value of TNF-α concentration in sample well) / (mean value of TNF-α concentration in positive control well - mean value of TNF-α concentration in negative control well)] × 100%, wherein the positive control well was a well without a compound (DMSO-treated well), and the negative control well was a culture medium well.

Dose response curve fitting: Log(inhibitor) vs. response - Variable slope (four parameters) in GraphPad Prism 6 was used for fitting equation analysis of the compound concentration and the corresponding inhibition rate, and the curve was fitted to obtain the IC₅₀ value of the compound. The fitting calculation equation was Y = Bottom + (Top - Bottom) / (1 + 10^((LogIC₅₀ - X) * HillSlope)).

### 5. Experimental results:

| **Example** | **U937 cell activity test** |
|---|---|
| | **TNF-α IC₅₀(nM)** |
| 1-1 | 1.20 |
| 2-1 | 0.33 |
| 3-1 | 0.18 |
| 4-1 | 1.20 |
| 6-1 | 0.16 |
| 7-1 | 0.34 |
| 8-1 | 0.19 |
| 9-1 | 0.12 |
| 10-1 | 0.10 |
| 11-1 | 0.21 |
| 13-1 | 0.51 |
| 14-1 | 0.12 |
| 15-1 | 0.18 |
| 16-1 | 0.85 |

### 6. Experimental conclusion:

The above data show that the compounds of the examples of the present invention have a stronger ability to inhibit the expression level of TNF-α in U937 cells.

### Test Example 4. Pharmacokinetic assay in SD rats

### 1. Experimental purpose:

SD rats were used as test animals to study the pharmacokinetic behavior of the compounds of the present invention in the rat body (plasma) after oral administration at a dose of 5 mg/kg or 2 mg/kg and intravenous administration at a dose of 1 mg/kg.

### 2. Experimental scheme

### 2.1 Test drugs:

Compounds of the present invention, made in house.

### 2.2 Test animals:

3 SD rats/group, male, from Shanghai Jiesijie Experimental Animal Co., Ltd., animal production license number (SCXK (Shanghai) 2013-0006 N0.311620400001794).

### 2.3 Drug preparation:

PO, 10% solutol HS15 was dissolved by ultrasound and prepared into a clear solution or homogeneous suspension.

IV, 5% DMSO + 10% Solutol HS15 + 85% PBS was dissolved by ultrasound and prepared into a clear solution.

### 2.4 Administration scheme:

3 SD rats/group, male; After fasting overnight, the rats were administered p.o. at a dose of 5 mg/kg or 2 mg/kg with a dosage volume of 10 mL/kg and i.v. at a dose 1 mg/kg with a dosage volume of 5 mL/kg, respectively.

### 2.5 Sample collection:

At 0.25, 0.5, 1, 2, 4, 6, 8, and 24 h after oral administration in the rats and at 0.083, 0.25, 0.5, 1, 2, 4, 6, 8, and 24 h after intravenous administration in the rats, 0.2 mL of blood was collected from the jugular vein, placed in an EDTA-K₂ test tube, centrifuged at 4°C at 6000 rpm for 6 min to separate plasma, which was stored at -80°C.

### 2.6 Sample treatment:

1) 100 uL of acetonitrile was added to 20 uL of plasma sample for pelleting, and it was mixed and centrifuged at 3500 × g for 5-20 min.
2) The treated supernatant solution was taken and subjected to LC/MS/MS to analyze the concentrations of the test compounds. LC/MS/MS analysis instrument: AB Sciex API 4000 Qtrap.

### 2.7 Liquid phase analysis:

• Liquid phase conditions: Shimadzu LC-20AD pump
• Chromatographic column: Waters Xbridge C18 5 µm, 4.6 X 50 mm, mobile phase: liquid A was 0.1% aqueous formic acid solution, and liquid B was methanol
• Flow rate: 1.0 mL/min
• Elution time: 0-4.0 min, the eluent was as follows:

| Time/min | Liquid A | Liquid B |
|---|---|---|
| 0.2 | 90% | 10% |
| 1.2 | 2% | 98% |
| 3.0 | 2% | 98% |
| 3.1 | 90% | 10% |
| 4.0 | Stop | |

### 3. Test results and data processing:

The main pharmacokinetic parameters were calculated using WinNonlin 6.1.

| Example | Pharmacokinetic experiment (PO administration) | | | | | |
|---|---|---|---|---|---|---|
| | Dose | Time to peak | Plasma concentration | Area under the curve | Half-life | Mean residence time |
| | mg/kg | tₘₐₓ (ng/mL) | Cₘₐₓ (ng/mL) | AUC_{0-∞} (ng/mL × h) | t_{1/2} (h) | MRT (h) |
| 6-1 | 5 | 1 | 4177 | 14213 | 2.7 | 2.9 |
| 7-1 | 2 | 1 | 2157 | 7349 | 1.4 | 2.5 |
| 9-1 | 5 | 0.5 | 3757 | 22011 | 2.5 | 3.8 |

### 4. Experimental conclusion

The pharmacokinetic determination results in the SD rats showed that the compounds of the present invention had better PK properties.

## Claims

1. A compound represented by general formula (I), or a stereoisomer or pharmaceutically acceptable salt thereof: 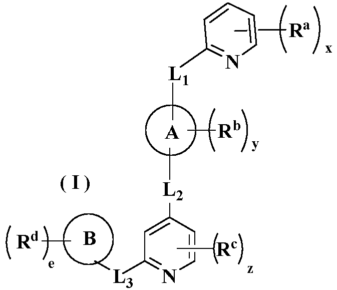 wherein:
ring A is selected from cycloalkyl, heterocyclyl, aryl, or heteroaryl;
ring B is selected from cycloalkyl, heterocyclyl, aryl, or heteroaryl;
L₁ is selected from a bond, substituted or unsubstituted alkenylene, substituted or unsubstituted alkynylene, -(CH₂)ₙ-, -(CH₂)ₙC(O)(CRₐₐR_{bb})ₙ₁-, -(CH₂)ₙC(O)NR_{cc}(CH₂)ₙ₁-, -(CH₂)ₙ(CRₐₐR_{bb})ₙ₁-, - (CRₐₐR_{bb})ₙO(CH₂)ₙ₁-, -(CH₂)ₙO(CRₐₐR_{bb})ₙ₁-, -(CRₐₐR_{bb})ₙS(CH₂)ₙ₁-, -(CH₂)ₙS(CRₐₐR_{bb})ₙ₁-, - (CRₐₐR_{bb})ₙ(CH₂)ₙ₁NR_{cc}-, -(CH₂)ₙNR_{cc}(CRₐₐR_{bb})ₙ₁-, -(CH₂)ₙNR_{cc}C(O)-, -(CH₂)ₙP(O)ₚRₐₐ-, -(CH₂)ₙS(O)ₘ-, - (CH₂)ₙS(O)ₘNR_{cc}-, and -(CH₂)ₙNR_{cc}S(O)ₘ-;
Rₐₐ, R_{bb}, and R_{cc} are each independently selected from hydrogen, deuterium, halogen, nitro, hydroxy, mercapto, cyano, amino, oxo, thio, carboxyl, alkyl, deuteroalkyl, haloalkyl, hydroxyalkyl, alkoxy, deuteroalkoxy, haloalkoxy, alkenyl, alkynyl, cycloalkyl, heterocyclyl, aryl, or
heteroaryl, wherein the amino, alkyl, deuteroalkyl, haloalkyl, hydroxyalkyl, alkoxy, deuteroalkoxy, haloalkoxy, alkenyl, alkynyl, cycloalkyl, heterocyclyl, aryl, and heteroaryl may be optionally further substituted;
or any two of Rₐₐ, R_{bb}, and R_{cc} are connected to form cycloalkyl, heterocyclyl, aryl, or heteroaryl, wherein the cycloalkyl, heterocyclyl, aryl, and heteroaryl may be optionally further substituted;
L₂ and L₃ are each independently selected from a bond, substituted or unsubstituted alkenyl, substituted or unsubstituted alkynyl, -(CH₂)ₙ₂-, -(CH₂)ₙ₂C(O)(CRₐ₁R_{b1})ₙ₃-, -(CH₂)ₙ₂C(O)NR_{c1}(CH₂)ₙ₃-, - (CH₂)ₙ₂(CRₐ₁R_{b1})ₙ₃-, -(CRₐ₁R_{b1})ₙ₂O(CH₂)ₙ₃-, -(CH₂)ₙ₂O(CRₐ₁R_{b1})ₙ₃-, -(CRₐ₁R_{b1})ₙ₂S(CH₂)ₙ₃-, - (CH₂)ₙ₂S(CRₐ₁R_{b1})ₙ₃-, -(CRₐ₁R_{b1})ₙ₂(CH₂)ₙ₃NR_{c1}-, -(CH₂)ₙ₂NR_{c1}(CRₐ₁R_{b1})ₙ₃-, -(CH₂)ₙ₂NR_{c1}C(O)-, - (CH₂)ₙ₂P(O)ₚ₁Rₐ₁-, -(CH₂)ₙ₂S(O)ₘ₁-, -(CH₂)ₙ₂S(O)ₘ₁NR_{c1}-, and -(CH₂)ₙ₂NR_{c1}S(O)ₘ₁-;
Rₐ₁, R_{b1}, and R_{c1} are each independently selected from hydrogen, deuterium, halogen, nitro, hydroxy, mercapto, cyano, amino, oxo, thio, carboxyl, alkyl, deuteroalkyl, haloalkyl, hydroxyalkyl, alkoxy, deuteroalkoxy, haloalkoxy, alkenyl, alkynyl, cycloalkyl, heterocyclyl, aryl, or
heteroaryl, wherein the amino, alkyl, deuteroalkyl, haloalkyl, hydroxyalkyl, alkoxy, deuteroalkoxy, haloalkoxy, alkenyl, alkynyl, cycloalkyl, heterocyclyl, aryl, and heteroaryl may be optionally further substituted;
each R^{a} is independently selected from hydrogen, deuterium, halogen, nitro, hydroxy, mercapto, cyano, amino, oxo, thio, carboxyl, alkyl, deuteroalkyl, haloalkyl, hydroxyalkyl, alkoxy, deuteroalkoxy, haloalkoxy, alkenyl, alkynyl, cycloalkyl, heterocyclyl, aryl, or heteroaryl, wherein
the amino, alkyl, deuteroalkyl, haloalkyl, alkoxy, haloalkoxy, hydroxyalkyl, alkenyl, alkynyl, cycloalkyl, heterocyclyl, aryl, and heteroaryl may be optionally further substituted;
each R^{b} is independently selected from hydrogen, deuterium, halogen, nitro, hydroxy, mercapto, cyano, amino, oxo, thio, carboxyl, alkyl, deuteroalkyl, haloalkyl, hydroxyalkyl, alkoxy, deuteroalkoxy, haloalkoxy, alkenyl, alkynyl, cycloalkyl, heterocyclyl, aryl, or heteroaryl, wherein
the amino, alkyl, deuteroalkyl, haloalkyl, alkoxy, haloalkoxy, hydroxyalkyl, alkenyl, alkynyl, cycloalkyl, heterocyclyl, aryl, and heteroaryl may be optionally further substituted;
each R^{c} is independently selected from hydrogen, deuterium, halogen, nitro, hydroxy, mercapto, cyano, amino, oxo, thio, carboxyl, alkyl, deuteroalkyl, haloalkyl, hydroxyalkyl, alkoxy, deuteroalkoxy, haloalkoxy, alkenyl, alkynyl, cycloalkyl, heterocyclyl, aryl, or heteroaryl, wherein
the amino, alkyl, deuteroalkyl, haloalkyl, alkoxy, haloalkoxy, hydroxyalkyl, alkenyl, alkynyl, cycloalkyl, heterocyclyl, aryl, and heteroaryl may be optionally further substituted;
R^{b} and R^{c} are connected to form cycloalkyl, heterocyclyl, aryl, or heteroaryl, wherein the cycloalkyl, heterocyclyl, aryl, and heteroaryl may be optionally further substituted;
each R^{d} is independently selected from hydrogen, deuterium, halogen, nitro, hydroxy, mercapto, cyano, amino, oxo, thio, carboxyl, alkyl, bridged cycloalkyl, deuteroalkyl, haloalkyl, hydroxyalkyl, alkoxy, deuteroalkoxy, haloalkoxy, alkenyl, alkynyl, cycloalkyl, heterocyclyl, aryl, heteroaryl, - (CRₑₑR_{ff})ₙ₄C(O)R_{gg}, -(CRₑₑR_{ff})ₙ₄NRₕₕC(O)R_{gg}, -(CRₑₑR_{ff})ₙ₄S(O)₂R_{gg}, -(CRₑₑR_{ff})ₙ₄C(O)NRₕₕR_{gg}, or - (CRₑₑR_{ff})ₙ₄S(O)₂NRₕₕR_{gg}, wherein the amino, alkyl, bridged cycloalkyl, deuteroalkyl, haloalkyl, alkoxy, haloalkoxy, hydroxyalkyl, alkenyl, alkynyl, cycloalkyl, heterocyclyl, aryl, and heteroaryl may be optionally further substituted;
Rₑₑ, R_{ff}, R_{gg}, and Rₕₕ are each independently selected from hydrogen, deuterium, halogen, nitro, hydroxy, mercapto, cyano, amino, carboxyl, C₁₋₈ alkyl, C₁₋₈ deuteroalkyl, C₁₋₈ haloalkyl, C₁₋₈ hydroxyalkyl, C₁₋₈ alkoxy, C₁₋₈ deuteroalkoxy, C₁₋₈ haloalkoxy, C₂₋₈ alkenyl, C₂₋₈ alkynyl, C₃₋₁₂ cycloalkyl, 3- to 12-membered heterocyclyl, C₆₋₁₄ aryl, or 5- to 14-membered heteroaryl;
preferably, Rₑₑ, R_{ff}, R_{gg}, and Rₕₕ are each independently selected from hydrogen, deuterium, halogen, nitro, hydroxy, mercapto, cyano, amino, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₈ cycloalkyl, 3- to 8-membered heterocyclyl, C₆₋₁₀ aryl, or 5- to 10-membered heteroaryl;
or Rₑₑ and R_{ff} are connected to form cycloalkyl, heterocyclyl, aryl, or heteroaryl, wherein the cycloalkyl, heterocyclyl, aryl, and heteroaryl may be optionally further substituted;
n4 is 0, 1, 2, 3, or 4;
preferably, each R^{d} is independently selected from hydrogen, deuterium, halogen, nitro, hydroxy, mercapto, cyano, amino, oxo, thio, carboxyl, alkyl, deuteroalkyl, haloalkyl, hydroxyalkyl, alkoxy, deuteroalkoxy, haloalkoxy, alkenyl, alkynyl, cycloalkyl, heterocyclyl, aryl, or
heteroaryl, wherein the amino, alkyl, deuteroalkyl, haloalkyl, alkoxy, haloalkoxy, hydroxyalkyl, alkenyl, alkynyl, cycloalkyl, heterocyclyl, aryl, and heteroaryl may be optionally further substituted;
or any two adjacent or non-adjacent R^{d} are connected to form cycloalkyl, heterocyclyl, aryl, or heteroaryl, wherein the cycloalkyl, heterocyclyl, aryl, and heteroaryl may be optionally further substituted;
or L₃ and R^{d} are connected to form cycloalkyl, heterocyclyl, aryl, or heteroaryl, wherein the cycloalkyl, heterocyclyl, aryl, and heteroaryl may be optionally further substituted;
or R^{c} and R^{d} are connected to form cycloalkyl, heterocyclyl, aryl, or heteroaryl, wherein the cycloalkyl, heterocyclyl, aryl, and heteroaryl may be optionally further substituted;
x is an integer of 0-6;
y is an integer of 0-6;
z is an integer of 0-6;
e is an integer of 0-6;
n is 0, 1, 2, or 3;
n1 is 0, 1, 2, or 3;
n2 is 0, 1, 2, or 3;
n3 is 0, 1, 2, or 3;
m is 0, 1, 2, or 3;
p is 0, 1, 2, or 3;
m1 is 0, 1, 2, or 3; and
p1 is 0, 1, 2, or 3.

2. The compound, or the stereoisomer or pharmaceutically acceptable salt thereof according to claim 1, wherein the compound is further as represented by general formula (I'-1): 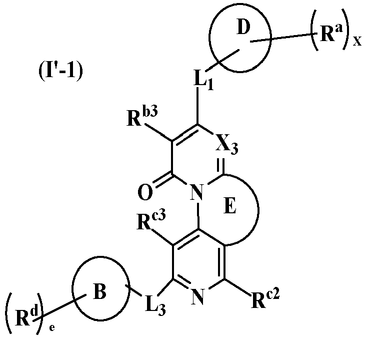 wherein:
ring D is selected from cycloalkyl, heterocyclyl, aryl, or heteroaryl;
ring E is heterocyclyl;
ring B is selected from cycloalkyl, heterocyclyl, aryl, or heteroaryl;
X₃ is selected from N or CR^{b1};
L₁ is selected from a bond, substituted or unsubstituted alkenylene, substituted or unsubstituted alkynylene, -(CH₂)ₙ-, -(CH₂)ₙC(O)(CRₐₐR_{bb})ₙ₁-, -(CH₂)ₙC(O)NR_{cc}(CH₂)ₙ₁-, -(CH₂)ₙ(CRₐₐR_{bb})ₙ₁-, - (CRₐₐR_{bb})ₙO(CH₂)ₙ₁-, -(CH₂)ₙO(CRₐₐR_{bb})ₙ₁-, -(CRₐₐR_{bb})ₙS(CH₂)ₙ₁-, -(CH₂)ₙS(CRₐₐR_{bb})ₙ₁-, - (CRₐₐR_{bb})ₙ(CH₂)ₙ₁NR_{cc}-, -(CH₂)ₙNR_{cc}(CRₐₐR_{bb})ₙ₁-, -(CH₂)ₙNR_{cc}C(O)-, -(CH₂)ₙP(O)ₚRₐₐ-, -(CH₂)ₙS(O)ₘ-, - (CH₂)ₙS(O)ₘNR_{cc}-, and -(CH₂)ₙNR_{cc}S(O)ₘ-;
Rₐₐ, R_{bb}, and R_{cc} are each independently selected from hydrogen, deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl, wherein the amino, C₁₋₆ alkyl, C₁₋₆ deuteroalkyl, C₁₋₆ haloalkyl, C₁₋₆ alkoxy, C₁₋₆ haloalkoxy, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
L₃ is selected from a bond, substituted or unsubstituted alkenyl, substituted or unsubstituted alkynyl, -(CH₂)ₙ₂-, -(CH₂)ₙ₂C(O)(CRₐ₁R_{b1})ₙ₃-, -(CH₂)ₙ₂C(O)NR_{c1}(CH₂)ₙ₃-, -(CH₂)ₙ₂(CRₐ₁R_{b1})ₙ₃-, - (CRₐ₁R_{b1})ₙ₂O(CH₂)ₙ₃-, -(CH₂)ₙ₂O(CRₐ₁R_{b1})ₙ₃-, -(CRₐ₁R_{b1})ₙ₂S(CH₂)ₙ₃-, -(CH₂)ₙ₂S(CRₐ₁R_{b1})ₙ₃-, - (CRₐ₁R_{b1})ₙ₂(CH₂)ₙ₃NR_{c1}-, -(CH₂)ₙ₂NR_{c1}(CRₐ₁R_{b1})ₙ₃-, -(CH₂)ₙ₂NR_{c1}C(O)-, -(CH₂)ₙ₂P(O)ₚ₁Rₐ₁-, - (CH₂)ₙ₂S(O)ₘ₁-, -(CH₂)ₙ₂S(O)ₘ₁NR_{c1}-, and -(CH₂)ₙ₂NR_{c1}S(O)ₘ₁-;
Rₐ₁, R_{b1}, and R_{c1} are each independently selected from hydrogen, deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl, wherein the amino, C₁₋₆ alkyl, C₁₋₆ deuteroalkyl, C₁₋₆ haloalkyl, C₁₋₆ alkoxy, C₁₋₆ haloalkoxy, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
each R^{a} is independently selected from hydrogen, deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl, wherein the amino, C₁₋₆ alkyl, C₁₋₆ deuteroalkyl, C₁₋₆ haloalkyl, C₁₋₆ alkoxy, C₁₋₆ haloalkoxy, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
or any two adjacent or non-adjacent R^{a} are connected to form C₃₋₁₂ cycloalkyl, 3- to 12-membered heterocyclyl, C₆₋₁₄ aryl, or 5- to 14-membered heteroaryl, wherein the C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
or L₁ and R^{a} are connected to form C₃₋₁₂ cycloalkyl, 3- to 12-membered heterocyclyl, C₆₋₁₄ aryl, or 5- to 14-membered heteroaryl, wherein the C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
each R^{b1} or R^{b3} is independently selected from hydrogen, deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl, wherein the amino, C₁₋₆ alkyl, C₁₋₆ deuteroalkyl, C₁₋₆ haloalkyl, C₁₋₆ alkoxy, C₁₋₆ haloalkoxy, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
each R^{c2} or R^{c3} is independently selected from hydrogen, deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl, wherein the amino, C₁₋₆ alkyl, C₁₋₆ deuteroalkyl, C₁₋₆ haloalkyl, C₁₋₆ alkoxy, C₁₋₆ haloalkoxy, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
each R^{d} is independently selected from hydrogen, deuterium, halogen, nitro, hydroxy, mercapto, cyano, amino, carboxyl, C₁₋₆ alkyl, C₁₋₆ deuteroalkyl, C₁₋₆ bridged cycloalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, - (CRₑₑR_{ff})ₙ₄C(O)R_{gg}, -(CRₑₑR_{ff})ₙ₄NRₕₕC(O)R_{gg}, -(CRₑₑR_{ff})ₙ₄NRₕₕOR_{gg}, -(CRₑₑR_{ff})ₙ₄S(O)₂R_{gg}, - (CRₑₑR_{ff})ₙ₄S(O)R_{gg}, -(CRₑₑR_{ff})ₙ₄SR_{gg}, -(CRₑₑR_{ff})ₙ₄C(O)NRₕₕR_{gg}, -(CRₑₑR_{ff})ₙ₄S(O)NRₕₕR_{gg}, - (CRₑₑR_{ff})ₙ₄SNRₕₕR_{gg}, or -(CRₑₑR_{ff})ₙ₄S(O)₂NRₕₕR_{gg}, wherein the amino, C₁₋₆ alkyl, C₁₋₆ deuteroalkyl, C₁₋₆ bridged cycloalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
Rₑₑ, R_{ff}, R_{gg}, and Rₕₕ are each independently selected from hydrogen, deuterium, halogen, nitro, hydroxy, mercapto, cyano, amino, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl;
or Rₑₑ and R_{ff} are connected to form C₃₋₁₂ cycloalkyl, 3- to 12-membered heterocyclyl, C₆₋₁₄ aryl, or 5- to 14-membered heteroaryl, wherein the C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
x is an integer of 0-6;
e is an integer of 0-6;
n is 0, 1, 2, or 3;
n1 is 0, 1, 2, or 3;
n2 is 0, 1, 2, or 3;
n3 is 0, 1, 2, or 3;
n4 is 0, 1, 2, 3, or 4;
n6 is 0, 1, 2, 3, or 4;
m is 0, 1, 2, or 3;
p is 0, 1, 2, or 3;
m1 is 0, 1, 2, or 3; and
p1 is 0, 1, 2, or 3.

3. The compound, or the stereoisomer or pharmaceutically acceptable salt thereof according to claim 2, wherein ring D is selected from C₃₋₁₂ cycloalkyl, 3- to 12-membered heterocyclyl, C₆₋₁₄ aryl, or 5- to 14-membered heteroaryl; preferably, ring D is selected from C₆₋₁₀ aryl, 5- to 6-membered monocyclic heteroaryl, or 7- to 10-membered bicyclic heteroaryl; and more preferably, ring D is selected from pyrazolyl, thienyl, thiazolyl, oxazolyl, pyrimidinyl, pyridinyl, phenyl,

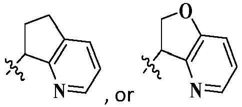

4. The compound, or the stereoisomer or pharmaceutically acceptable salt thereof according to any one of claims 2-3, wherein ring E is 6- to 12-membered heterocyclyl; and preferably, ring E is selected from

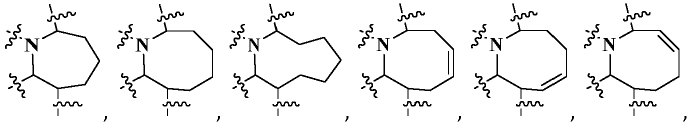

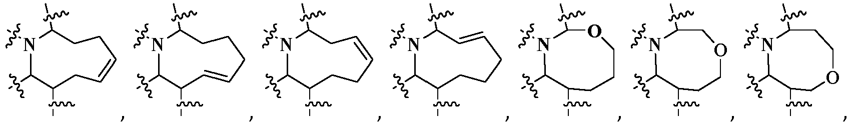

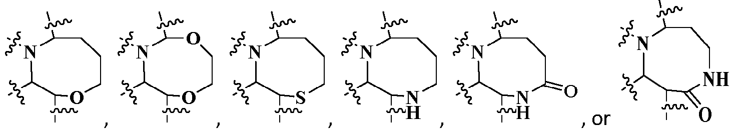

5. The compound, or the stereoisomer or pharmaceutically acceptable salt thereof according to any one of claims 1-4, wherein the compound is further as represented by general formula (XIV): 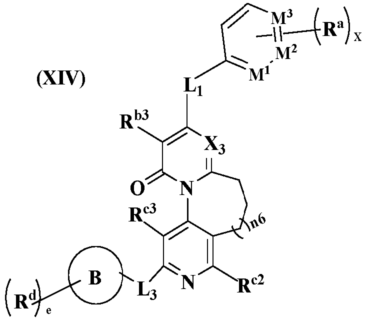 wherein:
M¹ is selected from C, CH, or N, or is absent;
M² is selected from C, CH, or N, or is absent;
M³ is selected from C, CH, or N, or is absent;
X₃ is selected from N or CR^{b1};
ring B is selected from cycloalkyl, heterocyclyl, aryl, or heteroaryl;
L₁ is selected from a bond, substituted or unsubstituted alkenylene, substituted or unsubstituted alkynylene, -(CH₂)ₙ-, -(CH₂)ₙC(O)(CRₐₐR_{bb})ₙ₁-, -(CH₂)ₙC(O)NR_{cc}(CH₂)ₙ₁-, -(CH₂)ₙ(CRₐₐR_{bb})ₙ₁-, - (CRₐₐR_{bb})ₙO(CH₂)ₙ₁-, -(CH₂)ₙO(CRₐₐR_{bb})ₙ₁-, -(CRₐₐR_{bb})ₙS(CH₂)ₙ₁-, -(CH₂)ₙS(CRₐₐR_{bb})ₙ₁-, - (CRₐₐR_{bb})ₙ(CH₂)ₙ₁NR_{cc}-, -(CH₂)ₙNR_{cc}(CRₐₐR_{bb})ₙ₁-, -(CH₂)ₙNR_{cc}C(O)-, -(CH₂)ₙP(O)ₚRₐₐ-, -(CH₂)ₙS(O)ₘ-, - (CH₂)ₙS(O)ₘNR_{cc}-, and -(CH₂)ₙNR_{cc}S(O)ₘ-;
Rₐₐ, R_{bb}, and R_{cc} are each independently selected from hydrogen, deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl, wherein the amino, C₁₋₆ alkyl, C₁₋₆ deuteroalkyl, C₁₋₆ haloalkyl, C₁₋₆ alkoxy, C₁₋₆ haloalkoxy, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
L₃ is selected from a bond, substituted or unsubstituted alkenyl, substituted or unsubstituted alkynyl, -(CH₂)ₙ₂-, -(CH₂)ₙ₂C(O)(CRₐ₁R_{b1})ₙ₃-, -(CH₂)ₙ₂C(O)NR_{c1}(CH₂)ₙ₃-, -(CH₂)ₙ₂(CRₐ₁R_{b1})ₙ₃-, - (CRₐ₁R_{b1})ₙ₂O(CH₂)ₙ₃-, -(CH₂)ₙ₂O(CRₐ₁R_{b1})ₙ₃-, -(CRₐ₁R_{b1})ₙ₂S(CH₂)ₙ₃-, -(CH₂)ₙ₂S(CRₐ₁R_{b1})ₙ₃-, - (CRₐ₁R_{b1})ₙ₂(CH₂)ₙ₃NR_{c1}-, -(CH₂)ₙ₂NR_{c1}(CRₐ₁R_{b1})ₙ₃-, -(CH₂)ₙ₂NR_{c1}C(O)-, -(CH₂)ₙ₂P(O)ₚ₁Rₐ₁-, - (CH₂)ₙ₂S(O)ₘ₁-, -(CH₂)ₙ₂S(O)ₘ₁NR_{c1}-, and -(CH₂)ₙ₂NR_{c1}S(O)ₘ₁-;
Rₐ₁, R_{b1}, and R_{c1} are each independently selected from hydrogen, deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl, wherein the amino, C₁₋₆ alkyl, C₁₋₆ deuteroalkyl, C₁₋₆ haloalkyl, C₁₋₆ alkoxy, C₁₋₆ haloalkoxy, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
each R^{a} is independently selected from hydrogen, deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl, wherein the amino, C₁₋₆ alkyl, C₁₋₆ deuteroalkyl, C₁₋₆ haloalkyl, C₁₋₆ alkoxy, C₁₋₆ haloalkoxy, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
or any two adjacent or non-adjacent R^{a} are connected to form C₃₋₁₂ cycloalkyl, 3- to 12-membered heterocyclyl, C₆₋₁₄ aryl, or 5- to 14-membered heteroaryl, wherein the C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
or L₁ and R^{a} are connected to form C₃₋₁₂ cycloalkyl, 3- to 12-membered heterocyclyl, C₆₋₁₄ aryl, or 5- to 14-membered heteroaryl, wherein the C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
each R^{b1} or R^{b3} is independently selected from hydrogen, deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl, wherein the amino, C₁₋₆ alkyl, C₁₋₆ deuteroalkyl, C₁₋₆ haloalkyl, C₁₋₆ alkoxy, C₁₋₆ haloalkoxy, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
each R^{c2} or R^{c3} is independently selected from hydrogen, deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl, wherein the amino, C₁₋₆ alkyl, C₁₋₆ deuteroalkyl, C₁₋₆ haloalkyl, C₁₋₆ alkoxy, C₁₋₆ haloalkoxy, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
each R^{d} is independently selected from hydrogen, deuterium, halogen, nitro, hydroxy, mercapto, cyano, amino, carboxyl, C₁₋₆ alkyl, C₁₋₆ deuteroalkyl, C₁₋₆ bridged cycloalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, - (CRₑₑR_{ff})ₙ₄C(O)R_{gg}, -(CRₑₑR_{ff})ₙ₄NRₕₕC(O)R_{gg}, -(CRₑₑR_{ff})ₙ₄NRₕₕOR_{gg}, -(CRₑₑR_{ff})ₙ₄S(O)₂R_{gg}, - (CRₑₑR_{ff})ₙ₄S(O)R_{gg}, -(CRₑₑR_{ff})ₙ₄SR_{gg}, -(CRₑₑR_{ff})ₙ₄C(O)NRₕₕR_{gg}, -(CRₑₑR_{ff})ₙ₄S(O)NRₕₕR_{gg}, - (CRₑₑR_{ff})ₙ₄SNRₕₕR_{gg}, or -(CRₑₑR_{ff})ₙ₄S(O)₂NRₕₕR_{gg}, wherein the amino, C₁₋₆ alkyl, C₁₋₆ deuteroalkyl, C₁₋₆ bridged cycloalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl,
or 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
Rₑₑ, R_{ff}, R_{gg}, and Rₕₕ are each independently selected from hydrogen, deuterium, halogen, nitro, hydroxy, mercapto, cyano, amino, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl;
or Rₑₑ and R_{ff} are connected to form C₃₋₁₂ cycloalkyl, 3- to 12-membered heterocyclyl, C₆₋₁₄ aryl, or 5- to 14-membered heteroaryl, wherein the C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
x is an integer of 0-6;
e is an integer of 0-6;
n is 0, 1, 2, or 3;
n1 is 0, 1, 2, or 3;
n2 is 0, 1, 2, or 3;
n3 is 0, 1, 2, or 3;
n4 is 0, 1, 2, 3, or 4;
n6 is 0, 1, 2, 3, or 4;
m is 0, 1, 2, or 3;
p is 0, 1, 2, or 3;
m1 is 0, 1, 2, or 3; and
p1 is 0, 1, 2, or 3.

6. The compound, or the stereoisomer or pharmaceutically acceptable salt thereof according to claim 1, wherein ring A is selected from C₃₋₁₂ cycloalkyl, 3- to 12-membered heterocyclyl, C₆₋₁₄ aryl, or 5- to 14-membered heteroaryl;
preferably, ring A is selected from 6- to 12-membered bicyclic heterocyclyl, 5- to 6-membered monocyclic heteroaryl, or 7- to 10-membered bicyclic heteroaryl; and
more preferably, ring A is selected from pyrazolyl, thienyl, thiazolyl, oxazolyl, pyrimidinyl, pyridinyl, phenyl, tetrahydroquinolinyl, benzocyclopentyl, pyrrolopyrimidinyl, pyrrolopyridinyl, pyrazolopyridine, or imidazopyridine.

7. The compound, or the stereoisomer or pharmaceutically acceptable salt thereof according to any one of claims 1-6, wherein ring B is selected from C₃₋₁₂ cycloalkyl, 3- to 12-membered heterocyclyl, C₆₋₁₄ aryl, or 5- to 14-membered heteroaryl;
preferably, ring B is selected from 3-7-membered monocyclic heterocyclyl, 6- to 12-membered bicyclic heterocyclyl, 5-membered monocyclic heteroaryl, 6-membered monocyclic heteroaryl, or 7- to 14-membered heteroaryl fused ring;
more preferably, ring B is selected from 5-membered monocyclic nitrogen-containing heteroaryl, 6-membered monocyclic nitrogen-containing heteroaryl, 5- to 6-membered heteroaryl fused to 5- to 6-membered cycloalkyl, 5-to 6-membered heteroaryl fused to 5- to 6-membered heteroaryl, or 5- to 6-membered heteroaryl fused to phenyl;
further preferably, ring B is selected from pyrimidine, pyridocyclohexyl, pyridocyclopentyl, pyrrolotriazinyl, pyrrolopyrimidinyl, pyridocyclopentenyl, pyridine, thiazole, pyrazole,
pyridopyrrolidone, pyridofuranone, pyridosulfolane, 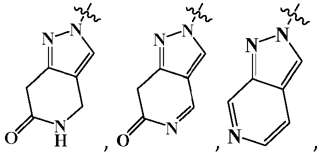
or 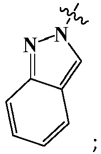
and
further more preferably, ring B is selected from pyrimidine, pyridocyclohexyl, pyridocyclopentyl, pyrrolotriazinyl, pyrrolopyrimidinyl, pyridocyclopentenyl, pyridine, thiazole, pyrazole, pyridopyrrolidone, pyridofuranone, or pyridosulfolane.

8. The compound, or the stereoisomer or pharmaceutically acceptable salt thereof according to claim 1, wherein the compound is further as represented by general formula (XV): 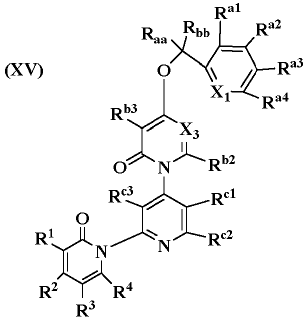 wherein:
X₁ is selected from N or CR^{a5};
X₃ is selected from N or CR^{b1};
each R^{a1}, R^{a2}, R^{a3}, R^{a4}, or R^{a5} is independently selected from hydrogen, deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl, wherein the amino, C₁₋₆ alkyl, C₁₋₆ deuteroalkyl, C₁₋₆ haloalkyl, C₁₋₆ alkoxy, C₁₋₆ haloalkoxy, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
or any two adjacent or non-adjacent R^{a1}, R^{a2}, R^{a3}, R^{a4}, and R^{a5} are connected to form C₃₋₁₂ cycloalkyl, 3- to 12-membered heterocyclyl, C₆₋₁₄ aryl, or 5- to 14-membered heteroaryl, wherein the C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered each heteroaryl;
each Rₐₐ or R_{bb} is independently selected from hydrogen, deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl, wherein
the amino, C₁₋₆ alkyl, C₁₋₆ deuteroalkyl, C₁₋₆ haloalkyl, C₁₋₆ alkoxy, C₁₋₆ haloalkoxy, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
or Rₐₐ and R_{bb} are connected to form C₃₋₁₂ cycloalkyl, 3- to 12-membered heterocyclyl, C₆₋₁₄ aryl, or 5- to 14-membered heteroaryl, wherein the C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
each R^{b1}, R^{b2}, or R^{b3} is independently selected from hydrogen, deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl, wherein the amino, C₁₋₆ alkyl, C₁₋₆ deuteroalkyl, C₁₋₆ haloalkyl, C₁₋₆ alkoxy, C₁₋₆ haloalkoxy, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
or the substituents R^{b1} and R^{b2} are connected to form C₃₋₁₂ cycloalkyl, 3- to 12-membered heterocyclyl, C₆₋₁₄ aryl, or 5- to 14-membered heteroaryl, wherein the C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
each R^{c1}, R^{c2}, or R^{c3} is independently selected from hydrogen, deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl, wherein the amino, C₁₋₆ alkyl, C₁₋₆ deuteroalkyl, C₁₋₆ haloalkyl, C₁₋₆ alkoxy, C₁₋₆ haloalkoxy, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
or the substituents R^{c1} and R^{c2} are connected to form C₃₋₁₂ cycloalkyl, 3- to 12-membered heterocyclyl, C₆₋₁₄ aryl, or 5- to 14-membered heteroaryl, wherein the C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
or the substituents R^{c1} and R^{b2} are connected to form 3- to 12-membered heterocyclyl or 5- to 14-membered heteroaryl, wherein the 3- to 12-membered heterocyclyl and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
R¹ is selected from hydrogen, deuterium, halogen, nitro, hydroxy, mercapto, cyano, amino, oxo, thio, carboxyl, C₁₋₆ alkyl, C₁₋₆ deuteroalkyl, C₁₋₆ bridged cycloalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, -(CRₑₑR_{ff})ₙ₄C(O)R_{gg}, - (CRₑₑR_{ff})ₙ₄NRₕₕC(O)R_{gg}, -(CRₑₑR_{ff})ₙ₄NRₕₕOR_{gg}, -(CRₑₑR_{ff})ₙ₄S(O)₂R_{gg}, -(CRₑₑR_{ff})ₙ₄S(O)R_{gg}, -(CRₑₑR_{ff})ₙ₄SR_{gg}, -(CRₑₑR_{ff})ₙ₄C(O)NRₕₕR_{gg}, -(CRₑₑR_{ff})ₙ₄S(O)NRₕₕR_{gg}, -(CRₑₑR_{ff})ₙ₄SNRₕₕR_{gg}, or -(CRₑₑR_{ff})ₙ₄S(O)₂NRₕₕR_{gg}, wherein the amino, C₁₋₆ alkyl, C₁₋₆ deuteroalkyl, C₁₋₆ bridged cycloalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
Rₑₑ, R_{ff}, R_{gg}, and Rₕₕ are each independently selected from hydrogen, deuterium, halogen, nitro, hydroxy, mercapto, cyano, amino, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl; or Rₑₑ and R_{ff} are connected to form C₃₋₁₂ cycloalkyl, 3- to 12-membered heterocyclyl, C₆₋₁₄ aryl, or 5- to 14-membered heteroaryl, wherein the C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
each R², R³, or R⁴ is independently selected from hydrogen, deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl, wherein the amino, C₁₋₆ alkyl, C₁₋₆ deuteroalkyl, C₁₋₆ haloalkyl, C₁₋₆ alkoxy, C₁₋₆ haloalkoxy, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
or any two adjacent or non-adjacent R¹, R², R³, and R⁴ are connected to form C₃₋₁₂ cycloalkyl, 3-to 12-membered heterocyclyl, C₆₋₁₄ aryl, or 5- to 14-membered heteroaryl, wherein the C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
n4 is 0, 1, 2, 3, or 4; and
the 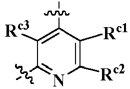 is not

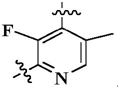

9. The compound, or the stereoisomer or pharmaceutically acceptable salt thereof according to any one of claims 1-8, wherein the compound is further as represented by general formula (XIV-A), (XIV-B), or (XIV-C): 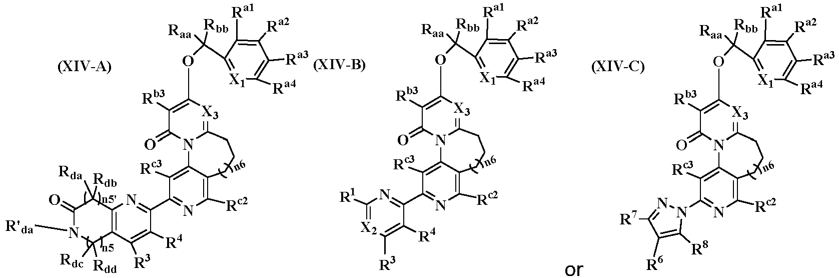 wherein:
X₁ is selected from N or CR^{a5};
X₂ is selected from N or CR²;
X₃ is selected from N or CR^{b1};
each R^{a1}, R^{a2}, R^{a3}, R^{a4}, or R^{a5} is independently selected from hydrogen, deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl, wherein the amino, C₁₋₆ alkyl, C₁₋₆ deuteroalkyl, C₁₋₆ haloalkyl, C₁₋₆ alkoxy, C₁₋₆ haloalkoxy, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
or any two adjacent or non-adjacent R^{a1}, R^{a2}, R^{a3}, R^{a4}, and R^{a5} are connected to form C₃₋₁₂ cycloalkyl, 3- to 12-membered heterocyclyl, C₆₋₁₄ aryl, or 5- to 14-membered heteroaryl, wherein the C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered each heteroaryl;
each Rₐₐ or R_{bb} is independently selected from hydrogen, deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl, wherein the amino, C₁₋₆ alkyl, C₁₋₆ deuteroalkyl, C₁₋₆ haloalkyl, C₁₋₆ alkoxy, C₁₋₆ haloalkoxy, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
or Rₐₐ and R_{bb} are connected to form C₃₋₁₂ cycloalkyl, 3- to 12-membered heterocyclyl, C₆₋₁₄ aryl, or 5- to 14-membered heteroaryl, wherein the C₃₋₁₂ cycloalkyl, 3- to 12-membered heterocyclyl, C₆₋₁₄ aryl, and 5- to 14-membered heteroaryl may be optionally further substituted with one or
more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
each R^{b1} or R^{b3} is independently selected from hydrogen, deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl, wherein the amino, C₁₋₆ alkyl, C₁₋₆ deuteroalkyl, C₁₋₆ haloalkyl, C₁₋₆ alkoxy, C₁₋₆ haloalkoxy, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
each R^{c2} or R^{c3} is independently selected from hydrogen, deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl, wherein the amino, C₁₋₆ alkyl, C₁₋₆ deuteroalkyl, C₁₋₆ haloalkyl, C₁₋₆ alkoxy, C₁₋₆ haloalkoxy, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
each R_{da}, R_{db}, or R'_{da} is independently selected from hydrogen, deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl, wherein the amino, C₁₋₆ alkyl, C₁₋₆ deuteroalkyl, C₁₋₆ haloalkyl, C₁₋₆ alkoxy, C₁₋₆ haloalkoxy, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl,
and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
or R_{da} and R_{db} are connected to form C₃₋₁₂ cycloalkyl, 3- to 12-membered heterocyclyl, C₆₋₁₄ aryl, or 5- to 14-membered heteroaryl, wherein the C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
each R_{dc} or R_{dd} is independently selected from hydrogen, deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl, wherein the amino, C₁₋₆ alkyl, C₁₋₆ deuteroalkyl, C₁₋₆ haloalkyl, C₁₋₆ alkoxy, C₁₋₆ haloalkoxy, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
or R_{dc} and R_{dd} are connected to form C₃₋₁₂ cycloalkyl, 3- to 12-membered heterocyclyl, C₆₋₁₄ aryl, or 5- to 14-membered heteroaryl, wherein the C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
R¹ is selected from hydrogen, deuterium, halogen, nitro, hydroxy, mercapto, cyano, amino, oxo, thio, carboxyl, C₁₋₆ alkyl, C₁₋₆ deuteroalkyl, C₁₋₆ bridged cycloalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, -(CRₑₑR_{ff})ₙ₄C(O)R_{gg}, - (CRₑₑR_{ff})ₙ₄NRₕₕC(O)R_{gg}, -(CRₑₑR_{ff})ₙ₄NRₕₕOR_{gg}, -(CRₑₑR_{ff})ₙ₄S(O)₂R_{gg}, -(CRₑₑR_{ff})ₙ₄S(O)R_{gg}, -(CRₑₑR_{ff})ₙ₄SR_{gg}, -(CRₑₑR_{ff})ₙ₄C(O)NRₕₕR_{gg}, -(CRₑₑR_{ff})ₙ₄S(O)NRₕₕR_{gg}, -(CRₑₑR_{ff})ₙ₄SNRₕₕR_{gg}, or -(CRₑₑR_{ff})ₙ₄S(O)₂NRₕₕR_{gg}, wherein the amino, C₁₋₆ alkyl, C₁₋₆ deuteroalkyl, C₁₋₆ bridged cycloalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
Rₑₑ, R_{ff}, R_{gg}, and Rₕₕ are each independently selected from hydrogen, deuterium, halogen, nitro, hydroxy, mercapto, cyano, amino, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl;
or Rₑₑ and R_{ff} are connected to form C₃₋₁₂ cycloalkyl, 3- to 12-membered heterocyclyl, C₆₋₁₄ aryl, or 5- to 14-membered heteroaryl, wherein the C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
each R², R³, or R⁴ is independently selected from hydrogen, deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl, wherein the amino, C₁₋₆ alkyl, C₁₋₆ deuteroalkyl, C₁₋₆ haloalkyl, C₁₋₆ alkoxy, C₁₋₆ haloalkoxy, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
or any two adjacent or non-adjacent R¹, R², R³, and R⁴ are connected to form C₃₋₁₂ cycloalkyl, 3-to 12-membered heterocyclyl, C₆₋₁₄ aryl, or 5- to 14-membered heteroaryl, wherein the C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
R⁷ is selected from hydrogen, deuterium, halogen, nitro, hydroxy, mercapto, cyano, amino, oxo, thio, carboxyl, C₁₋₆ alkyl, C₁₋₆ deuteroalkyl, C₁₋₆ bridged cycloalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, -(CRₑₑR_{ff})ₙ₄C(O)R_{gg}, - (CRₑₑR_{ff})ₙ₄NRₕₕC(O)R_{gg}, -(CR_{gg}R_{ff})ₙ₄NRₕₕOR_{gg}, -(CRₑₑR_{ff})ₙ₄S(O)₂R_{gg}, -(CRₑₑR_{ff})ₙ₄S(O)R_{gg}, -(CRₑₑR_{ff})ₙ₄SR_{gg}, -(CRₑₑR_{ff})ₙ₄C(O)NRₕₕR_{gg}, -(CRₑₑR_{ff})ₙ₄S(O)NRₕₕR_{gg}, -(CRₑₑR_{ff})ₙ₄SNRₕₕR_{gg}, or -(CRₑₑR_{ff})ₙ₄S(O)₂NRₕₕR_{gg}, wherein the amino, C₁₋₆ alkyl, C₁₋₆ deuteroalkyl, C₁₋₆ bridged cycloalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
Rₑₑ, R_{ff}, R_{gg}, and Rₕₕ are each independently selected from hydrogen, deuterium, halogen, nitro, hydroxy, mercapto, cyano, amino, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl;
or Rₑₑ and R_{ff} are connected to form C₃₋₁₂ cycloalkyl, 3- to 12-membered heterocyclyl, C₆₋₁₄ aryl, or 5- to 14-membered heteroaryl, wherein the C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
each R⁶ or R⁸ is independently selected from hydrogen, deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl, wherein the amino, C₁₋₆ alkyl, C₁₋₆ deuteroalkyl, C₁₋₆ haloalkyl, C₁₋₆ alkoxy, C₁₋₆ haloalkoxy, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
or any two adjacent or non-adjacent R⁶, R⁷, and R⁸ are connected to form C₃₋₁₂ cycloalkyl, 3- to 12-membered heterocyclyl, C₆₋₁₄ aryl, or 5- to 14-membered heteroaryl, wherein the C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
n4 is 0, 1, 2, 3, or 4;
n5 is 0, 1, or 2;
n5' is 0, 1, or 2;
n5 and n5' are not both 0; and
n6 is 0, 1, 2, 3, or 4.

10. The compound, or the stereoisomer or pharmaceutically acceptable salt thereof according to any one of claims 1-9, wherein the compound is further as represented by general formula (I'-1-1), (I'-1-2), (XIV-1), (XIV-2), (XIV-A-1), (XIV-A-2), (XIV-B-1), (XIV-B-2), (XIV-C-1), (XIV-C-2), (XV-1), or (XV-2):

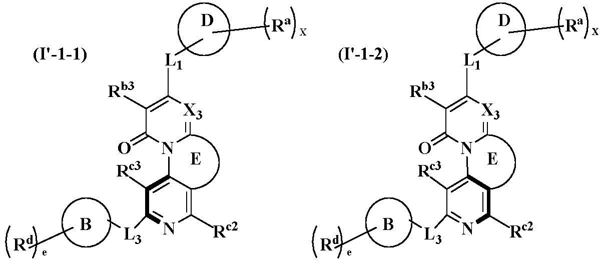

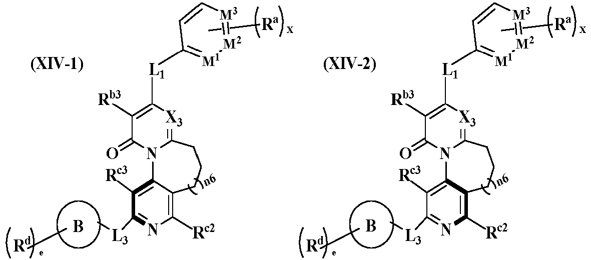

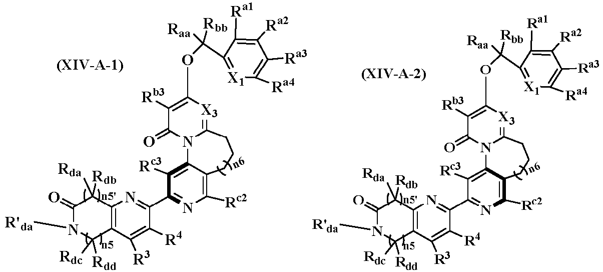

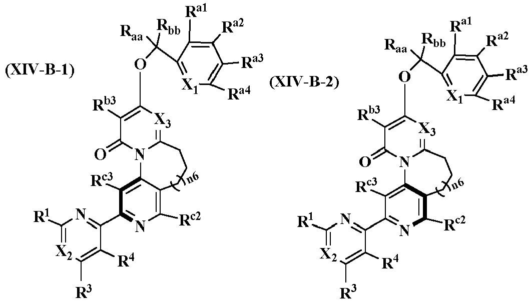

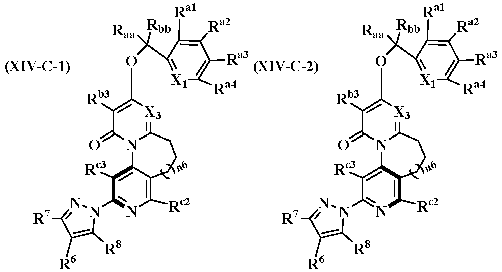

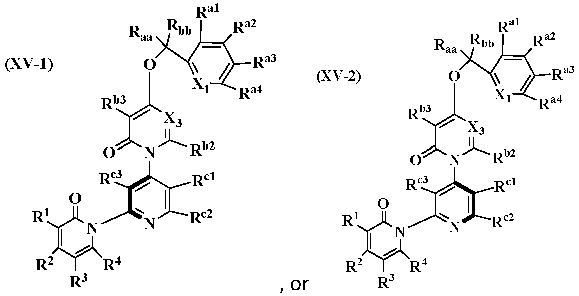

11. The compound, or the stereoisomer or pharmaceutically acceptable salt thereof according to any one of claims 1-7 or 10, wherein L₁ is selected from a bond, substituted or unsubstituted alkenylene, substituted or unsubstituted alkynylene, -(CH₂)ₙ-, -(CH₂)ₙC(O)(CRₐₐR_{bb})ₙ₁-, - (CH₂)ₙC(O)NR_{cc}(CH₂)ₙ₁-, -(CH₂)ₙ(CRₐₐR_{bb})ₙ₁-, -(CRₐₐR_{bb})ₙO(CH₂)ₙ₁-, -(CH₂)ₙO(CRₐₐR_{bb})ₙ₁-, - (CRₐₐR_{bb})ₙS(CH₂)ₙ₁-, -(CH₂)ₙS(CRₐₐR_{bb})ₙ₁-, -(CRₐₐR_{bb})ₙ(CH₂)ₙ₁NR_{cc}-, -(CH₂)ₙNR_{cc}(CRₐₐR_{bb})ₙ₁-, - (CH₂)ₙNR_{cc}C(O)-, -(CH₂)ₙP(O)ₚRₐₐ-, -(CH₂)ₙS(O)ₘ-, -(CH₂)ₙS(O)ₘNR_{cc}-, and -(CH₂)ₙNR_{cc}S(O)ₘ-;
preferably, L₁ is selected from a bond, substituted or unsubstituted alkenylene, substituted or unsubstituted alkynylene, -(CH₂)ₙ-, -(CH₂)ₙC(O)-, -(CRₐₐR_{bb})ₙO-, -O(CRₐₐR_{bb})ₙ₁-, -(CRₐₐR_{bb})ₙS-, - S(CRₐₐR_{bb})ₙ₁-, -(CH₂)ₙ₁NR_{cc}-, -NR_{cc}(CRₐₐR_{bb})ₙ₁-, or-NR_{cc}C(O)-;
more preferably, L₁ is selected from a bond, -CH₂-, -OCH₂-, -OCD₂-, -NH-, -C(O)NH-, -OCH(CH₃)-, - OC(CH₃)₂-, -NH-CH₂-, or 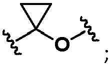
Rₐₐ, R_{bb}, and R_{cc} are each independently selected from hydrogen, deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl, wherein the amino, C₁₋₆ alkyl, C₁₋₆ deuteroalkyl, C₁₋₆ haloalkyl, C₁₋₆ alkoxy, C₁₋₆ haloalkoxy, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
preferably, Rₐₐ, R_{bb}, and R_{cc} are each independently selected from hydrogen, deuterium, halogen, nitro, hydroxy, mercapto, cyano, amino, oxo, thio, carboxyl, C₁₋₃ alkyl, C₁₋₃ deuteroalkyl, C₁₋₃ haloalkyl, C₁₋₃ hydroxyalkyl, C₁₋₃ alkoxy, C₁₋₃ deuteroalkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl, or 5- to 10-membered heteroaryl, wherein the amino, C₁₋₃ alkyl, C₁₋₃ deuteroalkyl, C₁₋₃ haloalkyl, C₁₋₃ alkoxy, C₁₋₃ haloalkoxy, C₁₋₃ hydroxyalkyl, C₂₋₄ alkenyl, C₂₋₄ alkynyl, C₃₋₈ cycloalkyl, 3- to 8-membered heterocyclyl, C₆₋₁₀ aryl, and 5- to 10-membered heteroaryl may be optionally further substituted with one or more substituents of deuterium, halogen, nitro, hydroxy, mercapto, cyano, amino, oxo, thio, carboxyl, C₁₋₃ alkyl, C₁₋₃ deuteroalkyl, C₁₋₃ haloalkyl, C₁₋₃ hydroxyalkyl, C₁₋₃ alkoxy, C₁₋₃ deuteroalkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl, and 5- to 10-membered heteroaryl;
or any two of Rₐₐ, R_{bb}, and R_{cc} are connected to form C₃₋₁₂ cycloalkyl, 3- to 12-membered heterocyclyl, C₆₋₁₄ aryl, or 5- to 14-membered heteroaryl, wherein the C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, and C₁₋₆ haloalkoxy; and
preferably, any two of Rₐₐ, R_{bb}, and R_{cc} are connected to form C₃₋₈ cycloalkyl, 3- to 8-membered heterocyclyl, C₆₋₁₀ aryl, or 5- to 10-membered heteroaryl, wherein the C₃₋₈ cycloalkyl, 3- to 8-membered heterocyclyl, C₆₋₁₀ aryl, and 5- to 10-membered heteroaryl may be optionally further substituted with one or more substituents of deuterium, halogen, nitro, hydroxy, mercapto, cyano, amino, oxo, thio, carboxyl, C₁₋₃ alkyl, C₁₋₃ deuteroalkyl, C₁₋₃ haloalkyl, C₁₋₃ hydroxyalkyl, C₁₋₃ alkoxy, C₁₋₃ deuteroalkoxy, and C₁₋₃ haloalkoxy.

12. The compound, or the stereoisomer or pharmaceutically acceptable salt thereof according to any one of claims 1-7 or 10-11, wherein each R^{a} is independently selected from hydrogen, deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl, wherein the amino, C₁₋₆ alkyl, C₁₋₆ deuteroalkyl, C₁₋₆ haloalkyl, C₁₋₆ alkoxy, C₁₋₆ haloalkoxy, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl; and
preferably, each R^{a} is independently selected from hydrogen, deuterium, halogen, nitro, hydroxy, mercapto, cyano, amino, oxo, thio, carboxyl, C₁₋₃ alkyl, C₁₋₃ deuteroalkyl, C₁₋₃ haloalkyl, C₁₋₃ hydroxyalkyl, C₁₋₃ alkoxy, C₁₋₃ deuteroalkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl, or 5- to 10-membered heteroaryl, wherein the amino, C₁₋₃ alkyl, C₁₋₃ deuteroalkyl, C₁₋₃ haloalkyl, C₁₋₃ alkoxy, C₁₋₃ haloalkoxy, C₁₋₃ hydroxyalkyl, C₂₋₄ alkenyl, C₂₋₄ alkynyl, C₃₋₈ cycloalkyl, 3- to 8-membered heterocyclyl, C₆₋₁₀ aryl, and 5- to 10-membered heteroaryl may be optionally further substituted with one or more substituents of deuterium, halogen, nitro, hydroxy, mercapto, cyano, amino, oxo, thio, carboxyl, C₁₋₃ alkyl, C₁₋₃ deuteroalkyl, C₁₋₃ haloalkyl, C₁₋₃ hydroxyalkyl, C₁₋₃ alkoxy, C₁₋₃ deuteroalkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl, and 5- to 10-membered heteroaryl.

13. The compound, or the stereoisomer or pharmaceutically acceptable salt thereof according to any one of claims 1, 6-7, or 11-12, wherein each R^{b} is independently selected from hydrogen, deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl, wherein the amino, C₁₋₆ alkyl, C₁₋₆ deuteroalkyl, C₁₋₆ haloalkyl, C₁₋₆ alkoxy, C₁₋₆ haloalkoxy, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl; and
preferably, each R^{b} is independently selected from hydrogen, deuterium, halogen, nitro, hydroxy, mercapto, cyano, amino, oxo, thio, carboxyl, C₁₋₃ alkyl, C₁₋₃ deuteroalkyl, C₁₋₃ haloalkyl, C₁₋₃ hydroxyalkyl, C₁₋₃ alkoxy, C₁₋₃ deuteroalkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl, or 5- to 10-membered heteroaryl, wherein the amino, C₁₋₃ alkyl, C₁₋₃ deuteroalkyl, C₁₋₃ haloalkyl, C₁₋₃ alkoxy, C₁₋₃ haloalkoxy, C₁₋₃ hydroxyalkyl, C₂₋₄ alkenyl, C₂₋₄ alkynyl, C₃₋₈ cycloalkyl, 3- to 8-membered heterocyclyl, C₆₋₁₀ aryl, and 5- to 10-membered heteroaryl may be optionally further substituted with one or more substituents of deuterium, halogen, nitro, hydroxy, mercapto, cyano, amino, oxo, thio, carboxyl, C₁₋₃ alkyl, C₁₋₃ deuteroalkyl, C₁₋₃ haloalkyl, C₁₋₃ hydroxyalkyl, C₁₋₃ alkoxy, C₁₋₃ deuteroalkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl, and 5- to 10-membered heteroaryl.

14. The compound, or the stereoisomer or pharmaceutically acceptable salt thereof according to any one of claims 1, 6-7, or 11-13, wherein each R^{c} is independently selected from hydrogen, deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl, wherein the amino, C₁₋₆ alkyl, C₁₋₆ deuteroalkyl, C₁₋₆ haloalkyl, C₁₋₆ alkoxy, C₁₋₆ haloalkoxy, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
preferably, each R^{c} is independently selected from hydrogen, deuterium, halogen, nitro, hydroxy, mercapto, cyano, amino, oxo, thio, carboxyl, C₁₋₃ alkyl, C₁₋₃ deuteroalkyl, C₁₋₃ haloalkyl, C₁₋₃ hydroxyalkyl, C₁₋₃ alkoxy, C₁₋₃ deuteroalkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl, or 5- to 10-membered heteroaryl, wherein the amino, C₁₋₃ alkyl, C₁₋₃ deuteroalkyl, C₁₋₃ haloalkyl, C₁₋₃ alkoxy, C₁₋₃ haloalkoxy, C₁₋₃ hydroxyalkyl, C₂₋₄ alkenyl, C₂₋₄ alkynyl, C₃₋₈ cycloalkyl, 3- to 8-membered heterocyclyl, C₆₋₁₀ aryl, and 5- to 10-membered heteroaryl may be optionally further substituted with one or more substituents of deuterium, halogen, nitro, hydroxy, mercapto, cyano, amino, oxo, thio, carboxyl, C₁₋₃ alkyl, C₁₋₃ deuteroalkyl, C₁₋₃ haloalkyl, C₁₋₃ hydroxyalkyl, C₁₋₃ alkoxy, C₁₋₃ deuteroalkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl, and 5- to 10-membered heteroaryl;
or R^{b} and R` are connected to form C₃₋₁₂ cycloalkyl, 3- to 12-membered heterocyclyl, C₆₋₁₄ aryl, or 5- to 14-membered heteroaryl, wherein C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl.

15. The compound, or the stereoisomer or pharmaceutically acceptable salt thereof according to any one of claims 1-7 or 10-14, wherein
each R^{d} is independently selected from hydrogen, deuterium, halogen, nitro, hydroxy, mercapto, cyano, amino, oxo, thio, carboxyl, C₁₋₆ alkyl, 5- to 10-membered bridged cycloalkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, -(CRₑₑR_{ff})ₙ₄C(O)R_{gg}, -(CRₑₑR_{ff})ₙ₄NRₕₕC(O)R_{gg}, -(CRₑₑR_{ff})ₙ₄S(O)₂R_{gg}, - (CRₑₑR_{ff})ₙ₄C(O)NRₕₕR_{gg}, or -(CRₑₑR_{ff})ₙ₄S(O)₂NRₕₕR_{gg}, wherein the amino, C₁₋₆ alkyl, 5- to 10-membered bridged cycloalkyl, C₁₋₆ deuteroalkyl, C₁₋₆ haloalkyl, C₁₋₆ alkoxy, C₁₋₆ haloalkoxy, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
Rₑₑ, R_{ff}, R_{gg}, and Rₕₕ are each independently selected from hydrogen, deuterium, halogen, nitro, hydroxy, mercapto, cyano, amino, carboxyl, C₁₋₈ alkyl, C₁₋₈ deuteroalkyl, C₁₋₈ haloalkyl, C₁₋₈ hydroxyalkyl, C₁₋₈ alkoxy, C₁₋₈ deuteroalkoxy, C₁₋₈ haloalkoxy, C₂₋₈ alkenyl, C₂₋₈ alkynyl, C₃₋₁₂ cycloalkyl, 3- to 12-membered heterocyclyl, C₆₋₁₄ aryl, or 5- to 14-membered heteroaryl; preferably, Rₑₑ, R_{ff}, R_{gg}, and Rₕₕ are each independently selected from hydrogen, deuterium, halogen, nitro, hydroxy, mercapto, cyano, amino, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₈ cycloalkyl, 3- to 8-membered heterocyclyl, C₆₋₁₀ aryl, or 5- to 10-membered heteroaryl;
or Rₑₑ and R_{ff} are connected to form cycloalkyl, heterocyclyl, aryl, or heteroaryl, wherein the cycloalkyl, heterocyclyl, aryl, and heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl; and
n4 is 0, 1, 2, 3, or 4;
preferably,
each R^{d} is independently selected from hydrogen, deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl, wherein the amino, C₁₋₆ alkyl, C₁₋₆ deuteroalkyl, C₁₋₆ haloalkyl, C₁₋₆ alkoxy, C₁₋₆ haloalkoxy, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl; and
preferably, each R^{d} is independently selected from hydrogen, deuterium, halogen, nitro, hydroxy, mercapto, cyano, amino, oxo, thio, carboxyl, C₁₋₃ alkyl, C₁₋₃ deuteroalkyl, C₁₋₃ haloalkyl, C₁₋₃ hydroxyalkyl, C₁₋₃ alkoxy, C₁₋₃ deuteroalkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl, or 5- to 10-membered heteroaryl, wherein the amino, C₁₋₃ alkyl, C₁₋₃ deuteroalkyl, C₁₋₃ haloalkyl, C₁₋₃ alkoxy, C₁₋₃ haloalkoxy, C₁₋₃ hydroxyalkyl, C₂₋₄ alkenyl, C₂₋₄ alkynyl, C₃₋₈ cycloalkyl, 3- to 8-membered heterocyclyl, C₆₋₁₀ aryl, and 5- to 10-membered heteroaryl may be optionally further substituted with one or more substituents of deuterium, halogen, nitro, hydroxy, mercapto, cyano, amino, oxo, thio, carboxyl, C₁₋₃ alkyl, C₁₋₃ deuteroalkyl, C₁₋₃ haloalkyl, C₁₋₃ hydroxyalkyl, C₁₋₃ alkoxy, C₁₋₃ deuteroalkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl, and 5- to 10-membered heteroaryl.

16. The compound, or the stereoisomer or pharmaceutically acceptable salt thereof according to any one of claims 8-10, wherein R² is selected from halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl, wherein the amino, C₁₋₆ alkyl, C₁₋₆ deuteroalkyl, C₁₋₆ haloalkyl, C₁₋₆ alkoxy, C₁₋₆ haloalkoxy, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
preferably, R² is selected from halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, or C₂₋₆ alkynyl; and
more preferably, R² is fluorine.

17. The compound, or the stereoisomer or pharmaceutically acceptable salt thereof according to any one of claims 8-10, wherein R⁶ is selected from halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 14-membered heteroaryl, wherein the amino, C₁₋₆ alkyl, C₁₋₆ deuteroalkyl, C₁₋₆ haloalkyl, C₁₋₆ alkoxy, C₁₋₆ haloalkoxy, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl may be optionally further substituted with one or more substituents of deuterium, halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 14-membered heteroaryl;
preferably, R⁶ is selected from halogen, nitro, hydroxy, mercapto, cyano, amino, oxo, thio, carboxyl, C₁₋₆ alkyl, C₁₋₆ deuteroalkyl, C₁₋₆ haloalkyl, C₁₋₆ hydroxyalkyl, C₁₋₆ alkoxy, C₁₋₆ deuteroalkoxy, C₁₋₆ haloalkoxy, C₂₋₆ alkenyl, or C₂₋₆ alkynyl; and
more preferably, R⁶ is fluorine.

18. The compound, or the stereoisomer or pharmaceutically acceptable salt thereof according to any one of claims 1-17, wherein the structure of the compound is as shown below:

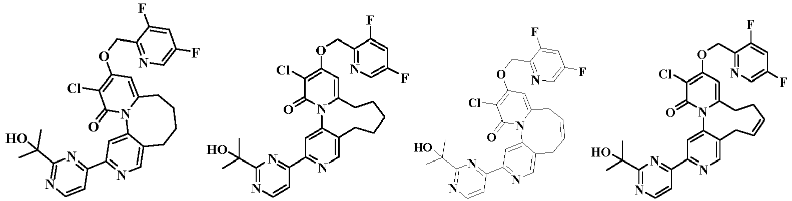

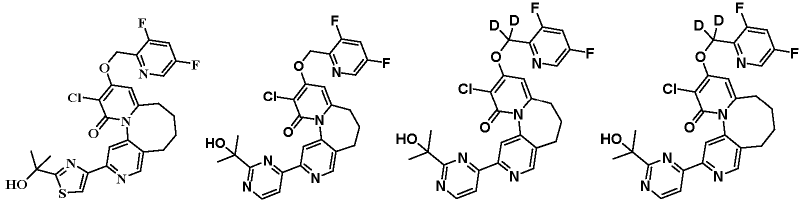

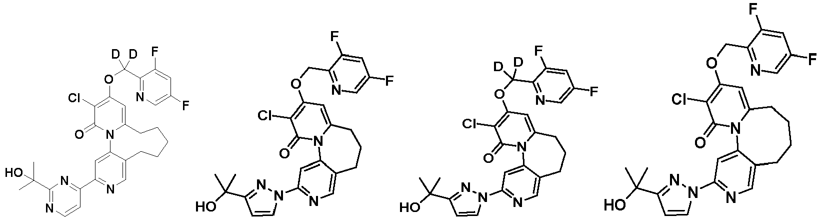

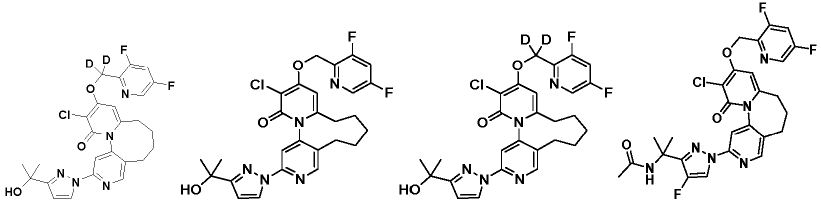

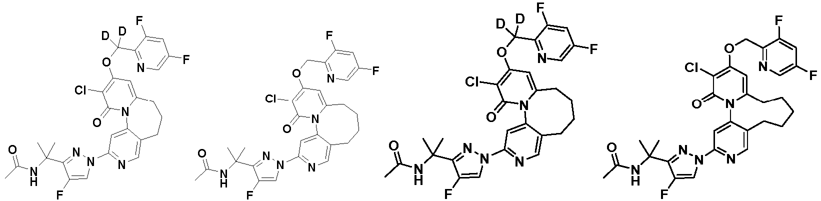

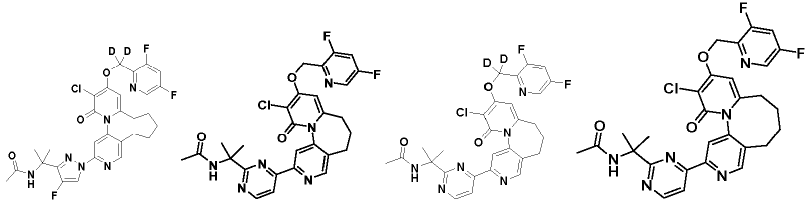

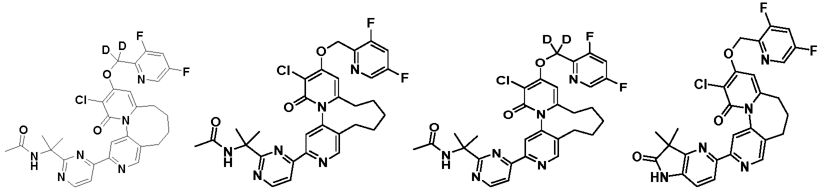

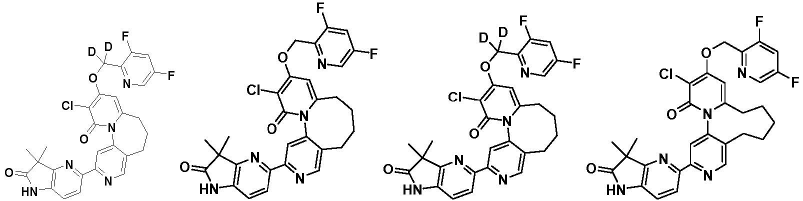

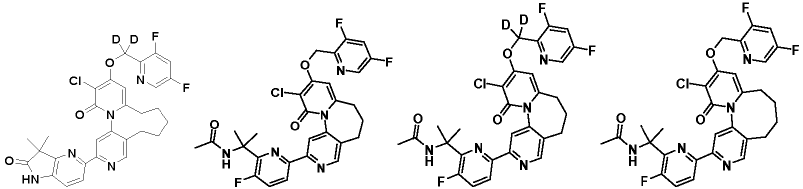

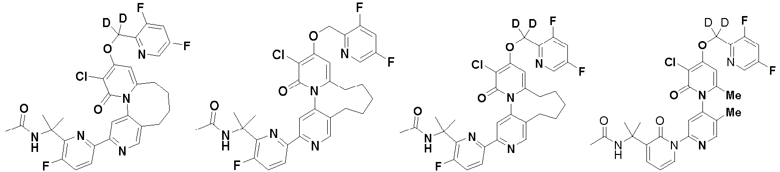

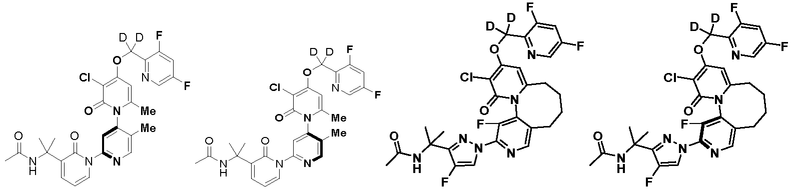

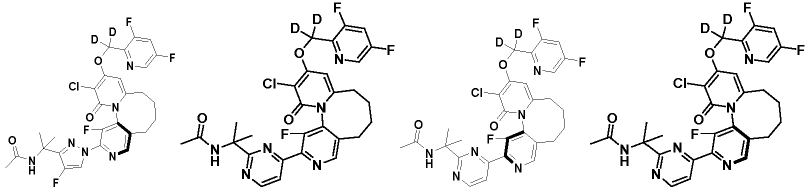

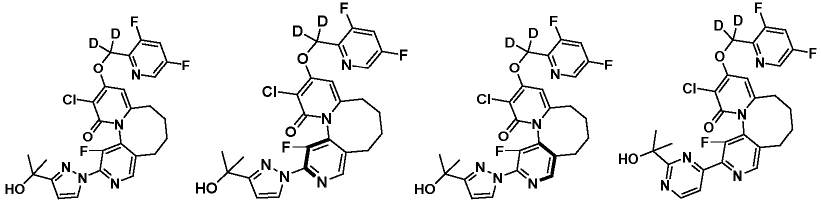

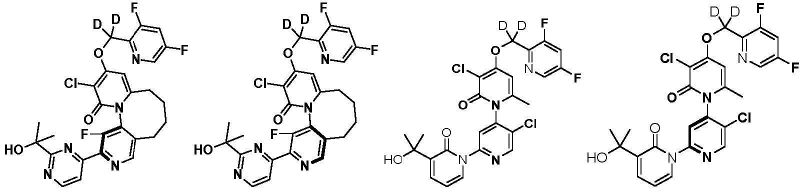

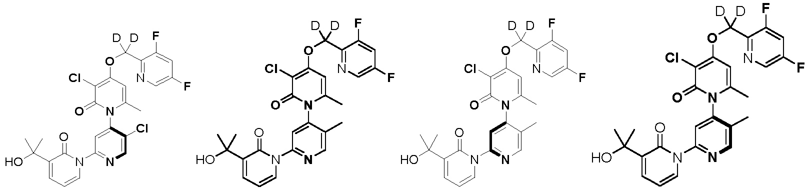

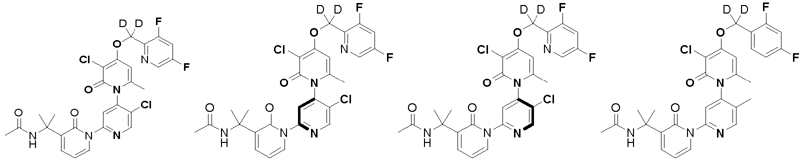

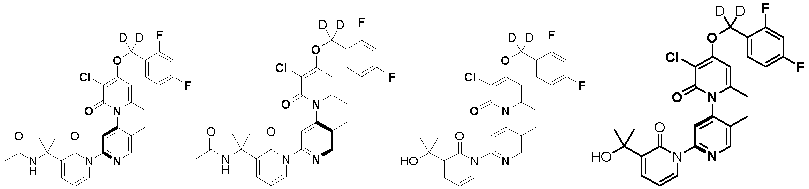

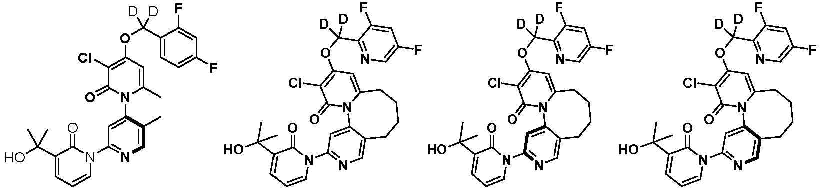

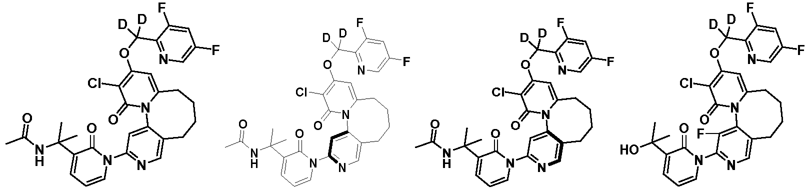

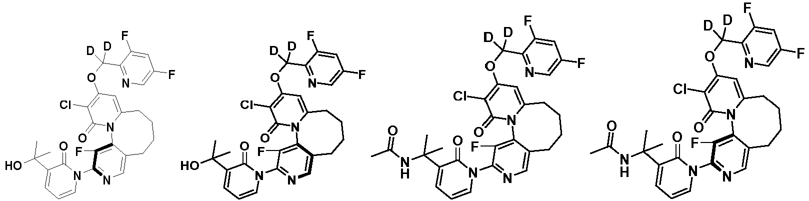

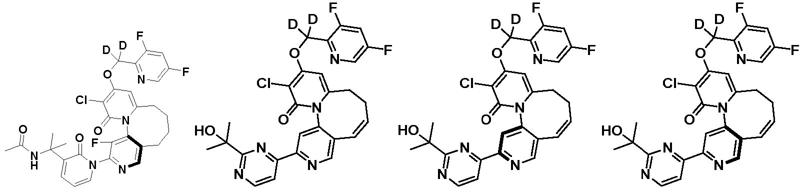

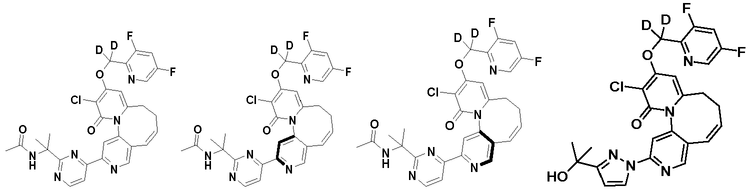

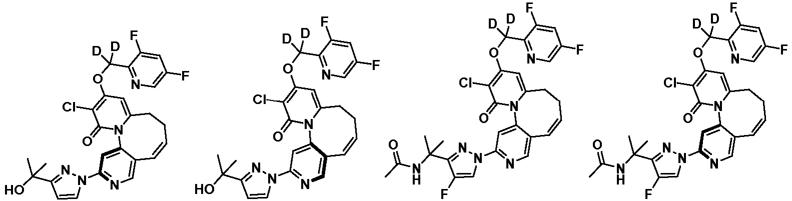

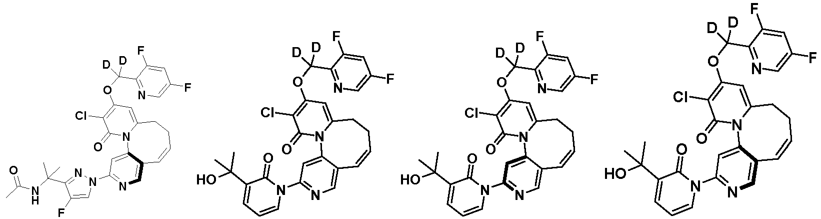

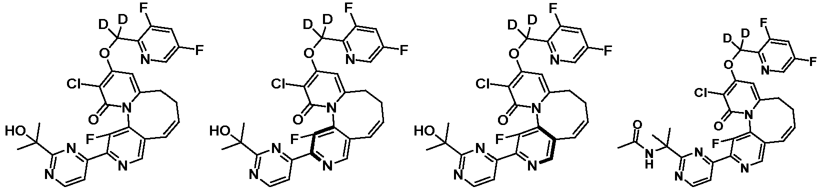

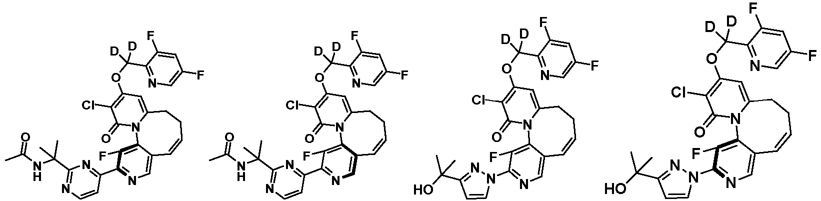

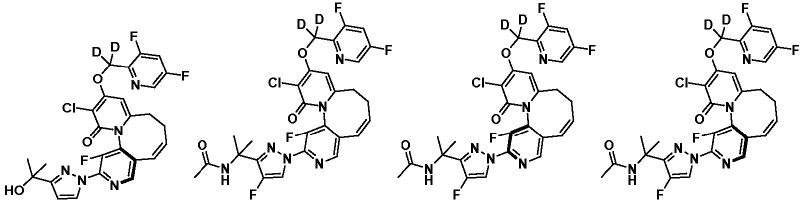

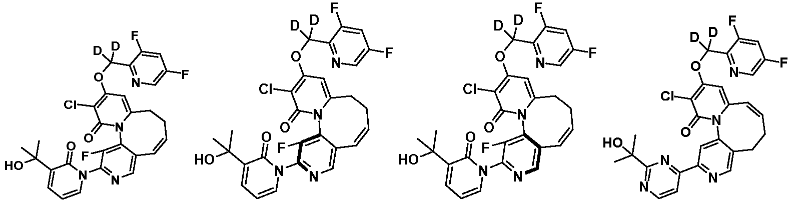

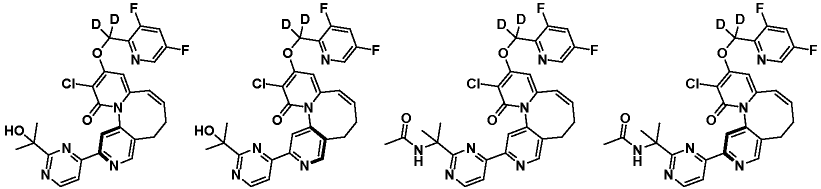

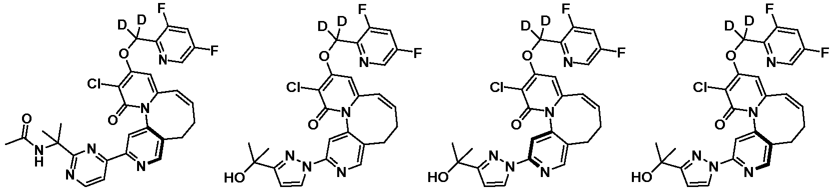

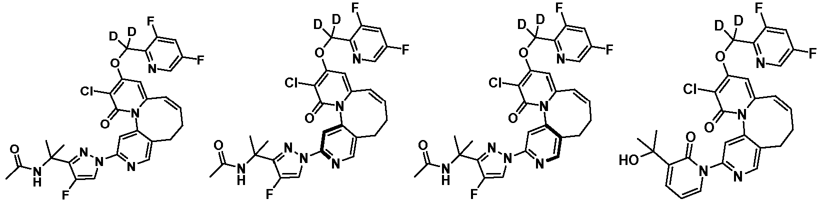

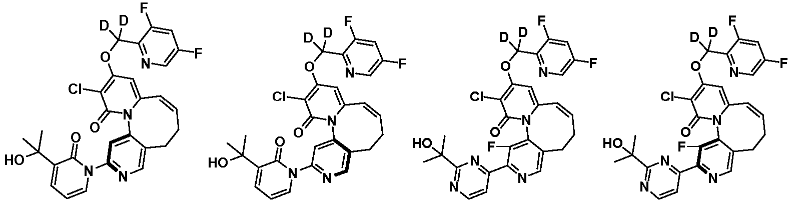

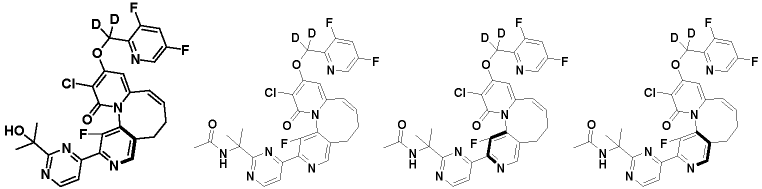

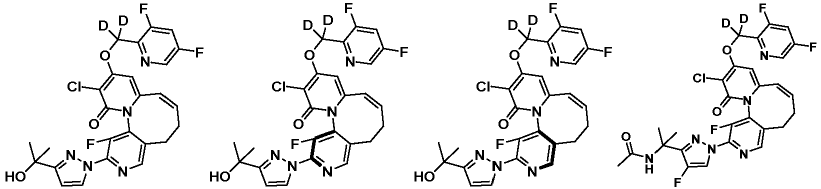

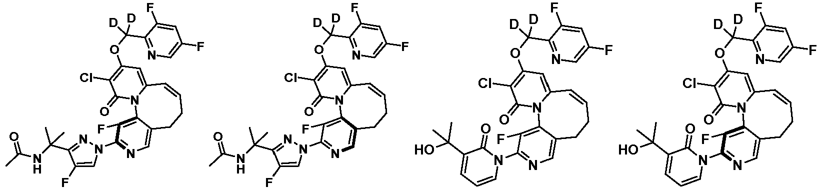

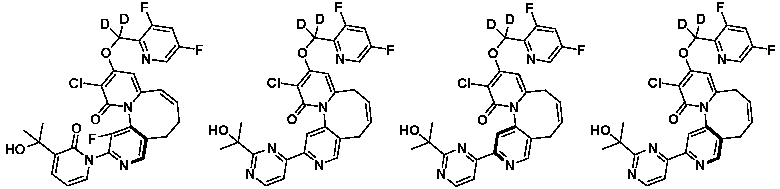

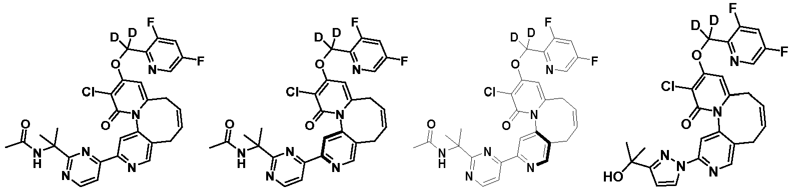

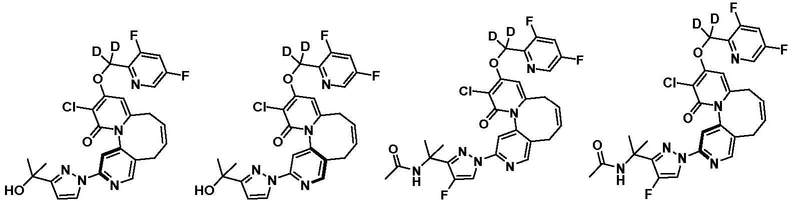

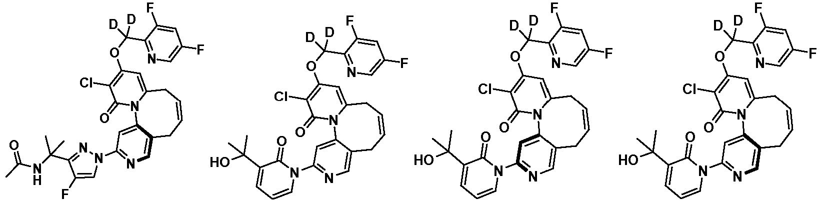

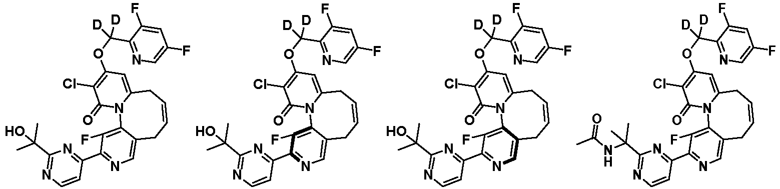

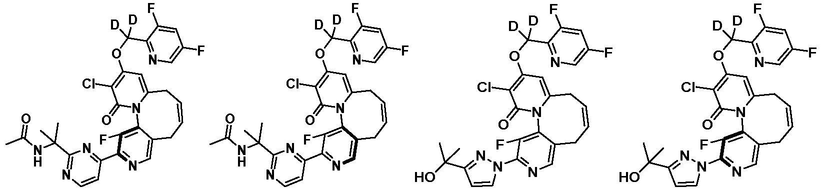

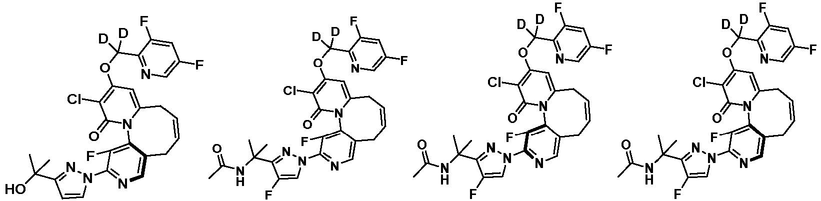

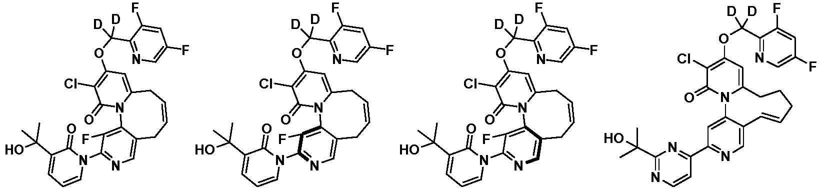

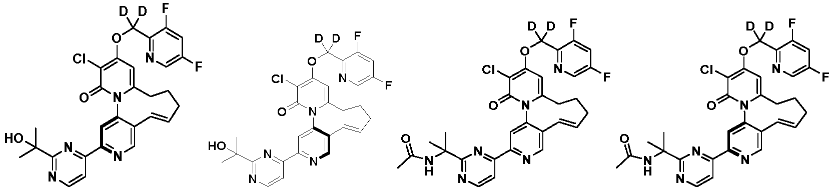

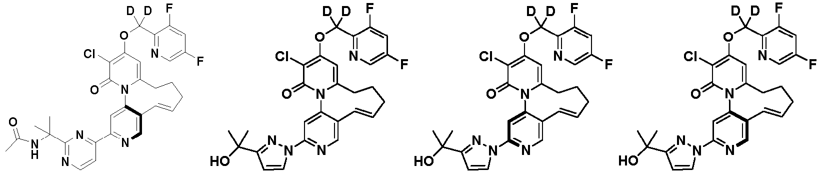

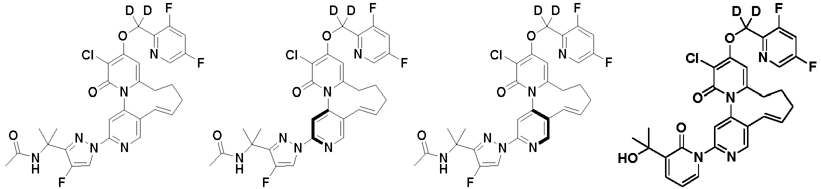

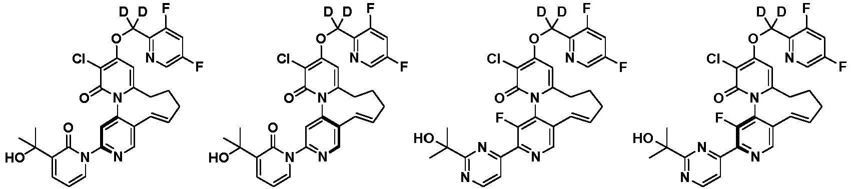

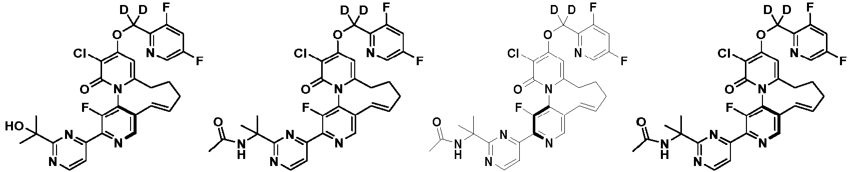

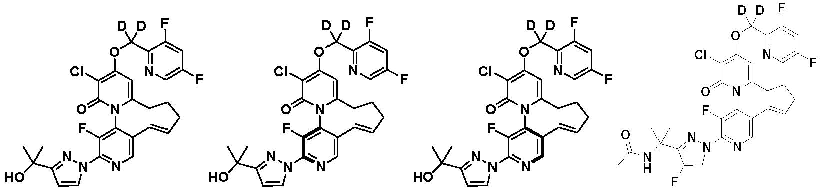

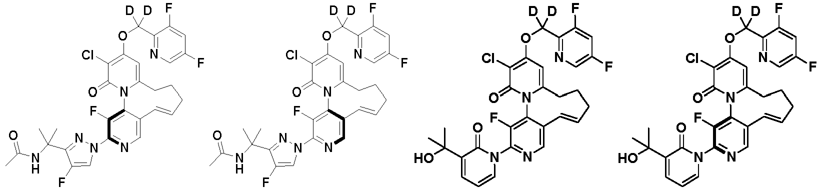

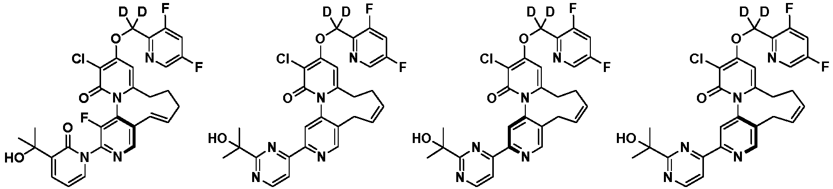

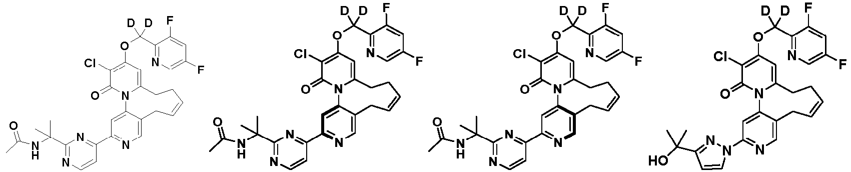

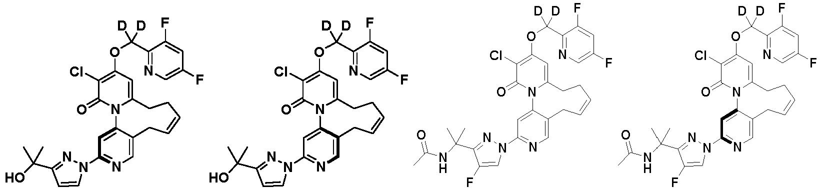

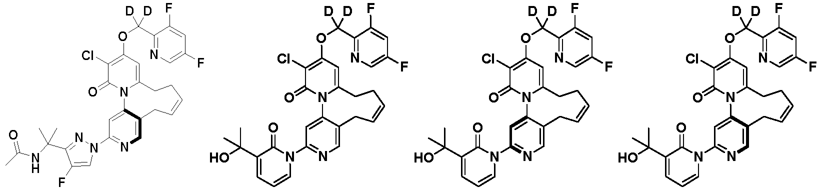

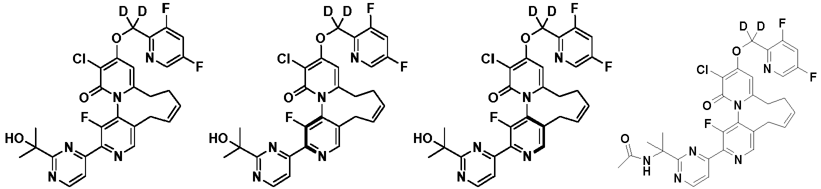

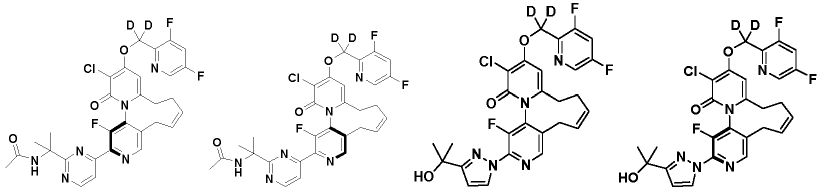

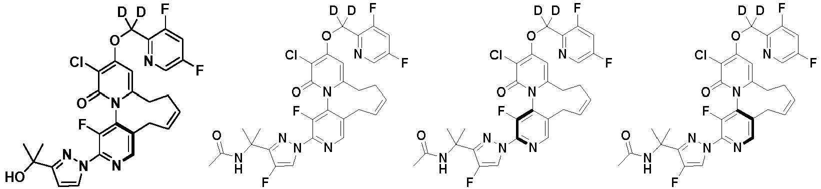

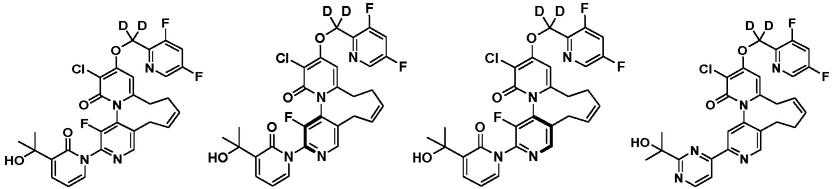

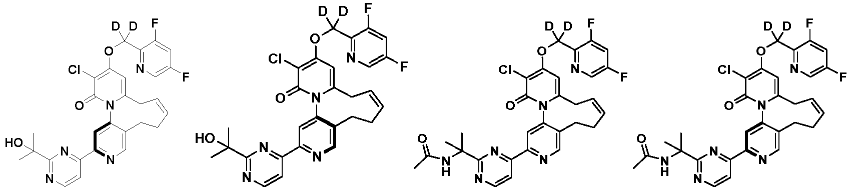

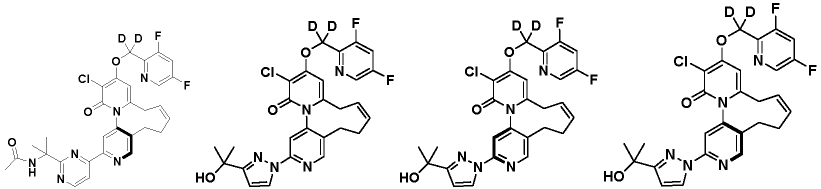

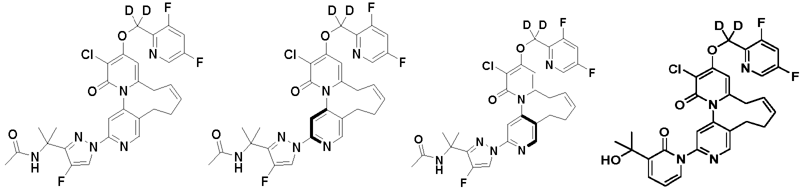

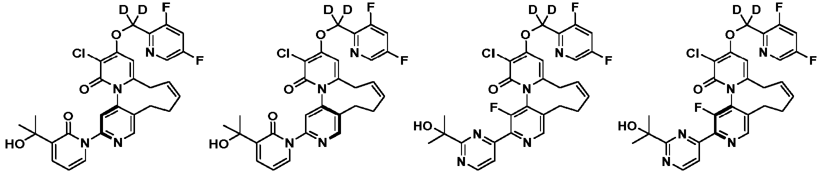

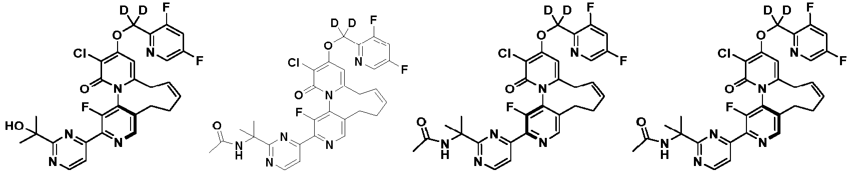

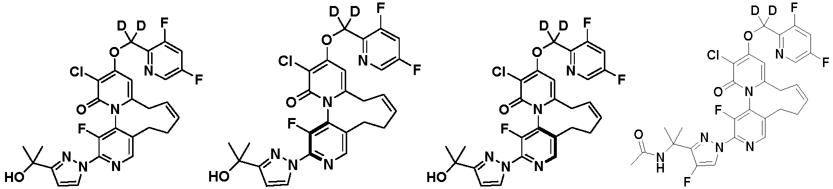

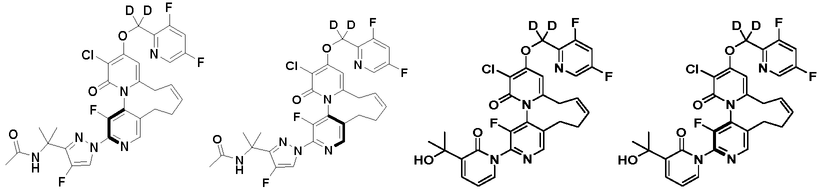

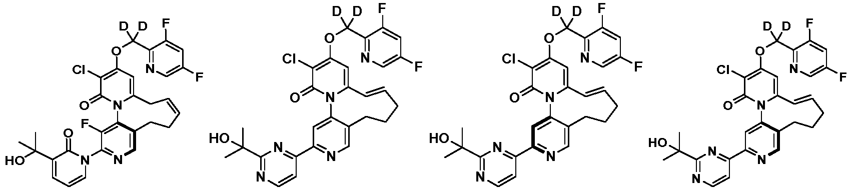

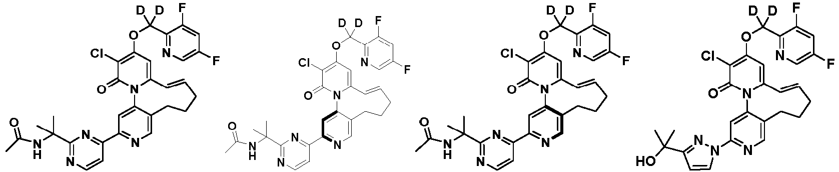

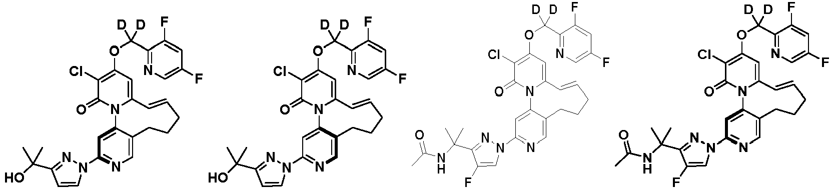

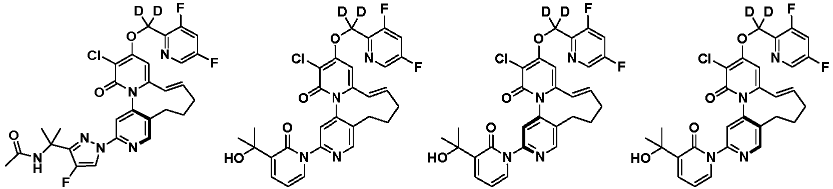

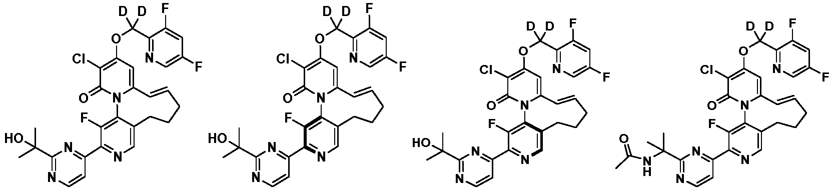

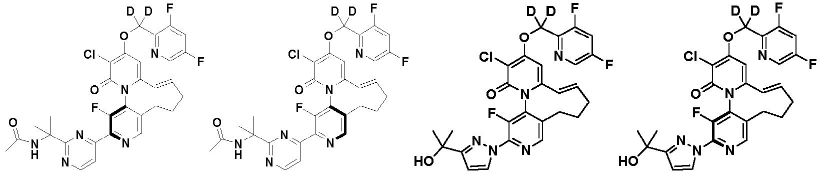

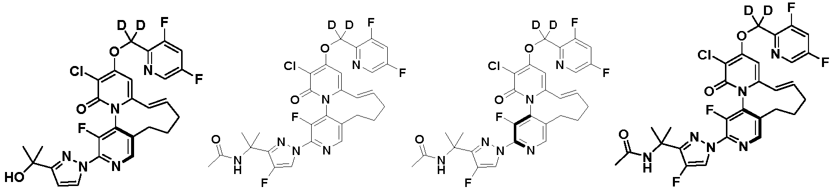

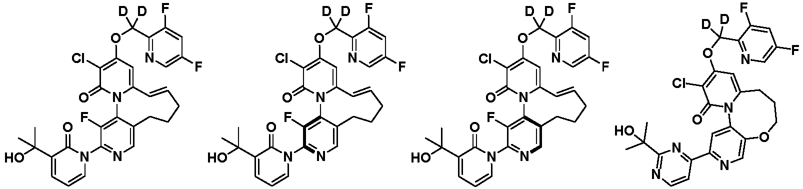

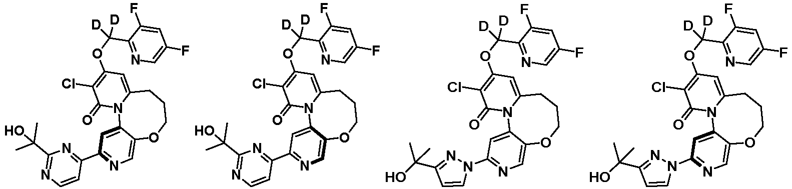

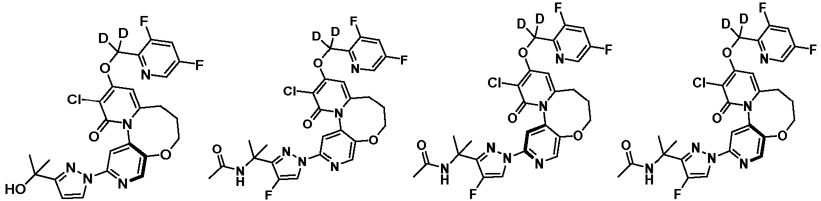

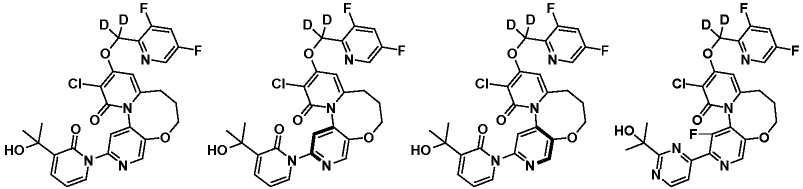

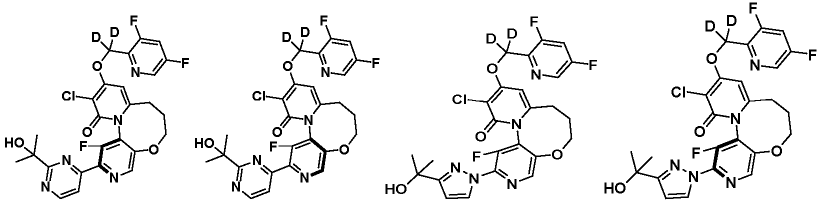

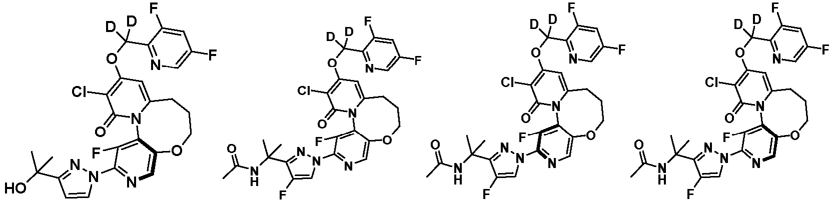

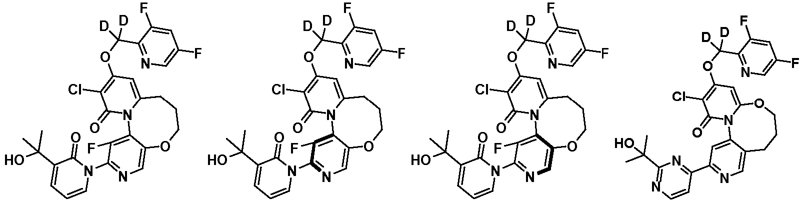

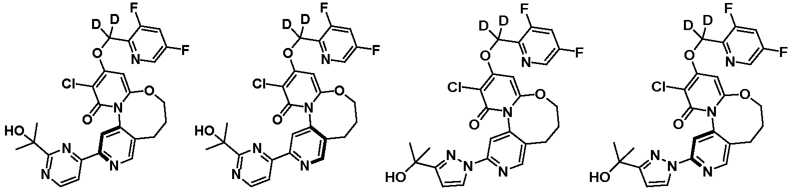

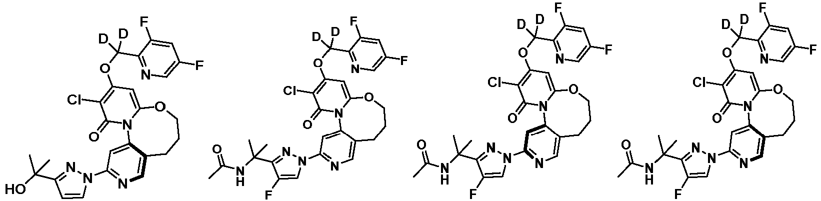

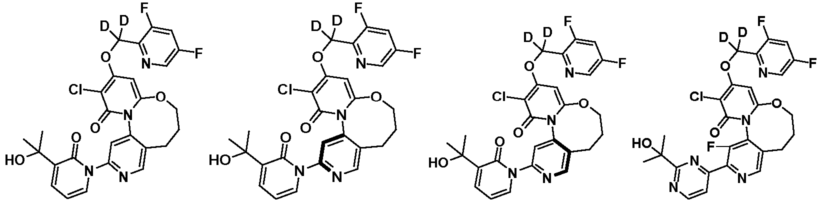

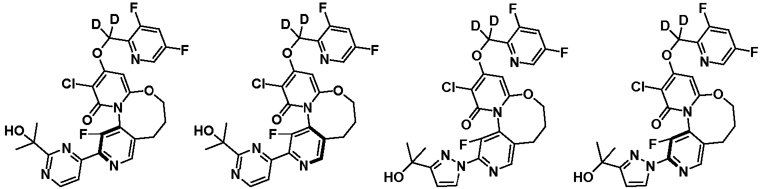

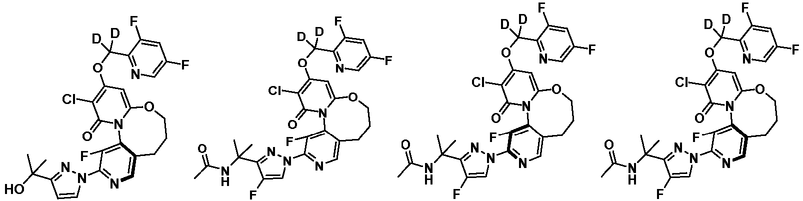

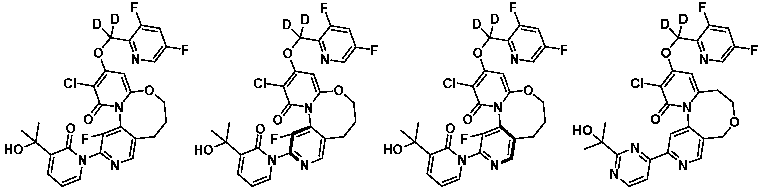

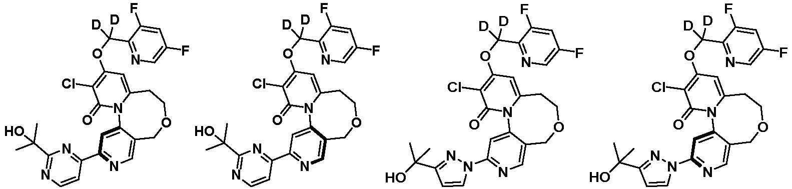

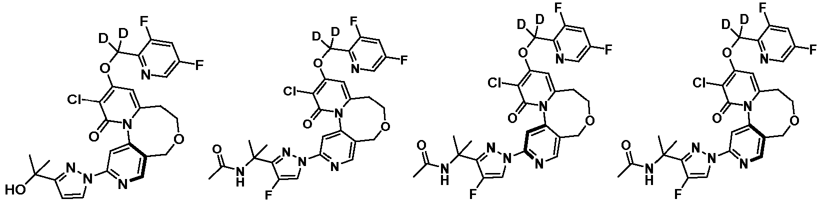

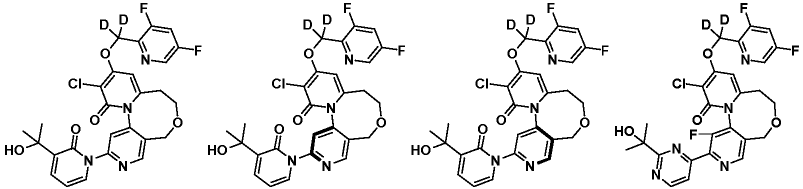

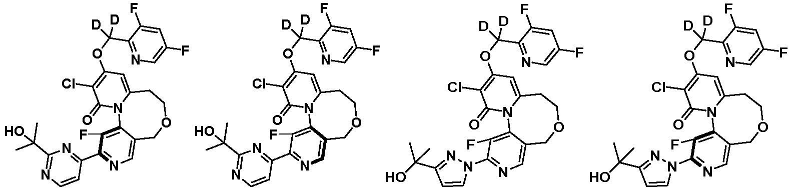

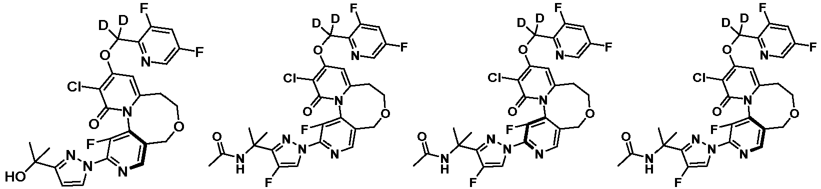

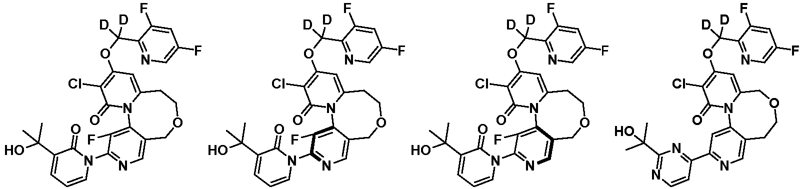

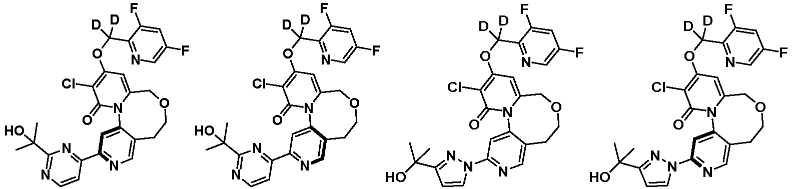

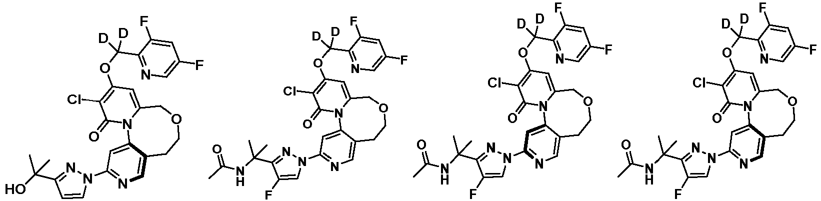

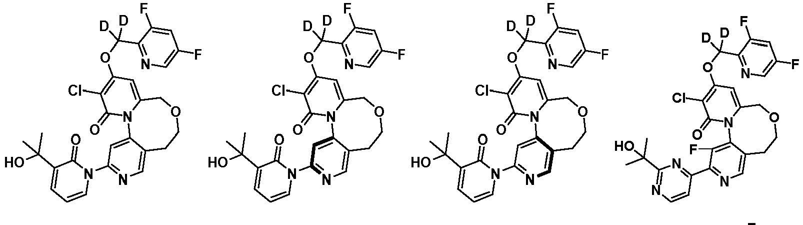

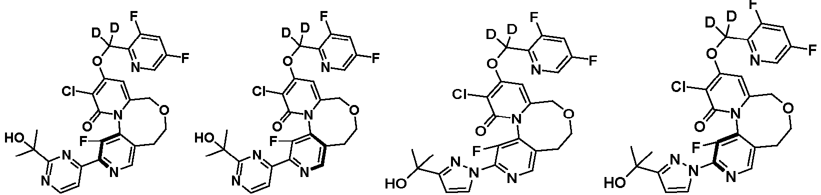

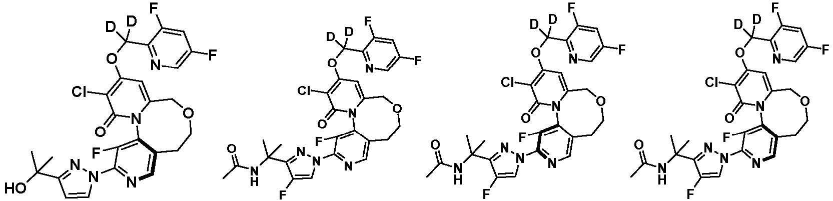

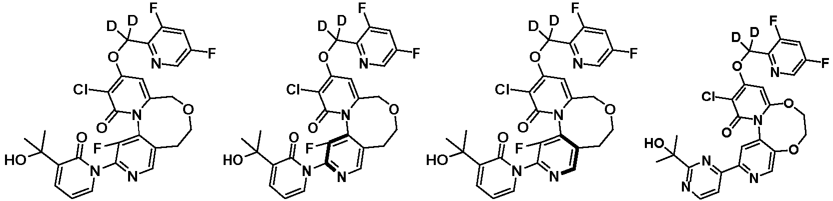

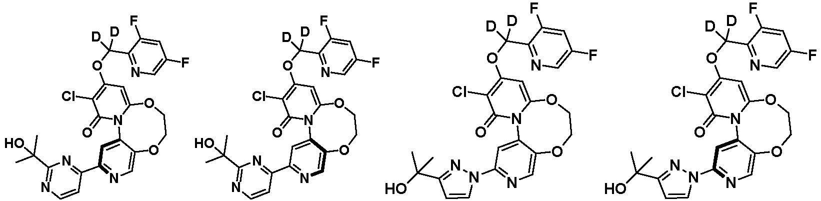

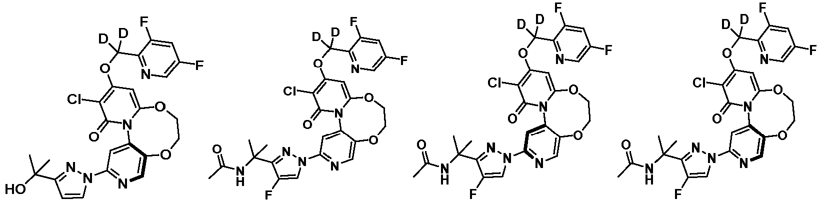

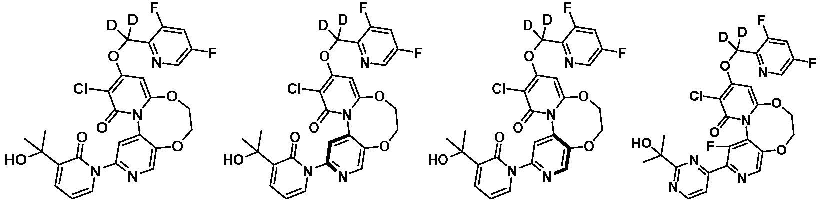

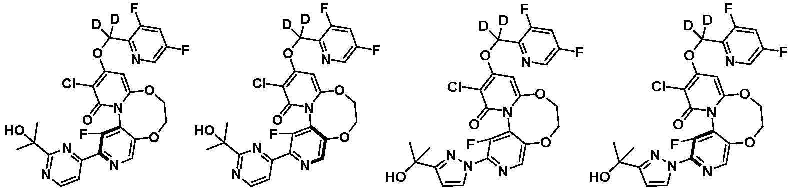

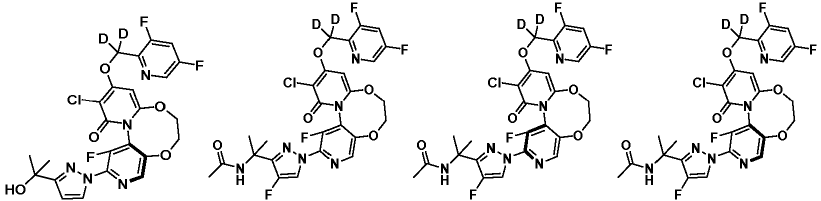

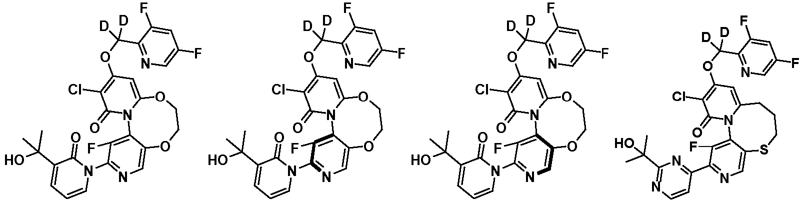

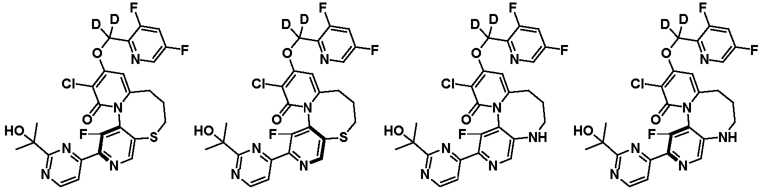

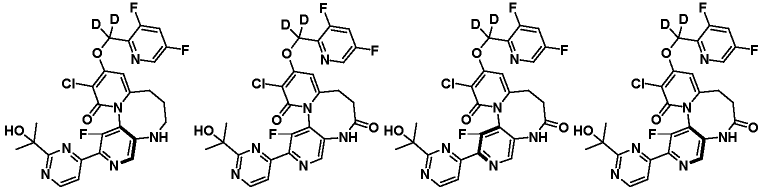

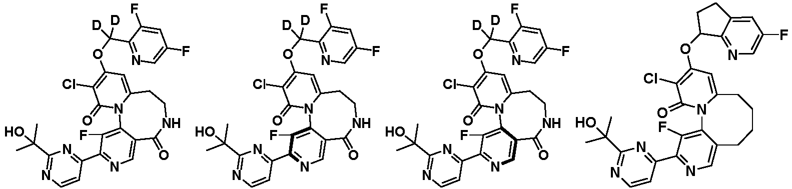

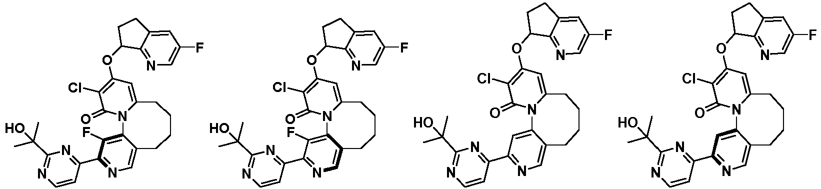

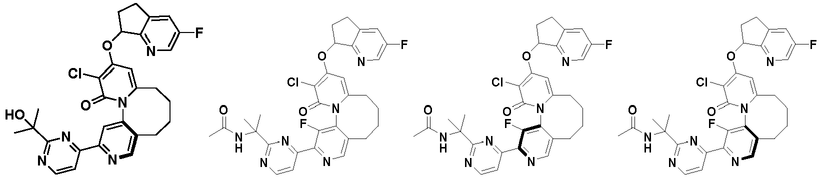

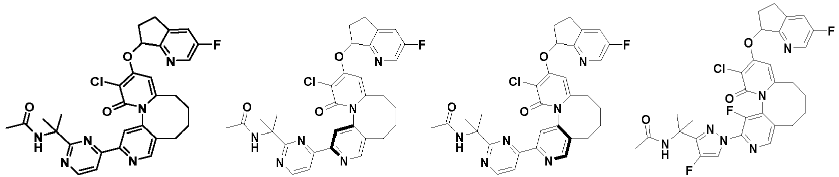

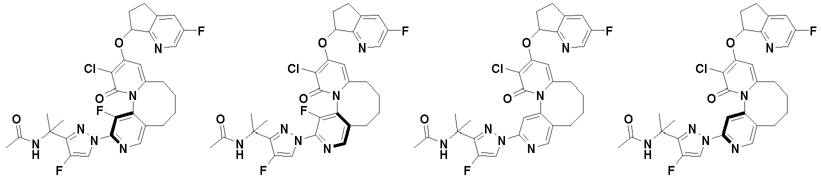

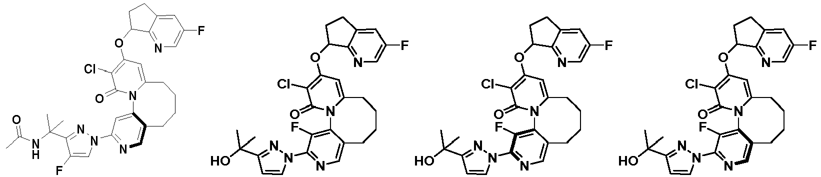

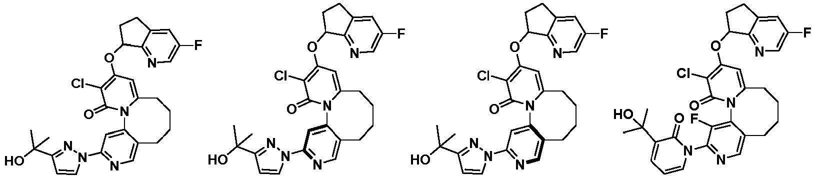

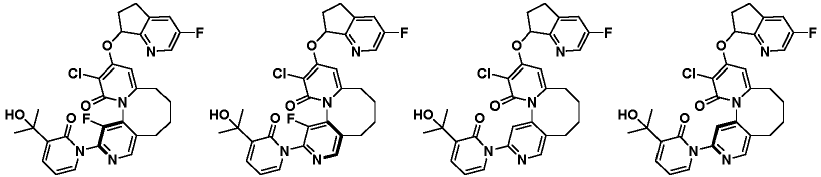

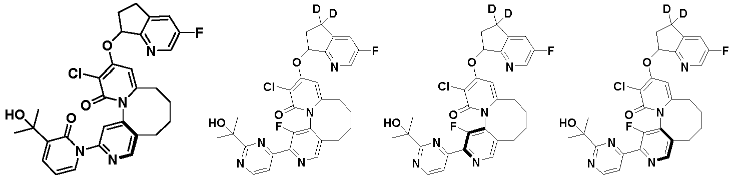

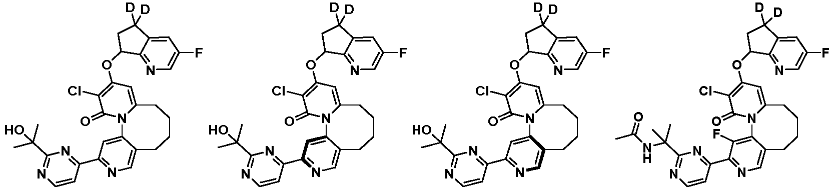

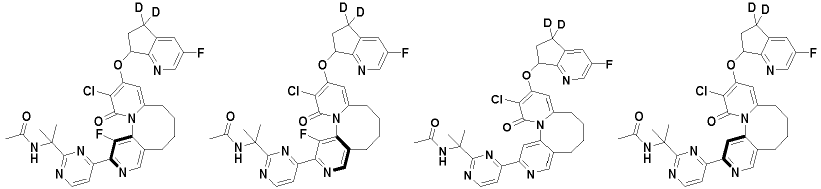

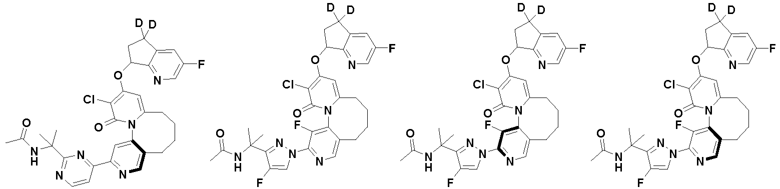

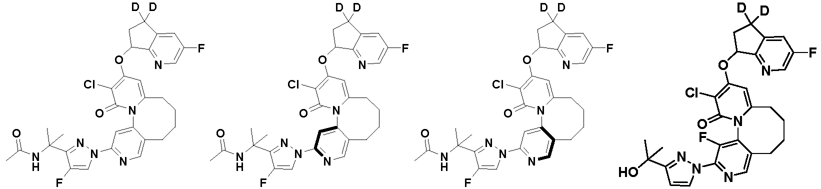

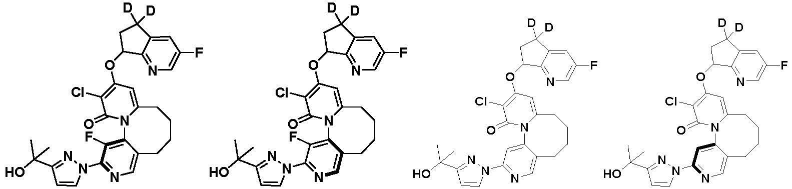

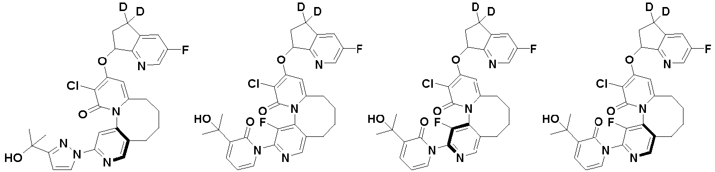

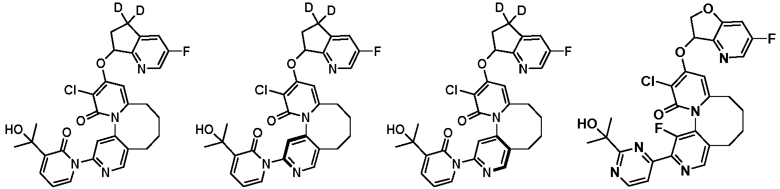

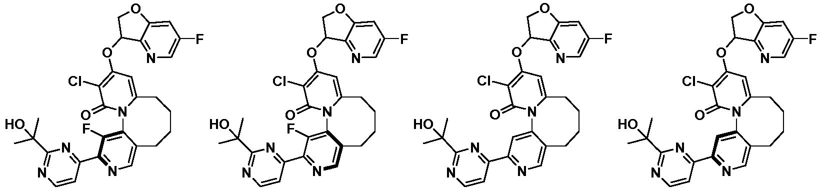

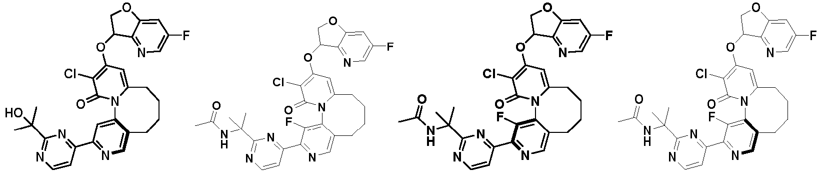

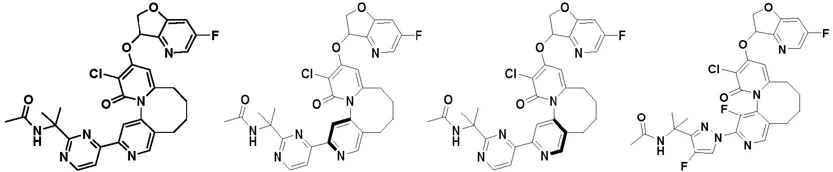

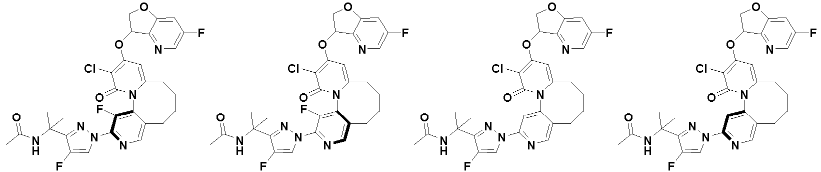

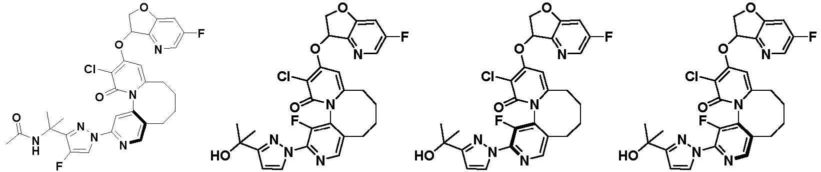

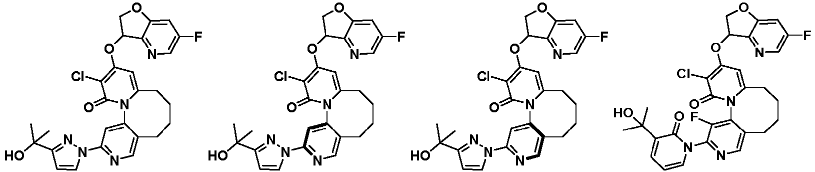

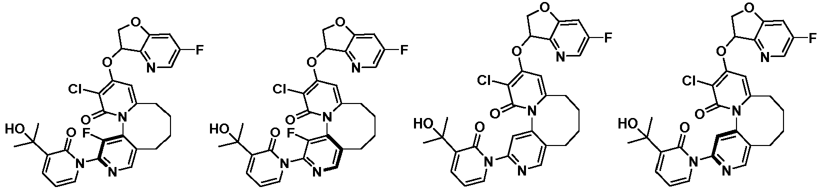

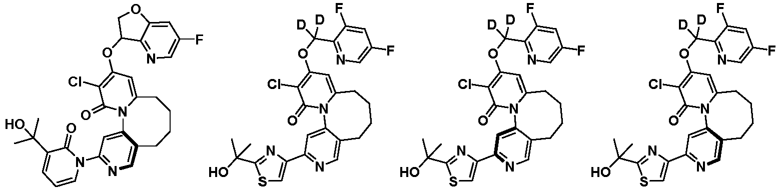

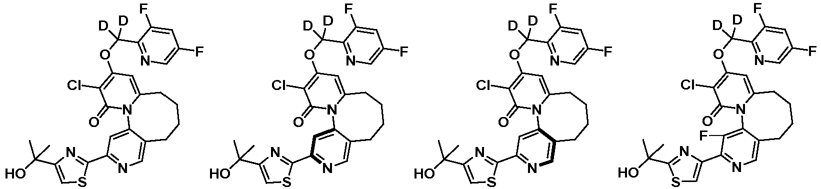

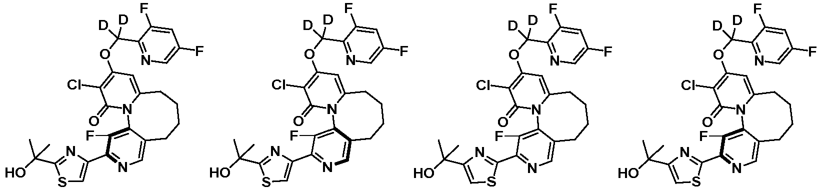

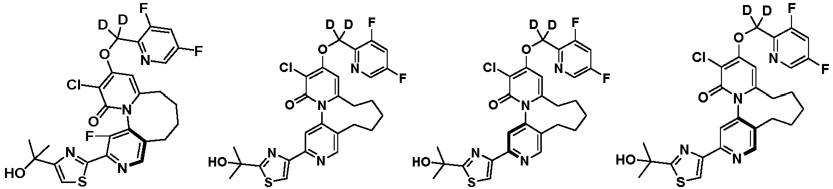

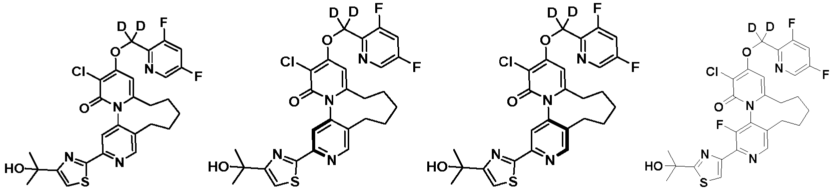

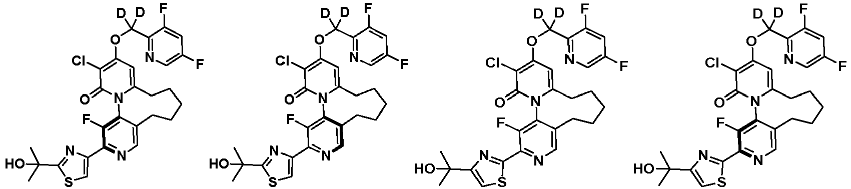

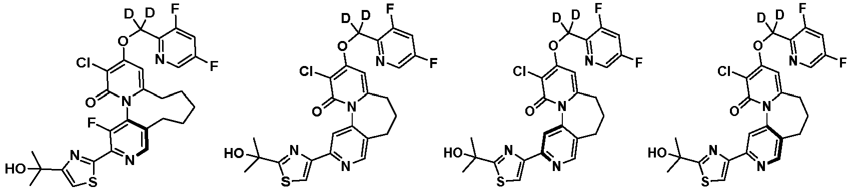

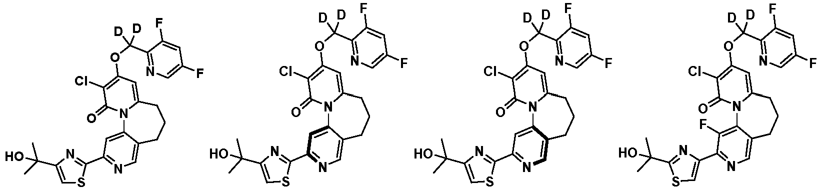

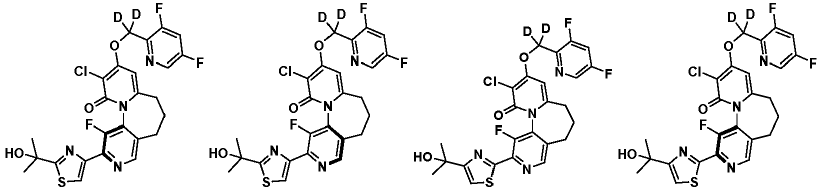

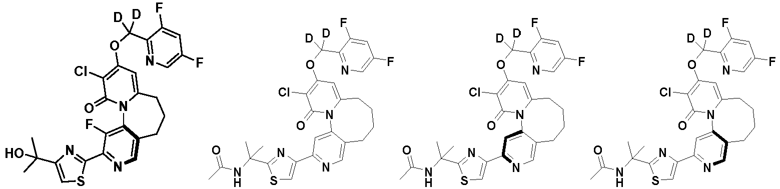

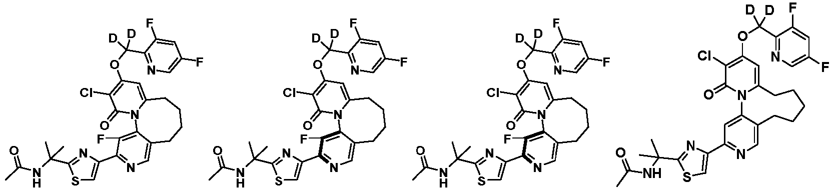

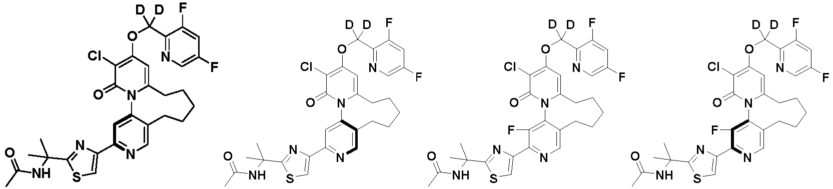

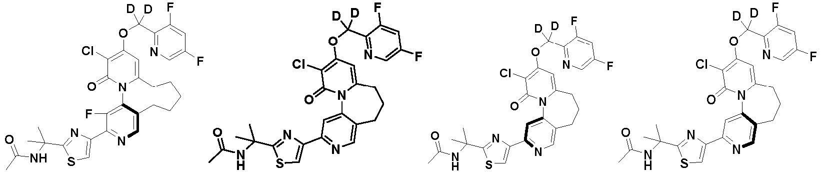

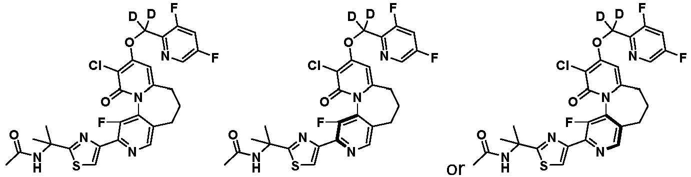

19. A compound represented by general formula (M-1) or (M-2), or a stereoisomer or pharmaceutically acceptable salt thereof: 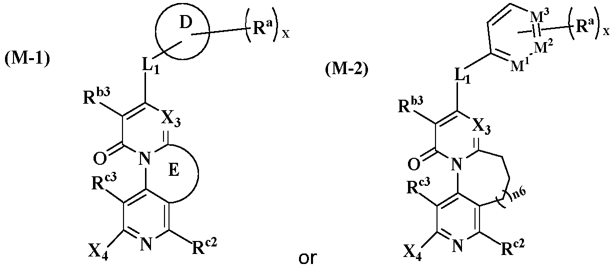 wherein:
X₄ is selected from halogen, preferably chlorine or bromine;
ring D and ring E are as defined in claim 2;
M¹, M², M³, and n6 are as defined in claim 5; and
L₁, R^{a}, R^{b3}, R^{c2}, R^{c3}, X₃, and x are as defined in claim 2 or 5.

20. A method for preparing the compound of general formula (I'-1), or the stereoisomer and pharmaceutically acceptable salt thereof according to claim 2, wherein the method comprises the following steps: 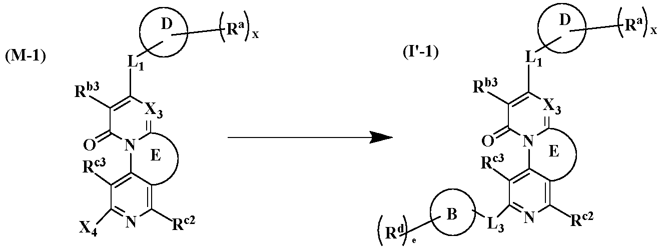
reacting a compound represented by general formula (M-1) to obtain a target compound represented by general formula (I'-1);
wherein
X₄ is selected from halogen, preferably chlorine or bromine; and ring D, ring E, ring B, L₁, L₃, R^{a}, R^{b3}, R^{c2}, R^{c3}, X₃, R^{d}, x, and e are as defined in claim 2.

21. A method for preparing the compound of general formula (XIV), or the stereoisomer and pharmaceutically acceptable salt thereof according to claim 5, wherein the method comprises the following steps: 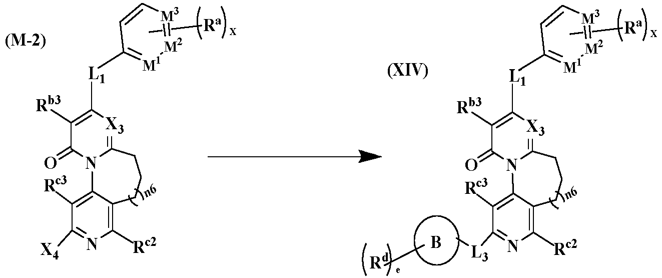
reacting a compound represented by general formula (M-2) to obtain a target compound represented by general formula (XIV);
wherein
X₄ is selected from halogen, preferably chlorine or bromine; and
M¹, M², M³, L₁, L₃, R^{a}, R^{b3}, R^{c2}, R^{c3}, X₃, R^{d}, x, e, and n6 are as defined in claim 5.

22. A method for preparing the compound of general formula (XV), or the stereoisomer and pharmaceutically acceptable salt thereof according to claim 8, wherein the method comprises the following steps: 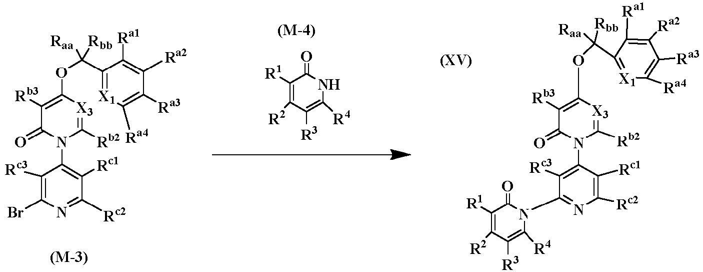
reacting general formula (M-3) with general formula (M-4) to obtain a target compound represented by general formula (XV);
wherein
X₁, X₃, R^{a1}, R^{a2}, R^{a3}, R^{a4}, Rₐₐ, R_{bb}, R^{b2}, R^{b3}, R^{c1}, R^{c2}, R^{c3}, R¹, R², R³, and R⁴ are as defined in claim 8.

23. A pharmaceutical composition comprising a therapeutically effective dose of the compound, or the stereoisomer or pharmaceutically acceptable salt thereof according to any one of claims 1-17, and one or more pharmaceutically acceptable carriers, diluents, or excipients.

24. Use of the compound, or the stereoisomer or pharmaceutically acceptable salt thereof according to any one of claims 1-18, or the pharmaceutical composition according to claim 23 in the preparation of a medicament for the treatment of a p38 kinase-mediated disease.

25. Use of the compound, or the stereoisomer or pharmaceutically acceptable salt thereof according to any one of claims 1-18, or the pharmaceutical composition according to claim 23 in the preparation of a medicament for the treatment of autoimmune diseases, chronic inflammatory diseases, acute inflammatory conditions, autoinflammatory diseases, atherosclerosis, diabetes, fibrotic diseases, metabolic diseases, cancers, tumors, leukemia, and lymphoma.
